# EUROPEAN PATENT APPLICATION

(11) **EP 4 851 290 A2**
(43) Date of publication of application: **09.12.2026**
(21) Application number: 26178050.6
(22) Date of filing: 11.05.2026
(51) Int. Cl.: C12N 15/113, A61K 31/7088

(54) **SIRNA DUPLEXE AND CONJUGATE FOR TARGETED REGULATION OF INHBE GENE EXPRESSION AND USE THEREOF**

(30) Priority: 12.05.2025 CN 202510608772; 13.05.2025 CN 202510614795; 10.03.2026 CN 202610282198
(71) Applicant: Beijing Youcare Kechuang Pharmaceutical Technology Co., Ltd., Daxing District, Beijing 100176 (CN)
(72) Inventor: HUANG, Zeao, Beijing 100176 (CN); SONG, Gengshen, Beijing 100176 (CN); JI, Guangshen, Beijing 100176 (CN); YANG, Shuo, Beijing 100176 (CN); YU, Fei, Beijing 100176 (CN); FU, Rui, Beijing 100176 (CN); WU, Yucheng, Beijing 100176 (CN)
(74) Representative: Dehns

(57) **Abstract**

Disclosed herein are siRNA duplexe for targeted regulation of INHBE gene expression and use thereof in the prevention and treatment of metabolic diseases. The present disclosure also relates to corresponding conjugates and pharmaceutical compositions. Results from cell-based and animal experiments demonstrate that the oligonucleotide duplexes of the present disclosure can significantly inhibit INHBE gene expression, and thus may be used for the manufacture of medicaments for preventing or treating atherosclerosis and other related diseases.

## Description

The present application claims priority to Chinese Patent Application No. 2025106087721 filed on May 12, 2025, Chinese Patent Application No. 2025106147953 filed on May 13, 2025, and Chinese Patent Application No. 202610282198X filed on March 10, 2026. The contents of the above Chinese Patent Applications are incorporated herein by reference in their entirety.

### TECHNICAL FIELD

The present disclosure belongs to the field of biomedicine, and specifically relates to siRNA duplexes and conjugates for targeted regulation of INHBE gene expression and the use thereof in the prevention and treatment of metabolic diseases.

### BACKGROUND

Nucleic acid drugs, particularly oligonucleotide drugs, have been widely used due to their simple synthesis and high biological activity. Oligonucleotide drugs typically include antisense oligonucleotides (ASOs), small interfering RNAs (siRNAs), microRNAs (miRNAs), and aptamers.

Oligonucleotides are short DNA or RNA molecules or oligomers that can bind to their complementary oligonucleotides, DNA, or RNA in a sequence-specific manner to form duplexes or, although less commonly, hybrids of a higher order under certain circumstances. Oligonucleotides can bind sequence-specifically to complementary RNA strands and, upon hybridization, can induce RNase H-mediated cleavage of the target RNA. In natural oligonucleotides, nucleotides are linked via phosphodiester bonds. Under physiological conditions, oligonucleotides are particularly sensitive to nucleases. Therefore, when preparing oligonucleotide drugs, natural, unstructured, and unmodified oligonucleotide are readily and rapidly degraded *in vivo,* resulting in very limited biological activity and consequently poor druggability. Modification of oligonucleotides is an effective approach to enhance their activity, which can improve their resistance to nucleases and their affinity for RNA, and better facilitate endocytosis and tissue targeting, thereby effectively regulating the expression of target genes.

The basic structure of a nucleotide can typically be divided into four components: a base, a ribose, a phosphate backbone, and terminal groups. Modifications of these four components may include, for example, the following:
Base modifications: These mainly include three types: purine modifications, pyrimidine modifications, and base substitutions. Purine modifications include N6-methyladenosine, N1-methyladenosine, and 7-methylguanosine modifications. Pyrimidine modifications include 3-methyluridine, 5-methyluridine, 5-methylcytidine, N4-acetylcytidine, pseudouridine, thiouridine, propynyluridine, and dihydrouridine modifications.

2) Ribose modifications: These mainly include modifications and substitutions of functional groups at specific positions on the ribose ring. Ribose modifications include, but are not limited to, 2'-modifications, 4'-modifications, 5'-modifications, and isomeric modifications. The most common 2'-modifications of ribose are 2'-OMe (2'-O-methyl) and 2'-F (2'-fluoro) modifications. Compared to natural oligonucleotides, oligonucleotides incorporating both 2'-OMe and 2'-F modifications exhibit higher Tm values, greater serum stability, and improved biological activity.

3) Phosphate backbone modifications: The primary modification patterns include, but are not limited to, phosphorothioate modifications; methylphosphonate, selenophosphate, boranophosphate, and dithiophosphate modifications; substitution of the bridging oxygen atom in the phosphodiester linkage with a sulfur atom; and complete substitution of the phosphodiester linkage between nucleosides with a non-phosphorus-containing group, such as substitution of the phosphorus atom with a carbon, sulfur, or nitrogen atom, thereby forming structures such as guanidinium linkages, S-methylthiourea linkages, or nitrate linkages.

4) Terminal modifications: These include, but are not limited to, covalent attachment of specific groups to the 5' end and/or 3' end of the sense strand, and phosphorylation modification at the 5' end of the antisense strand.

Inhibin βE (INHBE) protein is a member of the transforming growth factor-β (TGF-β) superfamily. It is predominantly and specifically expressed in hepatocytes and is a secreted protein. Following proteolytic cleavage, the INHBE protein dimerizes to form Activin E, which may subsequently inhibit lipolysis by activating specific signaling pathways (*e.g*., ALK7), thereby promoting lipid accumulation.

Although drugs such as semaglutide are currently available for the treatment of metabolic disorders such as diabetes and obesity, semaglutide is associated with issues such as poor patient compliance and significant side effects. Therefore, there is a need for alternative therapies and combination therapies for patients suffering from diseases associated with INHBE gene expression.

Furthermore, based on research on INHBE in the art, in addition to diabetes and obesity, diseases associated with INHBE gene expression include, for example, hyperlipidemia or dyslipidemia, insulin resistance or elevated or altered insulin levels during fasting or metabolic challenge, hepatic fat deposition or fatty liver disease, non-alcoholic steatohepatitis, elevated blood pressure and/or hypertension, metabolic syndrome, coronary artery disease and other atherosclerotic conditions, as well as complications associated with these diseases. Therefore, there is a clinical need for oligonucleotide drugs for the treatment of diseases associated with INHBE gene expression.

### SUMMARY

The present disclosure provides siRNAs and conjugates for targeted regulation of INHBE gene expression and the use thereof (*e.g*., in the manufacture of medicaments for the treatment of metabolic disorders). In the present disclosure, by modifying the siRNA core sequences, multiple siRNA derivatives that exhibit significant inhibitory effects on INHBE gene expression are identified, thereby providing corresponding siRNA conjugates.

The technical solutions of the present disclosure include, but are not limited to, the following:
In one aspect, the present disclosure provides an siRNA duplex comprising an oligonucleotide duplex formed by pairing of a sense strand and an antisense strand.

In another aspect, the present disclosure provides a conjugate for reducing the expression of INHBE, comprising the siRNA duplex described above and a conjugate moiety attached thereto.

In another aspect, the present disclosure provides a nucleic acid-protein complex comprising a duplex region of the siRNA duplex described above or an antisense strand of the duplex region, and a nuclease.

In another aspect, the present disclosure provides a recombinant vector comprising a nucleic acid molecule encoding the siRNA duplex described above.

In some embodiments, the vector backbone of the recombinant vector is selected from recombinant virus-derived circular RNA vectors, tRNA scaffolds, rRNA scaffolds, and chimeric tRNA/pre-miRNA vectors.

In another aspect, the present disclosure provides a recombinant cell that synthesizes and secretes the siRNA duplex described above.

In some embodiments, the recombinant cell is selected from *Rhodovulum sulfidophilum* and ribonuclease III-deficient *Corynebacterium glutamicum.*

In another aspect, the present disclosure provides a method of preparing an siRNA duplex, comprising culturing the recombinant cell described above or chemical synthesis.

In another aspect, the present disclosure provides a pharmaceutical composition comprising the siRNA duplex described above, the conjugate described above, or the nucleic acid-protein complex described above, and a pharmaceutically acceptable carrier.

In another aspect, the present disclosure provides a kit comprising kit A, wherein the kit A comprises one or more of the siRNA, conjugate, or pharmaceutical composition described above.

In some embodiments, the kit further comprises kit B, wherein the kit B comprises one or more of the following:
(1) other agents that reduce INHBE gene expression or compositions comprising the agents that reduce INHBE gene expression;
(2) one or more selected from the group consisting of insulin, glucagon-like peptide-1 agonists, sulfonylureas, meglitinides, biguanides, thiazolidinediones, α-glucosidase inhibitors, SGLT2 inhibitors, DPP-4 inhibitors, and HMG-CoA reductase inhibitors.

In another aspect, the present disclosure provides a method of inhibiting INHBE gene expression or inhibiting INHBE replication, comprising contacting a target cell with one or more of the siRNA duplex, conjugate, pharmaceutical composition, or kit described above.

In some embodiments, the method is for non-diagnostic or non-therapeutic purposes.

In some embodiments, the method is performed *in vivo* or *in vitro.*

In another aspect, the present disclosure provides the use of the siRNA duplex, conjugate, recombinant vector, recombinant cell, or pharmaceutical composition described above in the manufacture of a medicament for preventing and/or treating a disease associated with INHBE gene expression.

The disease associated with INHBE gene expression is selected from diseases caused by overexpression of INHBE protein, pathogenic mutations in the INHBE gene, abnormal metabolism of INHBE protein, and abnormal interactions between INHBE and another substance.

In another aspect, the present disclosure provides the siRNA duplex, conjugate, pharmaceutical composition, or kit described above for use in the treatment and/or prevention of a disease associated with INHBE gene expression.

In another aspect, the present disclosure provides a method of treating and/or preventing a disease associated with INHBE gene expression, comprising administering to a subject in need thereof an effective amount of the siRNA duplex, conjugate, or pharmaceutical composition described above, or administering the kit described above to the subject in need thereof.

In another aspect, the present disclosure provides the siRNA duplex, conjugate, pharmaceutical composition, or kit described above for use in the treatment of metabolic disorders and cardiovascular and cerebrovascular diseases.

In another aspect, the present disclosure provides a method of treating metabolic disorders and cardiovascular and cerebrovascular diseases, comprising administering to a subject in need thereof an effective amount of the siRNA duplex, nucleic acid-protein complex, or pharmaceutical composition described above, or administering the kit described above to the subject in need thereof.

Those skilled in the art will appreciate that the disease associated with INHBE gene expression of the present disclosure includes, but is not limited to, type 2 diabetes; hyperlipidemia or dyslipidemia (elevated or altered circulating levels of low-density lipoprotein cholesterol (LDL-C), triglycerides, very low-density lipoprotein cholesterol (VLDL-C), apolipoprotein B, or other lipid components); obesity (particularly abdominal obesity); lipodystrophy (e.g., inability to deposit fat locally in adipose depots (partial lipodystrophy) or inability to deposit fat systemically (lipoatrophy)); insulin resistance or elevated or altered insulin levels during fasting or metabolic challenge; hepatic fat deposition or fatty liver disease and complications thereof (*e.g*., cirrhosis, fibrosis, or liver inflammation); non-alcoholic steatohepatitis; other types of liver inflammation; elevated or altered liver enzyme levels or liver injury; elevated or altered biomarkers of inflammation or fat deposition in the liver; elevated blood pressure and/or hypertension; elevated blood glucose or hyperglycemia; metabolic syndrome; coronary artery disease and other atherosclerotic conditions; as well as complications associated with each of the aforementioned diseases or conditions; and any other diseases associated with INHBE gene expression, including yet unidentified related disorders, pathologies, or syndromes.

Based on common knowledge in the art, the aforementioned preferred conditions may be combined in any manner to obtain preferred examples of the present disclosure.

The present disclosure has at least the following advantages:
(1) The siRNA core sequences of the present disclosure exhibit significant inhibitory effects on INHBE gene expression, with inhibition rates of greater than 50%.
(2) The siRNA derivatives obtained using modification templates exhibit inhibition rates of greater than 70%.

### DETAILED DESCRIPTION OF PREFERRED EMBODIMENTS

To facilitate understanding of the present disclosure, certain terms are first defined. In addition, it should be noted that whenever a value or a range of values for a parameter is recited, it is intended that intermediate values and ranges between the recited values are also intended to be encompassed within the scope of the present disclosure.

As used herein, the articles "a" and "an" refer to one or more than one (*i.e.*, at least one) of the grammatical object of the article. By way of example, "an element" refers to one element or more than one element, such as multiple elements.

The term "including" as used herein means, and is used interchangeably with, the phrase "including, but not limited to".

The term "or" as used herein means, and is used interchangeably with, the term "and/or", unless the context clearly indicates otherwise.

As used herein, the term "about" or "approximately", as applied to one or more target values, refers to a value that is similar to the stated reference value. In certain embodiments, unless otherwise specified or clearly evident from the context, the term "approximately" or "about" refers to a value within 20%, 19%, 18%, 17%, 16%, 15%, 14%, 13%, 12%, 11%, 10%, 9%, 8%, 7%, 6%, 5%, 4%, 3%, 2%, 1%, or less of the stated reference value in either direction (greater than or less than) (unless such a range would exceed 100% of the possible value).

As used herein, "INHBE" refers to the inhibin βE gene or protein. Those skilled in the art can readily distinguish, based on the context, whether a given reference to INHBE refers to the gene or the protein.

The term "INHBE gene" may refer to a wild-type INHBE gene or a mutant INHBE gene containing sequence variations. Numerous sequence variations in the INHBE gene have been identified and can be found in databases such as NCBI dbSNP and UniProt (see, for example, ncbi.nlm.nih.gov/snp).

"G", "C", "A", and "U" each generally represent nucleotides containing guanine, cytosine, adenine, and uracil as their bases, respectively. "T" and "dT" are used interchangeably herein and refer to a deoxyribonucleotide in which the nucleobase is thymine, such as deoxyribothymine, 2'-deoxythymidine, or thymidine. However, it should be understood that the term "ribonucleotide", "nucleotide", or "deoxyribonucleotide" may also refer to a modified nucleotide (as described in further detail below) or a surrogate replacement moiety. Those skilled in the art will readily appreciate that guanine, cytosine, adenine, and uracil may be replaced by other moieties without substantially altering the base pairing properties of an oligonucleotide (including a nucleotide comprising such a replacement moiety). For example, without limitation, a nucleotide containing inosine as its base may pair with a nucleotide containing adenine, cytosine, or uracil. Hence, nucleotides containing uracil, guanine, or adenine may be replaced in the nucleotide sequences of the present disclosure by a nucleotide containing, for example, inosine. Sequences containing such replacement moieties are applicable to, but are not limited to, double-stranded ribonucleic acids, double-stranded ribonucleic acid derivatives, double-stranded ribonucleic acid conjugates, pharmaceutical compositions, and methods of the present disclosure.

The terms "complementary", "fully complementary", and "substantially complementary", as used herein, may refer to base pairing between the sense and antisense strands of an siRNA, or between the antisense strand of an siRNA and a target sequence, as understood from the context in which they are used. In some embodiments of the present disclosure, a first nucleotide sequence may be considered complementary to a second nucleotide sequence if the two sequences exhibit at least 70%, 75%, 80%, 85%, 90%, 95%, 96%, 97%, 98%, or 99% sequence complementarity. In an exemplary embodiment, 18 out of 20 nucleobases in the first nucleotide sequence pair with a corresponding region of the second nucleotide sequence, thereby achieving 90% complementarity.

The terms "double-stranded ribonucleic acid", "double-stranded RNA (dsRNA) molecule", "dsRNA", and "RNA duplex" are used interchangeably and refer to a complex of ribonucleic acid molecules having a double-stranded structure, comprising two antiparallel, complementary or substantially complementary nucleic acid strands, which possess "sense" and "antisense" orientations relative to a target gene, such as the INHBE gene. In some examples, double-stranded ribonucleic acid (dsRNA) triggers degradation of a target RNA, such as mRNA, through a post-transcriptional gene silencing mechanism (referred to herein as RNA interference or RNAi). As used herein, the term "siRNA duplex" in some cases also typically refers to the technical meaning as defined above.

As is well known in the art, the terms "siRNA duplex", "double-stranded RNAi agent", "RNAi agent", "small interfering ribonucleic acid", or "siRNA" refer to small RNAi molecules. These are a class of double-stranded RNA molecules, also known in the art as short interfering RNA or silencing RNA. siRNA typically comprises a sense strand (also referred to as a passenger strand) and an antisense strand (also referred to as a guide strand), each of which is 17 to 30 nucleotides in length, typically 19 to 25 nucleotides in length, wherein the antisense strand is complementary (e.g., at least 95% complementary, such as fully complementary) to a target nucleic acid (suitably a mature mRNA sequence), and the sense strand is complementary to the antisense strand, such that the sense and antisense strands form a duplex or duplex region. The sense and antisense strands of an siRNA may form a blunt-ended duplex, or may form a duplex containing a 3' overhang, which may be, for example, of 1, 2, or 3 nucleotides in length, similar to products generated by Dicer, which can form substrates for RISC *in vivo.* Effective extended forms of Dicer substrates have been described in US 8,349,809 and US 8,513,207, which are incorporated herein by reference. In some examples, both the sense strand and the antisense strand have a 3' overhang of two nucleotides in length. Accordingly, the duplex region may be, for example, 17, 18, 19, 20, 21, 22, 23, 24, or 25 nucleotides in length, such as 19, 20, 21, 22, or 23 nucleotides in length.

Moreover, as used herein, "siRNA" in some cases also refers to a "core sequence". As used herein, the term "core sequence" in some cases specifically refers to an siRNA duplex in which each nucleotide in the double-stranded ribonucleic acid is an unmodified nucleotide, and it also appears in the context as "motif" or "siRNA motif". Accordingly, in the present disclosure, the terms "siRNA", "core sequence", "motif", and "siRNA motif" are used interchangeably, and their meanings also include the corresponding nucleotide arrangement of the siRNA duplex to which they refer. In the present disclosure, those skilled in the art can clearly understand the precise technical meaning of these terms based on the context. Furthermore, the 5'-terminal nucleotide of the antisense strand of the motif may be linked to a 5'-phosphate group or a 5'-phosphate derivative, or may not be linked to a 5'-phosphate group or a 5'-phosphate derivative.

As used herein, the term "siRNA derivative" refers to a double-stranded ribonucleic acid comprising at least one modified nucleotide, and in some cases also appears as a "double-stranded ribonucleic acid derivative". In the present disclosure, the siRNA motif is modified using different modification patterns to prepare corresponding siRNA derivatives. For example, in some embodiments, the motif is modified using an alternating modification pattern, thereby obtaining an alternately modified siRNA derivative. In other embodiments, the motif is modified using a specific modification template, thereby obtaining an siRNA derivative with the specific modification template. In still other embodiments, the motif is modified using the anti-off-target modification pattern described herein, thereby obtaining an siRNA derivative with anti-off-target modifications. In some cases, multiple different modification patterns may be simultaneously applied to the same siRNA motif, thereby obtaining corresponding siRNA derivatives with multiple modification patterns.

As used herein, the term "siRNA conjugate" refers to a double-stranded ribonucleic acid conjugate or a conjugate of a double-stranded ribonucleic acid derivative obtained by attaching a conjugate moiety to a double-stranded ribonucleic acid or a double-stranded ribonucleic acid derivative. Preferably, the term "siRNA conjugate" refers to a conjugate of a double-stranded ribonucleic acid derivative.

As used herein, the term "siRNA" in some cases not only refers to the unmodified siRNA duplex (or siRNA motif) described above, but may also refer to the corresponding siRNA derivative and/or siRNA conjugate. For example, in contexts involving, but not limited to, therapeutic methods or therapeutic agents, "siRNA" broadly refers to at least one of an siRNA motif, an siRNA derivative, and/or an siRNA conjugate. Those skilled in the art can clearly understand the specific technical meaning based on the context.

The term "antisense strand" refers to the strand of a double-stranded ribonucleic acid (*e.g.*, the siRNA duplex described herein) that includes a region substantially complementary to a target sequence. As used herein, the term "region of complementarity" refers to a region on the antisense strand that is substantially complementary to a sequence defined herein (*e.g.*, a target sequence). When the region of complementarity is not fully complementary to the target sequence, mismatches may occur in the internal or terminal regions of the molecule. Typically, mismatches are most tolerated in the terminal regions, for example, within 5, 4, 3, 2, or 1 nucleotides from the 5' and/or 3' ends.

As used herein, the term "sense strand" refers to the strand of a double-stranded ribonucleic acid that includes a region substantially complementary to the antisense strand (as defined herein).

The term "alternating modification" refers to a modification pattern in which nucleotides are modified with 2'-O-methyl (2'-OMe) and 2'-fluoro (2'-F) alternately according to the nucleotide arrangement of a double-stranded ribonucleic acid. For example, for the antisense strand of an siRNA, odd-numbered positions (*i.e.*, positions 1, 3, 5, 7, 11, 13, 15, 17, 19, 21, and 23) are modified with 2'-O-methyl, while even-numbered positions (*i.e.*, positions 2, 4, 6, 8, 10, 12, 14, 16, 18, 20, and 22) are modified with 2'-fluoro. For the sense strand complementary to the antisense strand, the positions corresponding to those modified with 2'-O-methyl on the antisense strand are modified with 2'-fluoro, while the positions corresponding to those modified with 2'-fluoro on the antisense strand are modified with 2'-O-methyl on the sense strand.

For RNA interference (RNAi), inhibition of a target gene is achieved by loading the antisense strand of an siRNA into the AGO2 protein to form an RNA-induced silencing complex (RISC), which then cleaves the mRNA transcript of the gene. Loading of the RNA-induced silencing complex (RISC) requires phosphorylation at the 5' end of the antisense strand (5'-phosphate). 5' phosphorylation may occur naturally in cells through the action of cleavage and polyadenylation factor I subunit 1 (Clp1), or may be introduced through chemical synthesis. The term "natural 5' phosphorylation" or "simple direct 5' phosphorylation" refers to phosphorylation at the 5' end of the antisense strand of siRNA occurring in the intracellular environment, rather than being introduced through chemical synthesis.

As used herein, the term "conjugate moiety" refers to a GalNAc derivative attached to an oligonucleotide. In some cases, the conjugate moiety comprises a targeting moiety (also referred to as a ligand), and optionally a linker; for example, a GalNAc derivative attached to an oligonucleotide via a linker (*e.g.*, a divalent, trivalent, or tetravalent branched linker arm); for another example, a GalNAc derivative attached to an oligonucleotide via a monovalent linker arm. In most cases, the terms "ligand" and "conjugate moiety" have meanings well known in the art.

As used herein, the term "inhibiting" is used interchangeably with "reducing", "silencing", "downregulating", "suppressing", and other similar terms, and includes any level of inhibition. As used herein, the term "regulating" in some cases refers to "inhibiting", and those skilled in the art can clearly understand the intended meaning based on the context.

As used herein, the phrase "inhibiting expression of INHBE" includes inhibition of expression of any INHBE gene (*e.g.*, a mouse INHBE gene, a rat INHBE gene, a monkey INHBE gene, or a human INHBE gene) as well as variants (*e.g*., naturally occurring variants) or mutants of the INHBE gene. Thus, the INHBE gene may be a wild-type INHBE gene, a mutant INHBE gene, or a transgenic INHBE gene in the context of genetically manipulated cells, cell populations, or organisms.

"Inhibiting INHBE gene expression" includes any level of inhibition of the INHBE gene, *e.g.*, at least partial inhibition of INHBE gene expression, such as inhibition of at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 8%, at least about 85%, at least about 90%, at least about 91%, at least about 92%, at least about 93%, at least about 94%, at least about 95%, at least about 96%, at least about 97%, at least about 98%, or at least about 99%.

INHBE gene expression may be assessed based on the level of any variable associated with INHBE gene expression, such as INHBE mRNA levels or INHBE protein levels. The inhibition may be assessed by a reduction in the absolute or relative level of one or more of these variables compared to a control level. The control level may be any type of control level utilized in the art, *e.g.,* a pre-dose baseline level or a level determined from a similar subject, cell, or sample that is untreated or treated with a control (*e.g.*, a buffer-only control or an inert control).

As used herein, the term "regulating" in some cases may carry the same meaning as "inhibiting". Accordingly, "regulating INHBE gene expression" may mean "inhibiting INHBE gene expression." Those skilled in the art can clearly understand the specific technical meaning based on the context.

As used herein, a "patient" or "subject" is intended to include humans or non-human animals, preferably mammals, such as monkeys. Preferably, the subject or patient is a human.

As used herein, a "disease associated with INHBE gene expression" is intended to include any disease associated with the INHBE gene or protein. The disease may be caused, for example, by overproduction of the INHBE protein, by mutations in the INHBE gene, by abnormal cleavage of the INHBE protein, or by abnormal interaction between INHBE and other proteins or other endogenous or exogenous substances. Exemplary INHBE-associated diseases include metabolic disorders related to body fat distribution, including, but not limited to, type 2 diabetes; hyperlipidemia or dyslipidemia (elevated or altered circulating levels of low-density lipoprotein cholesterol (LDL-C), triglycerides, very low-density lipoprotein cholesterol (VLDL-C), apolipoprotein B, or other lipid components); obesity (particularly abdominal obesity); lipodystrophy (*e.g*., inability to deposit fat locally in adipose depots (partial lipodystrophy) or inability to deposit fat systemically (lipoatrophy)); insulin resistance or elevated or altered insulin levels during fasting or metabolic challenge; hepatic fat deposition or fatty liver disease and complications thereof (*e.g*., cirrhosis, fibrosis, or liver inflammation); non-alcoholic steatohepatitis; other types of liver inflammation; elevated or altered liver enzyme levels or liver injury; elevated or altered biomarkers of inflammation or fat deposition in the liver; elevated blood pressure and/or hypertension; elevated blood glucose or hyperglycemia; metabolic syndrome; coronary artery disease and other atherosclerotic conditions; as well as complications associated with each of the aforementioned conditions.

As used herein, a "therapeutically effective amount" is intended to include an amount of an RNAi agent that, when administered to a patient for treating an INHBE-associated disease, is sufficient to treat the disease (*e.g*., by attenuating, ameliorating, or maintaining the existing disease or one or more symptoms of the disease). The "therapeutically effective amount" may vary depending on the RNAi agent, how the agent is administered, the disease and its severity, medical history, age, weight, family history, genetic makeup, stage of pathological processes mediated by the expression of INHBE, type of prior or concomitant therapy (if any), and other individual characteristics of the patient to be treated.

As used herein, a "prophylactically effective amount" is intended to include an amount of an RNAi agent that, when administered to a subject who has not yet experienced or exhibited symptoms of an INHBE-associated disease, but who may be susceptible to the disease, is sufficient to prevent or ameliorate the disease or one or more symptoms of the disease. Amelioration of the disease includes slowing the progression of the disease or reducing the severity of later-onset disease. The "prophylactically effective amount" may vary depending on the RNAi agent, how the agent is administered, degree of risk of the disease, medical history, age, weight, family history, genetic makeup, type of prior or concomitant therapy (if any), and other individual characteristics of the patient to be treated.

The "therapeutically effective amount" or "prophylactically effective amount" also includes an amount of an RNAi agent that produces a desired local or systemic effect at a reasonable benefit/risk ratio applicable to any treatment. The RNAi agent used in the methods of the present disclosure may be administered in an amount sufficient to achieve a reasonable benefit/risk ratio applicable to such treatment.

As used herein, the term "sample" includes a collection of similar fluids, cells, or tissues isolated from a subject, as well as fluids, cells, or tissues present in the subject. Examples of biological fluids include blood, serum and serous fluid, plasma, cerebrospinal fluid, ocular fluid, lymph, urine, saliva, *etc.* Tissue samples may include samples from tissues, organs, or localized regions. For example, samples may be derived from specific organs, parts of organs, or fluids or cells within these organs. In certain examples, samples may be derived from the liver (*e.g.*, the entire liver or certain segments of the liver, or certain types of cells in the liver, such as hepatocytes). In a preferred example, a "sample derived from a subject" refers to blood or plasma drawn from the subject. In other examples, a "sample derived from a subject" refers to liver tissue (or subcomponents thereof) derived from the subject.

As used herein, unless otherwise specified, when referring to any nucleotide position on any strand of an siRNA motif, siRNA derivative, siRNA conjugate, siRNA duplex, *etc.*, the positions are indicated in the 5' to 3' direction.

In one aspect, the present disclosure provides an siRNA duplex for targeted regulation of INHBE gene expression, comprising a sense strand and an antisense strand that form a reverse complementary duplex region, wherein the antisense strand comprises at least 15, 16, 17, 18, or 19 consecutive nucleotides complementary to the target sequence of SEQ ID NO: 907 (UUGGAGUGAAGAGACCAAGAUGAAGUUUCCCAG), or wherein the antisense strand comprises a fragment of at least 15, 16, 17, 18, or 19 consecutive nucleotides from any one of the sequences of SEQ ID NOs: 13 to 24, 134 to 236, 238 to 240, and 252 to 260, or a modified fragment thereof.

In some embodiments, the target sequence is derived from the transcript of inhibin βE (INHBE) with the NCBI accession number NM_031479.5.

In some embodiments, the antisense strand comprises the sequence of SEQ ID NO: 906 (UCAUCUUGGUCUC). For example, the nucleotide sequence of the antisense strand is set forth in any one of SEQ ID NOs: 13 to 16 and 18 to 23.

In some embodiments, the antisense strand comprises at least 15, 16, 17, 18, or 19 consecutive nucleotides complementary to the target sequence of SEQ ID NO: 907. For example, the nucleotide sequence of the antisense strand is set forth in any one of SEQ ID NOs: 13 to 16 and 18 to 23.

In some embodiments, the reverse complementary duplex region is 17 to 21 bp in length, such as 20 or 21 bp in length.

In some embodiments, the sense strand comprises a fragment of at least 17, 18, or 19 consecutive nucleotides from any one of the sequences of SEQ ID NOs: 1 to 12, 25 to 127, 129 to 131, and 243 to 251, or a modified fragment thereof.

In some embodiments, the sense strand comprises the sequence of SEQ ID NO: 905 (GAGACCAAGAUGA).

In some embodiments, the sense strand and the antisense strand each independently comprise 19 to 23 nucleotides; preferably, the sense strand comprises 19 to 21 nucleotides, and the antisense strand comprises 21 to 23 nucleotides.

In some embodiments, the siRNA duplex is an siRNA for inhibiting INHBE gene expression.

In some embodiments, the siRNA duplex comprises any one oligonucleotide duplex selected from the following pairings of a sense strand and an antisense strand, or a combination of at least two thereof:
the sense strand has the sequence of SEQ ID NO: 1 or a modified sequence thereof; and the antisense strand has the sequence of SEQ ID NO: 13 or a modified sequence thereof;
the sense strand has the sequence of SEQ ID NO: 2 or a modified sequence thereof; and the antisense strand has the sequence of SEQ ID NO: 14 or a modified sequence thereof;
the sense strand has the sequence of SEQ ID NO: 3 or a modified sequence thereof; and the antisense strand has the sequence of SEQ ID NO: 15 or a modified sequence thereof;
the sense strand has the sequence of SEQ ID NO: 4 or a modified sequence thereof; and the antisense strand has the sequence of SEQ ID NO: 16 or a modified sequence thereof;
the sense strand has the sequence of SEQ ID NO: 5 or a modified sequence thereof; and the antisense strand has the sequence of SEQ ID NO: 17 or a modified sequence thereof;
the sense strand has the sequence of SEQ ID NO: 6 or a modified sequence thereof; and the antisense strand has the sequence of SEQ ID NO: 18 or a modified sequence thereof;
the sense strand has the sequence of SEQ ID NO: 7 or a modified sequence thereof; and the antisense strand has the sequence of SEQ ID NO: 19 or a modified sequence thereof;
the sense strand has the sequence of SEQ ID NO: 8 or a modified sequence thereof; and the antisense strand has the sequence of SEQ ID NO: 20 or a modified sequence thereof;
the sense strand has the sequence of SEQ ID NO: 9 or a modified sequence thereof; and the antisense strand has the sequence of SEQ ID NO: 21 or a modified sequence thereof;
the sense strand has the sequence of SEQ ID NO: 10 or a modified sequence thereof; and the antisense strand has the sequence of SEQ ID NO: 22 or a modified sequence thereof;
the sense strand has the sequence of SEQ ID NO: 11 or a modified sequence thereof; and the antisense strand has the sequence of SEQ ID NO: 23 or a modified sequence thereof;
the sense strand has the sequence of SEQ ID NO: 12 or a modified sequence thereof; and the antisense strand has the sequence of SEQ ID NO: 24 or a modified sequence thereof;
the sense strand has the sequence of SEQ ID NO: 25 or a modified sequence thereof; and the antisense strand has the sequence of SEQ ID NO: 134 or a modified sequence thereof;
the sense strand has the sequence of SEQ ID NO: 26 or a modified sequence thereof; and the antisense strand has the sequence of SEQ ID NO: 135 or a modified sequence thereof;
the sense strand has the sequence of SEQ ID NO: 27 or a modified sequence thereof; and the antisense strand has the sequence of SEQ ID NO: 136 or a modified sequence thereof;
the sense strand has the sequence of SEQ ID NO: 28 or a modified sequence thereof; and the antisense strand has the sequence of SEQ ID NO: 137 or a modified sequence thereof;
the sense strand has the sequence of SEQ ID NO: 29 or a modified sequence thereof; and the antisense strand has the sequence of SEQ ID NO: 138 or a modified sequence thereof;
the sense strand has the sequence of SEQ ID NO: 30 or a modified sequence thereof; and the antisense strand has the sequence of SEQ ID NO: 139 or a modified sequence thereof;
the sense strand has the sequence of SEQ ID NO: 31 or a modified sequence thereof; and the antisense strand has the sequence of SEQ ID NO: 140 or a modified sequence thereof;
the sense strand has the sequence of SEQ ID NO: 32 or a modified sequence thereof; and the antisense strand has the sequence of SEQ ID NO: 141 or a modified sequence thereof;
the sense strand has the sequence of SEQ ID NO: 33 or a modified sequence thereof; and the antisense strand has the sequence of SEQ ID NO: 142 or a modified sequence thereof;
the sense strand has the sequence of SEQ ID NO: 34 or a modified sequence thereof; and the antisense strand has the sequence of SEQ ID NO: 143 or a modified sequence thereof;
the sense strand has the sequence of SEQ ID NO: 35 or a modified sequence thereof; and the antisense strand has the sequence of SEQ ID NO: 144 or a modified sequence thereof;
the sense strand has the sequence of SEQ ID NO: 36 or a modified sequence thereof; and the antisense strand has the sequence of SEQ ID NO: 145 or a modified sequence thereof;
the sense strand has the sequence of SEQ ID NO: 37 or a modified sequence thereof; and the antisense strand has the sequence of SEQ ID NO: 146 or a modified sequence thereof;
the sense strand has the sequence of SEQ ID NO: 38 or a modified sequence thereof; and the antisense strand has the sequence of SEQ ID NO: 147 or a modified sequence thereof;
the sense strand has the sequence of SEQ ID NO: 39 or a modified sequence thereof; and the antisense strand has the sequence of SEQ ID NO: 148 or a modified sequence thereof;
the sense strand has the sequence of SEQ ID NO: 40 or a modified sequence thereof; and the antisense strand has the sequence of SEQ ID NO: 149 or a modified sequence thereof;
the sense strand has the sequence of SEQ ID NO: 41 or a modified sequence thereof; and the antisense strand has the sequence of SEQ ID NO: 150 or a modified sequence thereof;
the sense strand has the sequence of SEQ ID NO: 42 or a modified sequence thereof; and the antisense strand has the sequence of SEQ ID NO: 151 or a modified sequence thereof;
the sense strand has the sequence of SEQ ID NO: 43 or a modified sequence thereof; and the antisense strand has the sequence of SEQ ID NO: 152 or a modified sequence thereof;
the sense strand has the sequence of SEQ ID NO: 44 or a modified sequence thereof; and the antisense strand has the sequence of SEQ ID NO: 153 or a modified sequence thereof;
the sense strand has the sequence of SEQ ID NO: 45 or a modified sequence thereof; and the antisense strand has the sequence of SEQ ID NO: 154 or a modified sequence thereof;
the sense strand has the sequence of SEQ ID NO: 46 or a modified sequence thereof; and the antisense strand has the sequence of SEQ ID NO: 155 or a modified sequence thereof;
the sense strand has the sequence of SEQ ID NO: 47 or a modified sequence thereof; and the antisense strand has the sequence of SEQ ID NO: 156 or a modified sequence thereof;
the sense strand has the sequence of SEQ ID NO: 48 or a modified sequence thereof; and the antisense strand has the sequence of SEQ ID NO: 157 or a modified sequence thereof;
the sense strand has the sequence of SEQ ID NO: 49 or a modified sequence thereof; and the antisense strand has the sequence of SEQ ID NO: 158 or a modified sequence thereof;
the sense strand has the sequence of SEQ ID NO: 50 or a modified sequence thereof; and the antisense strand has the sequence of SEQ ID NO: 159 or a modified sequence thereof;
the sense strand has the sequence of SEQ ID NO: 51 or a modified sequence thereof; and the antisense strand has the sequence of SEQ ID NO: 160 or a modified sequence thereof;
the sense strand has the sequence of SEQ ID NO: 52 or a modified sequence thereof; and the antisense strand has the sequence of SEQ ID NO: 161 or a modified sequence thereof;
the sense strand has the sequence of SEQ ID NO: 53 or a modified sequence thereof; and the antisense strand has the sequence of SEQ ID NO: 162 or a modified sequence thereof;
the sense strand has the sequence of SEQ ID NO: 54 or a modified sequence thereof; and the antisense strand has the sequence of SEQ ID NO: 163 or a modified sequence thereof;
the sense strand has the sequence of SEQ ID NO: 55 or a modified sequence thereof; and the antisense strand has the sequence of SEQ ID NO: 164 or a modified sequence thereof;
the sense strand has the sequence of SEQ ID NO: 56 or a modified sequence thereof; and the antisense strand has the sequence of SEQ ID NO: 165 or a modified sequence thereof;
the sense strand has the sequence of SEQ ID NO: 57 or a modified sequence thereof; and the antisense strand has the sequence of SEQ ID NO: 166 or a modified sequence thereof;
the sense strand has the sequence of SEQ ID NO: 58 or a modified sequence thereof; and the antisense strand has the sequence of SEQ ID NO: 167 or a modified sequence thereof;
the sense strand has the sequence of SEQ ID NO: 59 or a modified sequence thereof; and the antisense strand has the sequence of SEQ ID NO: 168 or a modified sequence thereof;
the sense strand has the sequence of SEQ ID NO: 60 or a modified sequence thereof; and the antisense strand has the sequence of SEQ ID NO: 169 or a modified sequence thereof;
the sense strand has the sequence of SEQ ID NO: 61 or a modified sequence thereof; and the antisense strand has the sequence of SEQ ID NO: 170 or a modified sequence thereof;
the sense strand has the sequence of SEQ ID NO: 62 or a modified sequence thereof; and the antisense strand has the sequence of SEQ ID NO: 171 or a modified sequence thereof;
the sense strand has the sequence of SEQ ID NO: 63 or a modified sequence thereof; and the antisense strand has the sequence of SEQ ID NO: 172 or a modified sequence thereof;
the sense strand has the sequence of SEQ ID NO: 64 or a modified sequence thereof; and the antisense strand has the sequence of SEQ ID NO: 173 or a modified sequence thereof;
the sense strand has the sequence of SEQ ID NO: 65 or a modified sequence thereof; and the antisense strand has the sequence of SEQ ID NO: 174 or a modified sequence thereof;
the sense strand has the sequence of SEQ ID NO: 66 or a modified sequence thereof; and the antisense strand has the sequence of SEQ ID NO: 175 or a modified sequence thereof;
the sense strand has the sequence of SEQ ID NO: 67 or a modified sequence thereof; and the antisense strand has the sequence of SEQ ID NO: 176 or a modified sequence thereof;
the sense strand has the sequence of SEQ ID NO: 68 or a modified sequence thereof; and the antisense strand has the sequence of SEQ ID NO: 177 or a modified sequence thereof;
the sense strand has the sequence of SEQ ID NO: 69 or a modified sequence thereof; and the antisense strand has the sequence of SEQ ID NO: 178 or a modified sequence thereof;
the sense strand has the sequence of SEQ ID NO: 70 or a modified sequence thereof; and the antisense strand has the sequence of SEQ ID NO: 179 or a modified sequence thereof;
the sense strand has the sequence of SEQ ID NO: 71 or a modified sequence thereof; and the antisense strand has the sequence of SEQ ID NO: 180 or a modified sequence thereof;
the sense strand has the sequence of SEQ ID NO: 72 or a modified sequence thereof; and the antisense strand has the sequence of SEQ ID NO: 181 or a modified sequence thereof;
the sense strand has the sequence of SEQ ID NO: 73 or a modified sequence thereof; and the antisense strand has the sequence of SEQ ID NO: 182 or a modified sequence thereof;
the sense strand has the sequence of SEQ ID NO: 74 or a modified sequence thereof; and the antisense strand has the sequence of SEQ ID NO: 183 or a modified sequence thereof;
the sense strand has the sequence of SEQ ID NO: 75 or a modified sequence thereof; and the antisense strand has the sequence of SEQ ID NO: 184 or a modified sequence thereof;
the sense strand has the sequence of SEQ ID NO: 76 or a modified sequence thereof; and the antisense strand has the sequence of SEQ ID NO: 185 or a modified sequence thereof;
the sense strand has the sequence of SEQ ID NO: 77 or a modified sequence thereof; and the antisense strand has the sequence of SEQ ID NO: 186 or a modified sequence thereof;
the sense strand has the sequence of SEQ ID NO: 78 or a modified sequence thereof; and the antisense strand has the sequence of SEQ ID NO: 187 or a modified sequence thereof;
the sense strand has the sequence of SEQ ID NO: 79 or a modified sequence thereof; and the antisense strand has the sequence of SEQ ID NO: 188 or a modified sequence thereof;
the sense strand has the sequence of SEQ ID NO: 80 or a modified sequence thereof; and the antisense strand has the sequence of SEQ ID NO: 189 or a modified sequence thereof;
the sense strand has the sequence of SEQ ID NO: 81 or a modified sequence thereof; and the antisense strand has the sequence of SEQ ID NO: 190 or a modified sequence thereof;
the sense strand has the sequence of SEQ ID NO: 82 or a modified sequence thereof; and the antisense strand has the sequence of SEQ ID NO: 191 or a modified sequence thereof;
the sense strand has the sequence of SEQ ID NO: 83 or a modified sequence thereof; and the antisense strand has the sequence of SEQ ID NO: 192 or a modified sequence thereof;
the sense strand has the sequence of SEQ ID NO: 84 or a modified sequence thereof; and the antisense strand has the sequence of SEQ ID NO: 193 or a modified sequence thereof;
the sense strand has the sequence of SEQ ID NO: 85 or a modified sequence thereof; and the antisense strand has the sequence of SEQ ID NO: 194 or a modified sequence thereof;
the sense strand has the sequence of SEQ ID NO: 86 or a modified sequence thereof; and the antisense strand has the sequence of SEQ ID NO: 195 or a modified sequence thereof;
the sense strand has the sequence of SEQ ID NO: 87 or a modified sequence thereof; and the antisense strand has the sequence of SEQ ID NO: 196 or a modified sequence thereof;
the sense strand has the sequence of SEQ ID NO: 88 or a modified sequence thereof; and the antisense strand has the sequence of SEQ ID NO: 197 or a modified sequence thereof;
the sense strand has the sequence of SEQ ID NO: 89 or a modified sequence thereof; and the antisense strand has the sequence of SEQ ID NO: 198 or a modified sequence thereof;
the sense strand has the sequence of SEQ ID NO: 90 or a modified sequence thereof; and the antisense strand has the sequence of SEQ ID NO: 199 or a modified sequence thereof;
the sense strand has the sequence of SEQ ID NO: 91 or a modified sequence thereof; and the antisense strand has the sequence of SEQ ID NO: 200 or a modified sequence thereof;
the sense strand has the sequence of SEQ ID NO: 92 or a modified sequence thereof; and the antisense strand has the sequence of SEQ ID NO: 201 or a modified sequence thereof;
the sense strand has the sequence of SEQ ID NO: 93 or a modified sequence thereof; and the antisense strand has the sequence of SEQ ID NO: 202 or a modified sequence thereof;
the sense strand has the sequence of SEQ ID NO: 94 or a modified sequence thereof; and the antisense strand has the sequence of SEQ ID NO: 203 or a modified sequence thereof;
the sense strand has the sequence of SEQ ID NO: 95 or a modified sequence thereof; and the antisense strand has the sequence of SEQ ID NO: 204 or a modified sequence thereof;
the sense strand has the sequence of SEQ ID NO: 96 or a modified sequence thereof; and the antisense strand has the sequence of SEQ ID NO: 205 or a modified sequence thereof;
the sense strand has the sequence of SEQ ID NO: 97 or a modified sequence thereof; and the antisense strand has the sequence of SEQ ID NO: 206 or a modified sequence thereof;
the sense strand has the sequence of SEQ ID NO: 98 or a modified sequence thereof; and the antisense strand has the sequence of SEQ ID NO: 207 or a modified sequence thereof;
the sense strand has the sequence of SEQ ID NO: 99 or a modified sequence thereof; and the antisense strand has the sequence of SEQ ID NO: 208 or a modified sequence thereof;
the sense strand has the sequence of SEQ ID NO: 100 or a modified sequence thereof; and the antisense strand has the sequence of SEQ ID NO: 209 or a modified sequence thereof;
the sense strand has the sequence of SEQ ID NO: 101 or a modified sequence thereof; and the antisense strand has the sequence of SEQ ID NO: 210 or a modified sequence thereof;
the sense strand has the sequence of SEQ ID NO: 102 or a modified sequence thereof; and the antisense strand has the sequence of SEQ ID NO: 211 or a modified sequence thereof;
the sense strand has the sequence of SEQ ID NO: 103 or a modified sequence thereof; and the antisense strand has the sequence of SEQ ID NO: 212 or a modified sequence thereof;
the sense strand has the sequence of SEQ ID NO: 104 or a modified sequence thereof; and the antisense strand has the sequence of SEQ ID NO: 213 or a modified sequence thereof;
the sense strand has the sequence of SEQ ID NO: 105 or a modified sequence thereof; and the antisense strand has the sequence of SEQ ID NO: 214 or a modified sequence thereof;
the sense strand has the sequence of SEQ ID NO: 106 or a modified sequence thereof; and the antisense strand has the sequence of SEQ ID NO: 215 or a modified sequence thereof;
the sense strand has the sequence of SEQ ID NO: 107 or a modified sequence thereof; and the antisense strand has the sequence of SEQ ID NO: 216 or a modified sequence thereof;
the sense strand has the sequence of SEQ ID NO: 108 or a modified sequence thereof; and the antisense strand has the sequence of SEQ ID NO: 217 or a modified sequence thereof;
the sense strand has the sequence of SEQ ID NO: 109 or a modified sequence thereof; and the antisense strand has the sequence of SEQ ID NO: 218 or a modified sequence thereof;
the sense strand has the sequence of SEQ ID NO: 110 or a modified sequence thereof; and the antisense strand has the sequence of SEQ ID NO: 219 or a modified sequence thereof;
the sense strand has the sequence of SEQ ID NO: 111 or a modified sequence thereof; and the antisense strand has the sequence of SEQ ID NO: 220 or a modified sequence thereof;
the sense strand has the sequence of SEQ ID NO: 112 or a modified sequence thereof; and the antisense strand has the sequence of SEQ ID NO: 221 or a modified sequence thereof;
the sense strand has the sequence of SEQ ID NO: 113 or a modified sequence thereof; and the antisense strand has the sequence of SEQ ID NO: 222 or a modified sequence thereof;
the sense strand has the sequence of SEQ ID NO: 114 or a modified sequence thereof; and the antisense strand has the sequence of SEQ ID NO: 223 or a modified sequence thereof;
the sense strand has the sequence of SEQ ID NO: 115 or a modified sequence thereof; and the antisense strand has the sequence of SEQ ID NO: 224 or a modified sequence thereof;
the sense strand has the sequence of SEQ ID NO: 116 or a modified sequence thereof; and the antisense strand has the sequence of SEQ ID NO: 225 or a modified sequence thereof;
the sense strand has the sequence of SEQ ID NO: 117 or a modified sequence thereof; and the antisense strand has the sequence of SEQ ID NO: 226 or a modified sequence thereof;
the sense strand has the sequence of SEQ ID NO: 118 or a modified sequence thereof; and the antisense strand has the sequence of SEQ ID NO: 227 or a modified sequence thereof;
the sense strand has the sequence of SEQ ID NO: 119 or a modified sequence thereof; and the antisense strand has the sequence of SEQ ID NO: 228 or a modified sequence thereof;
the sense strand has the sequence of SEQ ID NO: 120 or a modified sequence thereof; and the antisense strand has the sequence of SEQ ID NO: 229 or a modified sequence thereof;
the sense strand has the sequence of SEQ ID NO: 121 or a modified sequence thereof; and the antisense strand has the sequence of SEQ ID NO: 230 or a modified sequence thereof;
the sense strand has the sequence of SEQ ID NO: 122 or a modified sequence thereof; and the antisense strand has the sequence of SEQ ID NO: 231 or a modified sequence thereof;
the sense strand has the sequence of SEQ ID NO: 123 or a modified sequence thereof; and the antisense strand has the sequence of SEQ ID NO: 232 or a modified sequence thereof;
the sense strand has the sequence of SEQ ID NO: 124 or a modified sequence thereof; and the antisense strand has the sequence of SEQ ID NO: 233 or a modified sequence thereof;
the sense strand has the sequence of SEQ ID NO: 125 or a modified sequence thereof; and the antisense strand has the sequence of SEQ ID NO: 234 or a modified sequence thereof;
the sense strand has the sequence of SEQ ID NO: 126 or a modified sequence thereof; and the antisense strand has the sequence of SEQ ID NO: 235 or a modified sequence thereof;
the sense strand has the sequence of SEQ ID NO: 127 or a modified sequence thereof; and the antisense strand has the sequence of SEQ ID NO: 236 or a modified sequence thereof; the sense strand has the sequence of SEQ ID NO: 129 or a modified sequence thereof; and the antisense strand has the sequence of SEQ ID NO: 238 or a modified sequence thereof;
the sense strand has the sequence of SEQ ID NO: 130 or a modified sequence thereof; and the antisense strand has the sequence of SEQ ID NO: 239 or a modified sequence thereof;
the sense strand has the sequence of SEQ ID NO: 131 or a modified sequence thereof; and the antisense strand has the sequence of SEQ ID NO: 240 or a modified sequence thereof;
the sense strand has the sequence of SEQ ID NO: 243 or a modified sequence thereof; and the antisense strand has the sequence of SEQ ID NO: 252 or a modified sequence thereof;
the sense strand has the sequence of SEQ ID NO: 244 or a modified sequence thereof; and the antisense strand has the sequence of SEQ ID NO: 253 or a modified sequence thereof;
the sense strand has the sequence of SEQ ID NO: 245 or a modified sequence thereof; and the antisense strand has the sequence of SEQ ID NO: 254 or a modified sequence thereof;
the sense strand has the sequence of SEQ ID NO: 246 or a modified sequence thereof; and the antisense strand has the sequence of SEQ ID NO: 255 or a modified sequence thereof;
the sense strand has the sequence of SEQ ID NO: 247 or a modified sequence thereof; and the antisense strand has the sequence of SEQ ID NO: 256 or a modified sequence thereof;
the sense strand has the sequence of SEQ ID NO: 248 or a modified sequence thereof; and the antisense strand has the sequence of SEQ ID NO: 257 or a modified sequence thereof;
the sense strand has the sequence of SEQ ID NO: 249 or a modified sequence thereof; and the antisense strand has the sequence of SEQ ID NO: 258 or a modified sequence thereof;
the sense strand has the sequence of SEQ ID NO: 250 or a modified sequence thereof; and the antisense strand has the sequence of SEQ ID NO: 259 or a modified sequence thereof;
the sense strand has the sequence of SEQ ID NO: 251 or a modified sequence thereof; and the antisense strand has the sequence of SEQ ID NO: 260 or a modified sequence thereof.

In some embodiments, the sense strand differs from any one of the sequences of SEQ ID NOs: 1 to 12, 25 to 127, 129 to 131, and 243 to 251 by 1 to 3 nucleotides.

In some embodiments, the antisense strand differs from any one of the sequences of SEQ ID NOs: 13 to 24, 134 to 236, 238 to 240, and 252 to 260 by 1 to 3 nucleotides.

In some preferred embodiments, the nucleotide sequence of the sense strand is set forth in any one of SEQ ID NOs: 1 to 12, and the corresponding nucleotide sequence of the antisense strand is set forth in SEQ ID NOs: 13 to 24, respectively.

In some embodiments, the number of nucleotides in the sense strand is either the same as or different from the number of nucleotides in the antisense strand.

In some embodiments, the sense strand comprises 19 nucleotides and the antisense strand comprises 19 nucleotides.

In some embodiments, the sense strand comprises 20 nucleotides and the antisense strand comprises 19 nucleotides.

In some embodiments, the sense strand comprises 20 nucleotides and the antisense strand comprises 20 nucleotides.

In some embodiments, the sense strand comprises 19 nucleotides and the antisense strand comprises 20 nucleotides.

In some embodiments, the sense strand comprises 19 nucleotides and the antisense strand comprises 21 nucleotides.

In some embodiments, the sense strand comprises 20 nucleotides and the antisense strand comprises 21 nucleotides.

In some embodiments, the sense strand comprises 21 nucleotides and the antisense strand comprises 21 nucleotides.

In some embodiments, the sense strand comprises 19 nucleotides and the antisense strand comprises 22 nucleotides.

In some embodiments, the sense strand comprises 20 nucleotides and the antisense strand comprises 22 nucleotides.

In some embodiments, the sense strand comprises 21 nucleotides and the antisense strand comprises 22 nucleotides.

In some embodiments, the sense strand comprises 19 nucleotides and the antisense strand comprises 23 nucleotides.

In some embodiments, the sense strand comprises 20 nucleotides and the antisense strand comprises 23 nucleotides.

In some embodiments, the sense strand comprises 21 nucleotides and the antisense strand comprises 23 nucleotides.

In some embodiments, the sense strand comprises 23 nucleotides and the antisense strand comprises 23 nucleotides.

In some embodiments, the sense strand and the antisense strand each independently comprise at least one modified nucleotide.

In some embodiments, the at least one modified nucleotide is any one selected from the group consisting of a deoxy-nucleotide, a 3'-deoxy-thymidine (dT) nucleotide, a 2'-O-methyl-modified nucleotide, a 2'-fluoro-modified nucleotide, a 2'-deoxy-modified nucleotide, a locked nucleotide, an unlocked nucleotide, a conformationally restricted nucleotide, a constrained ethyl nucleotide, an abasic nucleotide, a 2'-amino-modified nucleotide, a 2'-O-allyl-modified nucleotide, a 2'-C-alkyl-modified nucleotide, a 2'-hydroxy-modified nucleotide, a 2'-methoxyethyl-modified nucleotide, a 2'-O-alkyl-modified nucleotide, a morpholino nucleotide, a phosphoramidate, a nucleotide comprising a non-natural base, a tetrahydropyran modified nucleotide, a 1,5-anhydrohexitol-modified nucleotide, a cyclohexenyl-modified nucleotide, a nucleotide comprising a phosphorothioate group, a nucleotide comprising a methylphosphonate group, a nucleotide comprising a 5'-phosphate group, and a nucleotide comprising a 5'-phosphate mimic, or a combination of at least two thereof.

In some embodiments, at least one strand comprises a 3' overhang of at least one nucleotide.

In some embodiments, at least one strand comprises a 3' overhang of at least two nucleotides.

In some embodiments, nucleotides on the sense and antisense strands comprise a modification at the 2' position of the ribose of the nucleotide.

In some embodiments, the modification at the 2' position of the ribose of the nucleotide is any one selected from 2'-O-methyl modification, 2'-methoxyethyl modification, 2'-fluoro modification, 2'-benzyloxy modification, 2'-methylcarbonylamino modification, and 2'-pyridinylmethoxy modification, or a combination thereof.

In some embodiments, the modification at the 2' position of the ribose of each nucleotide is a combination of 2'-O-methyl modification and 2'-fluoro modification.

In some embodiments, the modification at the 2' position of the ribose of each nucleotide is an alternating combination of 2'-O-methyl modification and 2'-fluoro modification.

In some embodiments, the modification pattern at the 2' position of the ribose of each nucleotide is as follows: on the sense strand, all odd-numbered positions are modified with 2'-fluoro, and all even-numbered positions are modified with 2'-O-methyl; and on the antisense strand, all odd-numbered positions are modified with 2'-O-methyl, and all even-numbered positions are modified with 2'-fluoro.

In some embodiments, nucleotides are linked to one another via a 3',5'-phosphodiester bond.

In some embodiments, nucleotides are linked to one another via a 3',5'-phosphorothioate bond.

In some embodiments, the siRNA duplex has alternating 2'-fluoro and 2'-O-methyl modifications or template modifications.

In some embodiments, the 5' carbon atom of the nucleotide glycoside at the 5' end of the modified antisense strand phosphorylated, wherein the phosphorylation of the 5' carbon atom of the nucleotide glycoside includes, but is not limited to, the following 5'-phosphorylation groups: 5'-vinylphosphonate group (5'-E-VP), 5'-methylphosphonate group (5'-MP), 5'-C-methylphosphonate group, 5'-phosphorothioate group (5'-PS), and 5'-phosphate group (5'-P), the structures of which are as follows: 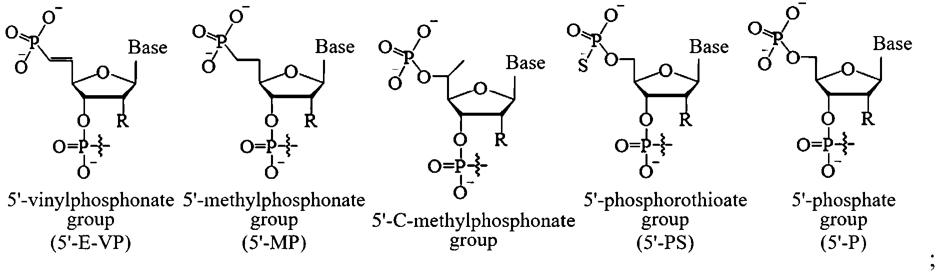
wherein R is hydrogen, hydroxy, amino, C₁₋₄ alkyl, aryl, C₁₋₄ alkoxy, C₁₋₄ alkylcarbonylamino, or halogen;
the base is selected from adenine, guanine, cytosine, thymine, and uracil.

In some embodiments, there is a 3',5'-phosphorothioate bond between nucleotides at positions 1 to 2 from the 3' and/or 5' ends of the sense strand and/or the antisense strand of the siRNA derivative. For example, in some embodiments, a stereochemically pure 3',5'-phosphorothioate bond is formed. In some embodiments, one, two, or three 3',5'-phosphorothioate bonds may be present among nucleotides at positions 1 to 4 from the 5' end of the sense strand and/or the antisense strand, and one, two, or three 3',5'-phosphorothioate bonds may be present among nucleotides at positions 1 to 4 from the 3' end of the antisense strand.

In some embodiments, the antisense strand adopts any one of the following modification patterns:

| **No.** | **Antisense strand (AS) 5'-3'** | | | | | | | | | | | |
|---|---|---|---|---|---|---|---|---|---|---|---|---|
| **A** | **1** | **2** | **3** | **4** | **5** | **6** | **7** | **8** | **9** | **10** | **11** | **12** |
| | 2'-OMe +PS +EVP | **2'-F +PS** | 2'-OMe | 2'-OMe | 2'-OMe | **2'-F** | 2'-OMe | 2'-OMe | 2'-OMe | 2'-OMe | 2'-OMe | 2'-OMe |
| | **13** | **14** | **15** | **16** | 17 | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **B** | **1** | **2** | **3** | **4** | **5** | **6** | **7** | **8** | **9** | **10** | **11** | **12** |
| | 2'-OMe +PS +EVP | **2'-F +PS** | **2'-F** | **2'-F** | 2'-OMe | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **C** | **1** | **2** | **3** | **4** | **5** | **6** | **7** | **8** | **9** | **10** | **11** | **12** |
| | 2'-OMe +PS +EVP | **2'-F +PS** | 2'-OMe | **2'-F** | **2'-F** | **2'-F** | 2'-OMe | 2'-OMe | 2'-OMe | 2'-OMe | 2'-OMe | 2'-OMe |
| | 13 | 14 | 15 | 16 | 17 | 18 | 19 | 20 | 21 | +22 | 23 | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **D** | **1** | **2** | **3** | **4** | **5** | **6** | **7** | **8** | **9** | **10** | **11** | **12** |
| | 2'-OMe +PS +EVP | **2'-F +PS** | 2'-OMe | 2'-OMe | **2'-F** | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **E** | **1** | **2** | 3 | **4** | **5** | 6 | 7 | 8 | 9 | 10 | 11 | 12 |
| | 2'-OMe +PS +EVP | **2'-F +PS** | **2'-F** | 2'-OMe | 2'-OMe | **2'-F** | 2'-OMe | 2'-OMe | 2'-OMe | 2'-OMe | 2'-OMe | 2'-OMe |
| | 13 | 14 | 15 | 16 | 17 | 18 | 19 | 20 | 21 | 22 | 23 | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **F** | 1 | 2 | 3 | 4 | 5 | 6 | 7 | 8 | 9 | 10 | 11 | 12 |
| | 2'-OMe +PS +EVP | **2'-F +PS** | 2'-OMe | 2'-OMe | 2'-OMe | **2'-F** | 2'-OMe | **2'-F** | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **G** | **1** | **2** | **3** | **4** | **5** | **6** | **7** | **8** | **9** | **10** | **11** | **12** |
| | 2'-OMe +PS +EVP | **2'-F +PS** | 2'-OMe | **2'-F** | **2'-F** | **2'-F** | 2'-OMe | **2'-F** | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **H** | **1** | **2** | **3** | **4** | **5** | **6** | **7** | **8** | **9** | **10** | **11** | **12** |
| | 2'-OMe +PS +EVP | **2'-F +PS** | **2'-F** | 2'-OMe | 2'-OMe | **2'-F** | 2'-OMe | **2'-F** | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |

and/or, the sense strand adopts any one of the following modification patterns:

| No. | **Sense strand (SS) 5'-3'** | | | | | | | | | | |
|---|---|---|---|---|---|---|---|---|---|---|---|
| **a** | **1** | **2** | **3** | **4** | **5** | **6** | **7** | 8 | **9** | **10** | **11** |
| | 2'-OMe +PS | 2'-OMe +PS | 2'-OMe | 2'-OMe | 2'-OMe | 2'-OMe | **2'-F** | 2'-OMe | **2'-F** | **2'-F** | **2'-F** |
| | **12** | **13** | **14** | **15** | **16** | **17** | **18** | **19** | **20** | **21** | |
| | 2'-OMe | 2'-OMe | 2'-OMe | 2'-OMe | 2'-OMe | **2'-F** | 2'-OMe | 2'-OMe | 2'-OMe | 2'-OMe | |
| **b** | **1** | **2** | **3** | **4** | **5** | **6** | **7** | **8** | **9** | **10** | **11** |
| | 2'-OMe +PS | 2'-OMe +PS | 2'-OMe | 2'-OMe | 2'-OMe | 2'-OMe | **2'-F** | 2'-OMe | **2'-F** | 2'-OMe | **2'-F** |
| | **12** | **13** | **14** | **15** | **16** | **17** | **18** | **19** | **20** | **21** | |
| | 2'-OMe | 2'-OMe | 2'-OMe | 2'-OMe | 2'-OMe | **2'-F** | 2'-OMe | 2'-OMe | 2'-OMe | 2'-OMe | |

wherein 2'-OMe represents a 2'-O-methyl modification; 2'-F represents a 2'-fluoro modification; PS indicates that, starting from the 5' end, the nucleotide at the position is linked to the adjacent nucleotide on the right via a 3',5'-phosphorothioate bond; EVP represents a 5'-vinyl-(E)-phosphonate modification.

In some embodiments, the antisense strand is modified according to modification pattern A, and the sense strand is modified according to modification pattern a;
the antisense strand is modified according to modification pattern B, and the sense strand is modified according to modification pattern a;
the antisense strand is modified according to modification pattern C, and the sense strand is modified according to modification pattern a;
the antisense strand is modified according to modification pattern C, and the sense strand is modified according to modification pattern b;
the antisense strand is modified according to modification pattern D, and the sense strand is modified according to modification pattern b;
the antisense strand is modified according to modification pattern E, and the sense strand is modified according to modification pattern b;
the antisense strand is modified according to modification pattern F, and the sense strand is modified according to modification pattern b;
the antisense strand is modified according to modification pattern G, and the sense strand is modified according to modification pattern b; or
the antisense strand is modified according to modification pattern H, and the sense strand is modified according to modification pattern b.

In some embodiments, the antisense strand adopts any one of the following modification patterns:

| No. | **Antisense strand (AS) 5'-3'** | | | | | | | | | | |
|---|---|---|---|---|---|---|---|---|---|---|---|
| **A'** | **1** | **2** | **3** | **4** | **5** | **6** | **7** | **8** | **9** | **10** | **11** |
| | 2'-OMe +PS +EVP | **2'-F +PS** | 2'-OMe | 2'-OMe | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **B'** | **1** | **2** | **3** | **4** | **5** | **6** | **7** | **8** | **9** | **10** | **11** |
| | 2'-OMe +PS +EVP | **2'-F +PS** | **2'-F** | **2'-F** | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **C'** | 1 | 2 | 3 | 4 | 5 | 6 | 7 | 8 | 9 | 10 | 11 |
| | 2'-OMe +PS +EVP | **2'-F +PS** | 2'-OMe | **2'-F** | **2'-F** | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **D'** | **1** | **2** | **3** | **4** | 5 | **6** | **7** | **8** | **9** | **10** | **11** |
| | 2'-OMe +PS +EVP | **2'-F +PS** | 2'-OMe | 2'-OMe | **2'-F** | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | 22 |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **E'** | **1** | **2** | **3** | **4** | **5** | **6** | **7** | **8** | **9** | **10** | **11** |
| | 2'-OMe +PS +EVP | **2'-F +PS** | **2'-F** | 2'-OMe | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **F'** | **1** | **2** | **3** | **4** | **5** | **6** | **7** | **8** | **9** | **10** | **11** |
| | 2'-OMe +PS +EVP | **2'-F +PS** | 2'-OMe | 2'-OMe | 2'-OMe | **2'-F** | 2'-OMe | **2'-F** | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **G'** | **1** | **2** | **3** | **4** | **5** | **6** | **7** | **8** | **9** | **10** | **11** |
| | 2'-OMe +PS +EVP | **2'-F +PS** | 2'-OMe | **2'-F** | **2'-F** | **2'-F** | 2'-OMe | **2'-F** | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **H'** | **1** | **2** | **3** | **4** | **5** | **6** | **7** | **8** | **9** | **10** | **11** |
| | 2'-OMe +PS +EVP | **2'-F +PS** | **2'-F** | 2'-OMe | 2'-OMe | **2'-F** | 2'-OMe | **2'-F** | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **I'** | **1** | **2** | **3** | **4** | **5** | **6** | **7** | **8** | **9** | **10** | **11** |
| | 2'-OMe +PS | **2'-F +PS** | 2'-OMe | 2'-OMe | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |

and/or, the sense strand adopts any one of the following modification patterns:

| No. | **Sense strand (SS) 5'-3'** | | | | | | | | | |
|---|---|---|---|---|---|---|---|---|---|---|
| **a'** | **1** | **2** | **3** | **4** | **5** | **6** | **7** | 8 | **9** | **10** |
| | 2'-OMe+PS | 2'-OMe+PS | 2'-OMe | 2'-OMe | 2'-OMe | **2'-F** | 2'-OMe | **2'-F** | **2'-F** | **2'-F** |
| | **11** | **12** | **13** | **14** | **15** | **16** | **17** | **18** | **19** | **20** |
| | 2'-OMe | 2'-OMe | 2'-OMe | 2'-OMe | 2'-OMe | **2'-F** | 2'-OMe | 2'-OMe | 2'-OMe | 2'-OMe |
| **b'** | **1** | **2** | **3** | **4** | **5** | **6** | **7** | **8** | **9** | **10** |
| | 2'-OMe+PS | 2'-OMe+PS | 2'-OMe | 2'-OMe | 2'-OMe | **2'-F** | 2'-OMe | **2'-F** | 2'-OMe | **2'-F** |
| | **11** | **12** | **13** | **14** | **15** | **16** | **17** | **18** | **19** | **20** |
| | 2'-OMe | 2'-OMe | 2'-OMe | 2'-OMe | 2'-OMe | **2'-F** | 2'-OMe | 2'-OMe | 2'-OMe | 2'-OMe |
| **c'** | **1** | **2** | **3** | **4** | **5** | **6** | **7** | **8** | **9** | **10** |
| | 2'-OMe+PS | 2'-OMe+PS | 2'-OMe | 2'-OMe | 2'-OMe | **2'-F** | 2'-OMe | **2'-F** | **2'-F** | **2'-F** |
| | **11** | **12** | **13** | **14** | **15** | **16** | **17** | **18** | **19** | **20** |
| | 2'-OMe | 2'-OMe | 2'-OMe | 2'-OMe | 2'-OMe | 2'-OMe | 2'-OMe | 2'-OMe | 2'-OMe | 2'-OMe |

wherein 2'-OMe represents a 2'-O-methyl modification; 2'-F represents a 2'-fluoro modification; PS indicates that, starting from the 5' end, the nucleotide at the position is linked to the adjacent nucleotide on the right via a 3',5'-phosphorothioate bond; EVP represents a 5'-vinyl-(E)-phosphonate modification.

In some embodiments, the antisense strand is modified according to modification pattern A', and the sense strand is modified according to modification pattern a';
the antisense strand is modified according to modification pattern B', and the sense strand is modified according to modification pattern a';
the antisense strand is modified according to modification pattern C', and the sense strand is modified according to modification pattern a';
the antisense strand is modified according to modification pattern C', and the sense strand is modified according to modification pattern b';
the antisense strand is modified according to modification pattern D', and the sense strand is modified according to modification pattern b';
the antisense strand is modified according to modification pattern E', and the sense strand is modified according to modification pattern b';
the antisense strand is modified according to modification pattern F', and the sense strand is modified according to modification pattern b';
the antisense strand is modified according to modification pattern G', and the sense strand is modified according to modification pattern b'; or
the antisense strand is modified according to modification pattern H', and the sense strand is modified according to modification pattern b'.

In some embodiments, the siRNA duplex is any one selected from the following, or a combination of at least two thereof:

| siRNA ID | SEO ID NO of sense motif | Sense modification pattern | SEQ ID NO of antisense motif | Antisense modification pattern |
|---|---|---|---|---|
| si075-DV29P | 1 | b | 13 | C |
| si751-DV29P | 2 | b | 14 | C |
| si753-DV29P | 3 | b | 15 | C |
| si791-DV29P | 4 | b | 16 | C |
| si014-DV29P | 5 | b | 17 | C |
| si076-DV29P | 6 | b | 18 | C |
| si077-DV29P | 7 | b | 19 | C |
| si078-DV29P | 8 | b | 20 | C |
| si080-DV29P | 9 | b | 21 | C |
| si752-DV29SP | 10 | b' | 22 | C' |
| si079-DV29SP | 11 | b' | 23 | C' |
| si047-DV29SP | 12 | b' | 24 | C' |
| si001-DV29P | 25 | b | 134 | C |
| si002-DV29P | 26 | b | 135 | C |
| si003-DV29P | 27 | b | 136 | C |
| si004-DV29P | 28 | b | 137 | C |
| si005-DV29SP | 29 | b' | 138 | C' |
| si006-DV29SP | 30 | b' | 139 | C' |
| si007-DV29P | 31 | b | 140 | C |
| si008-DV29P | 32 | b | 141 | C |
| si009-DV29P | 33 | b | 142 | C |
| si010-DV29SP | 34 | b' | 143 | C' |
| si011-DV29SP | 35 | b' | 144 | C' |
| si012-DV29P | 36 | b | 145 | C |
| si013-DV29P | 37 | b | 146 | C |
| si015-DV29SP | 38 | b' | 147 | C' |
| si016-DV29P | 39 | b | 148 | C |
| si017-DV29P | 40 | b | 149 | C |
| si018-DV29P | 41 | b | 150 | C |
| si019-DV29P | 42 | b | 151 | C |
| si020-DV29SP | 43 | b' | 152 | C' |
| si021-DV29P | 44 | b | 153 | C |
| si022-DV29P | 45 | b | 154 | C |
| si023-DV29P | 46 | b | 155 | C |
| si024-DV29SP | 47 | b' | 156 | C' |
| si025-DV29P | 48 | b | 157 | C |
| si026-DV29P | 49 | b | 158 | C |
| si027-DV29SP | 50 | b' | 159 | C' |
| si028-DV29P | 51 | b | 160 | C |
| si029-DV29P | 52 | b | 161 | C |
| si030-DV29SP | 53 | b' | 162 | C' |
| si031-DV29SP | 54 | b' | 163 | C' |
| si032-DV29P | 55 | b | 164 | C |
| si033-DV29SP | 56 | b' | 165 | C' |
| si034-DV29P | 57 | b | 166 | C |
| si035-DV29P | 58 | b | 167 | C |
| si036-DV29P | 59 | b | 168 | C |
| si037-DV29P | 60 | b | 169 | C |
| si038-DV29P | 61 | b | 170 | C |
| si039-DV29P | 62 | b | 171 | C |
| si040-DV29SP | 63 | b' | 172 | C' |
| si041-DV29SP | 64 | b' | 173 | C' |
| si042-DV29SP | 65 | b' | 174 | C' |
| si043-DV29SP | 66 | b' | 175 | C' |
| si044-DV29P | 67 | b | 176 | C |
| si045-DV29SP | 68 | b' | 177 | C' |
| si046-DV29SP | 69 | b' | 178 | C' |
| si048-DV29SP | 70 | b' | 179 | C' |
| si049-DV29SP | 71 | b' | 180 | C' |
| si050-DV29P | 72 | b | 181 | C |
| si051-DV29SP | 73 | b' | 182 | C' |
| si052-DV29P | 74 | b | 183 | C |
| si053-DV29P | 75 | b | 184 | C |
| si054-DV29P | 76 | b | 185 | C |
| si055-DV29P | 77 | b | 186 | C |
| si056-DV29SP | 78 | b' | 187 | C' |
| si057-DV29P | 79 | b | 188 | C |
| si058-DV29SP | 80 | b' | 189 | C' |
| si059-DV29P | 81 | b | 190 | C |
| si060-DV29P | 82 | b | 191 | C |
| si061-DV29P | 83 | b | 192 | C |
| si062-DV29P | 84 | b | 193 | C |
| si063-DV29P | 85 | b | 194 | C |
| si064-DV29P | 86 | b | 195 | C |
| si065-DV29P | 87 | b | 196 | C |
| si066-DV29SP | 88 | b' | 197 | C' |
| si067-DV29SP | 89 | b' | 198 | C' |
| si068-DV29P | 90 | b | 199 | C |
| si069-DV29P | 91 | b | 200 | C |
| si070-DV29P | 92 | b | 201 | C |
| si071-DV29P | 93 | b | 202 | C |
| si072-DV29P | 94 | b | 203 | C |
| si073-DV29P | 95 | b | 204 | C |
| si074-DV29P | 96 | b | 205 | C |
| si081-DV29P | 97 | b | 206 | C |
| si082-DV29P | 98 | b | 207 | C |
| si083-DV29P | 99 | b | 208 | C |
| si084-DV29P | 100 | b | 209 | C |
| si085-DV29P | 101 | b | 210 | C |
| si086-DV29P | 102 | b | 211 | C |
| si087-DV29P | 103 | b | 212 | C |
| si088-DV29P | 104 | b | 213 | C |
| si089-DV29P | 105 | b | 214 | C |
| si090-DV29P | 106 | b | 215 | C |
| si091-DV29P | 107 | b | 216 | C |
| si092-DV29P | 108 | b | 217 | C |
| si093-DV29P | 109 | b | 218 | C |
| si094-DV29P | 110 | b | 219 | C |
| si095-DV29P | 111 | b | 220 | C |
| si096-DV29P | 112 | b | 221 | C |
| si097-DV29P | 113 | b | 222 | C |
| si098-DV29P | 114 | b | 223 | C |
| si099-DV29P | 115 | b | 224 | C |
| si100-DV29P | 116 | b | 225 | C |
| si101-DV29P | 117 | b | 226 | C |
| si102-DV29P | 118 | b | 227 | C |
| si103-DV29P | 119 | b | 228 | C |
| si104-DV29P | 120 | b | 229 | C |
| si105-DV29P | 121 | b | 230 | C |
| si106-DV29P | 122 | b | 231 | C |
| si107-DV29P | 123 | b | 232 | C |
| si108-DV29P | 124 | b | 233 | C |
| si0481-DV29SP | 125 | b' | 234 | C' |
| siA029-DV29P | 126 | b | 235 | C |
| siA036-DV29P | 127 | b | 236 | C |
| siA043-DV29P | 129 | b | 238 | C |
| siA045-DV29P | 130 | b | 239 | C |
| siA048-DV29P | 131 | b | 240 | C |
| si754-DV29SP | 243 | b' | 252 | C' |
| si755-DV29SP | 244 | b' | 253 | C' |
| si756-DV29SP | 245 | b' | 254 | C' |
| si792-DV29SP | 246 | b' | 255 | C' |
| si141-DV29SP | 247 | b' | 256 | C' |
| si761-DV29SP | 248 | b' | 257 | C' |
| si771-DV29SP | 249 | b' | 258 | C' |
| si781-DV29SP | 250 | b' | 259 | C' |
| si801-DV29SP | 251 | b' | 260 | C' |
| si075-DV25P | 1 | a | 13 | A |
| si751-DV25P | 2 | a | 14 | A |
| si753-DV25P | 3 | a | 15 | A |
| si791-DV25P | 4 | a | 16 | A |
| si014-DV25P | 5 | a | 17 | A |
| si076-DV25P | 6 | a | 18 | A |
| si077-DV25P | 7 | a | 19 | A |
| si078-DV25P | 8 | a | 20 | A |
| si080-DV25P | 9 | a | 21 | A |
| si752-DV25SP | 10 | a' | 22 | A' |
| si079-DV25SP | 11 | a' | 23 | A' |
| si047-DV25SP | 12 | a' | 24 | A' |
| si075-DV26P | 1 | a | 13 | B |
| si751-DV26P | 2 | a | 14 | B |
| si753-DV26P | 3 | a | 15 | B |
| si791-DV26P | 4 | a | 16 | B |
| si014-DV26P | 5 | a | 17 | B |
| si076-DV26P | 6 | a | 18 | B |
| si077-DV26P | 7 | a | 19 | B |
| si078-DV26P | 8 | a | 20 | B |
| si080-DV26P | 9 | a | 21 | B |
| si752-DV26SP | 10 | a' | 22 | B' |
| si079-DV26SP | 11 | a' | 23 | B' |
| si047-DV26SP | 12 | a' | 24 | B' |
| si075-DV27P | 1 | a | 13 | C |
| si751-DV27P | 2 | a | 14 | C |
| si753-DV27P | 3 | a | 15 | C |
| si791-DV27P | 4 | a | 16 | C |
| si014-DV27P | 5 | a | 17 | C |
| si076-DV27P | 6 | a | 18 | C |
| si077-DV27P | 7 | a | 19 | C |
| si078-DV27P | 8 | a | 20 | C |
| si080-DV27P | 9 | a | 21 | C |
| si752-DV27SP | 10 | a' | 22 | C' |
| si079-DV27SP | 11 | a' | 23 | C' |
| si047-DV27SP | 12 | a' | 24 | C' |
| si075-DV32P | 1 | b | 13 | D |
| si751-DV32P | 2 | b | 14 | D |
| si753-DV32P | 3 | b | 15 | D |
| si791-DV32P | 4 | b | 16 | D |
| si014-DV32P | 5 | b | 17 | D |
| si076-DV32P | 6 | b | 18 | D |
| si077-DV32P | 7 | b | 19 | D |
| si078-DV32P | 8 | b | 20 | D |
| si080-DV32P | 9 | b | 21 | D |
| si752-DV32SP | 10 | b' | 22 | D' |
| si079-DV32SP | 11 | b' | 23 | D' |
| si047-DV32SP | 12 | b' | 24 | D' |
| si075-DV34P | 1 | b | 13 | E |
| si751-DV34P | 2 | b | 14 | E |
| si753-DV34P | 3 | b | 15 | E |
| si791-DV34P | 4 | b | 16 | E |
| si014-DV34P | 5 | b | 17 | E |
| si076-DV34P | 6 | b | 18 | E |
| si077-DV34P | 7 | b | 19 | E |
| si078-DV34P | 8 | b | 20 | E |
| si080-DV34P | 9 | b | 21 | E |
| si752-DV34SP | 10 | b' | 22 | E' |
| si079-DV34SP | 11 | b' | 23 | E' |
| si047-DV34SP | 12 | b' | 24 | E' |
| si075-DV41P | 1 | b | 13 | F |
| si751-DV41P | 2 | b | 14 | F |
| si791-DV41P | 4 | b | 16 | F |
| si080-DV41P | 9 | b | 21 | F |
| si079-DV41SP | 11 | b' | 23 | F' |
| si075-DV42P | 1 | b | 13 | G |
| si751-DV42P | 2 | b | 14 | G |
| si791-DV42P | 4 | b | 16 | G |
| si080-DV42P | 9 | b | 21 | G |
| si079-DV42SP | 11 | b' | 23 | G' |
| si075-DV43P | 1 | b | 13 | H |
| si751-DV43P | 2 | b | 14 | H |
| si791-DV43P | 4 | b | 16 | H |
| si080-DV43P | 9 | b | 21 | H |
| si079-DV43SP | 11 | b' | 23 | H' |
| si075-DV22 | 1 | c | 13 | I |
| si751-DV22 | 2 | c | 14 | I |
| si753-DV22 | 3 | c | 15 | I |
| si791-DV22 | 4 | c | 16 | I |
| si014-DV22 | 5 | c | 17 | I |
| si076-DV22 | 6 | c | 18 | I |
| si077-DV22 | 7 | c | 19 | I |
| si078-DV22 | 8 | c | 20 | I |
| si080-DV22 | 9 | c | 21 | I |
| si752-DV22S | 10 | c' | 22 | I' |
| si079-DV22S | 11 | c' | 23 | I' |
| si047-DV22S | 12 | c' | 24 | I' |
| si754-DV26SP | 243 | a' | 252 | B' |
| si754-DV27SP | 243 | a' | 252 | C' |
| si754-DV25SP | 243 | a' | 252 | A' |
| si754-DV32SP | 243 | b' | 252 | D' |
| si754-DV34SP | 243 | b' | 252 | E' |
| si754-DV22S | 243 | c' | 252 | I' |
| si755-DV26SP | 244 | a' | 253 | B' |
| si755-DV27SP | 244 | a' | 253 | C' |
| si755-DV25SP | 244 | a' | 253 | A' |
| si755-DV32SP | 244 | b' | 253 | D' |
| si755-DV34SP | 244 | b' | 253 | E' |
| si755-DV22S | 244 | c' | 253 | I' |
| si756-DV26SP | 245 | a' | 254 | B' |
| si756-DV27SP | 245 | a' | 254 | C' |
| si756-DV25SP | 245 | a' | 254 | A' |
| si756-DV32SP | 245 | b' | 254 | D' |
| si756-DV34SP | 245 | b' | 254 | E' |
| si756-DV22S | 245 | c' | 254 | I' |
| si792-DV26SP | 246 | a' | 255 | B' |
| si792-DV27SP | 246 | a' | 255 | C' |
| si792-DV25SP | 246 | a' | 255 | A' |
| si792-DV32SP | 246 | b' | 255 | D' |
| si792-DV34SP | 246 | b' | 255 | E' |
| si792-DV22S | 246 | c' | 255 | I' |
| si141-DV26SP | 247 | a' | 256 | B' |
| si141-DV27SP | 247 | a' | 256 | C' |
| si141-DV25SP | 247 | a' | 256 | A' |
| si141-DV32SP | 247 | b' | 256 | D' |
| si141-DV34SP | 247 | b' | 256 | E' |
| si141-DV22S | 247 | c' | 256 | I' |
| si761-DV26SP | 248 | a' | 257 | B' |
| si761-DV27SP | 248 | a' | 257 | C' |
| si761-DV25SP | 248 | a' | 257 | A' |
| si761-DV32SP | 248 | b' | 257 | D' |
| si761-DV34SP | 248 | b' | 257 | E' |
| si761-DV22S | 248 | c' | 257 | I' |
| si771-DV26SP | 249 | a' | 258 | B' |
| si771-DV27SP | 249 | a' | 258 | C' |
| si771-DV25SP | 249 | a' | 258 | A' |
| si771-DV32SP | 249 | b' | 258 | D' |
| si771-DV34SP | 249 | b' | 258 | E' |
| si771-DV22S | 249 | c' | 258 | I' |
| si781-DV26SP | 250 | a' | 259 | B' |
| si781-DV27SP | 250 | a' | 259 | C' |
| si781-DV25SP | 250 | a' | 259 | A' |
| si781-DV32SP | 250 | b' | 259 | D' |
| si781-DV34SP | 250 | b' | 259 | E' |
| si781-DV22S | 250 | c' | 259 | I' |
| si801-DV26SP | 251 | a' | 260 | B' |
| si801-DV27SP | 251 | a' | 260 | C' |
| si801-DV25SP | 251 | a' | 260 | A' |
| si801-DV32SP | 251 | b' | 260 | D' |
| si801-DV34SP | 251 | b' | 260 | E' |
| si801-DV22S | 251 | c' | 260 | I' |

In some embodiments, the siRNA duplex comprises any one oligonucleotide duplex selected from the pairings of a sense strand and an antisense strand shown in Table 5 and Table 12, or a combination of at least two thereof.

In some embodiments, any one or at least two positions selected from positions 2 to 11 from the 5' end of the antisense strand and/or the sense strand are modified with a modifying moiety, wherein the modifying moiety is selected from UNA, GNA, and DNA, wherein the structures of UNA and GNA are as follows: 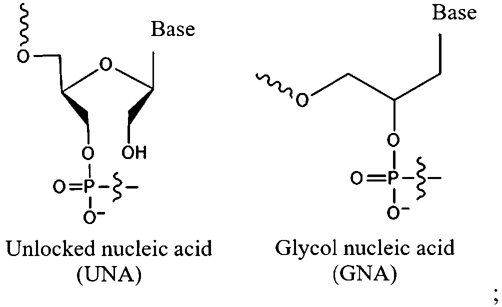 wherein the base is selected from adenine, guanine, cytosine, thymine, and uracil.

In some preferred embodiments, the nucleotide at position 7, or at positions 7 and 11, of the antisense strand is replaced with DNA, while the nucleotide at the position of the sense strand that is complementary to position 7 of the antisense strand is also replaced with DNA.

The present disclosure provides an siRNA duplex, wherein the siRNA duplex comprises any one oligonucleotide duplex selected from the pairings of a sense strand and an antisense strand shown in Table 5, or a combination of at least two thereof.

In some preferred embodiments, the siRNA duplex comprises any one oligonucleotide duplex selected from the following pairings of a sense strand and an antisense strand, or a combination of at least two thereof:
the sense strand has the sequence of SEQ ID NO: 261 or a modified sequence thereof; and the antisense strand has the sequence of SEQ ID NO: 391 or a modified sequence thereof;
the sense strand has the sequence of SEQ ID NO: 262 or a modified sequence thereof; and the antisense strand has the sequence of SEQ ID NO: 392 or a modified sequence thereof;
the sense strand has the sequence of SEQ ID NO: 263 or a modified sequence thereof; and the antisense strand has the sequence of SEQ ID NO: 393 or a modified sequence thereof;
the sense strand has the sequence of SEQ ID NO: 264 or a modified sequence thereof; and the antisense strand has the sequence of SEQ ID NO: 394 or a modified sequence thereof;
the sense strand has the sequence of SEQ ID NO: 265 or a modified sequence thereof; and the antisense strand has the sequence of SEQ ID NO: 395 or a modified sequence thereof;
the sense strand has the sequence of SEQ ID NO: 266 or a modified sequence thereof; and the antisense strand has the sequence of SEQ ID NO: 396 or a modified sequence thereof;
the sense strand has the sequence of SEQ ID NO: 267 or a modified sequence thereof; and the antisense strand has the sequence of SEQ ID NO: 397 or a modified sequence thereof;
the sense strand has the sequence of SEQ ID NO: 268 or a modified sequence thereof; and the antisense strand has the sequence of SEQ ID NO: 398 or a modified sequence thereof;
the sense strand has the sequence of SEQ ID NO: 269 or a modified sequence thereof; and the antisense strand has the sequence of SEQ ID NO: 399 or a modified sequence thereof;
the sense strand has the sequence of SEQ ID NO: 270 or a modified sequence thereof; and the antisense strand has the sequence of SEQ ID NO: 400 or a modified sequence thereof;
the sense strand has the sequence of SEQ ID NO: 271 or a modified sequence thereof; and the antisense strand has the sequence of SEQ ID NO: 401 or a modified sequence thereof;
the sense strand has the sequence of SEQ ID NO: 272 or a modified sequence thereof; and the antisense strand has the sequence of SEQ ID NO: 401 or a modified sequence thereof.

The present disclosure also provides an siRNA duplex, wherein the siRNA duplex comprises any one oligonucleotide duplex selected from the pairings of a sense strand and an antisense strand shown in Table 12, or a combination of at least two thereof.

The present disclosure provides an siRNA conjugate comprising an siRNA duplex described herein and a conjugate moiety linked to the siRNA duplex.

The double-stranded ribonucleic acid or double-stranded ribonucleic acid derivative of the present disclosure may optionally be linked to one or more conjugate moieties. The conjugate moiety may be attached to the sense strand, the antisense strand, or both strands at the 3' end, the 5' end, or both ends. For example, the conjugate moiety is attached to the 3' end or 5' end of the sense strand. In a preferred example, the conjugate moiety is attached to the 3' end of the sense strand. In one example, the conjugate moiety has an arbitrary GalNAc structure.

In some embodiments, the conjugate moiety comprises one or more GalNAc derivatives attached via a bivalent or trivalent branched linker arm.

Typically, the conjugate moiety comprises at least one pharmaceutically acceptable targeting moiety, or further comprises a linker, wherein the siRNA, the linker, and the targeting moiety are sequentially linked. In some embodiments, the number of targeting moieties is 1 to 6. In some embodiments, the number of targeting moieties is 2 to 4. In some embodiments, the number of targeting moieties is 3. The conjugate moiety may be covalently or non-covalently linked to the siRNA molecule, wherein the linkage site may be located at the 3' end or 5' end of the sense strand, at the 5' end of the antisense strand, or within the internal sequence of the siRNA. In some embodiments, the linkage site is located at the 3' end of the sense strand of the siRNA.

In some embodiments, the pharmaceutically acceptable targeting moiety may be a conventional ligand in the field of siRNA delivery, such as various ligands described in WO2009082607A2, the contents of which are incorporated herein by reference in their entirety.

In some embodiments, each ligand is independently selected from a ligand capable of binding to a cell surface receptor. In some embodiments, at least one ligand is a ligand capable of binding to a hepatocyte surface receptor. In some embodiments, at least one ligand is a ligand capable of binding to a mammalian cell surface receptor. In some embodiments, at least one ligand is a ligand capable of binding to a human hepatocyte surface receptor. In some embodiments, at least one ligand is a ligand capable of binding to a hepatocyte surface asialoglycoprotein receptor (ASGPR). The types of these ligands are well known to those skilled in the art, and their general function is to specifically bind to receptors on the surface of target cells, thereby mediating the delivery of siRNA conjugated to the ligand into the target cells.

In some embodiments, the pharmaceutically acceptable targeting moiety in the siRNA conjugate may be galactose or N-acetylgalactosamine, wherein the galactose or N-acetylgalactosamine molecule may be monovalent, divalent, trivalent, or tetravalent. That is, when the siRNA molecule forms an siRNA conjugate with a conjugate moiety containing a galactose or N-acetylgalactosamine molecule as the targeting moiety, the molar ratio of the siRNA molecule to the galactose or N-acetylgalactosamine molecule in the siRNA conjugate is 1:1, 1:2, 1:3, or 1:4. In some embodiments, when the siRNA is conjugated with a conjugate moiety containing N-acetylgalactosamine, the N-acetylgalactosamine molecule is trivalent.

The targeting moiety may be linked to the siRNA molecule via a suitable linker, and those skilled in the art may select an appropriate linker based on the specific type of targeting moiety. The types of these linkers and targeting moieties, as well as the modes of attachment to siRNA may be found in WO2015006740A2, the contents of which are incorporated herein by reference in their entirety.

In some embodiments, the structure of the conjugate moiety is, for example, as follows: 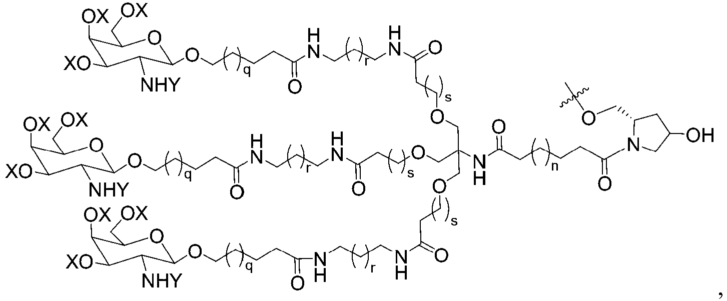 wherein X is a hydroxyl protecting group or H, the hydroxyl protecting group being selected from acetyl, benzoyl, and isobutyryl; Y is an amino protecting group or H, the amino protecting group being selected from formyl, acetyl, propionyl, n-butyryl, and isobutyryl; n is an integer from 0 to 20; q, r, and s are independently integers from 1 to 7.

In some embodiments, the structure of the conjugate moiety is, for example, as follows:

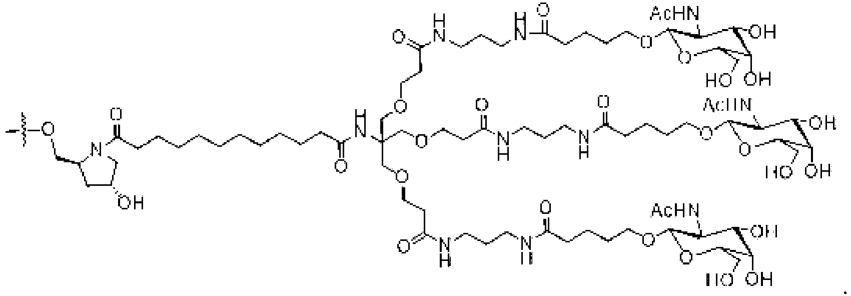

In some embodiments, the conjugate moiety is, for example, as follows: 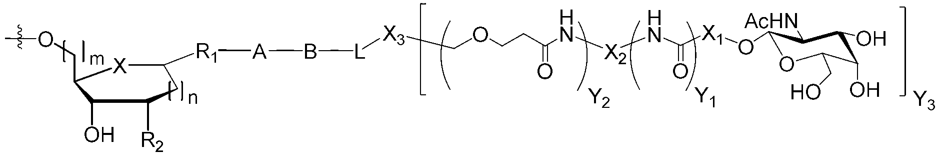
wherein X is oxygen, -N(Y)-, or sulfur;
Y is C₁₋₄ alkyl or C₆₋₁₀ aryl;
R₁ is oxygen or sulfur;
R₂ is hydrogen, -NH₂, C₁₋₄ alkyl, C_{6-1O} aryl, C₁₋₄ alkoxy, or halogen;
A is -(CH₂)ₐ-, -(CH₂CH₂O)_{b}-, -((CH₂)_{c}NHCO)_{d}-, or -((CH₂)_{c}CONH)_{d}-, wherein a is an integer from 1 to 15, b is an integer from 1 to 7, c is an integer from 1 to 7, and d is an integer from 1 to 5;
B is -(CH₂)ₑ-, wherein e is an integer from 0 to 7;
L is -CONH- or -NHCO-;
X₁ is -(CH₂)_{f}- or -(CH₂CH₂O)_{f}CH₂-, wherein f is an integer from 1 to 5;
X₂ is -(CH₂)_{g}-, wherein g is an integer from 1 to 6;
X₃ is oxygen or sulfur;
Y₁ is 0 or 1;
Y₂ is 0, 1, or 2;
when Y₃ is 1, X₄ is CH₂; when Y₃ is 2, X₄ is CH; when Y₃ is 3, X₃ is carbon;
m is an integer from 0 to 4;
n is an integer from 0 to 4.

In some embodiments, the conjugate moiety has, for example, any one of the following structures: 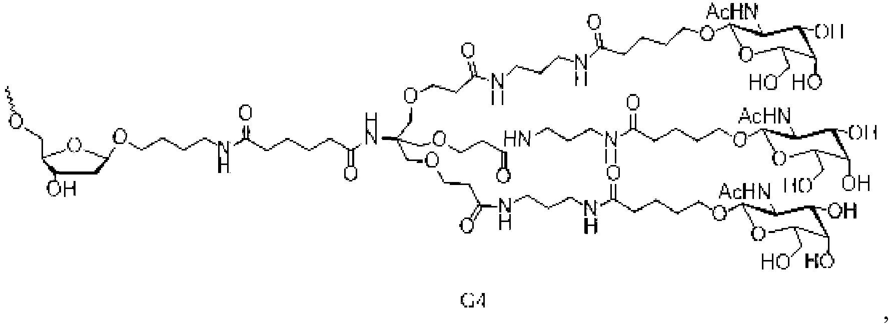 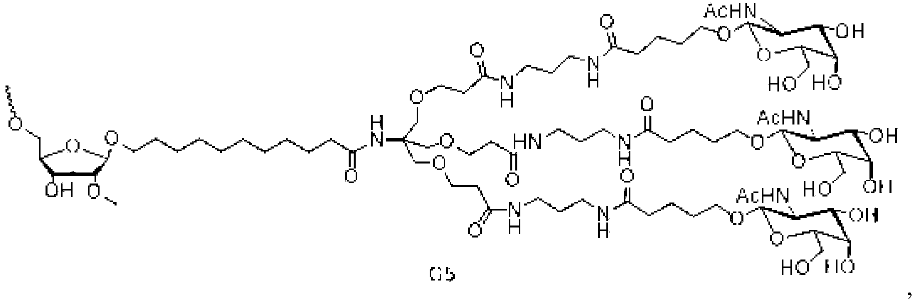 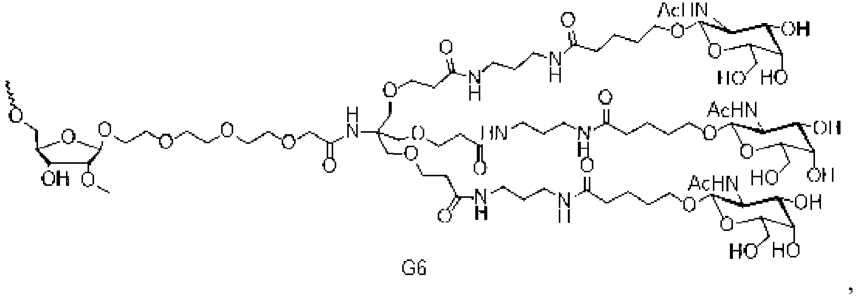 or

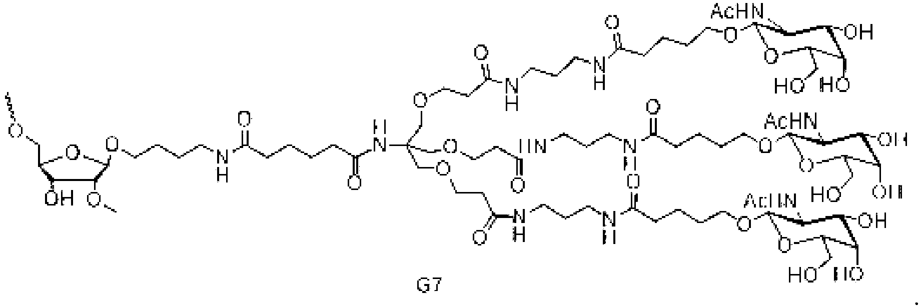

In some embodiments, the siRNA conjugate of the present disclosure may have any one of the following structures: 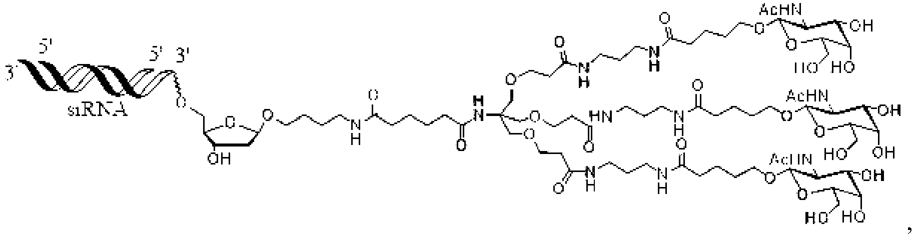 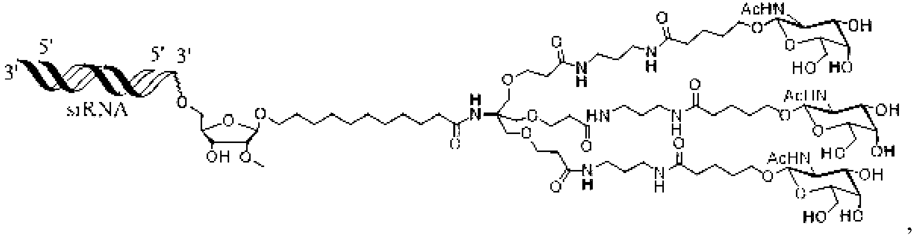 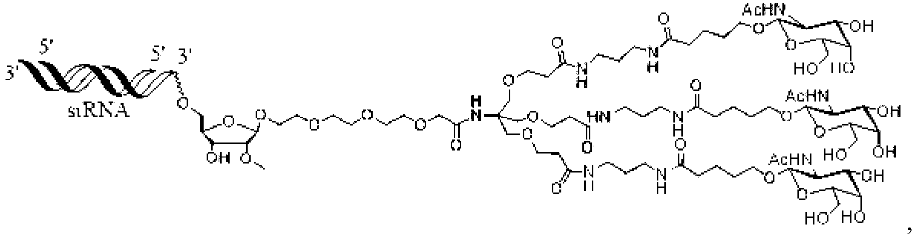 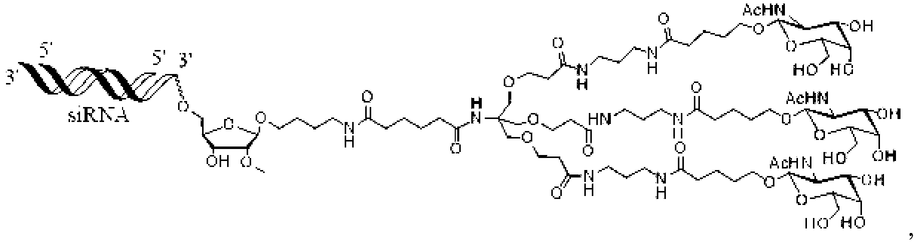 or

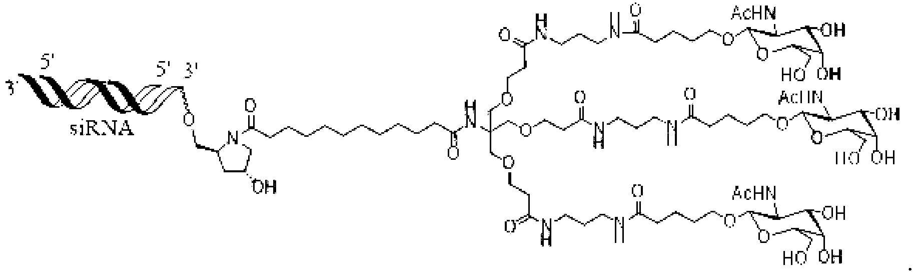

In some embodiments, the conjugate moiety has, for example, the following structure: 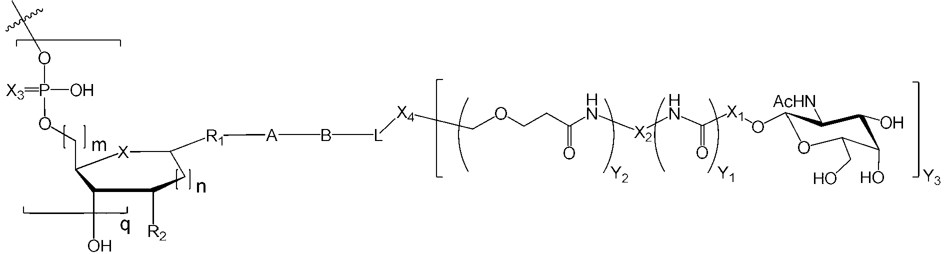
wherein X is oxygen, -N(Y)-, or sulfur;
Y is C₁₋₄ alkyl or C₆₋₁₀ aryl;
R₁ is oxygen or sulfur;
R₂ is hydrogen, -NH₂, C₁₋₄ alkyl, C₆₋₁₀ aryl, C₁₋₄ alkoxy, or halogen;
A is -(CH₂)ₐ-, -(CH₂CH₂O)_{b}-, -((CH₂)_{c}NHCO)_{d}-, or -((CH₂)_{c}CONH)_{d}-, wherein a is an integer from 1 to 15, b is an integer from 1 to 7, c is an integer from 1 to 7, and d is an integer from 1 to 5;
B is -(CH₂)ₑ-, wherein e is an integer from 0 to 7;
L is -CONH- or -NHCO-;
X₁ is -(CH₂)_{f}- or -(CH₂CH₂O)_{f}CH₂-, wherein f is an integer from 1 to 5;
X₂ is -(CH₂)_{g}-, wherein g is an integer from 1 to 6;
X₃ is oxygen or sulfur;
Y₁ is 0 or 1;
Y₂ is 0, 1, or 2;
when Y₃ is 1, X₄ is CH₂; when Y₃ is 2, X₄ is CH; when Y₃ is 3, X₃ is carbon;
m is an integer from 0 to 4;
n is an integer from 0 to 4;
q is an integer from 0 to 4.

In some embodiments, the conjugate moiety has, for example, any one of the following structures: 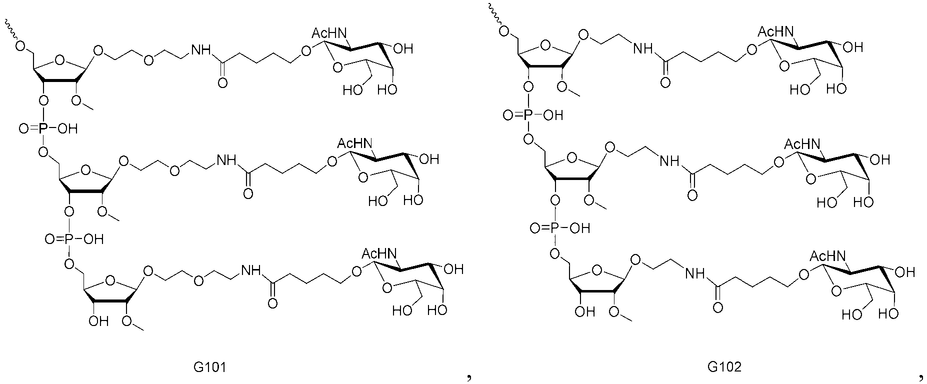 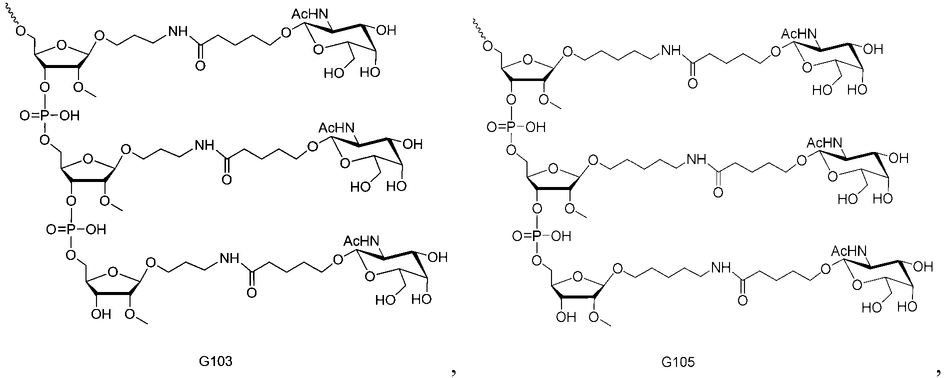 or

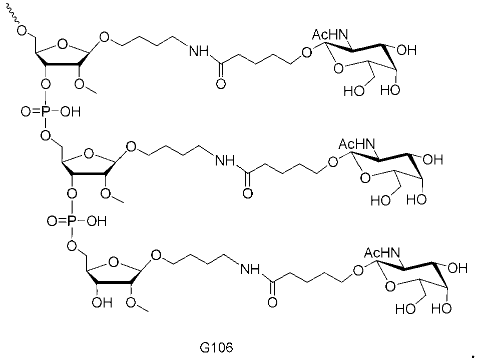

In some embodiments, the siRNA conjugate of the present disclosure has, for example, any one of the following structures: 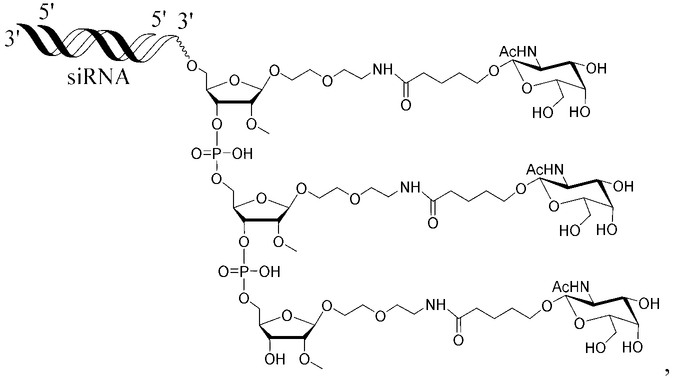 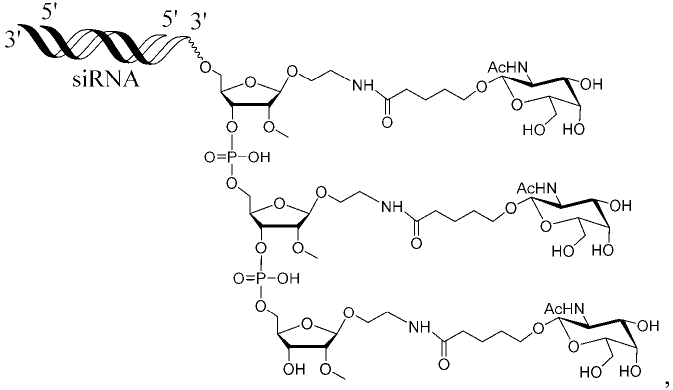 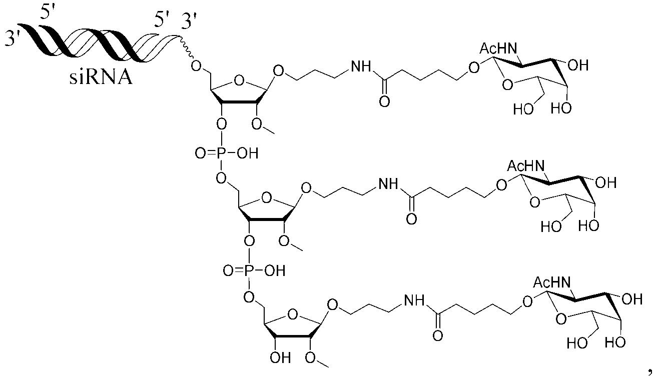 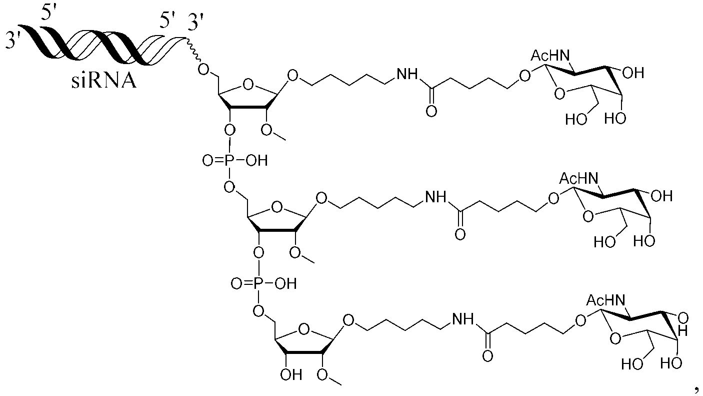 or

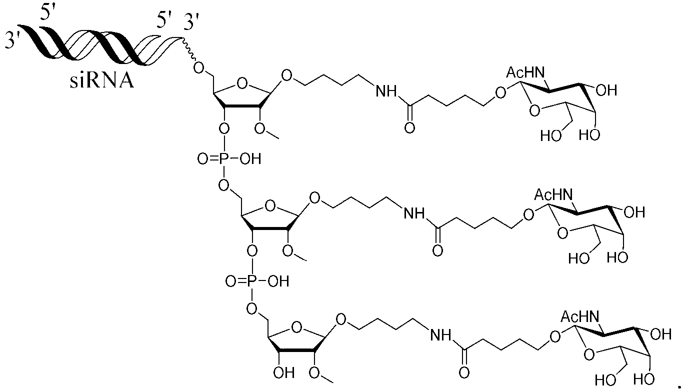

In some embodiments, either or both of the sense and antisense strands of the siRNA duplex may be linked to the conjugate moiety G4, G5, G6, G7, G101, G102, G103, G105, or G106.

In some embodiments, either or both of the sense strand and the antisense strand are linked to the conjugate moiety G5.

In some embodiments, the conjugate moiety is attached to the 3' end of the sense strand.

In some embodiments, the siRNA conjugate comprises any one oligonucleotide duplex selected from the pairings of a sense strand and an antisense strand shown in Table 5, Table 12, and Table 16, or a combination of at least two thereof.

In some preferred embodiments, the siRNA conjugate comprises any one siRNA duplex selected from the pairings of a sense strand and an antisense strand shown in Table 16, or a combination of at least two thereof.

The present disclosure also provides a nucleic acid-protein complex comprising a duplex region of the siRNA duplex or siRNA conjugate described above or an antisense strand of the duplex region, and a nuclease.

In the present disclosure, the term "nucleic acid-protein complex" refers to an RNA-induced silencing complex (RISC) formed by binding of siRNA to Argonaute protein (AGO). The siRNA duplex is then unwound into a sense strand and an antisense strand. The sense strand is degraded, while the antisense strand (guide strand) directs the RISC to bind to a target mRNA homologous to the siRNA via base pairing. The RISC possesses nuclease activity, and the siRNA-guided RISC cleaves the homologous single-stranded mRNA, resulting in loss of mRNA function, *i.e.*, preventing translation into protein and thereby achieving gene "silencing".

The present disclosure also provides a recombinant vector comprising a nucleic acid molecule encoding the siRNA duplex described herein.

In some embodiments, the vector backbone of the recombinant vector is selected from recombinant virus-derived circular RNA vectors, tRNA scaffolds, rRNA scaffolds, and chimeric tRNA/pre-miRNA vectors.

The present disclosure also provides a recombinant cell comprising the siRNA duplex or recombinant vector described above.

In some embodiments, the recombinant cell is selected from *Rhodovulum sulfidophilum* and ribonuclease III-deficient *Corynebacterium glutamicum.*

As used herein, the term "recombinant vector" preferably refers to a vector comprising a regulatory sequence operably linked to a nucleotide sequence encoding the sense strand contained in the nucleic acid molecule of the present disclosure. The term "recombinant cell" refers to a cell into which at least one recombinant vector capable of expressing the nucleic acid molecule or at least one strand of the nucleic acid molecule has been introduced.

The present disclosure also provides a method of preparing the siRNA described herein, comprising culturing the recombinant cell described above and/or obtaining the siRNA by means of chemical synthesis.

The present disclosure also provides a pharmaceutical composition comprising the siRNA duplex or siRNA conjugate described herein, and a pharmaceutically acceptable carrier.

In one example, provided herein is a pharmaceutical composition comprising the siRNA described herein and a pharmaceutically acceptable carrier. The pharmaceutical composition comprising the siRNA duplex or siRNA conjugate may be used for treating a disease or disorder associated with the expression or activity of the INHBE gene, such as metabolic disorders. Such pharmaceutical compositions are formulated based on the mode of delivery. One example is a composition formulated for systemic administration via parenteral delivery, *e.g*., via subcutaneous (s.c.) injection.

The pharmaceutical composition comprising the siRNA duplex or siRNA conjugate described herein may be, for example, a solution with or without a buffer, or a composition comprising a pharmaceutically acceptable carrier. Such compositions include, for example, aqueous or crystalline compositions, liposomal formulations, micellar formulations, emulsions, and gene therapy vectors.

In the method of the present disclosure, the siRNA duplex or siRNA conjugate may be administered in a solution. A free siRNA may be administered in an unbuffered solution, *e.g*., in saline or in water. Alternatively, the free siRNA may also be administered in a suitable buffered solution. The buffered solution may comprise acetate, citrate, prolamin, carbonate, or phosphate, or any combination thereof. In a preferred example, the buffered solution is phosphate-buffered saline (PBS). The pH and osmolarity of the buffered solution comprising the iRNA agent may be adjusted such that it is suitable for administration to a subject.

In some examples, the buffered solution further comprises an agent for controlling the osmolarity of the solution, such that the osmolarity is maintained at a desired value, *e.g.,* at the physiological value of human plasma. Solutes that may be added to the buffered solution to control the osmolarity include, but are not limited to, proteins, peptides, amino acids, non-metabolized polymers, vitamins, ions, sugars, metabolites, organic acids, lipids, or salts. In some examples, the agent for controlling the osmolarity of the solution is a salt. In certain examples, the agent for controlling the osmolarity of the solution is sodium chloride or potassium chloride.

The pharmaceutical composition of the present disclosure may be administered at a dose sufficient to inhibit INHBE gene expression. Generally, suitable doses of the siRNA of the present disclosure range from about 0.001 to about 200.0 mg/kg body weight per day, typically from about 1 to 50 mg/kg body weight per day. For example, the RNAi agent (e.g., siRNA duplex) may be administered at a single dose of about 0.01, 0.05, 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 31, 32, 33, 34, 34, 35, 36, 37, 38, 39, 40, 41, 42, 43, 44, 45, 46, 47, 48, 49, or 50 mg/kg.

The pharmaceutical composition may be administered once daily, or multiple times at various intervals ranging from 1 to 365 days, or the siRNA may be administered as two, three, or more sub-doses at appropriate intervals within a year, or even using continuous infusion or delivery via a controlled-release formulation. In such cases, the amount of siRNA in each sub-dose must be correspondingly reduced in order to achieve the total daily dose. Dosage units may also be compounded for delivery over several days, *e.g*., using a conventional sustained-release formulation that provides sustained release of siRNA over a period of several days. Sustained-release formulations are well known in the art and are particularly useful for delivering agents to specific sites, and thus may be used with the agents of the present disclosure. In such examples, the dosage unit contains a corresponding plurality of daily doses.

In other examples, a single dose of the pharmaceutical composition may be long-acting, such that subsequent doses are administered at intervals of no more than 3, 4, or 5 days, or no more than 1, 2, 3, or 4 weeks. In some examples of the present disclosure, a single dose of the pharmaceutical composition of the present disclosure is administered once weekly. In other examples of the present disclosure, a single dose of the pharmaceutical composition of the present disclosure is administered once every two months.

Those skilled in the art will appreciate that certain factors may influence the dosage and dosing schedule required for effective treatment of a subject, including, but not limited to, the severity of the disease or disorder, prior treatments, the general health and/or age of the subject, and other existing diseases. Furthermore, treating a subject with a therapeutically effective amount of the composition may involve a single treatment or a series of treatments. As described elsewhere herein, the effective dose and *in vivo* half-life for each siRNA encompassed by the present disclosure may be estimated using conventional methods or on the basis of *in vivo* testing using suitable animal models.

The pharmaceutical composition of the present disclosure may be administered in various ways depending upon whether local or systemic treatment is desired and upon the area to be treated. Administration may be topical *(e.g.,* via a transdermal patch); pulmonary; *e.g.,* via inhalation or insufflation of a powder or aerosol, including via a nebulizer; intratracheal; intranasal; epidermal and transdermal; oral; or parenteral. Parenteral administration includes intravenous, intra-arterial, subcutaneous, intraperitoneal, or intramuscular injection or infusion; subdermal administration, *e.g.,* via an implantable device; or intracranial administration, *e.g.,* intraparenchymal, intrathecal, or intraventricular administration.

The siRNA for use in the compositions and methods of the present disclosure may be formulated for delivery in membranous molecular assemblies, such as liposomes or micelles. As used herein, the term "liposome" refers to a vesicle composed of amphipathic lipids arranged in at least one lipid bilayer *(e.g.,* one bilayer or a plurality of bilayers), having an external membrane formed by lipophilic materials and an internal aqueous compartment. The lipophilic material separates the internal aqueous compartment from the external aqueous environment, which typically does not contain the siRNA composition (although in some instances it may). Liposomes are useful for transferring and delivering active ingredients to sites of action. Because liposomal membranes are structurally similar to biological membranes, when liposomes are applied to a tissue, the liposomal bilayer fuses with the bilayer of the cell membrane. As the fusion of liposomes with cells proceeds, the internal aqueous contents, including the siRNA, are delivered into the cell, where the siRNA can specifically bind to a target RNA and mediate RNA interference (RNAi). In some cases, these liposomes are also specifically targeted, *e.g.,* to direct the siRNA to particular cell types.

Liposomes containing RNAi agents may be prepared by a variety of methods. In one example, a lipid component of the liposome is dissolved in a detergent such that micelles are formed with the lipid component. For example, the lipid component may be an amphipathic cationic lipid or a lipid conjugate. The detergent may have a high critical micelle concentration and may be nonionic. Exemplary detergents include cholate, CHAPS, octyl glucoside, deoxycholate, and lauroyl sarcosinate. The RNAi agent is then added to the micelles containing the lipid component. The cationic groups on the lipid interact with the RNAi agent and condense around the RNAi agent to form liposomes. After condensation, the detergent is removed, *e.g*., by dialysis, to obtain a liposomal formulation of the RNAi agent.

The RNAi agent, such as the siRNA duplex described herein, may be fully encapsulated in a lipid formulation (*e.g*., a lipid nanoparticle (LNP) or another nucleic acid-lipid particle).

As used herein, the term "LNP" refers to a stable nucleic acid-lipid particle. The LNP comprises a cationic lipid, a non-cationic lipid, and a lipid that prevents aggregation of the particles (*e.g.*, a PEG-lipid conjugate). LNPs are extremely useful for synthetic applications, as they exhibit a prolonged circulation lifetime following intravenous (i.v.) injection and accumulate at distal sites (*e.g*., sites physically separated from the site of administration).

In one example, the mass ratio of lipid to drug is about 1:1 to about 50:1, about 1:1 to about 25:1, about 3:1 to about 15:1, about 4:1 to about 10:1, about 5:1 to about 9:1, or about 6:1 to about 9:1.

In some preferred embodiments, the lipid nanoparticle comprises a cationic lipid, a neutral lipid, a structured lipid, and a polymer-conjugated lipid.

In some preferred embodiments, the cationic lipid is a compound having the structure of formula (I), or an N-oxide, a solvate, a pharmaceutically acceptable salt, or a stereoisomer thereof, wherein G₁ is C₁₋₆ alkylene; G₂ is C₂₋₈ alkylene; G₃ is C₁₋₃ alkylene; L₁ is C₆₋₁₅ linear alkyl; L₂ is C₁₂₋₂₅ branched alkyl; for example, YK-009 having the structure of formula (I-I) (see patent CN114044741B).

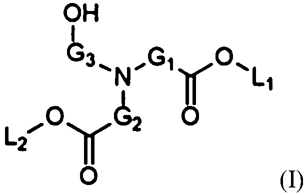

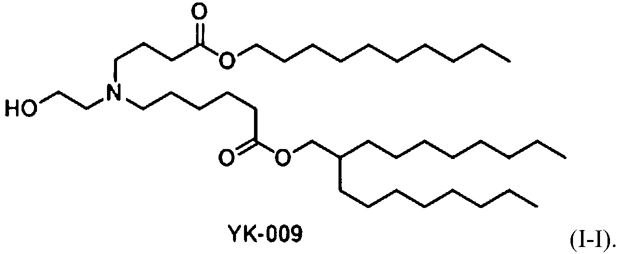

In some preferred embodiments, the cationic lipid is a compound having the structure of formula (II), or an N-oxide, a solvate, a pharmaceutically acceptable salt, or a stereoisomer thereof, wherein G₁ is C₂₋₈ alkylene; G₂ is C₂₋₈ alkylene; L₁ is -C(O)O- or -OC(O)-; L₂ is -C(O)O- or - OC(O)-; R₁ is C₆₋₂₅ linear or branched alkyl; R₂ is C₆₋₂₅ linear or branched alkyl; G₃ is HO(CH₂)₂- or HO(CH₂)₃-; G₄ is HO(CH₂)₂- or HO(CH₂)₃-; L is (CH₂)₂-, -(CH₂)₃-, or -(CH₂)₄-; for example, YK-401 having the structure of formula (II-I), YK-402 having the structure of formula (II-II), or YK-407 having the structure of formula (II-III) (see patent CN115784921B, the contents of which are incorporated herein by reference in their entirety, particularly including the general formulas and specific compounds described therein).

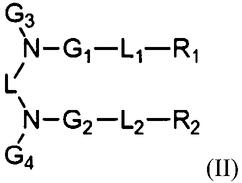

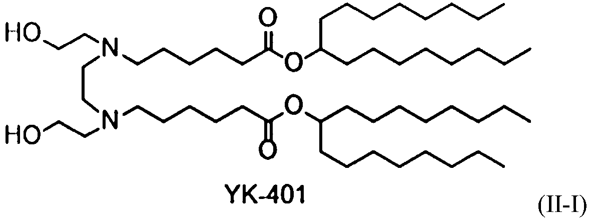

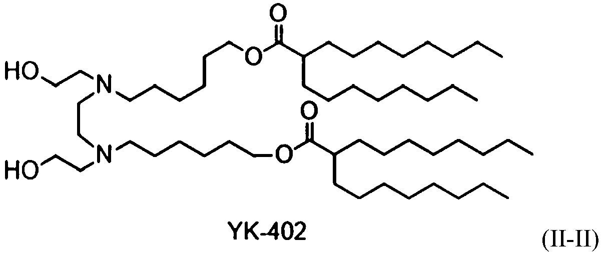

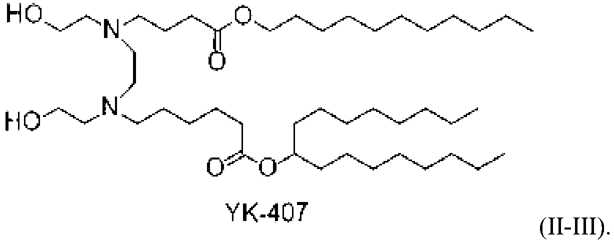

In some preferred embodiments, the cationic lipid is a compound having the structure of formula (III), or an N-oxide, a solvate, a pharmaceutically acceptable salt, or a stereoisomer thereof, wherein G₁ is C₁₋₆ alkylene; G₂ is C₂₋₈ alkylene; R₁ is C₆₋₂₀ linear or branched alkyl; R₂ is C₁₂₋₂₅ branched alkyl; G₃ is HO(CH₂)₂N(CH₃)(CH₂)₂-, HO(CH₂)₂N(CH₂CH₃)(CH₂)₂-, (HO(CH₂)₂)₂N(CH₂)₂-, CH₃O(CH₂)₂N(CH₃)(CH₂)₂-, (CH₃)₂N(CH₂)₃SC(O)O(CH₂)₂-, (CH₃)₂N(CH₂)₃SC(O)-, CH₃NH(CH₂)₂N(CH₃)(CH₂)₂-, or CH₃CH₂NH(CH₂)₂-; for example, YK-201 having the structure of formula (III-I) or YK-202 having the structure of formula (III-II) (see patent CN115677518B, the contents of which are incorporated herein by reference in their entirety, particularly including the general formulas and specific compounds described therein).

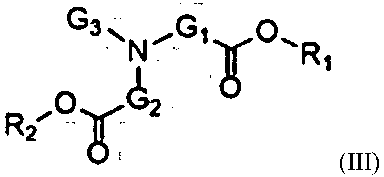

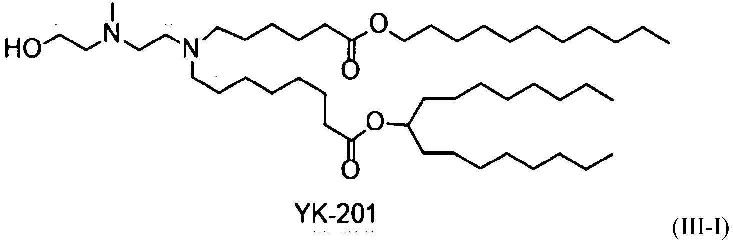

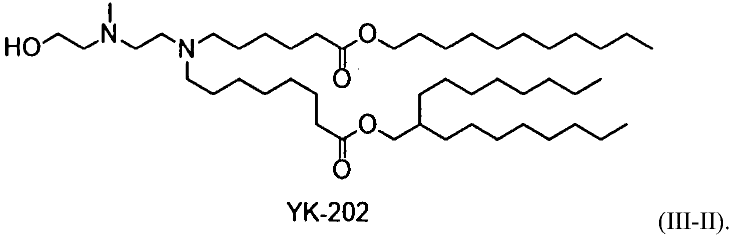

In some preferred embodiments, the cationic lipid is a compound having the structure of formula (IV), or an N-oxide, a solvate, a pharmaceutically acceptable salt, or a stereoisomer thereof, wherein G₁ is C₁₋₈ alkylene; G₂ is C₂₋₈ alkylene; R₁ is C₆₋₂₅ linear or branched alkyl; R₂ is C₁₂₋₂₅ linear or branched alkyl; G₃ is HO(CH₂)₂N(R₃)CH₂CH(OH)CH₂-, wherein R₃ is -CH₃, - CH₂CH₃, or -CH₂CH₂OH; for example, YK-305 having the structure of formula (IV-I) or YK-310 having the structure of formula (IV-II) (see patent CN115745820B, the contents of which are incorporated herein by reference in their entirety, particularly including the general formulas and specific compounds described therein).

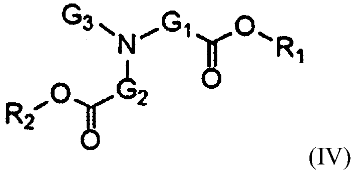

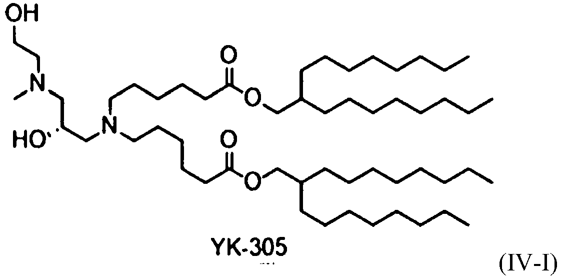

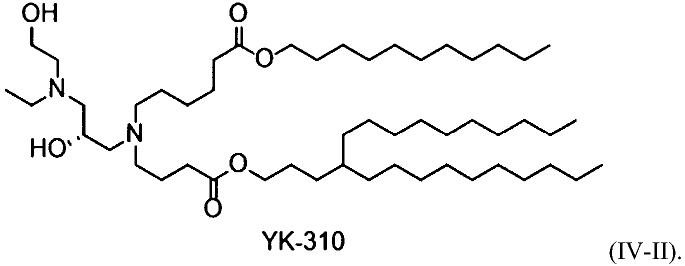

In some preferred embodiments, the cationic lipid is a compound having the structure of formula (V), or an N-oxide, a solvate, a pharmaceutically acceptable salt, or a stereoisomer thereof, wherein G¹ and G² are each independently unsubstituted C₆-C₁₀ alkylene; G³ is unsubstituted C₁-C₁₂ alkylene; R¹ and R² are each independently C₆-C₂₄ alkyl or C₆-C₂₄ alkenyl; R³ is OR⁵, N, - C(=O)OR⁴, -OC(=O)R⁴, or -NR⁵C(=O)R⁴; R⁴ is C₁-C₁₂ hydrocarbyl; and R⁵ is H or C₁-C₆ hydrocarbyl; for example, ALC0315 having the structure of formula (V-I) (see patent CN108368028B, the contents of which are incorporated herein by reference in their entirety, particularly including the general formulas and specific compounds described therein).

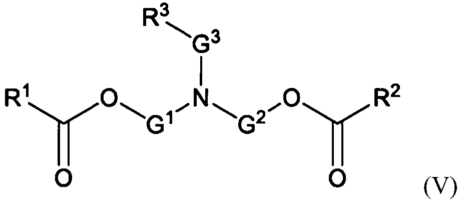

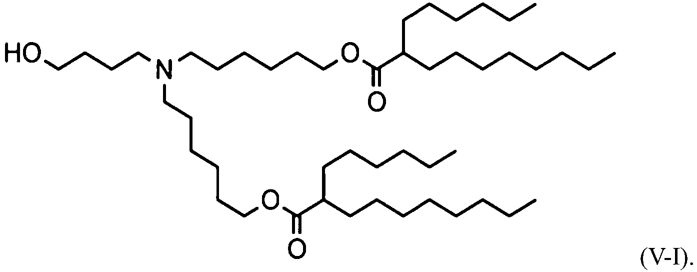

In some preferred embodiments, the cationic lipid is a compound having the structure of formula (VI), or an N-oxide, a solvate, a pharmaceutically acceptable salt, or a stereoisomer thereof, wherein R₄ is selected from -(CH₂)ₙQ and -(CH₂)ₙCHQR; Q is selected from the group consisting of -OR, -OH, -O(CH₂)ₙN(R)₂, -OC(O)R, -CX₃, -CN, -N(R)C(O)R, -N(H)C(O)R, - N(R)S(O)₂R, -N(H)S(O)₂R, -N(R)C(O)N(R)₂, -N(H)C(O)N(R)₂, -N(H)C(O)N(H)(R), - N(R)C(S)N(R)₂, -N(H)C(S)N(R)₂, -N(H)C(S)N(H)(R), -N(R)S(O)₂R₈, and heterocycle; n is 1, 2, or 3; for example, SM102 having the structure of formula (VI-I) (see patent CN110520409A, the contents of which are incorporated herein by reference in their entirety, particularly including the general formulas and specific compounds described therein).

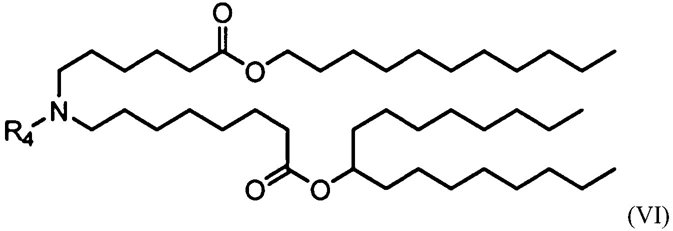

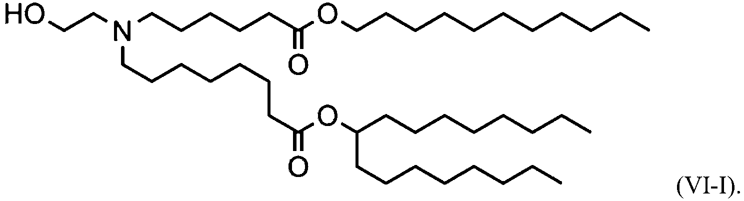

In some preferred embodiments, the cationic lipid is a compound having the structure of formula (VII), or an N-oxide, a solvate, a pharmaceutically acceptable salt, or a stereoisomer thereof (see patent CN102625696B, DLIN-MC3-DMA, the contents of which are incorporated herein by reference in their entirety, particularly including the general formulas and specific compounds described therein).

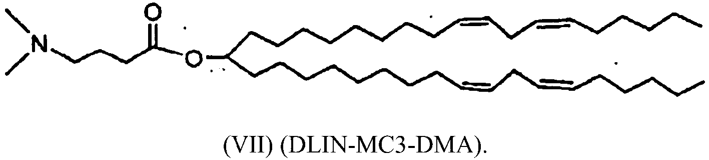

In some more preferred embodiments, the cationic lipid is any one selected from the group consisting of YK-009, YK-401, YK-305, ALC0315, SM102, and DLIN-MC3-DMA, or a combination of at least two thereof.

In some preferred embodiments, the molar ratio of the cationic lipid to the neutral lipid is 1:1 to 10:1.

In some preferred embodiments, the molar ratio of the cationic lipid to the structured lipid is 1:1 to 5:1.

In some preferred embodiments, the molar ratio of the cationic lipid, the neutral lipid, the structured lipid, and the polymer-conjugated lipid is (25 to 65):(5 to 25):(25 to 70):(0.5 to 5).

In some preferred embodiments, the molar ratio of the cationic lipid, the neutral lipid, the structured lipid, and the polymer-conjugated lipid is (25 to 65):(5 to 25):(25 to 45):(0.5 to 5).

In some more preferred embodiments, the molar ratio of the cationic lipid, the neutral lipid, the structured lipid, and the polymer-conjugated lipid is 50:10:38.5:1.5 or 49:10:39.5:1.5.

In some preferred embodiments, the neutral lipid is any one selected from the group consisting of phosphatidylcholine, phosphatidylethanolamine, sphingomyelin, ceramide, sterols, and derivatives thereof, or a combination of at least two thereof.

In some more preferred embodiments, the neutral lipid is any one selected from the group consisting of 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanolamine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), dipalmitoyl phosphatidylglycerol (DPPG), palmitoyl oleoyl phosphatidylethanolamine (POPE), distearoyl-phosphatidyl-ethanolamine (DSPE), dipalmitoyl phosphatidylethanolamine (DPPE), dimyristoyl phosphoethanolamine (DMPE), 1-stearoyl-2-oleoyl-stearoylethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyl oleoyl phosphatidylcholine, lysophosphatidylcholine, lysophosphatidylethanolamine (LPE), and mixtures thereof, or a combination of at least two thereof.

In some more preferred embodiments, the neutral lipid is DOPE and/or DSPC.

In some preferred embodiments, the structured lipid is any one selected from the group consisting of cholesterol, nonsterols, sitosterol, ergosterol, campesterol, stigmasterol, brassinosterol, tomatidine, ursolic acid, α-tocopherol, and corticosteroids, or a combination of at least two thereof.

In some more preferred embodiments, the structured lipid is cholesterol.

In some preferred embodiments, the polymer-conjugated lipid is any one selected from the group consisting of PEG-modified phosphatidylethanolamine, PEG-modified phosphatidic acid, PEG-modified ceramide, PEG-modified dialkylamine, PEG-modified diacylglycerol, and PEG-modified dialkylglycerol, or a combination of at least two thereof.

In some more preferred embodiments, the polymer-conjugated lipid is any one selected from the group consisting of distearoyl phosphatidylethanolamine polyethylene glycol 2000 (DSPE-PEG2000), dimyristoylglycero-3-methoxypolyethylene glycol 2000 (DMG-PEG2000), and methoxypolyethylene glycol ditetradecylacetamide (ALC-0159), or a combination of at least two thereof.

Examples of pharmaceutical compositions of the present disclosure include, but are not limited to, aqueous formulations, emulsion formulations, and liposome-containing formulations. These compositions may be derived from a variety of components, examples of which include, but are not limited to, preformed liquids, self-emulsifying solids, and self-emulsifying semisolids. For example, formulations targeting the liver are preferred when treating hepatic disorders (*e.g*., hepatocellular carcinoma).

The pharmaceutical formulations of the present disclosure (which may conveniently be presented in unit dosage forms) may be prepared using conventional techniques well known in the pharmaceutical industry. Such techniques include the step of bringing into association the active ingredients with the pharmaceutical carrier(s) or excipient(s). Generally, the formulations are prepared by uniformly and intimately bringing into association the active ingredients with a liquid carrier or a finely divided solid carrier or both, and then, if necessary, shaping the product.

The compositions of the present disclosure may be formulated into any of a variety of possible dosage forms, including, but not limited to, tablets, capsules, gelatin capsules, liquid syrups, soft capsules, suppositories, and enemas. The compositions of the present disclosure may also be formulated as suspensions in aqueous, non-aqueous, or mixed media. Aqueous suspensions may further comprise substances that increase the viscosity of the suspension, including, for example, sodium carboxymethylcellulose, sorbitol, and/or dextran. The suspension may also comprise a stabilizer.

Certain compositions of the present disclosure further incorporate a carrier compound into the formulation. As used herein, the term "carrier compound" or "carrier" may refer to a nucleic acid or analog thereof that is inert (*i.e*., lacks biological activity per se) but is recognized as a nucleic acid during *in vivo* processes that reduce the bioavailability of a biologically active nucleic acid, for example by degrading the biologically active nucleic acid or facilitating its removal from circulation. Co-administration of a nucleic acid and a carrier compound (typically the latter in excess) may result in a substantial reduction in the amount of nucleic acid recovered in the liver, kidney, or other extracirculatory reservoirs, presumably due to competition between the carrier compound and the nucleic acid for common receptors. For example, when co-administered with polyinosinic acid, dextran sulfate, polycytidylic acid, or 4-acetamido-4'-isothiocyano-stilbene-2,2'-disulfonic acid, the recovery of partially phosphorothioated dsRNA in hepatic tissue may be reduced (Miyao et al., DsRNA Res. Dev., 1995, 5:115-121; Takakura et al., DsRNA & Nucl. Acid Drug Dev., 1996, 6:177-183).

In contrast to carrier compounds, a "pharmaceutical carrier" or "excipient" refers to a pharmaceutically acceptable solvent, suspending agent, or any other pharmaceutically inert vehicle for delivering one or more nucleic acids to an animal. The excipient may be liquid or solid and is selected with reference to the intended mode of administration in order to provide the desired bulk, consistency, and other properties, when combined with the nucleic acid and other components of a particular pharmaceutical composition Typical pharmaceutical carriers include, but are not limited to, binders (*e.g*., pregelatinized corn starch, polyvinylpyrrolidone, or hydroxypropyl methylcellulose); fillers (*e.g.,* lactose and other sugars, microcrystalline cellulose, pectin, gelatin, calcium sulfate, ethyl cellulose, polyacrylates, or calcium hydrogen phosphate); lubricants (*e.g*., magnesium stearate, talc, silica, colloidal silicon dioxide, stearic acid, metallic stearate, hydrogenated vegetable oil, corn starch, polyethylene glycol, sodium benzoate, or sodium acetate); disintegrants (*e.g.,* starch or sodium starch glycolate); and wetting agents (*e.g.,* sodium lauryl sulfate).

Pharmaceutically acceptable organic or inorganic excipients suitable for non-parenteral administration that do not deleteriously react with nucleic acids may also be used to formulate the compositions of the present disclosure. Suitable pharmaceutically acceptable carriers include, but are not limited to, water, saline solutions, alcohols, polyethylene glycol, gelatin, lactose, amylose, magnesium stearate, talc, silicic acid, viscous paraffin, hydroxymethyl cellulose, and polyvinylpyrrolidone.

Formulations suitable for topical administration of nucleic acids may include sterile or non-sterile aqueous solutions or non-aqueous solutions in common solvents such as alcohols, or nucleic acid solutions in liquid or solid oil bases. These solutions may further include buffers, diluents, and other suitable additives. Pharmaceutically acceptable organic or inorganic excipients suitable for non-parenteral administration that do not deleteriously react with nucleic acids may be used.

Suitable pharmaceutically acceptable excipients include, but are not limited to, water, saline solutions, alcohols, polyethylene glycol, gelatin, lactose, amylose, magnesium stearate, talc, silicic acid, viscous paraffin, hydroxymethyl cellulose, and polyvinylpyrrolidone.

The present disclosure also provides methods of treating and/or preventing diseases and/or disorders that can be modulated by downregulating INHBE gene expression. The diseases and/or disorders include, but are not limited to, type 2 diabetes; hyperlipidemia or dyslipidemia (elevated or altered circulating levels of low-density lipoprotein cholesterol (LDL-C), triglycerides, very low-density lipoprotein cholesterol (VLDL-C), apolipoprotein B, or other lipid components); obesity (particularly abdominal obesity); lipodystrophy (*e.g*., inability to deposit fat locally in adipose depots (partial lipodystrophy) or inability to deposit fat systemically (lipoatrophy)); insulin resistance or elevated or altered insulin levels during fasting or metabolic challenge; hepatic fat deposition or fatty liver disease and complications thereof (*e.g*., cirrhosis, fibrosis, or liver inflammation); non-alcoholic steatohepatitis; other types of liver inflammation; elevated or altered liver enzyme levels or liver injury; elevated or altered biomarkers of inflammation or fat deposition in the liver; elevated blood pressure and/or hypertension; elevated blood glucose or hyperglycemia; metabolic syndrome; coronary artery disease and other atherosclerotic conditions; as well as complications associated with each of the aforementioned conditions; and any other diseases associated with elevated INHBE levels, including yet unidentified related disorders, pathologies, or syndromes.

The siRNA of the present disclosure may be administered to a subject using any route of administration known in the art, including, but not limited to, subcutaneous, intravenous, intramuscular, intraocular, intrabronchial, intrapleural, intraperitoneal, intra-arterial, lymphatic, or cerebrospinal administration, and any combination thereof. In a preferred example, the agents are administered subcutaneously.

In another example, the siRNA is administered in combination with an additional therapeutic agent. The siRNA and the additional therapeutic agent may be co-administered in the same composition, *e.g*., parenterally, or the additional therapeutic agent may be administered as part of a separate composition or by another method described herein.

Examples of additional therapeutic agents include agents known for treating INHBE-associated diseases or disorders. For example, additional therapeutic agents include: administering to a subject one or more siRNAs of the present disclosure; administering to a subject a non-INHBE RNAi therapeutic agent; and implementing behavioral modifications in a subject. In some embodiments, the non-INHBE RNAi therapeutic agent is any one selected from the group consisting of insulin, glucagon-like peptide-1 agonists, sulfonylureas, meglitinides, biguanides, thiazolidinediones, α-glucosidase inhibitors, SGLT2 inhibitors, DPP-4 inhibitors, HMG-CoA reductase inhibitors, and statins, or a combination of at least two thereof.

In one example, the iRNA agent is administered to a patient, followed by administration of the additional therapeutic agent (or vice versa). In another example, the iRNA agent and the additional therapeutic agent are administered simultaneously.

In another aspect, the present disclosure provides a kit comprising kit A, wherein the kit A comprises one or more of the siRNA duplex, siRNA conjugate, nucleic acid-protein complex, or pharmaceutical composition described above.

In some embodiments, the kit further comprises kit B, wherein the kit B comprises one or more of the following:
(1) other agents that reduce INHBE gene expression or compositions comprising the agents that reduce INHBE gene expression;
(2) one or more selected from the group consisting of insulin, glucagon-like peptide-1 agonists, sulfonylureas, meglitinides, biguanides, thiazolidinediones, α-glucosidase inhibitors, SGLT2 inhibitors, DPP-4 inhibitors, HMG-CoA reductase inhibitors, and statins.

As used herein, "other agents that reduce INHBE gene expression or compositions comprising the agents that reduce INHBE gene expression" refer to agents that do not contain the siRNA duplex, siRNA conjugate, or pharmaceutical composition provided by the present disclosure.

The present disclosure also provides the use of the siRNA duplex, siRNA conjugate, recombinant vector, recombinant cell, or pharmaceutical composition described above in the manufacture of a medicament for preventing and/or treating a disease associated with INHBE gene expression.

In some embodiments, the disease associated with INHBE gene expression is selected from the following diseases: type 2 diabetes; hyperlipidemia or dyslipidemia (elevated or altered circulating levels of low-density lipoprotein cholesterol (LDL-C), triglycerides, very low-density lipoprotein cholesterol (VLDL-C), apolipoprotein B, or other lipid components); obesity (particularly abdominal obesity); lipodystrophy (*e.g*., inability to deposit fat locally in adipose depots (partial lipodystrophy) or inability to deposit fat systemically (lipoatrophy)); insulin resistance or elevated or altered insulin levels during fasting or metabolic challenge; hepatic fat deposition or fatty liver disease and complications thereof (*e.g*., cirrhosis, fibrosis, or liver inflammation); non-alcoholic steatohepatitis; other types of liver inflammation; elevated or altered liver enzyme levels or liver injury; elevated or altered biomarkers of inflammation or fat deposition in the liver; elevated blood pressure and/or hypertension; elevated blood glucose or hyperglycemia; metabolic syndrome; coronary artery disease and other atherosclerotic conditions; as well as complications associated with each of the aforementioned conditions.

The present disclosure also provides a method of reducing INHBE gene expression or inhibiting INHBE replication for non-prophylactic and/or non-therapeutic purposes, comprising administering one or more of the siRNA, conjugate, pharmaceutical composition, and kit described above to a sample.

As used herein, "non-prophylactic and/or non-therapeutic purposes" refers to reducing INHBE gene expression or inhibiting INHBE replication for research purposes, for example, in a laboratory setting.

The present disclosure also provides the siRNA, conjugate, pharmaceutical composition, and/or kit described herein for use in the prevention and/or treatment of a disease associated with INHBE gene expression.

The present disclosure also provides methods of treating and/or preventing diseases and disorders that can be modulated by downregulating INHBE gene expression, such as metabolic disorders related to body fat distribution, including, but not limited to, type 2 diabetes; hyperlipidemia or dyslipidemia (elevated or altered circulating levels of low-density lipoprotein cholesterol (LDL-C), triglycerides, very low-density lipoprotein cholesterol (VLDL-C), apolipoprotein B, or other lipid components); obesity (particularly abdominal obesity); lipodystrophy (*e.g*., inability to deposit fat locally in adipose depots (partial lipodystrophy) or inability to deposit fat systemically (lipoatrophy)); insulin resistance or elevated or altered insulin levels during fasting or metabolic challenge; hepatic fat deposition or fatty liver disease and complications thereof (*e.g*., cirrhosis, fibrosis, or liver inflammation); non-alcoholic steatohepatitis; other types of liver inflammation; elevated or altered liver enzyme levels or liver injury; elevated or altered biomarkers of inflammation or fat deposition in the liver; elevated blood pressure and/or hypertension; elevated blood glucose or hyperglycemia; metabolic syndrome; coronary artery disease and other atherosclerotic conditions; as well as complications associated with each of the aforementioned conditions.

In some embodiments, the method comprises administering to a subject in need thereof a therapeutically and/or prophylactically effective amount of the siRNA, conjugate, and/or pharmaceutical composition described herein, or administering the kit described herein to the subject in need thereof.

The RNAi agent of the present disclosure may be administered to a subject using any route of administration known in the art, including, but not limited to, subcutaneous, intravenous, intramuscular, intraocular, intrabronchial, intrapleural, intraperitoneal, intra-arterial, lymphatic, or cerebrospinal administration, and any combination thereof. In a preferred example, the agents are administered subcutaneously.

In another example, the siRNA is administered in combination with an additional therapeutic agent. The siRNA and the additional therapeutic agent may be co-administered in the same composition, *e.g.*, parenterally, or the additional therapeutic agent may be administered as part of a separate composition or by another method described herein.

Examples of additional therapeutic agents include agents known for treating metabolic disorders or cardiovascular and cerebrovascular diseases, including, but not limited to, insulin, glucagon-like peptide-1 agonists (*e.g*., exenatide, liraglutide, dulaglutide, semaglutide, and pramlintide), sulfonylureas (*e.g.,* chlorpropamide, glipizide), meglitinides (*e.g.,* repaglinide, nateglinide), biguanides (*e.g.,* metformin), thiazolidinediones (*e.g.,* rosiglitazone, troglitazone), α-glucosidase inhibitors (*e.g.,* acarbose and miglitol), SGLT2 inhibitors (*e.g.,* dapagliflozin), DPP-4 inhibitors (*e.g*., linagliptin), or HMG-CoA reductase inhibitors, such as statins, including atorvastatin (Lipitor), fluvastatin (Lescol), lovastatin (Mevacor), lovastatin extended-release (Altoprev), pitavastatin (Livalo), pravastatin (Pravachol), rosuvastatin (Crestor), and simvastatin (Zocor).

In one example, an siRNA agent is administered to a patient, followed by administration of the additional therapeutic agent (or vice versa). In another example, the siRNA agent and the additional therapeutic agent are administered simultaneously.

The present disclosure is further illustrated below by way of examples, but is not thereby limited to the scope of the examples provided. In the following examples, experimental methods without specified conditions are selected according to conventional methods and conditions, or according to product instructions.

Unless otherwise specified, the reagents and raw materials used in the present disclosure are commercially available.

The abbreviations for nucleotides used herein are as follows:
A = adenosine-3'-phosphate
Am = 2'-O-methyladenosine-3'-phosphate
Ams = 2'-O-methyladenosine-3'-phosphorothioate
Af = 2'-fluoroadenosine-3'-phosphate
Afs = 2'-fluoroadenosine-3'-phosphorothioate
G = guanosine-3'-phosphate
Gm = 2'-O-methylguanosine-3'-phosphate
Gms = 2'-O-methylguanosine-3'-phosphorothioate
Gf = 2'-fluoroguanosine-3'-phosphate
Gfs = 2'-fluoroguanosine-3'-phosphorothioate
C = cytidine-3'-phosphate
Cm = 2'-O-methylcytidine-3'-phosphate
Cms = 2'-O-methylcytidine-3'-phosphorothioate
Cf = 2'-fluorocytidine-3'-phosphate
Cfs = 2'-fluorocytidine-3'-phosphorothioate
U = uridine-3'-phosphate
Um = 2'-O-methyluridine-3'-phosphate
Ums = 2'-O-methyluridine-3'-phosphorothioate
Uf = 2'-fluorouridine-3'-phosphate
Ufs = 2'-fluorouridine-3'-phosphorothioate
AmsEVP = 5'-vinyl-(E)-phosphonate-2'-O-methyladenosine-3'-phosphorothioate
UmsEVP = 5'-vinyl-(E)-phosphonate-2'-O-methyluridine-3'-phosphorothioate
Agna = adenosine-glycol nucleic acid
Cgna = cytidine-glycol nucleic acid
Ggna = guanosine-glycol nucleic acid
Tgna = thymidine-glycol nucleic acid
Ugna = uridine-glycol nucleic acid

The coding rules for siRNA motifs (or motifs), siRNA derivatives, and siRNA conjugates used herein are illustrated as follows:
The siRNA motif is an unmodified siRNA.

Sequences with alternating 2'-O-methyl (2'-OMe) and 2'-fluoro (2'-F) modifications are designated by adding the suffix "-AL" after the motif code. For example, the codes for alternately modified derivatives are si075-AL, si751-AL, and the like.

For template-modified siRNA derivatives, the modification templates disclosed herein, such as DV25P to DV29P, DV32P to DV34P, DV41P to DV43P, and DV25SP to DV43SP, are employed. The coding rule involves adding the name of the corresponding modification template for the siRNA derivative after the motif code. For example, for the motif si751, a derivative obtained using the template DV25P is designated as si751-DV25P.

For example, for siRNA conjugates, the name of the conjugate moiety, such as "G103" or "G101", is appended to the end of the code for the corresponding derivative. For example, when the derivative si751-DV25P is conjugated with the conjugate moiety G101, the corresponding code is si751-DV25PG101. Some conjugates, in addition to template modifications, may further incorporate anti-off-target modifications (*e.g*., d7B or d7B11). The coding rule for such conjugates incorporating both template modifications and anti-off-target modifications involves sequentially adding the names of the corresponding template and anti-off-target modification after the motif code, followed by appending the name of the conjugate moiety, such as "G103" or "G101", at the end.

Examples of codes for siRNA duplexes (motifs, derivatives, and conjugates) in the present disclosure

| Type of siRNA duplex | siRNA motif | Template-modified siRNA derivative | Template-modified and anti-off-target modified siRNA derivative | Template-modified and anti-off-target modified siRNA conjugate |
|---|---|---|---|---|
| Examples of codes | si079 | si079-DV29SP | si079-DV34SPd7B | si079-DV34SPd7BG101 |

### Examples

In the following examples, for experimental data used in comparison among groups, *P <* 0.05 is considered to indicate a statistically significant difference.

Additional objectives, features, and advantages of the present disclosure will become apparent from the following detailed description. However, it should be understood that the detailed description and specific examples, although indicating specific embodiments of the present disclosure, are provided for illustrative purposes only, since various changes and modifications made within the spirit and scope of the present disclosure will become apparent to those skilled in the art upon reading the detailed description.

Unless otherwise specified, the experimental techniques and methods used in the following examples are conventional methods. For example, experimental methods for which specific conditions are not indicated in the following examples are typically performed under conventional conditions, such as those described in Sambrook *et al.*, *Molecular Cloning: A Laboratory Manual* (New York: Cold Spring Harbor Laboratory Press, 1989), or according to the conditions recommended by the respective manufacturers. Unless otherwise specified, the materials and reagents used in the examples are commercially available.

Those skilled in the art will appreciate that in various examples of the present disclosure, when the test substance in an experiment is an siRNA conjugate, the experimental data and results (including, but not limited to, inhibition rate, IC₅₀, and IC₄₀) can accordingly reflect the inhibition rate, IC₅₀, IC₄₀, and other parameters of the corresponding siRNA derivative of the siRNA conjugate. There is no obstacle to understanding for those skilled in the art.

### Example 1: Inhibition of INHBE gene expression by siRNA motifs

In this example, siRNA motifs listed in Table 1 were designed and synthesized based on the INHBE mRNA sequence (NM_031479.5). These siRNA motifs were transfected into Huh7 cells via lipid nanoparticles (LNPs). The inhibitory effects of the siRNA motifs on INHBE gene expression were evaluated by qPCR, thereby identifying siRNA motifs with favorable inhibitory activity.

### 1. Experimental materials

Test samples: siRNA motifs listed in Table 1.

Cell line: hepatocellular carcinoma cell line, Huh7 cells (Guangzhou Darui Biotechnology, DR-Cell-01).

Solvents: sterile nuclease-free water, Gibco Opti-MEM.

### 2. Synthesis of siRNA motifs

Instruments and reagents: 192 P DNA/RNA automatic synthesizer (Tsingke), using a universal solid-phase support consisting of cross-linked polystyrene beads; model: Primer Support 5G Unylinker 350 (manufactured by Cytiva).

### Preparation method:

Nucleotide monomers were dissolved in acetonitrile at a concentration of 0.15 M, including DMT-A-2'-O-TBDMS phosphoramidite monomer (Formula 1), DMT-C-2'-O-TBDMS phosphoramidite monomer (Formula 2), DMT-G-2'-O-TBDMS phosphoramidite monomer (Formula 3), and DMT-U-2'-O-TBDMS phosphoramidite monomer (Formula 4).

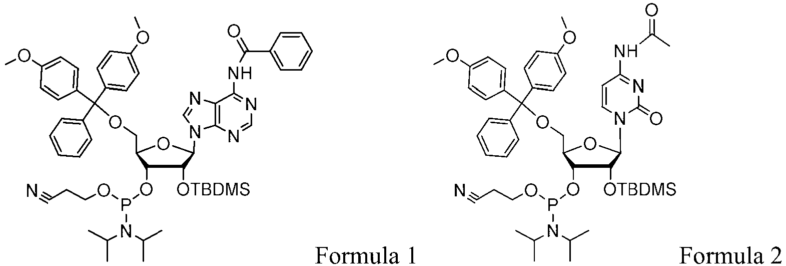

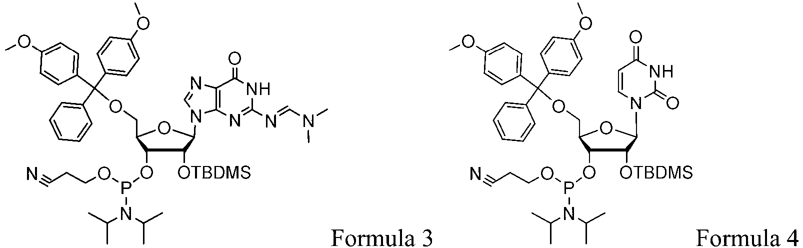

Nucleotide monomers were sequentially attached in the 3' to 5' direction according to the nucleotide arrangement using the solid-phase phosphoramidite method. The process of attaching each nucleotide monomer involves four steps: deprotection, coupling, oxidation or sulfurization, and hydroxyl protection. Specifically, when two nucleotides are linked via a phosphate linkage, the process of attaching a subsequent nucleotide monomer involves four steps: deprotection, coupling, oxidation, and hydroxyl protection. When two nucleotides are linked via a phosphorothioate linkage, the process of attaching a subsequent nucleotide monomer involves four steps: deprotection, coupling, sulfurization, and hydroxyl protection.

The preparation was carried out according to the following steps:

The solid-phase support was loaded into the designated position of the synthesizer, followed by multiple synthesis cycles to obtain the desired product, each cycle consisting of (1) deprotection, (2) coupling, (3) oxidation/sulfurization, and (4) hydroxyl protection. The cycle process and reagents used are described as follows:

### (1) Deprotection

3% dichloroacetic acid in toluene was used as a deprotection reagent to remove the DMT protecting group, followed by washing with acetonitrile.

### (2) Coupling

Each nucleotide monomer in acetonitrile was coupled using 0.25 M 5-ethylthio-1H-tetrazole as an activator, followed by washing with acetonitrile.

### (3) Oxidation/sulfurization

Oxidation: 0.05 M iodine in pyridine/water (90/10) was used as an oxidant for oxidation, followed by washing with acetonitrile.

Sulfurization: 3% xanthane hydride in pyridine was used as a sulfurizing agent for sulfurization, followed by washing with acetonitrile.

### (4) Hydroxyl protection

10% acetic anhydride in tetrahydrofuran (CAP A) and tetrahydrofuran/pyridine/N-methylimidazole (74/10/16, v/v/v) (CAP B) were used as hydroxyl protecting reagents for hydroxyl protection, followed by washing with acetonitrile.

The aforementioned steps were repeated according to the specified nucleotide arrangement to obtain a product with the specific sequence arrangement in either the sense strand or the antisense strand.

(5) 3% dichloroacetic acid in toluene was used as a deprotection reagent to remove the DMT protecting group from the last nucleotide, followed by washing with acetonitrile.

### (6) Ammonolysis and purification

The solid-phase support was transferred to a reactor and treated with concentrated aqueous ammonia (25 to 28% w/w) at 60°C for 12 hours. The system was then cooled to room temperature, and the mixture was filtered. The filter cake was washed with a mixture of purified water and ethanol. The filtrates were combined, subjected to column chromatography, concentrated, and lyophilized to obtain a 2'-O-TBDMS-protected product.

### (7) Removal of TBDMS protecting groups

The resulting product was treated with DMSO and triethylamine hydrofluoride at 60°C for 2 hours. An aqueous ammonium acetate solution was then added to the reaction mixture and vortexed thoroughly. The resulting mixture was added with anhydrous ethanol, vortexed thoroughly, followed by crystallization at -20°C for 8 to 12 hours. After centrifugation, the supernatant was discarded, and the precipitate was washed with anhydrous ethanol to obtain an unmodified single-stranded product.

### (8) Annealing

The sense and antisense strands of the resulting siRNA motif were mixed at a molar ratio of 1:1, heated to 95°C and maintained for 3 minutes, and slowly cooled to room temperature to form a double-stranded siRNA motif.

The siRNAs listed in Table 1 were synthesized according to the method described above.

**Table 1: siRNA motifs**

| **siRNA ID** | **Sense strand 5'-3'** | **SEQ ID NO:** | **Antisense strand 5'-3'** | **SEQ ID NO:** |
|---|---|---|---|---|
| si075 | GGAGUGAAGAGACCAAGAUGA | 1 | UCAUCUUGGUCUCUUCACUCCAA | 13 |
| si751 | AGUGAAGAGACCAAGAUGAAA | 2 | UUUCAUCUUGGUCUCUUCACUCC | 14 |
| si753 | GAAGAGACCAAGAUGAAGUUU | 3 | AAACUUCAUCUUGGUCUCUUCAC | 15 |
| si791 | GAGACCAAGAUGAAGUUUCCA | 4 | UGGAAACUUCAUCUUGGUCUCUU | 16 |
| si014 | AGCUAUUUUCAUAAUAAUACU | 5 | AGUAUUAUUAUGAAAAUAGCUUU | 17 |
| si076 | GAGUGAAGAGACCAAGAUGAA | 6 | UUCAUCUUGGUCUCUUCACUCCA | 18 |
| si077 | GUGAAGAGACCAAGAUGAAGU | 7 | ACUUCAUCUUGGUCUCUUCACUC | 19 |
| si078 | AAGAGACCAAGAUGAAGUUUA | 8 | UAAACUUCAUCUUGGUCUCUUCA | 20 |
| si080 | GACCAAGAUGAAGUUUCCCAA | 9 | UUGGGAAACUUCAUCUUGGUCUC | 21 |
| si752 | GAAGAGACCAAGAUGAAGUU | 10 | AACUUCAUCUUGGUCUCUUCAC | 22 |
| si079 | GAGACCAAGAUGAAGUUUCA | 11 | UGAAACUUCAUCUUGGUCUCUU | 23 |
| si047 | GAGACAAGCAUUUAUACUUU | 12 | AAAGUAUAAAUGCUUGUCUCCC | 24 |
| si001 | GAGGUCCCUUUUCAGGGGUGU | 25 | ACACCCCUGAAAAGGGACCUCCC | 134 |
| si002 | CCUUUUCAGGGGUGUCCACAA | 26 | UUGUGGACACCCCUGAAAAGGGA | 135 |
| si003 | CUUUUCAGGGGUGUCCACAAA | 27 | UUUGUGGACACCCCUGAAAAGGG | 136 |
| si004 | UCAGGGGUGUCCACAAAGUCA | 28 | UGACUUUGUGGACACCCCUGAAA | 137 |
| si005 | GGGGUGUCCACAAAGUCAAA | 29 | UUUGACUUUGUGGACACCCCUG | 138 |
| si006 | UCCACAAAGUCAAAGCUAUU | 30 | AAUAGCUUUGACUUUGUGGACA | 139 |
| si007 | UCCACAAAGUCAAAGCUAUUU | 31 | AAAUAGCUUUGACUUUGUGGACA | 140 |
| si008 | CAAAGUCAAAGCUAUUUUCAU | 32 | AUGAAAAUAGCUUUGACUUUGUG | 141 |
| si009 | AAGUCAAAGCUAUUUUCAUAA | 33 | UUAUGAAAAUAGCUUUGACUUUG | 142 |
| si010 | GUCAAAGCUAUUUUCAUAAU | 34 | AUUAUGAAAAUAGCUUUGACUU | 143 |
| si011 | UCAAAGCUAUUUUCAUAAUA | 35 | UAUUAUGAAAAUAGCUUUGACU | 144 |
| si012 | CAAAGCUAUUUUCAUAAUAAU | 36 | AUUAUUAUGAAAAUAGCUUUGAC | 145 |
| si013 | AAAGCUAUUUUCAUAAUAAUA | 37 | UAUUAUUAUGAAAAUAGCUUUGA | 146 |
| si015 | CUAUUUUCAUAAUAAUACUA | 38 | UAGUAUUAUUAUGAAAAUAGCU | 147 |
| si016 | CUAUUUUCAUAAUAAUACUAA | 39 | UUAGUAUUAUUAUGAAAAUAGCU | 148 |
| si017 | AUUUUCAUAAUAAUACUAACA | 40 | UGUUAGUAUUAUUAUGAAAAUAG | 149 |
| si018 | UUUUCAUAAUAAUACUAACAU | 41 | AUGUUAGUAUUAUUAUGAAAAUA | 150 |
| si019 | UUCAUAAUAAUACUAACAUGU | 42 | ACAUGUUAGUAUUAUUAUGAAAA | 151 |
| si020 | AUAAUAAUACUAACAUGUUA | 43 | UAACAUGUUAGUAUUAUUAUGA | 152 |
| si021 | UAAUAAUACUAACAUGUUAUU | 44 | AAUAACAUGUUAGUAUUAUUAUG | 153 |
| si022 | AAUAAUACUAACAUGUUAUUU | 45 | AAAUAACAUGUUAGUAUUAUUAU | 154 |
| si023 | UACUAACAUGUUAUUUGCCUU | 46 | AAGGCAAAUAACAUGUUAGUAUU | 155 |
| si024 | UAACAUGUUAUUUGCCUUUU | 47 | AAAAGGCAAAUAACAUGUUAGU | 156 |
| si025 | ACAUGUUAUUUGCCUUUUGAA | 48 | UUCAAAAGGCAAAUAACAUGUUA | 157 |
| si026 | AUGUUAUUUGCCUUUUGAAUU | 49 | AAUUCAAAAGGCAAAUAACAUGU | 158 |
| si027 | UUAUUUGCCUUUUGAAUUCU | 50 | AGAAUUCAAAAGGCAAAUAACA | 159 |
| si028 | UAUUUGCCUUUUGAAUUCUCA | 51 | UGAGAAUUCAAAAGGCAAAUAAC | 160 |
| si029 | UUUGCCUUUUGAAUUCUCAUU | 52 | AAUGAGAAUUCAAAAGGCAAAUA | 161 |
| si030 | GCCUUUUGAAUUCUCAUUAU | 53 | AUAAUGAGAAUUCAAAAGGCAA | 162 |
| si031 | UUUUGAAUUCUCAUUAUCUU | 54 | AAGAUAAUGAGAAUUCAAAAGG | 163 |
| si032 | UUUGAAUUCUCAUUAUCUUAA | 55 | UUAAGAUAAUGAGAAUUCAAAAG | 164 |
| si033 | GAAUUCUCAUUAUCUUAAAA | 56 | UUUUAAGAUAAUGAGAAUUCAA | 165 |
| si034 | AAUUCUCAUUAUCUUAAAAUU | 57 | AAUUUUAAGAUAAUGAGAAUUCA | 166 |
| si035 | UUCUCAUUAUCUUAAAAUUGU | 58 | ACAAUUUUAAGAUAAUGAGAAUU | 167 |
| si036 | CUCAUUAUCUUAAAAUUGUAU | 59 | AUACAAUUUUAAGAUAAUGAGAA | 168 |
| si037 | CUUAAAAUUGUAUUGUGGAGU | 60 | ACUCCACAAUACAAUUUUAAGAU | 169 |
| si038 | UAAAAUUGUAUUGUGGAGUUU | 61 | AAACUCCACAAUACAAUUUUAAG | 170 |
| si039 | UUGUAUUGUGGAGUUUUCCAA | 62 | UUGGAAAACUCCACAAUACAAUU | 171 |
| si040 | CCACUGGGAGACAAGCAUUU | 63 | AAAUGCUUGUCUCCCAGUGGGU | 172 |
| si041 | CACUGGGAGACAAGCAUUUA | 64 | UAAAUGCUUGUCUCCCAGUGGG | 173 |
| si042 | ACUGGGAGACAAGCAUUUAU | 65 | AUAAAUGCUUGUCUCCCAGUGG | 174 |
| si043 | CUGGGAGACAAGCAUUUAUA | 66 | UAUAAAUGCUUGUCUCCCAGUG | 175 |
| si044 | CUGGGAGACAAGCAUUUAUAA | 67 | UUAUAAAUGCUUGUCUCCCAGUG | 176 |
| si045 | GGGAGACAAGCAUUUAUACU | 68 | AGUAUAAAUGCUUGUCUCCCAG | 177 |
| si046 | GGAGACAAGCAUUUAUACUU | 69 | AAGUAUAAAUGCUUGUCUCCCA | 178 |
| si048 | GACAAGCAUUUAUACUUUCU | 70 | AGAAAGUAUAAAUGCUUGUCUC | 179 |
| si049 | CAAGCAUUUAUACUUUCUUU | 71 | AAAGAAAGUAUAAAUGCUUGUC | 180 |
| si050 | CAAGCAUUUAUACUUUCUUUA | 72 | UAAAGAAAGUAUAAAUGCUUGUC | 181 |
| si051 | AGCAUUUAUACUUUCUUUCU | 73 | AGAAAGAAAGUAUAAAUGCUUG | 182 |
| si052 | CAUUUAUACUUUCUUUCUUCU | 74 | AGAAGAAAGAAAGUAUAAAUGCU | 183 |
| si053 | AUUUAUACUUUCUUUCUUCUU | 75 | AAGAAGAAAGAAAGUAUAAAUGC | 184 |
| si054 | UUUAUACUUUCUUUCUUCUUU | 76 | AAAGAAGAAAGAAAGUAUAAAUG | 185 |
| si055 | CUGGCUUAUACUUUCUUAAUA | 77 | UAUUAAGAAAGUAUAAGCCAGGC | 186 |
| si056 | GCUUAUACUUUCUUAAUAAA | 78 | UUUAUUAAGAAAGUAUAAGCCA | 187 |
| si057 | GCUUAUACUUUCUUAAUAAAA | 79 | UUUUAUUAAGAAAGUAUAAGCCA | 188 |
| si058 | UUAUACUUUCUUAAUAAAAA | 80 | UUUUUAUUAAGAAAGUAUAAGC | 189 |
| si059 | UAUACUUUCUUAAUAAAAAGA | 81 | UCUUUUUAUUAAGAAAGUAUAAG | 190 |
| si060 | AUACUUUCUUAAUAAAAAGGA | 82 | UCCUUUUUAUUAAGAAAGUAUAA | 191 |
| si061 | UACUUUCUUAAUAAAAAGGAA | 83 | UUCCUUUUUAUUAAGAAAGUAUA | 192 |
| si062 | ACUUUCUUAAUAAAAAGGAGA | 84 | UCUCCUUUUUAUUAAGAAAGUAU | 193 |
| si063 | CUUUCUUAAUAAAAAGGAGAA | 85 | UUCUCCUUUUUAUUAAGAAAGUA | 194 |
| si064 | UUUCUUAAUAAAAAGGAGAAA | 86 | UUUCUCCUUUUUAUUAAGAAAGU | 195 |
| si065 | UUGCUGCCUCUUUCCAUUCUA | 87 | UAGAAUGGAAAGAGGCAGCAAUG | 196 |
| si066 | UCUUUCCAUUCUGCCGUCUU | 88 | AAGACGGCAGAAUGGAAAGAGG | 197 |
| si067 | CUUUCCAUUCUGCCGUCUUA | 89 | UAAGACGGCAGAAUGGAAAGAG | 198 |
| si068 | UCCAUUCUGCCGUCUUCAGCA | 90 | UGCUGAAGACGGCAGAAUGGAAA | 199 |
| si069 | CCGUCUUCAGCCUCCUCAAAA | 91 | UUUUGAGGAGGCUGAAGACGGCA | 200 |
| si070 | CGUCUUCAGCCUCCUCAAAGA | 92 | UCUUUGAGGAGGCUGAAGACGGC | 201 |
| si071 | GGGCUUUGGAGUGAAGAGACA | 93 | UGUCUCUUCACUCCAAAGCCCCA | 202 |
| si072 | GGCUUUGGAGUGAAGAGACCA | 94 | UGGUCUCUUCACUCCAAAGCCCC | 203 |
| si073 | UUUGGAGUGAAGAGACCAAGA | 95 | UCUUGGUCUCUUCACUCCAAAGC | 204 |
| si074 | UGGAGUGAAGAGACCAAGAUA | 96 | UAUCUUGGUCUCUUCACUCCAAA | 205 |
| si081 | CAAGAUGAAGUUUCCCAGGCA | 97 | UGCCUGGGAAACUUCAUCUUGGU | 206 |
| si082 | AAGAUGAAGUUUCCCAGGCAA | 98 | UUGCCUGGGAAACUUCAUCUUGG | 207 |
| si083 | CUGGCAAUAUGACUCACUUGA | 99 | UCAAGUGAGUCAUAUUGCCAGGU | 208 |
| si084 | GGCAAUAUGACUCACUUGACA | 100 | UGUCAAGUGAGUCAUAUUGCCAG | 209 |
| si085 | GCAAUAUGACUCACUUGACCA | 101 | UGGUCAAGUGAGUCAUAUUGCCA | 210 |
| si086 | CAAUAUGACUCACUUGACCCA | 102 | UGGGUCAAGUGAGUCAUAUUGCC | 211 |
| si087 | AUAUGACUCACUUGACCCCUA | 103 | UAGGGGUCAAGUGAGUCAUAUUG | 212 |
| si088 | UAUGACUCACUUGACCCCUAU | 104 | AUAGGGGUCAAGUGAGUCAUAUU | 213 |
| si089 | AUGACUCACUUGACCCCUAUA | 105 | UAUAGGGGUCAAGUGAGUCAUAU | 214 |
| si090 | AGCAGGCUUGAAGCAGGGUAA | 106 | UUACCCUGCUUCAAGCCUGCUGG | 215 |
| si091 | GCAGGCUUGAAGCAGGGUAAA | 107 | UUUACCCUGCUUCAAGCCUGCUG | 216 |
| si092 | GGCUUGAAGCAGGGUAAGCAA | 108 | UUGCUUACCCUGCUUCAAGCCUG | 217 |
| si093 | GCUUGAAGCAGGGUAAGCAGA | 109 | UCUGCUUACCCUGCUUCAAGCCU | 218 |
| si094 | CCUUAAGGGAAGGUCAAGAGA | 110 | UCUCUUGACCUUCCCUUAAGGUA | 219 |
| si095 | UCUUAAUAAAAAGGAGAAAGA | 111 | UCUUUCUCCUUUUUAUUAAGAAA | 220 |
| si096 | CUUAAUAAAAAGGAGAAAGAA | 112 | UUCUUUCUCCUUUUUAUUAAGAA | 221 |
| si097 | UUAAUAAAAAGGAGAAAGAAA | 113 | UUUCUUUCUCCUUUUUAUUAAGA | 222 |
| si098 | AAUAAAAAGGAGAAAGAAAAU | 114 | AUUUUCUUUCUCCUUUUUAUUAA | 223 |
| si099 | AUAAAAAGGAGAAAGAAAAUA | 115 | UAUUUUCUUUCUCCUUUUUAUUA | 224 |
| si100 | UAAAAAGGAGAAAGAAAAUCA | 116 | UGAUUUUCUUUCUCCUUUUUAUU | 225 |
| si101 | AAAAAGGAGAAAGAAAAUCAA | 117 | UUGAUUUUCUUUCUCCUUUUUAU | 226 |
| si102 | AAAAGGAGAAAGAAAAUCAAA | 118 | UUUGAUUUUCUUUCUCCUUUUUA | 227 |
| si103 | AAAGGAGAAAGAAAAUCAACA | 119 | UGUUGAUUUUCUUUCUCCUUUUU | 228 |
| si104 | AAAGUCAAAGCUAUUUUCAUA | 120 | UAUGAAAAUAGCUUUGACUUUGU | 229 |
| si105 | AAGCUAUUUUCAUAAUAAUAA | 121 | UUAUUAUUAUGAAAAUAGCUUUG | 230 |
| si106 | AUAAUACUAACAUGUUAUUUA | 122 | UAAAUAACAUGUUAGUAUUAUUA | 231 |
| si107 | AUUCUCAUUAUCUUAAAAUUA | 123 | UAAUUUUAAGAUAAUGAGAAUUC | 232 |
| si108 | UCAUUAUCUUAAAAUUGUAUU | 124 | AAUACAAUUUUAAGAUAAUGAGA | 233 |
| si481 | CAAAGCUAUUUUCAUAAUAA | 125 | UUAUUAUGAAAAUAGCUUUGAC | 234 |
| siA029 | UGGCAAUAUGACUCACUUGAA | 126 | UUCAAGUGAGUCAUAUUGCCAGG | 235 |
| siA036 | CUUAAGGGAAGGUCAAGAGGA | 127 | UCCUCUUGACCUUCCCUUAAGGU | 236 |
| siA043 | UUAUACUUUCUUAAUAAAAAA | 129 | UUUUUUAUUAAGAAAGUAUAAGC | 238 |
| siA045 | UAAUAAAAAGGAGAAAGAAAA | 130 | UUUUCUUUCUCCUUUUUAUUAAG | 239 |
| siA048 | UCAAAGCUAUUUUCAUAAUAA | 131 | UUAUUAUGAAAAUAGCUUUGACU | 240 |
| si754 | GAGUGAAGAGACCAAGAUGA | 243 | UCAUCUUGGUCUCUUCACUCCA | 252 |
| si755 | GUGAAGAGACCAAGAUGAAA | 244 | UUUCAUCUUGGUCUCUUCACUC | 253 |
| si756 | AAGAGACCAAGAUGAAGUUU | 245 | AAACUUCAUCUUGGUCUCUUCA | 254 |
| si792 | AGACCAAGAUGAAGUUUCCA | 246 | UGGAAACUUCAUCUUGGUCUCU | 255 |
| si141 | GCUAUUUUCAUAAUAAUACU | 247 | AGUAUUAUUAUGAAAAUAGCUU | 256 |
| si761 | AGUGAAGAGACCAAGAUGAA | 248 | UUCAUCUUGGUCUCUUCACUCC | 257 |
| si771 | UGAAGAGACCAAGAUGAAGU | 249 | ACUUCAUCUUGGUCUCUUCACU | 258 |
| si781 | AGAGACCAAGAUGAAGUUUA | 250 | UAAACUUCAUCUUGGUCUCUUC | 259 |
| si801 | ACCAAGAUGAAGUUUCCCAA | 251 | UUGGGAAACUUCAUCUUGGUCU | 260 |

### 3. Experimental methods

The experimental method for evaluating the inhibitory effect of siRNA motifs on INHBE gene expression is the same as that described in other examples of the present disclosure, wherein the final siRNA concentration per well is 0.07 nM, with the only difference being that the test samples are siRNA motifs.

### 4. Experimental results

The inhibition rates of INHBE mRNA expression by the core sequences are shown in Table 2 below.

**Table 2**

| **siRNA ID** | **Inhibition rate (%)** | **siRNA ID** | **Inhibition rate (%)** | **siRNA ID** | **Inhibition rate (%)** |
|---|---|---|---|---|---|
| si752 | 97.7 | si039 | 79.6 | si075 | 74.2 |
| si751 | 96.5 | si043 | 79.5 | si753 | 71.5 |
| si141 | 93.7 | si076 | 77.7 | si080 | 71.3 |
| si781 | 87.8 | si078 | 77.6 | si801 | 67 |
| si079 | 86.4 | si047 | 77.6 | si014 | 66.3 |
| si761 | 84.1 | si050 | 77.1 | si756 | 45.5 |
| si755 | 81.3 | si754 | 76.5 | si792 | 30.9 |
| si771 | 79.7 | si791 | 76.4 | / | / |
| si049 | 79.7 | si077 | 75.3 | / | / |

### Example 2: Inhibition of INHBE gene expression by DV29P-modified siRNA derivatives

### 2.1 Design and synthesis of DV29P-modified siRNA derivatives

To enhance the inhibitory efficiency and stability of the sequences, the siRNA motifs described in Example 1 were modified using the template DV29P or DV29SP (the modification types are shown in Tables 3 and 4 below). The sequences of the siRNA derivatives modified using the template DV29P or DV29SP are shown in Table 5.

**Table 3: Example of modification template DV29P**

| No. | **Antisense strand (AS) 5'-3'** | | | | | | | | | | | | |
|---|---|---|---|---|---|---|---|---|---|---|---|---|---|
| **C** | **1** | **2** | **3** | | **4** | **5** | **6** | **7** | **8** | **9** | **10** | **11** | **12** |
| | 2'-OMe +PS +EVP | **2'-F +PS** | 2'-OMe | | **2'-F** | **2'-F** | **2'-F** | 2'-OMe | 2'-OMe | 2'-OMe | 2'-OMe | 2'-OMe | 2'-OMe |
| | **13** | **14** | **15** | | **16** | **17** | **18** | **19** | **20** | **21** | **22** | **23** | |
| | 2'-OMe | **2'-F** | 2'-OMe | | **2'-F** | 2'-OMe | 2'-OMe | 2'-OMe | 2'-OMe | 2'-OMe +PS | 2'-OMe +PS | 2'-OMe | |

| No. | **Sense strand (SS) 5'-3'** | | | | | | | | | | | | |
|---|---|---|---|---|---|---|---|---|---|---|---|---|---|
| **b** | **1** | **2** | | **3** | | **4** | **5** | **6** | **7** | **8** | **9** | **10** | **11** |
| | 2'-OMe +PS | 2'-OMe +PS | | 2'-OMe | | 2'-OMe | 2'-OMe | 2'-OMe | **2'-F** | 2'-OMe | **2'-F** | 2'-OMe | **2'-F** |
| | **12** | **13** | | **14** | | **15** | **16** | **17** | **18** | **19** | **20** | **21** | |
| | 2'-OMe | 2'-OMe | | 2'-OMe | | 2'-OMe | 2'-OMe | **2'-F** | 2'-OMe | 2'-OMe | 2'-OMe | 2'-OMe | |

**Table 4: Example of modification template DV29SP**

| No. | **Antisense strand (AS) 5'-3'** | | | | | | | | | | | |
|---|---|---|---|---|---|---|---|---|---|---|---|---|
| **C'** | **1** | **2** | **3** | **4** | **5** | **6** | **7** | **8** | **9** | **10** | **11** | **12** |
| | 2'-OMe +PS +EVP | **2'-F +PS** | 2'-OMe | **2'-F** | **2'-F** | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | | |
| | 2'-OMe | **2'-F** | 2'-OMe | **2'-F** | 2'-OMe | 2'-OMe | 2'-OMe | 2'-OME +PS | 2'-OMe +PS | 2'-OMe | | |

| No. | **Sense strand (SS) 5'-3'** | | | | | | | | | | | |
|---|---|---|---|---|---|---|---|---|---|---|---|---|
| **b'** | **1** | **2** | | **3** | **4** | **5** | **6** | **7** | **8** | **9** | | **10** |
| | 2'-OMe +PS | 2'-OMe +PS | | 2'-OMe | 2'-OMe | 2'-OMe | **2'-F** | 2'-OMe | **2'-F** | 2'-OMe | | **2'-F** |
| | **11** | **12** | | **13** | **14** | **15** | **16** | **17** | **18** | **19** | | **20** |
| | 2'-OMe | 2'-OMe | | 2'-OMe | 2'-OMe | 2'-OMe | **2'-F** | 2'-OMe | 2'-OMe | 2'-OMe | | 2'-OMe |

**Table 5: Sequences of siRNA derivatives modified with DV29P or DV29SP**

| **siRNA ID** | **Sense strand 5'-3'** | **SEQ ID NO:** | **Antisense strand 5'-3'** | **SEQ ID NO:** |
|---|---|---|---|---|
| si075-DV29P | Gms-Gms-Am-Gm-Um-Gm-Af-Am-Gf- Am-Gf-Am-Cm-Cm-Am-Am-Gf-Am- Um-Gm-Am | 261 | UmsEVP-Cfs-Am-Uf-Cf-Uf-Um-Gm- Gm-Um-Cm-Um-Cm-Uf-Um-Cf-Am- Cm-Um-Cm-Cms-Ams-Am | 391 |
| si751-DV29P | Ams-Gms-Um-Gm-Am-Am-Gf-Am-Gf- Am-Cf-Cm-Am-Am-Gm-Am-Uf-Gm- Am-Am-Am | 262 | UmsEVP-Ufs-Um-Cf-Af-Uf-Cm-Um- Um-Gm-Gm-Um-Cm-Uf-Cm-Uf-Um- Cm-Am-Cm-Ums-Cms-Cm | 392 |
| si753-DV29P | Gms-Ams-Am-Gm-Am-Gm-Af-Cm-Cf- Am-Af-Gm-Am-Um-Gm-Am-Af-Gm- Um-Um-Um | 263 | AmsEVP-Afs-Am-Cf-Uf-Uf-Cm-Am- Um-Cm-Um-Um-Gm-Gf-Um-Cf-Um- Cm-Um-Um-Cms-Ams-Cm | 393 |
| si791-DV29P | Gms-Ams-Gm-Am-Cm-Cm-Af-Am-Gf- Am-Uf-Gm-Am-Am-Gm-Um-Uf-Um- Cm-Cm-Am | 264 | UmsEVP-Gfs-Gm-Af-Af-Af-Cm-Um- Um-Cm-Am-Um-Cm-Uf-Um-Gf-Gm- Um-Cm-Um-Cms-Ums-Um | 394 |
| si014-DV29P | Ams-Gms-Cm-Um-Am-Um-Uf-Um-Uf- Cm-Af-Um-Am-Am-Um-Am-Af-Um- Am-Cm-Um | 265 | AmsEVP-Gfs-Um-Af-Uf-Uf-Am-Um- Um-Am-Um-Gm-Am-Af-Am-Af-Um- Am-Gm-Cm-Ums-Ums-Um | 395 |
| si076-DV29P | Gms-Ams-Gm-Um-Gm-Am-Af-Gm-Af- Gm-Af-Cm-Cm-Am-Am-Gm-Af-Um- Gm-Am-Am | 266 | UmsEVP-Ufs-Cm-Af-Uf-Cf-Um-Um- Gm-Gm-Um-Cm-Um-Cf-Um-Uf-Cm- Am-Cm-Um-Cms-Cms-Am | 396 |
| si077-DV29P | Gms-Ums-Gm-Am-Am-Gm-Af-Gm-Af- Cm-Cf-Am-Am-Gm-Am-Um-Gf-Am- Am-Gm-Um | 267 | AmsEVP-Cfs-Um-Uf-Cf-Af-Um-Cm- Um-Um-Gm-Gm-Um-Cf-Um-Cf-Um- Um-Cm-Am-Cms-Ums-Cm | 397 |
| si078-DV29P | Ams-Ams-Gm-Am-Gm-Am-Cf-Cm-Af- Am-Gf-Am-Um-Gm-Am-Am-Gf-Um- Um-Um-Am | 268 | UmsEVP-Afs-Am-Af-Cf-Uf-Um-Cm- Am-Um-Cm-Um-Um-Gf-Gm-Uf-Cm- Um-Cm-Um-Ums-Cms-Am | 398 |
| si080-DV29P | Gms-Ams-Cm-Cm-Am-Am-Gf-Am-Uf- Gm-Af-Am-Gm-Um-Um-Um-Cf-Cm- Cm-Am-Am | 269 | UmsEVP-Ufs-Gm-Gf-Gf-Af-Am-Am- Cm-Um-Um-Cm-Am-Uf-Cm-Uf-Um- Gm-Gm-Um-Cms-Ums-Cm | 399 |
| si752-DV29SP | Gms-Ams-Am-Gm-Am-Gf-Am-Cf-Cm- Af-Am-Gm-Am-Um-Gm-Af-Am-Gm- Um-Um | 270 | AmsEVP-Afs-Cm-Uf-Uf-Cf-Am-Um- Cm-Um-Um-Gm-Gm-Uf-Cm-Uf-Cm- Um-Um-Cms-Ams-Cm | 400 |
| si079-DV29SP | Gms-Ams-Gm-Am-Cm-Cf-Am-Af-Gm- Af-Um-Gm-Am-Am-Gm-Uf-Um-Um- Cm-Am | 271 | UmsEVP-Gfs-Am-Af-Af-Cf-Um-Um- Cm-Am-Um-Cm-Um-Uf-Gm-Gf-Um- Cm-Um-Cms-Ums-Um | 401 |
| si047-DV29SP | Gms-Ams-Gm-Am-Cm-Af-Am-Gf-Cm- Af-Um-Um-Um-Am-Um-Af-Cm-Um- Um-Um | 272 | AmsEVP-Afs-Am-Gf-Uf-Af-Um-Am- Am-Am-Um-Gm-Cm-Uf-Um-Gf-Um- Cm-Um-Cms-Cms-Cm | 402 |
| si001-DV29P | Gms-Ams-Gm-Gm-Um-Cm-Cf-Cm-Uf- Um-Uf-Um-Cm-Am-Gm-Gm-Gf-Gm- Um-Gm-Um | 273 | AmsEVP-Cfs-Am-Cf-Cf-Cf-Cm-Um- Gm-Am-Am-Am-Am-Gf-Gm-Gf-Am- Cm-Cm-Um-Cms-Cms-Cm | 403 |
| si002-DV29P | Cms-Cms-Um-Um-Um-Um-Cf-Am-Gf- Gm-Gf-Gm-Um-Gm-Um-Cm-Cf-Am- Cm-Am-Am | 274 | UmsEVP-Ufs-Gm-Uf-Gf-Gf-Am-Cm- Am-Cm-Cm-Cm-Cm-Uf-Gm-Af-Am- Am-Am-Gm-Gms-Gms-Am | 404 |
| si003-DV29P | Cms-Ums-Um-Um-Um-Cm-Af-Gm-Gf- Gm-Gf-Um-Gm-Um-Cm-Cm-Af-Cm- Am-Am-Am | 275 | UmsEVP-Ufs-Um-Gf-Uf-Gf-Gm-Am- Cm-Am-Cm-Cm-Cm-Cf-Um-Gf-Am- Am-Am-Am-Gms-Gms-Gm | 405 |
| si004-DV29P | Ums-Cms-Am-Gm-Gm-Gm-Gf-Um-Gf- Um-Cf-Cm-Am-Cm-Am-Am-Af-Gm- Um-Cm-Am | 276 | UmsEVP-Gfs-Am-Cf-Uf-Uf-Um-Gm- Um-Gm-Gm-Am-Cm-Af-Cm-Cf-Cm- Cm-Um-Gm-Ams-Ams-Am | 406 |
| si005-DV29SP | Gms-Gms-Gm-Gm-Um-Gf-Um-Cf-Cm- Af-Cm-Am-Am-Am-Gm-Uf-Cm-Am- Am-Am | 277 | UmsEVP-Ufs-Um-Gf-Af-Cf-Um-Um- Um-Gm-Um-Gm-Gm-Af-Cm-Af-Cm- Cm-Cm-Cms-Ums-Gm | 407 |
| si006-DV29SP | Ums-Cms-Cm-Am-Cm-Af-Am-Af-Gm- Uf-Cm-Am-Am-Am-Gm-Cf-Um-Am- Um-Um | 278 | AmsEVP-Afs-Um-Af-Gf-Cf-Um-Um- Um-Gm-Am-Cm-Um-Uf-Um-Gf-Um- Gm-Gm-Ams-Cms-Am | 408 |
| si007-DV29P | Ums-Cms-Cm-Am-Cm-Am-Af-Am-Gf- Um-Cf-Am-Am-Am-Gm-Cm-Uf-Am- Um-Um-Um | 279 | AmsEVP-Afs-Am-Uf-Af-Gf-Cm-Um- Um-Um-Gm-Am-Cm-Uf-Um-Uf-Gm- Um-Gm-Gm-Ams-Cms-Am | 409 |
| si008-DV29P | Cms-Ams-Am-Am-Gm-Um-Cf-Am-Af- Am-Gf-Cm-Um-Am-Um-Um-Uf-Um- Cm-Am-Um | 280 | AmsEVP-Ufs-Gm-Af-Af-Af-Am-Um- Am-Gm-Cm-Um-Um-Uf-Gm-Af-Cm- Um-Um-Um-Gms-Ums-Gm | 410 |
| si009-DV29P | Ams-Ams-Gm-Um-Cm-Am-Af-Am-Gf- Cm-Uf-Am-Um-Um-Um-Um-Cf-Am- Um-Am-Am | 281 | UmsEVP-Ufs-Am-Uf-Gf-Af-Am-Am- Am-Um-Am-Gm-Cm-Uf-Um-Uf-Gm- Am-Cm-Um-Ums-Ums-Gm | 411 |
| si010-DV29SP | Gms-Ums-Cm-Am-Am-Af-Gm-Cf-Um- Af-Um-Um-Um-Um-Cm-Af-Um-Am- Am-Um | 282 | AmsEVP-Ufs-Um-Af-Uf-Gf-Am-Am- Am-Am-Um-Am-Gm-Cf-Um-Uf-Um- Gm-Am-Cms-Ums-Um | 412 |
| si011-DV29SP | Ums-Cms-Am-Am-Am-Gf-Cm-Uf-Am- Uf-Um-Um-Um-Cm-Am-Uf-Am-Am- Um-Am | 283 | UmsEVP-Afs-Um-Uf-Af-Uf-Gm-Am- Am-Am-Am-Um-Am-Gf-Cm-Uf-Um- Um-Gm-Ams-Cms-Um | 413 |
| si012-DV29P | Cms-Ams-Am-Am-Gm-Cm-Uf-Am-Uf- Um-Uf-Um-Cm-Am-Um-Am-Af-Um- Am-Am-Um | 284 | AmsEVP-Ufs-Um-Af-Uf-Uf-Am-Um- Gm-Am-Am-Am-Am-Uf-Am-Gf-Cm- Um-Um-Um-Gms-Ams-Cm | 414 |
| si013-DV29P | Ams-Ams-Am-Gm-Cm-Um-Af-Um-Uf- Um-Uf-Cm-Am-Um-Am-Am-Uf-Am- Am-Um-Am | 285 | UmsEVP-Afs-Um-Uf-Af-Uf-Um-Am- Um-Gm-Am-Am-Am-Af-Um-Af-Gm- Cm-Um-Um-Ums-Gms-Am | 415 |
| si015-DV29SP | Cms-Ums-Am-Um-Um-Uf-Um-Cf-Am- Uf-Am-Am-Um-Am-Am-Uf-Am-Cm- Um-Am | 286 | UmsEVP-Afs-Gm-Uf-Af-Uf-Um-Am- Um-Um-Am-Um-Gm-Af-Am-Af-Am- Um-Am-Gms-Cms-Um | 416 |
| si016-DV29P | Cms-Ums-Am-Um-Um-Um-Uf-Cm-Af- Um-Af-Am-Um-Am-Am-Um-Af-Cm- Um-Am-Am | 287 | UmsEVP-Ufs-Am-Gf-Uf-Af-Um-Um- Am-Um-Um-Am-Um-Gf-Am-Af-Am- Am-Um-Am-Gms-Cms-Um | 417 |
| si017-DV29P | Ams-Ums-Um-Um-Um-Cm-Af-Um-Af- Am-Uf-Am-Am-Um-Am-Cm-Uf-Am- Am-Cm-Am | 288 | UmsEVP-Gfs-Um-Uf-Af-Gf-Um-Am- Um-Um-Am-Um-Um-Af-Um-Gf-Am- Am-Am-Am-Ums-Ams-Gm | 418 |
| si018-DV29P | Ums-Ums-Um-Um-Cm-Am-Uf-Am-Af- Um-Af-Am-Um-Am-Cm-Um-Af-Am- Cm-Am-Um | 289 | AmsEVP-Ufs-Gm-Uf-Uf-Af-Gm-Um- Am-Um-Um-Am-Um-Uf-Am-Uf-Gm- Am-Am-Am-Ams-Ums-Am | 419 |
| si019-DV29P | Ums-Ums-Cm-Am-Um-Am-Af-Um-Af- Am-Uf-Am-Cm-Um-Am-Am-Cf-Am- Um-Gm-Um | 290 | AmsEVP-Cfs-Am-Uf-Gf-Uf-Um-Am- Gm-Um-Am-Um-Um-Af-Um-Uf-Am- Um-Gm-Am-Ams-Ams-Am | 420 |
| si020-DV29SP | Ams-Ums-Am-Am-Um-Af-Am-Uf-Am- Cf-Um-Am-Am-Cm-Am-Uf-Gm-Um- Um-Am | 291 | UmsEVP-Afs-Am-Cf-Af-Uf-Gm-Um- Um-Am-Gm-Um-Am-Uf-Um-Af-Um- Um-Am-Ums-Gms-Am | 421 |
| si021-DV29P | Ums-Ams-Am-Um-Am-Am-Uf-Am-Cf- Um-Af-Am-Cm-Am-Um-Gm-Uf-Um- Am-Um-Um | 292 | AmsEVP-Afs-Um-Af-Af-Cf-Am-Um- Gm-Um-Um-Am-Gm-Uf-Am-Uf-Um- Am-Um-Um-Ams-Ums-Gm | 422 |
| si022-DV29P | Ams-Ams-Um-Am-Am-Um-Af-Cm-Uf- Am-Af-Cm-Am-Um-Gm-Um-Uf-Am- Um-Um-Um | 293 | AmsEVP-Afs-Am-Uf-Af-Af-Cm-Am- Um-Gm-Um-Um-Am-Gf-Um-Af-Um- Um-Am-Um-Ums-Ams-Um | 423 |
| si023-DV29P | Ums-Ams-Cm-Um-Am-Am-Cf-Am-Uf- Gm-Uf-Um-Am-Um-Um-Um-Gf-Cm- Cm-Um-Um | 294 | AmsEVP-Afs-Gm-Gf-Cf-Af-Am-Am- Um-Am-Am-Cm-Am-Uf-Gm-Uf-Um- Am-Gm-Um-Ams-Ums-Um | 424 |
| si024-DV29SP | Ums-Ams-Am-Cm-Am-Uf-Gm-Uf-Um- Af-Um-Um-Um-Gm-Cm-Cf-Um-Um- Um-Um | 295 | AmsEVP-Afs-Am-Af-Gf-Gf-Cm-Am- Am-Am-Um-Am-Am-Cf-Am-Uf-Gm- Um-Um-Ams-Gms-Um | 425 |
| si025-DV29P | Ams-Cms-Am-Um-Gm-Um-Uf-Am-Uf- Um-Uf-Gm-Cm-Cm-Um-Um-Uf-Um- Gm-Am-Am | 296 | UmsEVP-Ufs-Cm-Af-Af-Af-Am-Gm- Gm-Cm-Am-Am-Am-Uf-Am-Af-Cm- Am-Um-Gm-Ums-Ums-Am | 426 |
| si026-DV29P | Ams-Ums-Gm-Um-Um-Am-Uf-Um-Uf- Gm-Cf-Cm-Um-Um-Um-Um-Gf-Am- Am-Um-Um | 297 | AmsEVP-Afs-Um-Uf-Cf-Af-Am-Am- Am-Gm-Gm-Cm-Am-Af-Am-Uf-Am- Am-Cm-Am-Ums-Gms-Um | 427 |
| si027-DV29SP | Ums-Ums-Am-Um-Um-Uf-Gm-Cf-Cm- Uf-Um-Um-Um-Gm-Am-Af-Um-Um- Cm-Um | 298 | AmsEVP-Gfs-Am-Af-Uf-Uf-Cm-Am- Am-Am-Am-Gm-Gm-Cf-Am-Af-Am- Um-Am-Ams-Cms-Am | 428 |
| si028-DV29P | Ums-Ams-Um-Um-Um-Gm-Cf-Cm-Uf- Um-Uf-Um-Gm-Am-Am-Um-Uf-Cm- Um-Cm-Am | 299 | UmsEVP-Gfs-Am-Gf-Af-Af-Um-Um- Cm-Am-Am-Am-Am-Gf-Gm-Cf-Am- Am-Am-Um-Ams-Ams-Cm | 429 |
| si029-DV29P | Ums-Ums-Um-Gm-Cm-Cm-Uf-Um-Uf- Um-Gf-Am-Am-Um-Um-Cm-Uf-Cm- Am-Um-Um | 300 | AmsEVP-Afs-Um-Gf-Af-Gf-Am-Am- Um-Um-Cm-Am-Am-Af-Am-Gf-Gm- Cm-Am-Am-Ams-Ums-Am | 430 |
| si030-DV29SP | Gms-Cms-Cm-Um-Um-Uf-Um-Gf-Am- Af-Um-Um-Cm-Um-Cm-Af-Um-Um- Am-Um | 301 | AmsEVP-Ufs-Am-Af-Uf-Gf-Am-Gm- Am-Am-Um-Um-Cm-Af-Am-Af-Am- Gm-Gm-Cms-Ams-Am | 431 |
| si031-DV29SP | Ums-Ums-Um-Um-Gm-Af-Am-Uf-Um- Cf-Um-Cm-Am-Um-Um-Af-Um-Cm- Um-Um | 302 | AmsEVP-Afs-Gm-Af-Uf-Af-Am-Um- Gm-Am-Gm-Am-Am-Uf-Um-Cf-Am- Am-Am-Ams-Gms-Gm | 432 |
| si032-DV29P | Ums-Ums-Um-Gm-Am-Am-Uf-Um-Cf- Um-Cf-Am-Um-Um-Am-Um-Cf-Um- Um-Am-Am | 303 | UmsEVP-Ufs-Am-Af-Gf-Af-Um-Am- Am-Um-Gm-Am-Gm-Af-Am-Uf-Um- Cm-Am-Am-Ams-Ams-Gm | 433 |
| si033-DV29SP | Gms-Ams-Am-Um-Um-Cf-Um-Cf-Am- Uf-Um-Am-Um-Cm-Um-Uf-Am-Am- Am-Am | 304 | UmsEVP-Ufs-Um-Uf-Af-Af-Gm-Am- Um-Am-Am-Um-Gm-Af-Gm-Af-Am- Um-Um-Cms-Ams-Am | 434 |
| si034-DV29P | Ams-Ams-Um-Um-Cm-Um-Cf-Am-Uf- Um-Af-Um-Cm-Um-Um-Am-Af-Am- Am-Um-Um | 305 | AmsEVP-Afs-Um-Uf-Uf-Uf-Am-Am- Gm-Am-Um-Am-Am-Uf-Gm-Af-Gm- Am-Am-Um-Ums-Cms-Am | 435 |
| si035-DV29P | Ums-Ums-Cm-Um-Cm-Am-Uf-Um-Af- Um-Cf-Um-Um-Am-Am-Am-Af-Um- Um-Gm-Um | 306 | AmsEVP-Cfs-Am-Af-Uf-Uf-Um-Um- Am-Am-Gm-Am-Um-Af-Am-Uf-Gm- Am-Gm-Am-Ams-Ums-Um | 436 |
| si036-DV29P | Cms-Ums-Cm-Am-Um-Um-Af-Um-Cf- Um-Uf-Am-Am-Am-Am-Um-Uf-Gm- Um-Am-Um | 307 | AmsEVP-Ufs-Am-Cf-Af-Af-Um-Um- Um-Um-Am-Am-Gm-Af-Um-Af-Am- Um-Gm-Am-Gms-Ams-Am | 437 |
| si037-DV29P | Cms-Ums-Um-Am-Am-Am-Af-Um-Uf- Gm-Uf-Am-Um-Um-Gm-Um-Gf-Gm- Am-Gm-Um | 308 | AmsEVP-Cfs-Um-Cf-Cf-Af-Cm-Am- Am-Um-Am-Cm-Am-Af-Um-Uf-Um- Um-Am-Am-Gms-Ams-Um | 438 |
| si038-DV29P | Ums-Ams-Am-Am-Am-Um-Uf-Gm-Uf- Am-Uf-Um-Gm-Um-Gm-Gm-Af-Gm- Um-Um-Um | 309 | AmsEVP-Afs-Am-Cf-Uf-Cf-Cm-Am- Cm-Am-Am-Um-Am-Cf-Am-Af-Um- Um-Um-Um-Ams-Ams-Gm | 439 |
| si039-DV29P | Ums-Ums-Gm-Um-Am-Um-Uf-Gm-Uf- Gm-Gf-Am-Gm-Um-Um-Um-Uf-Cm- Cm-Am-Am | 310 | UmsEVP-Ufs-Gm-Gf-Af-Af-Am-Am- Cm-Um-Cm-Cm-Am-Cf-Am-Af-Um- Am-Cm-Am-Ams-Ums-Um | 440 |
| si040-DV29SP | Cms-Cms-Am-Cm-Um-Gf-Gm-Gf-Am- Gf-Am-Cm-Am-Am-Gm-Cf-Am-Um- Um-Um | 311 | AmsEVP-Afs-Am-Uf-Gf-Cf-Um-Um- Gm-Um-Cm-Um-Cm-Cf-Cm-Af-Gm- Um-Gm-Gms-Gms-Um | 441 |
| si041-DV29SP | Cms-Ams-Cm-Um-Gm-Gf-Gm-Af-Gm- Af-Cm-Am-Am-Gm-Cm-Af-Um-Um- Um-Am | 312 | UmsEVP-Afs-Am-Af-Uf-Gf-Cm-Um- Um-Gm-Um-Cm-Um-Cf-Cm-Cf-Am- Gm-Um-Gms-Gms-Gm | 442 |
| si042-DV29SP | Ams-Cms-Um-Gm-Gm-Gf-Am-Gf-Am- Cf-Am-Am-Gm-Cm-Am-Uf-Um-Um- Am-Um | 313 | AmsEVP-Ufs-Am-Af-Af-Uf-Gm-Cm- Um-Um-Gm-Um-Cm-Uf-Cm-Cf-Cm- Am-Gm-Ums-Gms-Gm | 443 |
| si043-DV29SP | Cms-Ums-Gm-Gm-Gm-Af-Gm-Af-Cm- Af-Am-Gm-Cm-Am-Um-Uf-Um-Am- Um-Am | 314 | UmsEVP-Afs-Um-Af-Af-Af-Um-Gm- Cm-Um-Um-Gm-Um-Cf-Um-Cf-Cm- Cm-Am-Gms-Ums-Gm | 444 |
| si044-DV29P | Cms-Ums-Gm-Gm-Gm-Am-Gf-Am-Cf- Am-Af-Gm-Cm-Am-Um-Um-Uf-Am- Um-Am-Am | 315 | UmsEVP-Ufs-Am-Uf-Af-Af-Am-Um- Gm-Cm-Um-Um-Gm-Uf-Cm-Uf-Cm- Cm-Cm-Am-Gms-Ums-Gm | 445 |
| si045-DV29SP | Gms-Gms-Gm-Am-Gm-Af-Cm-Af-Am- Gf-Cm-Am-Um-Um-Um-Af-Um-Am- Cm-Um | 316 | AmsEVP-Gfs-Um-Af-Uf-Af-Am-Am- Um-Gm-Cm-Um-Um-Gf-Um-Cf-Um- Cm-Cm-Cms-Ams-Gm | 446 |
| si046-DV29SP | Gms-Gms-Am-Gm-Am-Cf-Am-Af-Gm- Cf-Am-Um-Um-Um-Am-Uf-Am-Cm- Um-Um | 317 | AmsEVP-Afs-Gm-Uf-Af-Uf-Am-Am- Am-Um-Gm-Cm-Um-Uf-Gm-Uf-Cm- Um-Cm-Cms-Cms-Am | 447 |
| si048-DV29SP | Gms-Ams-Cm-Am-Am-Gf-Cm-Af-Um- Uf-Um-Am-Um-Am-Cm-Uf-Um-Um- Cm-Um | 318 | AmsEVP-Gfs-Am-Af-Af-Gf-Um-Am- Um-Am-Am-Am-Um-Gf-Cm-Uf-Um- Gm-Um-Cms-Ums-Cm | 448 |
| si049-DV29SP | Cms-Ams-Am-Gm-Cm-Af-Um-Uf-Um- Af-Um-Am-Cm-Um-Um-Uf-Cm-Um- Um-Um | 319 | AmsEVP-Afs-Am-Gf-Af-Af-Am-Gm- Um-Am-Um-Am-Am-Af-Um-Gf-Cm- Um-Um-Gms-Ums-Cm | 449 |
| si050-DV29P | Cms-Ams-Am-Gm-Cm-Am-Uf-Um-Uf- Am-Uf-Am-Cm-Um-Um-Um-Cf-Um- Um-Um-Am | 320 | UmsEVP-Afs-Am-Af-Gf-Af-Am-Am- Gm-Um-Am-Um-Am-Af-Am-Uf-Gm- Cm-Um-Um-Gms-Ums-Cm | 450 |
| si051-DV29SP | Ams-Gms-Cm-Am-Um-Uf-Um-Af-Um- Af-Cm-Um-Um-Um-Cm-Uf-Um-Um- Cm-Um | 321 | AmsEVP-Gfs-Am-Af-Af-Gf-Am-Am- Am-Gm-Um-Am-Um-Af-Am-Af-Um- Gm-Cm-Ums-Ums-Gm | 451 |
| si052-DV29P | Cms-Ams-Um-Um-Um-Am-Uf-Am-Cf- Um-Uf-Um-Cm-Um-Um-Um-Cf-Um- Um-Cm-Um | 322 | AmsEVP-Gfs-Am-Af-Gf-Af-Am-Am- Gm-Am-Am-Am-Gm-Uf-Am-Uf-Am- Am-Am-Um-Gms-Cms-Um | 452 |
| si053-DV29P | Ams-Ums-Um-Um-Am-Um-Af-Cm-Uf- Um-Uf-Cm-Um-Um-Um-Cm-Uf-Um- Cm-Um-Um | 323 | AmsEVP-Afs-Gm-Af-Af-Gf-Am-Am- Am-Gm-Am-Am-Am-Gf-Um-Af-Um- Am-Am-Am-Ums-Gms-Cm | 453 |
| si054-DV29P | Ums-Ums-Um-Am-Um-Am-Cf-Um-Uf- Um-Cf-Um-Um-Um-Cm-Um-Uf-Cm- Um-Um-Um | 324 | AmsEVP-Afs-Am-Gf-Af-Af-Gm-Am- Am-Am-Gm-Am-Am-Af-Gm-Uf-Am- Um-Am-Am-Ams-Ums-Gm | 454 |
| si055-DV29P | Cms-Ums-Gm-Gm-Cm-Um-Uf-Am-Uf- Am-Cf-Um-Um-Um-Cm-Um-Uf-Am- Am-Um-Am | 325 | UmsEVP-Afs-Um-Uf-Af-Af-Gm-Am- Am-Am-Gm-Um-Am-Uf-Am-Af-Gm- Cm-Cm-Am-Gms-Gms-Cm | 455 |
| si056-DV29SP | Gms-Cms-Um-Um-Am-Uf-Am-Cf-Um- Uf-Um-Cm-Um-Um-Am-Af-Um-Am- Am-Am | 326 | UmsEVP-Ufs-Um-Af-Uf-Uf-Am-Am- Gm-Am-Am-Am-Gm-Uf-Am-Uf-Am- Am-Gm-Cms-Cms-Am | 456 |
| si057-DV29P | Gms-Cms-Um-Um-Am-Um-Af-Cm-Uf- Um-Uf-Cm-Um-Um-Am-Am-Uf-Am- Am-Am-Am | 327 | UmsEVP-Ufs-Um-Uf-Af-Uf-Um-Am- Am-Gm-Am-Am-Am-Gf-Um-Af-Um- Am-Am-Gm-Cms-Cms-Am | 457 |
| si058-DV29SP | Ums-Ums-Am-Um-Am-Cf-Um-Uf-Um- Cf-Um-Um-Am-Am-Um-Af-Am-Am- Am-Am | 328 | UmsEVP-Ufs-Um-Uf-Uf-Af-Um-Um- Am-Am-Gm-Am-Am-Af-Gm-Uf-Am- Um-Am-Ams-Gms-Cm | 458 |
| si059-DV29P | Ums-Ams-Um-Am-Cm-Um-Uf-Um-Cf- Um-Uf-Am-Am-Um-Am-Am-Af-Am- Am-Gm-Am | 329 | UmsEVP-Cfs-Um-Uf-Uf-Uf-Um-Am- Um-Um-Am-Am-Gm-Af-Am-Af-Gm- Um-Am-Um-Ams-Ams-Gm | 459 |
| si060-DV29P | Ams-Ums-Am-Cm-Um-Um-Uf-Cm-Uf- Um-Af-Am-Um-Am-Am-Am-Af-Am- Gm-Gm-Am | 330 | UmsEVP-Cfs-Cm-Uf-Uf-Uf-Um-Um- Am-Um-Um-Am-Am-Gf-Am-Af-Am- Gm-Um-Am-Ums-Ams-Am | 460 |
| si061-DV29P | Ums-Ams-Cm-Um-Um-Um-Cf-Um-Uf- Am-Af-Um-Am-Am-Am-Am-Af-Gm- Gm-Am-Am | 331 | UmsEVP-Ufs-Cm-Cf-Uf-Uf-Um-Um- Um-Am-Um-Um-Am-Af-Gm-Af-Am- Am-Gm-Um-Ams-Ums-Am | 461 |
| si062-DV29P | Ams-Cms-Um-Um-Um-Cm-Uf-Um-Af- Am-Uf-Am-Am-Am-Am-Am-Gf-Gm- Am-Gm-Am | 332 | UmsEVP-Cfs-Um-Cf-Cf-Uf-Um-Um- Um-Um-Am-Um-Um-Af-Am-Gf-Am- Am-Am-Gm-Ums-Ams-Um | 462 |
| si063-DV29P | Cms-Ums-Um-Um-Cm-Um-Uf-Am-Af- Um-Af-Am-Am-Am-Am-Gm-Gf-Am- Gm-Am-Am | 333 | UmsEVP-Ufs-Cm-Uf-Cf-Cf-Um-Um- Um-Um-Um-Am-Um-Uf-Am-Af-Gm- Am-Am-Am-Gms-Ums-Am | 463 |
| si064-DV29P | Ums-Ums-Um-Cm-Um-Um-Af-Am-Uf- Am-Af-Am-Am-Am-Gm-Gm-Af-Gm- Am-Am-Am | 334 | UmsEVP-Ufs-Um-Cf-Uf-Cf-Cm-Um- Um-Um-Um-Um-Am-Uf-Um-Af-Am- Gm-Am-Am-Ams-Gms-Um | 464 |
| si065-DV29P | Ums-Ums-Gm-Cm-Um-Gm-Cf-Cm-Uf- Cm-Uf-Um-Um-Cm-Cm-Am-Uf-Um- Cm-Um-Am | 335 | UmsEVP-Afs-Gm-Af-Af-Uf-Gm-Gm- Am-Am-Am-Gm-Am-Gf-Gm-Cf-Am- Gm-Cm-Am-Ams-Ums-Gm | 465 |
| si066-DV29SP | Ums-Cms-Um-Um-Um-Cf-Cm-Af-Um- Uf-Cm-Um-Gm-Cm-Cm-Gf-Um-Cm- Um-Um | 336 | AmsEVP-Afs-Gm-Af-Cf-Gf-Gm-Cm- Am-Gm-Am-Am-Um-Gf-Gm-Af-Am- Am-Gm-Ams-Gms-Gm | 466 |
| si067-DV29SP | Cms-Ums-Um-Um-Cm-Cf-Am-Uf-Um- Cf-Um-Gm-Cm-Cm-Gm-Uf-Cm-Um- Um-Am | 337 | UmsEVP-Afs-Am-Gf-Af-Cf-Gm-Gm- Cm-Am-Gm-Am-Am-Uf-Gm-Gf-Am- Am-Am-Gms-Ams-Gm | 467 |
| si068-DV29P | Ums-Cms-Cm-Am-Um-Um-Cf-Um-Gf- Cm-Cf-Gm-Um-Cm-Um-Um-Cf-Am- Gm-Cm-Am | 338 | UmsEVP-Gfs-Cm-Uf-Gf-Af-Am-Gm- Am-Cm-Gm-Gm-Cm-Af-Gm-Af-Am- Um-Gm-Gm-Ams-Ams-Am | 468 |
| si069-DV29P | Cms-Cms-Gm-Um-Cm-Um-Uf-Cm-Af- Gm-Cf-Cm-Um-Cm-Cm-Um-Cf-Am- Am-Am-Am | 339 | UmsEVP-Ufs-Um-Uf-Gf-Af-Gm-Gm- Am-Gm-Gm-Cm-Um-Gf-Am-Af-Gm- Am-Cm-Gm-Gms-Cms-Am | 469 |
| si070-DV29P | Cms-Gms-Um-Cm-Um-Um-Cf-Am-Gf- Cm-Cf-Um-Cm-Cm-Um-Cm-Af-Am- Am-Gm-Am | 340 | UmsEVP-Cfs-Um-Uf-Uf-Gf-Am-Gm- Gm-Am-Gm-Gm-Cm-Uf-Gm-Af-Am- Gm-Am-Cm-Gms-Gms-Cm | 470 |
| si071-DV29P | Gms-Gms-Gm-Cm-Um-Um-Uf-Gm-Gf- Am-Gf-Um-Gm-Am-Am-Gm-Af-Gm- Am-Cm-Am | 341 | UmsEVP-Gfs-Um-Cf-Uf-Cf-Um-Um- Cm-Am-Cm-Um-Cm-Cf-Am-Af-Am- Gm-Cm-Cm-Cms-Cms-Am | 471 |
| si072-DV29P | Gms-Gms-Cm-Um-Um-Um-Gf-Gm-Af- Gm-Uf-Gm-Am-Am-Gm-Am-Gf-Am- Cm-Cm-Am | 342 | UmsEVP-Gfs-Gm-Uf-Cf-Uf-Cm-Um- Um-Cm-Am-Cm-Um-Cf-Cm-Af-Am- Am-Gm-Cm-Cms-Cms-Cm | 472 |
| si073-DV29P | Ums-Ums-Um-Gm-Gm-Am-Gf-Um-Gf- Am-Af-Gm-Am-Gm-Am-Cm-Cf-Am- Am-Gm-Am | 343 | UmsEVP-Cfs-Um-Uf-Gf-Gf-Um-Cm- Um-Cm-Um-Um-Cm-Af-Cm-Uf-Cm- Cm-Am-Am-Ams-Gms-Cm | 473 |
| si074-DV29P | Ums-Gms-Gm-Am-Gm-Um-Gf-Am-Af- Gm-Af-Gm-Am-Cm-Cm-Am-Af-Gm- Am-Um-Am | 344 | UmsEVP-Afs-Um-Cf-Uf-Uf-Gm-Gm- Um-Cm-Um-Cm-Um-Uf-Cm-Af-Cm- Um-Cm-Cm-Ams-Ams-Am | 474 |
| si081-DV29P | Cms-Ams-Am-Gm-Am-Um-Gf-Am-Af- Gm-Uf-Um-Um-Cm-Cm-Cm-Af-Gm- Gm-Cm-Am | 345 | UmsEVP-Gfs-Cm-Cf-Uf-Gf-Gm-Gm- Am-Am-Am-Cm-Um-Uf-Cm-Af-Um- Cm-Um-Um-Gms-Gms-Um | 475 |
| si082-DV29P | Ams-Ams-Gm-Am-Um-Gm-Af-Am-Gf- Um-Uf-Um-Cm-Cm-Cm-Am-Gf-Gm- Cm-Am-Am | 346 | UmsEVP-Ufs-Gm-Cf-Cf-Uf-Gm-Gm- Gm-Am-Am-Am-Cm-Uf-Um-Cf-Am- Um-Cm-Um-Ums-Gms-Gm | 476 |
| si083-DV29P | Cms-Ums-Gm-Gm-Cm-Am-Af-Um-Af- Um-Gf-Am-Cm-Um-Cm-Am-Cf-Um- Um-Gm-Am | 347 | UmsEVP-Cfs-Am-Af-Gf-Uf-Gm-Am- Gm-Um-Cm-Am-Um-Af-Um-Uf-Gm- Cm-Cm-Am-Gms-Gms-Um | 477 |
| si084-DV29P | Gms-Gms-Cm-Am-Am-Um-Af-Um-Gf- Am-Cf-Um-Cm-Am-Cm-Um-Uf-Gm- Am-Cm-Am | 348 | UmsEVP-Gfs-Um-Cf-Af-Af-Gm-Um- Gm-Am-Gm-Um-Cm-Af-Um-Af-Um- Um-Gm-Cm-Cms-Ams-Gm | 478 |
| si085-DV29P | Gms-Cms-Am-Am-Um-Am-Uf-Gm-Af- Cm-Uf-Cm-Am-Cm-Um-Um-Gf-Am- Cm-Cm-Am | 349 | UmsEVP-Gfs-Gm-Uf-Cf-Af-Am-Gm- Um-Gm-Am-Gm-Um-Cf-Am-Uf-Am- Um-Um-Gm-Cms-Cms-Am | 479 |
| si086-DV29P | Cms-Ams-Am-Um-Am-Um-Gf-Am-Cf- Um-Cf-Am-Cm-Um-Um-Gm-Af-Cm- Cm-Cm-Am | 350 | UmsEVP-Gfs-Gm-Gf-Uf-Cf-Am-Am- Gm-Um-Gm-Am-Gm-Uf-Cm-Af-Um- Am-Um-Um-Gms-Cms-Cm | 480 |
| si087-DV29P | Ams-Ums-Am-Um-Gm-Am-Cf-Um-Cf- Am-Cf-Um-Um-Gm-Am-Cm-Cf-Cm- Cm-Um-Am | 351 | UmsEVP-Afs-Gm-Gf-Gf-Gf-Um-Cm- Am-Am-Gm-Um-Gm-Af-Gm-Uf-Cm- Am-Um-Am-Ums-Ums-Gm | 481 |
| si088-DV29P | Ums-Ams-Um-Gm-Am-Cm-Uf-Cm-Af- Cm-Uf-Um-Gm-Am-Cm-Cm-Cf-Cm- Um-Am-Um | 352 | AmsEVP-Ufs-Am-Gf-Gf-Gf-Gm-Um- Cm-Am-Am-Gm-Um-Gf-Am-Gf-Um- Cm-Am-Um-Ams-Ums-Um | 482 |
| si089-DV29P | Ams-Ums-Gm-Am-Cm-Um-Cf-Am-Cf- Um-Uf-Gm-Am-Cm-Cm-Cm-Cf-Um- Am-Um-Am | 353 | UmsEVP-Afs-Um-Af-Gf-Gf-Gm-Gm- Um-Cm-Am-Am-Gm-Uf-Gm-Af-Gm- Um-Cm-Am-Ums-Ams-Um | 483 |
| si090-DV29P | Ams-Gms-Cm-Am-Gm-Gm-Cf-Um-Uf- Gm-Af-Am-Gm-Cm-Am-Gm-Gf-Gm- Um-Am-Am | 354 | UmsEVP-Ufs-Am-Cf-Cf-Cf-Um-Gm- Cm-Um-Um-Cm-Am-Af-Gm-Cf-Cm- Um-Gm-Cm-Ums-Gms-Gm | 484 |
| si091-DV29P | Gms-Cms-Am-Gm-Gm-Cm-Uf-Um-Gf- Am-Af-Gm-Cm-Am-Gm-Gm-Gf-Um- Am-Am-Am | 355 | UmsEVP-Ufs-Um-Af-Cf-Cf-Cm-Um- Gm-Cm-Um-Um-Cm-Af-Am-Gf-Cm- Cm-Um-Gm-Cms-Ums-Gm | 485 |
| si092-DV29P | Gms-Gms-Cm-Um-Um-Gm-Af-Am-Gf- Cm-Af-Gm-Gm-Gm-Um-Am-Af-Gm- Cm-Am-Am | 356 | UmsEVP-Ufs-Gm-Cf-Uf-Uf-Am-Cm- Cm-Cm-Um-Gm-Cm-Uf-Um-Cf-Am- Am-Gm-Cm-Cms-Ums-Gm | 486 |
| si093-DV29P | Gms-Cms-Um-Um-Gm-Am-Af-Gm-Cf- Am-Gf-Gm-Gm-Um-Am-Am-Gf-Cm- Am-Gm-Am | 357 | UmsEVP-Cfs-Um-Gf-Cf-Uf-Um-Am- Cm-Cm-Cm-Um-Gm-Cf-Um-Uf-Cm- Am-Am-Gm-Cms-Cms-Um | 487 |
| si094-DV29P | Cms-Cms-Um-Um-Am-Am-Gf-Gm-Gf- Am-Af-Gm-Gm-Um-Cm-Am-Af-Gm- Am-Gm-Am | 358 | UmsEVP-Cfs-Um-Cf-Uf-Uf-Gm-Am- Cm-Cm-Um-Um-Cm-Cf-Cm-Uf-Um- Am-Am-Gm-Gms-Ums-Am | 488 |
| si095-DV29P | Ums-Cms-Um-Um-Am-Am-Uf-Am-Af- Am-Af-Am-Gm-Gm-Am-Gm-Af-Am- Am-Gm-Am | 359 | UmsEVP-Cfs-Um-Uf-Uf-Cf-Um-Cm- Cm-Um-Um-Um-Um-Uf-Am-Uf-Um- Am-Am-Gm-Ams-Ams-Am | 489 |
| si096-DV29P | Cms-Ums-Um-Am-Am-Um-Af-Am-Af- Am-Af-Gm-Gm-Am-Gm-Am-Af-Am- Gm-Am-Am | 360 | UmsEVP-Ufs-Cm-Uf-Uf-Uf-Cm-Um- Cm-Cm-Um-Um-Um-Uf-Um-Af-Um- Um-Am-Am-Gms-Ams-Am | 490 |
| si097-DV29P | Ums-Ums-Am-Am-Um-Am-Af-Am-Af- Am-Gf-Gm-Am-Gm-Am-Am-Af-Gm- Am-Am-Am | 361 | UmsEVP-Ufs-Um-Cf-Uf-Uf-Um-Cm- Um-Cm-Cm-Um-Um-Uf-Um-Uf-Am- Um-Um-Am-Ams-Gms-Am | 491 |
| si098-DV29P | Ams-Ams-Um-Am-Am-Am-Af-Am-Gf- Gm-Af-Gm-Am-Am-Am-Gm-Af-Am- Am-Am-Um | 362 | AmsEVP-Ufs-Um-Uf-Uf-Cf-Um-Um- Um-Cm-Um-Cm-Cm-Uf-Um-Uf-Um- Um-Am-Um-Ums-Ams-Am | 492 |
| si099-DV29P | Ams-Ums-Am-Am-Am-Am-Af-Gm-Gf- Am-Gf-Am-Am-Am-Gm-Am-Af-Am- Am-Um-Am | 363 | UmsEVP-Afs-Um-Uf-Uf-Uf-Cm-Um- Um-Um-Cm-Um-Cm-Cf-Um-Uf-Um- Um-Um-Am-Ums-Ums-Am | 493 |
| si100-DV29P | Ums-Ams-Am-Am-Am-Am-Gf-Gm-Af- Gm-Af-Am-Am-Gm-Am-Am-Af-Am- Um-Cm-Am | 364 | UmsEVP-Gfs-Am-Uf-Uf-Uf-Um-Cm- Um-Um-Um-Cm-Um-Cf-Cm-Uf-Um- Um-Um-Um-Ams-Ums-Um | 494 |
| si101-DV29P | Ams-Ams-Am-Am-Am-Gm-Gf-Am-Gf- Am-Af-Am-Gm-Am-Am-Am-Af-Um- Cm-Am-Am | 365 | UmsEVP-Ufs-Gm-Af-Uf-Uf-Um-Um- Cm-Um-Um-Um-Cm-Uf-Cm-Cf-Um- Um-Um-Um-Ums-Ams-Um | 495 |
| si102-DV29P | Ams-Ams-Am-Am-Gm-Gm-Af-Gm-Af- Am-Af-Gm-Am-Am-Am-Am-Uf-Cm- Am-Am-Am | 366 | UmsEVP-Ufs-Um-Gf-Af-Uf-Um-Um- Um-Cm-Um-Um-Um-Cf-Um-Cf-Cm- Um-Um-Um-Ums-Ums-Am | 496 |
| si103-DV29P | Ams-Ams-Am-Gm-Gm-Am-Gf-Am-Af- Am-Gf-Am-Am-Am-Am-Um-Cf-Am- Am-Cm-Am | 367 | UmsEVP-Gfs-Um-Uf-Gf-Af-Um-Um- Um-Um-Cm-Um-Um-Uf-Cm-Uf-Cm- Cm-Um-Um-Ums-Ums-Um | 497 |
| si104-DV29P | Ams-Ams-Am-Gm-Um-Cm-Af-Am-Af- Gm-Cf-Um-Am-Um-Um-Um-Uf-Cm- Am-Um-Am | 368 | UmsEVP-Afs-Um-Gf-Af-Af-Am-Am- Um-Am-Gm-Cm-Um-Uf-Um-Gf-Am- Cm-Um-Um-Ums-Gms-Um | 498 |
| si105-DV29P | Ams-Ams-Gm-Cm-Um-Am-Uf-Um-Uf- Um-Cf-Am-Um-Am-Am-Um-Af-Am- Um-Am-Am | 369 | UmsEVP-Ufs-Am-Uf-Uf-Af-Um-Um- Am-Um-Gm-Am-Am-Af-Am-Uf-Am- Gm-Cm-Um-Ums-Ums-Gm | 499 |
| si106-DV29P | Ams-Ums-Am-Am-Um-Am-Cf-Um-Af- Am-Cf-Am-Um-Gm-Um-Um-Af-Um- Um-Um-Am | 370 | UmsEVP-Afs-Am-Af-Uf-Af-Am-Cm- Am-Um-Gm-Um-Um-Af-Gm-Uf-Am- Um-Um-Am-Ums-Ums-Am | 500 |
| si107-DV29P | Ams-Ums-Um-Cm-Um-Cm-Af-Um-Uf- Am-Uf-Cm-Um-Um-Am-Am-Af-Am- Um-Um-Am | 371 | UmsEVP-Afs-Am-Uf-Uf-Uf-Um-Am- Am-Gm-Am-Um-Am-Af-Um-Gf-Am- Gm-Am-Am-Ums-Ums-Cm | 501 |
| si108-DV29P | Ums-Cms-Am-Um-Um-Am-Uf-Cm-Uf- Um-Af-Am-Am-Am-Um-Um-Gf-Um- Am-Um-Um | 372 | AmsEVP-Afs-Um-Af-Cf-Af-Am-Um- Um-Um-Um-Am-Am-Gf-Am-Uf-Am- Am-Um-Gm-Ams-Gms-Am | 502 |
| si0481-DV29SP | Cms-Ams-Am-Am-Gm-Cf-Um-Af-Um- Uf-Um-Um-Cm-Am-Um-Af-Am-Um- Am-Am | 373 | UmsEVP-Ufs-Am-Uf-Uf-Af-Um-Gm- Am-Am-Am-Am-Um-Af-Gm-Cf-Um- Um-Um-Gms-Ams-Cm | 503 |
| siA029-DV29P | Ums-Gms-Gm-Cm-Am-Am-Uf-Am-Uf- Gm-Af-Cm-Um-Cm-Am-Cm-Uf-Um- Gm-Am-Am | 374 | UmsEVP-Ufs-Cm-Af-Af-Gf-Um-Gm- Am-Gm-Um-Cm-Am-Uf-Am-Uf-Um- Gm-Cm-Cm-Ams-Gms-Gm | 504 |
| siA036-DV29P | Cms-Ums-Um-Am-Am-Gm-Gf-Gm-Af- Am-Gf-Gm-Um-Cm-Am-Am-Gf-Am- Gm-Gm-Am | 375 | UmsEVP-Cfs-Cm-Uf-Cf-Uf-Um-Gm- Am-Cm-Cm-Um-Um-Cf-Cm-Cf-Um- Um-Am-Am-Gms-Gms-Um | 505 |
| siA043-DV29P | Ums-Ums-Am-Um-Am-Cm-Uf-Um-Uf- Cm-Uf-Um-Am-Am-Um-Am-Af-Am- Am-Am-Am | 377 | UmsEVP-Ufs-Um-Uf-Uf-Uf-Am-Um- Um-Am-Am-Gm-Am-Af-Am-Gf-Um- Am-Um-Am-Ams-Gms-Cm | 507 |
| siA045-DV29P | Ums-Ams-Am-Um-Am-Am-Af-Am-Af- Gm-Gf-Am-Gm-Am-Am-Am-Gf-Am- Am-Am-Am | 378 | UmsEVP-Ufs-Um-Uf-Cf-Uf-Um-Um- Cm-Um-Cm-Cm-Um-Uf-Um-Uf-Um- Am-Um-Um-Ams-Ams-Gm | 508 |
| siA048-DV29P | Ums-Cms-Am-Am-Am-Gm-Cf-Um-Af- Um-Uf-Um-Um-Cm-Am-Um-Af-Am- Um-Am-Am | 379 | UmsEVP-Ufs-Am-Uf-Uf-Af-Um-Gm- Am-Am-Am-Am-Um-Af-Gm-Cf-Um- Um-Um-Gm-Ams-Cms-Um | 509 |
| si754-DV29SP | Gms-Ams-Gm-Um-Gm-Af-Am-Gf-Am- Gf-Am-Cm-Cm-Am-Am-Gf-Am-Um- Gm-Am | 382 | UmsEVP-Cfs-Am-Uf-Cf-Uf-Um-Gm- Gm-Um-Cm-Um-Cm-Uf-Um-Cf-Am- Cm-Um-Cms-Cms-Am | 512 |
| si755-DV29SP | Gms-Ums-Gm-Am-Am-Gf-Am-Gf-Am- Cf-Cm-Am-Am-Gm-Am-Uf-Gm-Am- Am-Am | 383 | UmsEVP-Ufs-Um-Cf-Af-Uf-Cm-Um- Um-Gm-Gm-Um-Cm-Uf-Cm-Uf-Um- Cm-Am-Cms-Ums-Cm | 513 |
| si756-DV29SP | Ams-Ams-Gm-Am-Gm-Af-Cm-Cf-Am- Af-Gm-Am-Um-Gm-Am-Af-Gm-Um- Um-Um | 384 | AmsEVP-Afs-Am-Cf-Uf-Uf-Cm-Am- Um-Cm-Um-Um-Gm-Gf-Um-Cf-Um- Cm-Um-Ums-Cms-Am | 514 |
| si792-DV29SP | Ams-Gms-Am-Cm-Cm-Af-Am-Gf-Am- Uf-Gm-Am-Am-Gm-Um-Uf-Um-Cm- Cm-Am | 385 | UmsEVP-Gfs-Gm-Af-Af-Af-Cm-Um- Um-Cm-Am-Um-Cm-Uf-Um-Gf-Gm- Um-Cm-Ums-Cms-Um | 515 |
| si141-DV29SP | Gms-Cms-Um-Am-Um-Uf-Um-Uf-Cm- Af-Um-Am-Am-Um-Am-Af-Um-Am- Cm-Um | 386 | AmsEVP-Gfs-Um-Af-Uf-Uf-Am-Um- Um-Am-Um-Gm-Am-Af-Am-Af-Um- Am-Gm-Cms-Ums-Um | 516 |
| si761-DV29SP | Ams-Gms-Um-Gm-Am-Af-Gm-Af-Gm- Af-Cm-Cm-Am-Am-Gm-Af-Um-Gm- Am-Am | 387 | UmsEVP-Ufs-Cm-Af-Uf-Cf-Um-Um- Gm-Gm-Um-Cm-Um-Cf-Um-Uf-Cm- Am-Cm-Ums-Cms-Cm | 517 |
| si771-DV29SP | Ums-Gms-Am-Am-Gm-Af-Gm-Af-Cm- Cf-Am-Am-Gm-Am-Um-Gf-Am-Am- Gm-Um | 388 | AmsEVP-Cfs-Um-Uf-Cf-Af-Um-Cm- Um-Um-Gm-Gm-Um-Cf-Um-Cf-Um- Um-Cm-Ams-Cms-Um | 518 |
| si781-DV29SP | Ams-Gms-Am-Gm-Am-Cf-Cm-Af-Am- Gf-Am-Um-Gm-Am-Am-Gf-Um-Um- Um-Am | 389 | UmsEVP-Afs-Am-Af-Cf-Uf-Um-Cm- Am-Um-Cm-Um-Um-Gf-Gm-Uf-Cm- Um-Cm-Ums-Ums-Cm | 519 |
| si801-DV29SP | Ams-Cms-Cm-Am-Am-Gf-Am-Uf-Gm- Af-Am-Gm-Um-Um-Um-Cf-Cm-Cm- Am-Am | 390 | UmsEVP-Ufs-Gm-Gf-Gf-Af-Am-Am- Cm-Um-Um-Cm-Am-Uf-Cm-Uf-Um- Gm-Gm-Ums-Cms-Um | 520 |

Instruments and reagents: 192 P DNA/RNA automatic synthesizer (Tsingke), using a universal solid-phase support consisting of cross-linked polystyrene beads; model: Primer Support 5G Unylinker 350 (manufactured by Cytiva).

### Preparation method:

Nucleotide monomers were dissolved in acetonitrile at a concentration of 0.15 M, including DMT-A-OMe phosphoramidite monomer (Formula 5), DMT-C-OMe phosphoramidite monomer (Formula 6), DMT-G-OMe phosphoramidite monomer (Formula 7), DMT-U-OMe phosphoramidite monomer (Formula 8), DMT-A-F phosphoramidite monomer (Formula 9), DMT-C-F phosphoramidite monomer (Formula 10), DMT-G-F phosphoramidite monomer (Formula 11), and DMT-U-F phosphoramidite monomer (Formula 12).

The base at the 5' end of the antisense strand (the last base) was synthesized using a monomer containing a phosphonate group at the 5' end, such as vinyl-(E)-phosphonate-A-OMe phosphoramidite monomer (Formula 13) or vinyl-(E)-phosphonate-U-OMe phosphoramidite monomer (Formula 14).

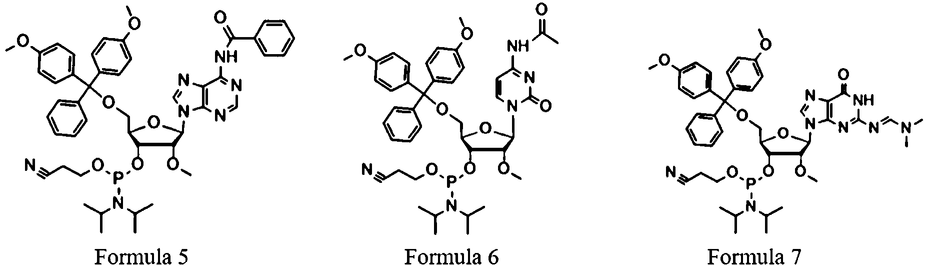

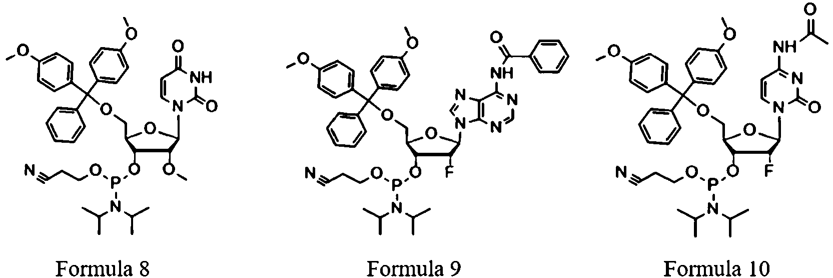

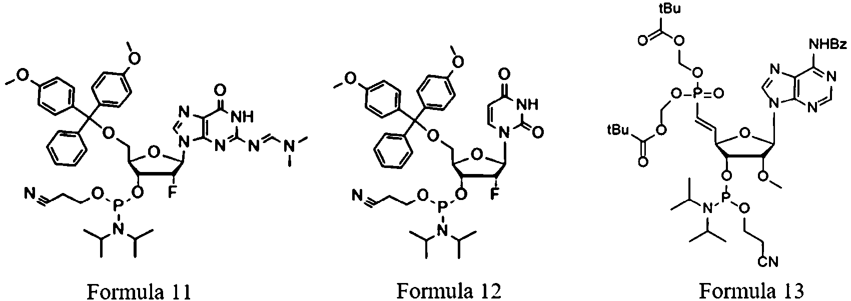

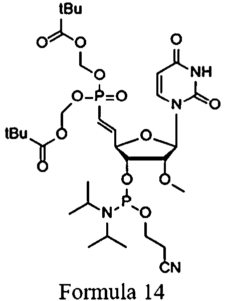

Nucleotide monomers were sequentially attached in the 3' to 5' direction according to the nucleotide arrangement using the solid-phase phosphoramidite method. The process of attaching each nucleotide monomer involves four steps: deprotection, coupling, oxidation or sulfurization, and hydroxyl protection. Specifically, when two nucleotides are linked via a phosphate linkage, the process of attaching a subsequent nucleotide monomer involves four steps: deprotection, coupling, oxidation, and hydroxyl protection. When two nucleotides are linked via a phosphorothioate linkage, the process of attaching a subsequent nucleotide monomer involves four steps: deprotection, coupling, sulfurization, and hydroxyl protection.

The preparation was carried out according to the following steps:
The solid-phase support was loaded into the designated position of the synthesizer, followed by multiple synthesis cycles to obtain the desired product, each cycle consisting of (1) deprotection, (2) coupling, (3) oxidation/sulfurization, and (4) hydroxyl protection. The cycle process and reagents used are described as follows:

### (1) Deprotection

3% dichloroacetic acid in toluene was used as a deprotection reagent to remove the DMT protecting group, followed by washing with acetonitrile.

### (2) Coupling

Each nucleotide monomer in acetonitrile was coupled using 0.25 M 5-ethylthio-1H-tetrazole as an activator, followed by washing with acetonitrile.

### (3) Oxidation/sulfurization

Oxidation: 0.05 M iodine in pyridine/water (90/10) was used as an oxidant for oxidation, followed by washing with acetonitrile.

Sulfurization: 3% xanthane hydride in pyridine was used as a sulfurizing agent for sulfurization, followed by washing with acetonitrile.

### (4) Hydroxyl protection

10% acetic anhydride in tetrahydrofuran (CAP A) and tetrahydrofuran/pyridine/N-methylimidazole (74/10/16, v/v/v) (CAP B) were used as hydroxyl protecting reagents for hydroxyl protection, followed by washing with acetonitrile.

The aforementioned steps were repeated according to the specified nucleotide arrangement to obtain a product with the specific sequence arrangement in either the sense strand or the antisense strand.

(5) 3% dichloroacetic acid in toluene was used as a deprotection reagent to remove the DMT protecting group from the last nucleotide, followed by washing with acetonitrile.

### (6) Ammonolysis and purification

The solid-phase support was transferred to a reactor and treated with concentrated aqueous ammonia (25 to 28% w/w) at 60°C for 12 hours. The system was then cooled to room temperature, and the mixture was transferred to a pressure filter. The filter cake was washed with a mixture of purified water and ethanol. The filtrates were combined, subjected to column chromatography, concentrated, and lyophilized to obtain a product.

### (7) Annealing

The purified sense and antisense strands were mixed at a molar ratio of 1:1, heated to 95°C and maintained for 3 minutes, and slowly cooled to room temperature to form a duplex.

### 2.2 Inhibition of INHBE gene expression

The DV29P-modified siRNA derivatives synthesized in Section 2.1 were transfected into Huh7 cells via lipid nanoparticles (LNPs). The inhibitory effects on INHBE gene expression were then evaluated by qPCR.

### (1) Experimental materials

Test samples: siRNA derivatives listed in Section 2.1.

Cell line: Huh7 cells.

Solvents: sterile nuclease-free water, Gibco Opti-MEM.

### (2) Experimental methods

The inhibitory effects of the test samples on INHBE mRNA expression in Huh7 cells were evaluated by qRT-PCR.

### (2.1) Cell culture

Subcultured Huh7 cells in the logarithmic growth phase were cultured in DMEM culture medium containing 10% fetal bovine serum (supplemented with 100× penicillin and 100× streptomycin at 10 µL/mL), and incubated in a cell incubator at 37°C with 5% CO₂. The medium was exchanged once daily. The cells were digested with 0.25% trypsin, followed by centrifugation at 800 rpm for 3 minutes. The supernatant was discarded, and fresh medium was added for continued culture.

### (2.2) Cell transfection

Preparation of transfection mixture: Lipofectamine RNAiMAX (Invitrogen, 13778150) was mixed with Opti-MEM at a volume ratio of 2:98 and vortexed thoroughly.

siRNA dilution: The siRNA samples were diluted to appropriate concentrations using Opti-MEM to prepare siRNA solutions to be tested.

Preparation of transfection reagent: 65 µL of siRNA solution (0.84 nM or 0.36 nM, diluted in Opti-MEM) was mixed with 65 µL of transfection mixture at a volume ratio of 1:1, vortexed thoroughly, followed by incubation at room temperature for 15 minutes to obtain lipid nanoparticles (LNPs). A 12.5 µL aliquot was taken for encapsulation efficiency analysis.

Blank control (transfection reagent): 65 µL of transfection mixture was added to 65 µL of Opti-MEM, vortexed thoroughly, followed by incubation at room temperature for 15 minutes.

The prepared transfection reagents with different siRNA concentrations were added at 100 µL per well to a 24-well cell culture plate pre-filled with 500 µL of cell suspension per well (containing 1.0 × 10⁵ cells per mL), resulting in a final siRNA concentration of 0.07 nM or 0.03 nM per well. After cross-mixing, the plate was incubated in a cell incubator at 37°C with 5% CO₂ for 40 hours.

### (2.3) INHBE mRNA analysis

### 1) RNA extraction

a. The culture medium was removed from a 12-well plate. 0.5 mL of 1× PBS was then added to each well to wash the cells, and the PBS was discarded. 0.5 mL of TRIzol reagent was added to each well, followed by pipetting to ensure complete lysis of the cells. The lysate was transferred to a 1.5 mL RNase-free EP tube, followed by incubation at room temperature for 5 minutes.
b. 0.1 mL of chloroform was added to each tube, shaken vigorously for 15 seconds, followed by incubation at room temperature for 5 minutes. After centrifugation at 12000 × g for 15 minutes at 4°C, 200 µL of the supernatant was transferred to a new EP tube.
c. An equal volume of isopropanol was added to the tube, which was gently mixed by inversion, followed by incubation at -20°C for 10 minutes. After centrifugation at 12000 × g for 15 minutes at 4°C, the supernatant was discarded.
d. 0.5 mL of 75% ethanol was added to gently wash the RNA pellet, followed by centrifugation at 12000 × g for 5 minutes at 4°C, and the supernatant was discarded. The washing step was repeated once, followed by centrifugation at 12000 × g for 1 minute at 4°C, and residual ethanol was thoroughly removed using a micropipette tip.
e. The residual ethanol was air-dried at room temperature for 2 to 3 minutes, followed by addition of 40 µL of RNase-free ddH₂O for dissolution.

### 2) Measurement of RNA concentration

RNA concentration was measured using a NanoDrop spectrophotometer, using 2 µL of RNase-free ddH₂O as a blank control and 2 µL of RNA sample for each measurement. The sample concentration was recorded.

### 3) Reverse transcription of INHBE mRNA

Reverse transcription reagent (Takara, RR092A), RNA solution, and water were mixed at a volume ratio of 2:5:3, followed by incubation in a PCR instrument at 37°C for 15 minutes and 85°C for 5 seconds, and finally held at 4°C. The resulting cDNA was diluted 5-fold with sterile nuclease-free water.

### 4) Quantitative analysis of INHBE mRNA

In a 15 mL centrifuge tube, qPCR reagent (Takara, RR420W), forward primer, and reverse primer were mixed at a volume ratio of 5:0.1:0.1 to obtain solution A.

The primer sequences are as follows:
hGAPDH-F: 5'-CTGGGCTACACTGAGCACC-3' (SEQ ID NO: 521)
hGAPDH-R: 5'-AAGTGTCGTTGAGGGCAATG-3' (SEQ ID NO: 522)
INHBE-F: 5'-TGTGTCCCTACTGCCCGAA-3' (SEQ ID NO: 523)
INHBE-R: 5'-CCATATCTGGCACATCCGTCTT-3' (SEQ ID NO: 524)

In a labeled 1.5 mL EP tube, diluted cDNA and water were mixed at a volume ratio of 1:3.8 to obtain solution B.

5.2 µL of solution A and 4.8 µL of solution B were added to each well of a 96-well PCR plate, which was sealed, then centrifuged at 3000 rpm for 1 minute, and subjected to instrumental analysis.
*The step was performed on ice under low-temperature conditions.

The plate was placed into the qPCR instrument and run according to the following program:
Pre-denaturation: 95°C for 30 seconds;
Cycling: 95°C for 5 seconds; 60°C for 34 seconds; 40 cycles;
Melting curve: 95°C for 15 seconds; 60°C for 60 seconds; 95°C for 15 seconds.

Total run time: approximately 2 hours. After analysis of the experimental results, 2^{-ΔΔCt} was calculated using the following formula:$Δ Ct = mean Ct value of target gene INHBE - mean Ct value of reference gene hGAPDH ;$$ΔΔ CT = Δ CT \left(test sample group\right) - Δ CT \left(RNAiMAX control group\right) ;$
Relative mRNA expression of the target gene = 2^{-ΔΔCt};
wherein the mean Ct value was automatically calculated using Quant Studio 5 software under default settings;
the RNAiMAX control group refers to samples without siRNA, as opposed to the test sample group.

### (2.4) Calculation of inhibition rate

INHBE mRNA expression rate (%) was calculated using the following formula:
$Expression rate = relative mRNA expression of target gene INHBE \times 100 % ;$
$Inhibition rate of INHBE gene expression \left(%\right) = 100 % - expression rate .$

### (3) Experimental results

In this example, the inhibition rate represents the average of three independent experiments. The inhibition rates of INHBE mRNA expression in Huh7 cells by these sequences are shown in Table 6.

The experimental results demonstrated that certain sequences modified using the template DV29P or DV29SP exhibited varying degrees of inhibitory effects on INHBE mRNA expression in Huh7 cells. Specifically, si079-DV29SP, si791-DV29P, si075-DV29P, and si751-DV29P exhibited inhibition rates exceeding 60%.

**Table 6**

| siRNA ID | Inhibition rate (%) | | siRNA ID | Inhibition rate (%) | | siRNA ID | Inhibition rate (%) | |
|---|---|---|---|---|---|---|---|---|
| | 0.03 nM | 0.07 nM | | 0.03 nM | 0.07 nM | | 0.03 nM | 0.07 nM |
| si079-DV29SP | 70.8 | 74.5 | si023-DV29P | 31.4 | 38.5 | si087-DV29P | 11.7 | 18.5 |
| si791-DV29P | 68.9 | 70.5 | si059-DV29P | 30.7 | 38.3 | si093-DV29P | 11.5 | 19.8 |
| si075-DV29P | 62.6 | 69.1 | si032-DV29P | 30.4 | 34.4 | si041-DV29SP | 11.4 | 18.1 |
| si751-DV29P | 62 | 69 | si013-DV29P | 28.5 | 29.9 | si054-DV29P | 11.3 | 15.5 |
| si752-DV29SP | 57.4 | 80.4 | si008-DV29P | 28.4 | 35.9 | si106-DV29P | 10.7 | 20.3 |
| si756-DV29SP | 55.9 | 61.1 | si006-DV29SP | 28.2 | 31.6 | si069-DV29P | 10.5 | 12.2 |
| si753-DV29P | 55.4 | 64.7 | si090-DV29P | 26.8 | 67.1 | si091-DV29P | 10.4 | 13.1 |
| si062-DV29P | 54.7 | 60.3 | si073-DV29P | 25.9 | 26.9 | si092-DV29P | 9.8 | 15.3 |
| si049-DV29SP | 54.6 | 63.7 | si005-DV29SP | 25.9 | 26.3 | si100-DV29P | 9.6 | 19.2 |
| si014-DV29P | 53.2 | 59.1 | si057-DV29P | 25.6 | 26.5 | si060-DV29P | 8.5 | 25.7 |
| siA043-DV29P | 52.9 | 54.4 | si021-DV29P | 25.2 | 29.7 | si040-DV29SP | 8.5 | 14.1 |
| si141-DV29SP | 52.6 | 55.3 | si082-DV29P | 24.5 | 33.2 | si063-DV29P | 7.8 | 16.1 |
| si076-DV29P | 51.5 | 64.4 | si070-DV29P | 24.5 | 28.3 | si103-DV29P | 6.7 | 16.1 |
| si080-DV29P | 51.5 | 59.8 | si007-DV29P | 24.4 | 33.8 | si027-DV29SP | 6.6 | 11.7 |
| si009-DV29P | 51.2 | 56.8 | si058-DV29SP | 24.2 | 25.8 | si038-DV29P | 6.5 | 16.5 |
| si078-DV29P | 50.4 | 50.9 | si031-DV29SP | 23 | 26.1 | siA036-DV29P | 5.9 | 10.7 |
| si754-DV29SP | 50.4 | 54.1 | si105-DV29P | 22.9 | 32.6 | si095-DV29P | 5.1 | 13.3 |
| si047-DV29SP | 50.1 | 51.1 | si024-DV29SP | 22.7 | 31.3 | si034-DV29P | 4.7 | 6 |
| si771-DV29SP | 48.8 | 57.7 | si026-DV29P | 22.5 | 28.2 | si030-DV29SP | 4.5 | 16.3 |
| si761-DV29SP | 48.7 | 53.7 | si099-DV29P | 22.4 | 36.5 | si098-DV29P | 3.4 | 13.2 |
| si801-DV29SP | 48.3 | 50.8 | si072-DV29P | 22.2 | 32 | si107-DV29P | 2.3 | 7.2 |
| si781-DV29SP | 47.8 | 56.1 | si071-DV29P | 21.8 | 28.8 | si065-DV29P | 1 | 4.9 |
| si792-DV29SP | 47.3 | 60.6 | siA029-DV29P | 21.7 | 25.4 | si094-DV29P | 0 | 3 |
| si104-DV29P | 47.2 | 52 | si017-DV29P | 20.8 | 36.8 | si019-DV29P | -0.7 | 0.4 |
| siA045-DV29P | 45.6 | 48.3 | si028-DV29P | 20.7 | 34.2 | si029-DV29P | -1.4 | 8.5 |
| si077-DV29P | 45.3 | 54.9 | si051-DV29SP | 20.6 | 22.5 | si085-DV29P | -1.5 | 3.1 |
| si755-DV29SP | 44.9 | 46.8 | si056-DV29SP | 19.9 | 25.1 | si039-DV29P | -3.2 | 3.5 |
| siA048-DV29P | 44.7 | 62.2 | si055-DV29P | 19.5 | 19.6 | si002-DV29P | -4.1 | 4.6 |
| si012-DV29P | 43.8 | 49.8 | si061-DV29P | 19.4 | 22.4 | si086-DV29P | -4.3 | -2.4 |
| si046-DV29SP | 43.4 | 64.5 | si068-DV29P | 19.2 | 23.2 | si018-DV29P | -4.4 | 3.9 |
| si101-DV29P | 42.2 | 49.1 | si053-DV29P | 17.1 | 26.1 | si066-DV29SP | -4.6 | 4.4 |
| si067-DV29SP | 41.7 | 45.8 | si036-DV29P | 16.6 | 36.4 | si064-DV29P | -4.8 | 1.7 |
| si011-DV29SP | 41.6 | 65 | si089-DV29P | 16.3 | 24.2 | si096-DV29P | -8.5 | 0.2 |
| si102-DV29P | 38.8 | 39.3 | si083-DV29P | 16.2 | 24.6 | si084-DV29P | -9.5 | 4.7 |
| si020-DV29SP | 38.8 | 43.4 | si001-DV29P | 16.1 | 19 | si097-DV29P | -12.1 | -8.7 |
| si048-DV29SP | 38.6 | 41.8 | si081-DV29P | 15.7 | 16 | si0481-DV29SP | -12.1 | -8.5 |
| si088-DV29P | 37.8 | 42 | si035-DV29P | 15.6 | 23.4 | si043-DV29SP | -17.3 | -11.7 |
| si010-DV29SP | 37 | 43 | si022-DV29P | 14.4 | 21.3 | si003-DV29P | -24.7 | -15.9 |
| si050-DV29P | 36.5 | 43 | si004-DV29P | 14.3 | 17.8 | si108-DV29P | -25.4 | -16 |
| si052-DV29P | 35.8 | 42.8 | si025-DV29P | 14.1 | 19.5 | si016-DV29P | -34.9 | -33 |
| si033-DV29SP | 34.7 | 37.9 | si044-DV29P | 13.5 | 15.2 | si037-DV29P | -52.3 | -47 |
| si074-DV29P | 33.2 | 41.2 | si042-DV29SP | 12.7 | 18 | | | |
| si015-DV29SP | 31.6 | 41.6 | si045-DV29SP | 12.5 | 13.4 | | | |

### (4) siRNAs with similar sequences may vary greatly in activity.

**Table7: Comparison of inhibition rates of INHBE by siRNA derivatives with similar base sequences**

| siRNA ID | Sense strand base sequence (5'-3') | Antisense strand base sequence (5'-3') | Inhibition rate (%) | Difference in inhibition rate (%) |
|---|---|---|---|---|
| si008-DV29P | CAAAGUCAAAGCUAUUU UCAU | AUGAAAAUAGCUUUGACUU UGUG | 28.4 | 22.8 |
| si009-DV29P | AAGUCAAAGCUAUUUC AUAA | UUAUGAAAUAGCUUUGAC UUUG | 51.2 | |
| si012-DV29P | CAAAGCUAUUUCAUAA UAAU | AUUAUUAUGAAAAUAGCUU UGAC | 43.8 | 15.3 |
| si013-DV29P | AAAGCUAUUUCAUAA UAAUA | UAUUAUUAUGAAAUAGCU UUGA | 28.5 | |
| si020-DV29SP | AUAAUAAUACUAACAU GUUA | UAACAUGUUAGUAUUAUUA UGA | 38.8 | 13.6 |
| si021-DV29P | UAAUAAUACUAACAUG UUAUU | AAUAACAUGUUAGUAUUAU UAUG | 25.2 | |
| si028-DV29P | UAUUUGCCUUUGAAU UCUCA | UGAGAAUUCAAAAGGCAAA UAAC | 62.0 | 63.4 |
| si029-DV29P | UUUGCCUUUUGAAUUCU CAUU | AAUGAGAAUUCAAAAGGCA AAUA | -1.4 | |
| si033-DV29SP | GAAUUCUCAUUAUCUUA AAA | UUUUAAGAUAAUGAGAAUU CAA | 34.7 | 30.0 |
| si034-DV29P | AAUUCUCAUUAUCUUAA AAUU | AAUUUUAAGAUAAUGAGAA UUCA | 4.7 | |
| si045-DV29SP | GGGAGACAAGCAUUUA UACU | AGUAUAAAUGCUUGUCUCC CAG | 12.5 | 30.9 |
| si046-DV29SP | GGAGACAAGCAUUUAU ACUU | AAGUAUAAAUGCUUGUCUC CCA | 43.4 | |

As shown in Table 7, certain siRNAs with similar sequences exhibited vastly different inhibitory effects on INHBE mRNA expression.

At a concentration of 0.03 nM:
si028-DV29P differs from si029-DV29P by only two terminal nucleotides, but the inhibition rate of si029-DV29P was 63.4% higher than that of si028-DV29P.
si033-DV29SP differs from si034-DV29P by only two terminal nucleotides, but the inhibition rate of si033-DV29P was 30.0% higher than that of si034-DV29P.
si045-DV29P differs from si046-DV29P by only one terminal nucleotide, but the inhibition rate of si045-DV29P was 30.9% lower than that of si046-DV29P.

### Example 3: Inhibition of INHBE gene expression by template-modified siRNA derivatives

In this example, the motifs described in Example 1 were modified using multiple modification templates, including DV25P, DV26P, DV27P, DV29P, DV32P, DV34P, DV25SP, DV26SP, DV27SP, DV29SP, DV32SP, DV34SP, DV41P to DV43P, or DV41SP to DV43SP.

Natural 5' phosphorylation or simple direct 5' phosphorylation may undergo dephosphorylation in cells. Directly 5'-phosphorylated oligonucleotide strands may undergo 90% dephosphorylation after 2 hours of circulation in blood and complete loss after 24 hours. A 5'-E-VP phosphorylation design was adopted, in which E-vinylphosphonate replaces the bridging oxygen, thereby improving phosphorylation and stability. The antisense strands of the modification templates DV25P to DV27P, DV29P, DV32P, DV34P, DV41P to DV43P, DV25SP to DV27SP, DV29SP, DV32SP, DV34SP, and DV41SP to DV43SP disclosed herein all contain a 5'-vinylphosphonate group.

### 1. Experimental materials

### Test samples:

The sequences listed in Table 12 were obtained by modifying the core sequences si075, si751, si753, si791, si014, si076, si077, si078, si080, si752, si079, si047, si754, si755, si756, si792, si141, si761, si771, si781, and si801 from Example 1 using different templates (DV25P, DV26P, DV27P, DV32P, DV34P, DV41P, DV42P, DV43P, DV22, DV25SP, DV26SP, DV27SP, DV32SP, DV34SP, DV41SP, DV42SP, DV43SP, and DV22S).

Cell line: Huh7 cells.

Solvents: sterile nuclease-free water, Gibco Opti-MEM.

Sequence synthesis: siRNA sequences were synthesized according to the method described in Example 1.

The modification rules for the templates disclosed herein are as follows:
(1) For compounds in which the sense and antisense strands are 23 and 21 nucleotides in length, respectively, the antisense strand adopts any one of the modification patterns shown in Table 8 below:

**Table 8: Modification of antisense strand**

| No. | **Antisense strand (AS) 5'-3'** | | | | | | | | | | | |
|---|---|---|---|---|---|---|---|---|---|---|---|---|
| **A** | **1** | **2** | **3** | **4** | **5** | **6** | **7** | **8** | **9** | **10** | **11** | **12** |
| | 2'-OMe +PS +EVP | **2'-F +PS** | 2'-OMe | 2'-OMe | 2'-OMe | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **B** | **1** | **2** | **3** | **4** | **5** | **6** | **7** | **8** | **9** | **10** | **11** | **12** |
| | 2'-OMe +PS +EVP | **2'-F +PS** | **2'-F** | **2'-F** | 2'-OMe | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **C** | **1** | **2** | **3** | **4** | **5** | **6** | **7** | **8** | **9** | **10** | **11** | **12** |
| | 2'-OMe +PS +EVP | **2'-F +PS** | 2'-OMe | **2'-F** | **2'-F** | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **D** | **1** | **2** | **3** | **4** | **5** | **6** | **7** | **8** | **9** | **10** | **11** | **12** |
| | 2'-OMe +PS +EVP | **2'-F +PS** | 2'-OMe | 2'-OMe | **2'-F** | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **E** | **1** | **2** | **3** | **4** | **5** | **6** | **7** | **8** | **9** | **10** | **11** | **12** |
| | 2'-OMe +PS +EVP | **2'-F +PS** | **2'-F** | 2'-OMe | 2'-OMe | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **F** | **1** | **2** | **3** | **4** | **5** | **6** | **7** | **8** | **9** | **10** | **11** | **12** |
| | 2'-OMe +PS +EVP | **2'-F +PS** | 2'-OMe | 2'-OMe | 2'-OMe | **2'-F** | 2'-OMe | **2'-F** | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **G** | **1** | **2** | **3** | **4** | **5** | **6** | **7** | **8** | **9** | **10** | **11** | **12** |
| | 2'-OMe +PS +EVP | **2'-F +PS** | 2'-OMe | **2'-F** | **2'-F** | **2'-F** | 2'-OMe | **2'-F** | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **H** | **1** | **2** | **3** | **4** | **5** | **6** | **7** | **8** | **9** | **10** | **11** | **12** |
| | 2'-OMe +PS +EVP | **2'-F +PS** | **2'-F** | 2'-OMe | 2'-OMe | **2'-F** | 2'-OMe | **2'-F** | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **I** | **1** | **2** | **3** | **4** | **5** | **6** | **7** | **8** | **9** | **10** | **11** | **12** |
| | 2'-OMe +PS | **2'-F +PS** | 2'-OMe | 2'-OMe | 2'-OMe | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |

The sense strand adopts any one of the modification patterns shown in Table 9 below:

**Table 9: Modification of sense strand**

| No. | **Sense strand (SS) 5'-3'** | | | | | | | | | | |
|---|---|---|---|---|---|---|---|---|---|---|---|
| **a** | **1** | **2** | **3** | **4** | **5** | **6** | **7** | **8** | **9** | **10** | **11** |
| | 2'-OMe +PS | 2'-OMe +PS | 2'-OMe | 2'-OMe | 2'-OMe | 2'-OMe | **2'-F** | 2'-OMe | **2'-F** | **2'-F** | **2'-F** |
| | **12** | **13** | **14** | **15** | **16** | **17** | **18** | **19** | **20** | **21** | |
| | 2'-OMe | 2'-OMe | 2'-OMe | 2'-OMe | 2'-OMe | **2'-F** | 2'-OMe | 2'-OMe | 2'-OMe | 2'-OMe | |
| **b** | **1** | **2** | **3** | **4** | **5** | **6** | **7** | **8** | **9** | **10** | **11** |
| | 2'-OMe +PS | 2'-OMe +PS | 2'-OMe | 2'-OMe | 2'-OMe | 2'-OMe | **2'-F** | 2'-OMe | **2'-F** | 2'-OMe | **2'-F** |
| | **12** | **13** | **14** | **15** | **16** | **17** | **18** | **19** | **20** | **21** | |
| | 2'-OMe | 2'-OMe | 2'-OMe | 2'-OMe | 2'-OMe | **2'-F** | 2'-OMe | 2'-OMe | 2'-OMe | 2'-OMe | |
| **c** | **1** | **2** | **3** | **4** | **5** | **6** | **7** | **8** | **9** | **10** | **11** |
| | 2'-OMe +PS | 2'-OMe +PS | 2'-OMe | 2'-OMe | 2'-OMe | 2'-OMe | **2'-F** | 2'-OMe | **2'-F** | **2'-F** | **2'-F** |
| | **12** | **13** | **14** | **15** | **16** | **17** | **18** | **19** | **20** | **21** | |
| | 2'-OMe | 2'-OMe | 2'-OMe | 2'-OMe | 2'-OMe | 2'-OMe | 2'-OMe | 2'-OMe | 2'-OMe | 2'-OMe | |

wherein 2'-OMe represents a 2'-O-methyl modification; 2'-F represents a 2'-fluoro modification; PS indicates that, starting from the 5' end, the nucleotide at the position is linked to the adjacent nucleotide on the right via a 3',5'-phosphorothioate bond; EVP represents a 5'-vinyl-(E)-phosphonate.

The siRNA modification template, in which the antisense strand was modified according to modification pattern A and the sense strand was modified according to modification pattern a, was designated as DV25P.

The siRNA modification template, in which the antisense strand was modified according to modification pattern B and the sense strand was modified according to modification pattern a, was designated as DV26P.

The siRNA modification template, in which the antisense strand was modified according to modification pattern C and the sense strand was modified according to modification pattern a, was designated as DV27P.

The siRNA modification template, in which the antisense strand was modified according to modification pattern C and the sense strand was modified according to modification pattern b, was designated as DV29P.

The siRNA modification template, in which the antisense strand was modified according to modification pattern D and the sense strand was modified according to modification pattern b, was designated as DV32P.

The siRNA modification template, in which the antisense strand was modified according to modification pattern E and the sense strand was modified according to modification pattern b, was designated as DV34P.

The siRNA modification template, in which the antisense strand was modified according to modification pattern F and the sense strand was modified according to modification pattern b, was designated as DV41P.

The siRNA modification template, in which the antisense strand was modified according to modification pattern G and the sense strand was modified according to modification pattern b, was designated as DV42P.

The siRNA modification template, in which the antisense strand was modified according to modification pattern H and the sense strand was modified according to modification pattern b, was designated as DV43P.

The siRNA modification template, in which the antisense strand was modified according to modification pattern I and the sense strand was modified according to modification pattern c, was designated as DV22.

(2) For compounds in which the sense and antisense strands are 20 and 22 nucleotides in length, respectively, the antisense strand adopts any one of the modification patterns shown in Table 10 below:

**Table 10: Modification of antisense strand**

| No. | **Antisense strand (AS) 5'-3'** | | | | | | | | | | |
|---|---|---|---|---|---|---|---|---|---|---|---|
| **A'** | **1** | **2** | **3** | **4** | **5** | **6** | **7** | **8** | **9** | **10** | **11** |
| | 2'-OMe +PS +EVP | **2'-F +PS** | 2'-OMe | 2'-OMe | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **B'** | **1** | **2** | **3** | **4** | **5** | **6** | **7** | **8** | **9** | **10** | **11** |
| | 2'-OMe +PS +EVP | **2'-F +PS** | **2'-F** | **2'-F** | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **C'** | **1** | **2** | **3** | **4** | **5** | **6** | **7** | **8** | **9** | **10** | **11** |
| | 2'-OMe +PS +EVP | **2'-F +PS** | 2'-OMe | **2'-F** | **2'-F** | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **D'** | **1** | **2** | **3** | **4** | **5** | **6** | **7** | **8** | **9** | **10** | **11** |
| | 2'-OMe +PS +EVP | **2'-F +PS** | 2'-OMe | 2'-OMe | **2'-F** | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **E'** | **1** | **2** | **3** | **4** | **5** | **6** | **7** | **8** | **9** | **10** | **11** |
| | 2'-OMe +PS +EVP | **2'-F +PS** | **2'-F** | 2'-OMe | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **F'** | **1** | **2** | **3** | **4** | **5** | **6** | **7** | **8** | **9** | **10** | **11** |
| | 2'-OMe +PS +EVP | **2'-F +PS** | 2'-OMe | 2'-OMe | 2'-OMe | **2'-F** | 2'-OMe | **2'-F** | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **G'** | **1** | **2** | **3** | **4** | **5** | **6** | **7** | **8** | **9** | **10** | **11** |
| | 2'-OMe +PS +EVP | **2'-F +PS** | 2'-OMe | **2'-F** | **2'-F** | **2'-F** | 2'-OMe | **2'-F** | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **H'** | **1** | **2** | **3** | **4** | **5** | **6** | **7** | **8** | **9** | **10** | **11** |
| | 2'-OMe +PS +EVP | **2'-F +PS** | **2'-F** | 2'-OMe | 2'-OMe | **2'-F** | 2'-OMe | **2'-F** | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **I'** | **1** | **2** | **3** | **4** | **5** | **6** | **7** | **8** | **9** | **10** | **11** |
| | 2'-OMe +PS | **2'-F +PS** | 2'-OMe | 2'-OMe | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |

wherein 2'-OMe represents a 2'-O-methyl modification; 2'-F represents a 2'-fluoro modification; PS indicates that, starting from the 5' end, the nucleotide at the position is linked to the adjacent nucleotide on the right via a 3',5'-phosphorothioate bond; EVP represents a 5'-vinyl-(E)-phosphonate.

The sense strand adopts any one of the modification patterns shown in Table 11 below:

**Table 11: Modification of sense strand**

| No. | **Sense strand (SS) 5'-3'** | | | | | | | | | |
|---|---|---|---|---|---|---|---|---|---|---|
| **a'** | **1** | **2** | **3** | **4** | **5** | **6** | **7** | **8** | **9** | **10** |
| | 2'-OMe+PS | 2'-OMe+PS | 2'-OMe | 2'-OMe | 2'-OMe | **2'-F** | 2'-OMe | **2'-F** | **2'-F** | **2'-F** |
| | **11** | **12** | **13** | **14** | **15** | **16** | **17** | **18** | **19** | **20** |
| | 2'-OMe | 2'-OMe | 2'-OMe | 2'-OMe | 2'-OMe | **2'-F** | 2'-OMe | 2'-OMe | 2'-OMe | 2'-OMe |
| **b'** | **1** | **2** | **3** | **4** | **5** | **6** | **7** | **8** | **9** | **10** |
| | 2'-OMe+PS | 2'-OMe+PS | 2'-OMe | 2'-OMe | 2'-OMe | **2'-F** | 2'-OMe | **2'-F** | 2'-OMe | **2'-F** |
| | **11** | **12** | **13** | **14** | **15** | **16** | **17** | **18** | **19** | **20** |
| | 2'-OMe | 2'-OMe | 2'-OMe | 2'-OMe | 2'-OMe | **2'-F** | 2'-OMe | 2'-OMe | 2'-OMe | 2'-OMe |
| **c'** | **1** | **2** | **3** | **4** | **5** | **6** | **7** | **8** | **9** | **10** |
| | 2'-OMe+PS | 2'-OMe+PS | 2'-OMe | 2'-OMe | 2'-OMe | **2'-F** | 2'-OMe | **2'-F** | **2'-F** | **2'-F** |
| | **11** | **12** | **13** | **14** | **15** | **16** | **17** | **18** | **19** | **20** |
| | 2'-OMe | 2'-OMe | 2'-OMe | 2'-OMe | 2'-OMe | 2'-OMe | 2'-OMe | 2'-OMe | 2'-OMe | 2'-OMe |

wherein 2'-OMe represents a 2'-O-methyl modification; 2'-F represents a 2'-fluoro modification; PS indicates that, starting from the 5' end, the nucleotide at the position is linked to the adjacent nucleotide on the right via a 3',5'-phosphorothioate bond; EVP represents a 5'-vinyl-(E)-phosphonate.

The siRNA modification template, in which the antisense strand was modified according to modification pattern A' and the sense strand was modified according to modification pattern a', was designated as DV25SP.

The siRNA modification template, in which the antisense strand was modified according to modification pattern B' and the sense strand was modified according to modification pattern a', was designated as DV26SP.

The siRNA modification template, in which the antisense strand was modified according to modification pattern C' and the sense strand was modified according to modification pattern a', was designated as DV27SP.

The siRNA modification template, in which the antisense strand was modified according to modification pattern C' and the sense strand was modified according to modification pattern b', was designated as DV29SP.

The siRNA modification template, in which the antisense strand was modified according to modification pattern D' and the sense strand was modified according to modification pattern b', was designated as DV32SP.

The siRNA modification template, in which the antisense strand was modified according to modification pattern E' and the sense strand was modified according to modification pattern b', was designated as DV34SP.

The siRNA modification template, in which the antisense strand was modified according to modification pattern F' and the sense strand was modified according to modification pattern b', was designated as DV41SP.

The siRNA modification template, in which the antisense strand was modified according to modification pattern G' and the sense strand was modified according to modification pattern b', was designated as DV42SP.

The siRNA modification template, in which the antisense strand was modified according to modification pattern H' and the sense strand was modified according to modification pattern b', was designated as DV43SP.

The siRNA modification template, in which the antisense strand was modified according to modification pattern I' and the sense strand was modified according to modification pattern c', was designated as DV22S.

The template-modified siRNA derivatives are detailed in Table 12 below. The synthesis methods for each sequence are as described above.

**Table 12: Sense and antisense sequences of siRNA derivatives**

| **siRNA ID** | **Sense sequence 5'-3'** | **SEQ ID NO:** | **Antisense sequence 5'-3'** | **SEQ ID NO:** |
|---|---|---|---|---|
| si075-DV25P | Gms-Gms-Am-Gm-Um-Gm-Af-Am- Gf-Af-Gf-Am-Cm-Cm-Am-Am-Gf- Am-Um-Gm-Am | 525 | UmsEVP-Cfs-Am-Um-Cm-Uf-Um-Gm- Gm-Um-Cm-Um-Cm-Uf-Um-Cf-Am-Cm- Um-Cm-Cms-Ams-Am | 687 |
| si751-DV25P | Ams-Gms-Um-Gm-Am-Am-Gf-Am- Gf-Af-Cf-Cm-Am-Am-Gm-Am-Uf- Gm-Am-Am-Am | 526 | UmsEVP-Ufs-Um-Cm-Am-Uf-Cm-Um- Um-Gm-Gm-Um-Cm-Uf-Cm-Uf-Um-Cm- Am-Cm-Ums-Cms-Cm | 688 |
| si753-DV25P | Gms-Ams-Am-Gm-Am-Gm-Af-Cm- Cf-Af-Af-Gm-Am-Um-Gm-Am-Af- Gm-Um-Um-Um | 527 | AmsEVP-Afs-Am-Cm-Um-Uf-Cm-Am- Um-Cm-Um-Um-Gm-Gf-Um-Cf-Um-Cm- Um-Um-Cms-Ams-Cm | 689 |
| si791-DV25P | Gms-Ams-Gm-Am-Cm-Cm-Af-Am- Gf-Af-Uf-Gm-Am-Am-Gm-Um-Uf- Um-Cm-Cm-Am | 528 | UmsEVP-Gfs-Gm-Am-Am-Af-Cm-Um- Um-Cm-Am-Um-Cm-Uf-Um-Gf-Gm-Um- Cm-Um-Cms-Ums-Um | 690 |
| si014-DV25P | Ams-Gms-Cm-Um-Am-Um-Uf-Um- Uf-Cf-Af-Um-Am-Am-Um-Am-Af- Um-Am-Cm-Um | 529 | AmsEVP-Gfs-Um-Am-Um-Uf-Am-Um- Um-Am-Um-Gm-Am-Af-Am-Af-Um-Am- Gm-Cm-Ums-Ums-Um | 691 |
| si076-DV25P | Gms-Ams-Gm-Um-Gm-Am-Af-Gm- Af-Gf-Af-Cm-Cm-Am-Am-Gm-Af- Um-Gm-Am-Am | 530 | UmsEVP-Ufs-Cm-Am-Um-Cf-Um-Um- Gm-Gm-Um-Cm-Um-Cf-Um-Uf-Cm-Am- Cm-Um-Cms-Cms-Am | 692 |
| si077-DV25P | Gms-Ums-Gm-Am-Am-Gm-Af-Gm- Af-Cf-Cf-Am-Am-Gm-Am-Um-Gf- Am-Am-Gm-Um | 531 | AmsEVP-Cfs-Um-Um-Cm-Af-Um-Cm- Um-Um-Gm-Gm-Um-Cf-Um-Cf-Um-Um- Cm-Am-Cms-Ums-Cm | 693 |
| si078-DV25P | Ams-Ams-Gm-Am-Gm-Am-Cf-Cm- Af-Af-Gf-Am-Um-Gm-Am-Am-Gf- Um-Um-Um-Am | 532 | UmsEVP-Afs-Am-Am-Cm-Uf-Um-Cm- Am-Um-Cm-Um-Um-Gf-Gm-Uf-Cm-Um- Cm-Um-Ums-Cms-Am | 694 |
| si080-DV25P | Gms-Ams-Cm-Cm-Am-Am-Gf-Am- Uf-Gf-Af-Am-Gm-Um-Um-Um-Cf- Cm-Cm-Am-Am | 533 | UmsEVP-Ufs-Gm-Gm-Gm-Af-Am-Am- Cm-Um-Um-Cm-Am-Uf-Cm-Uf-Um-Gm- Gm-Um-Cms-Ums-Cm | 695 |
| si752-DV25SP | Gms-Ams-Am-Gm-Am-Gf-Am-Cf- Cf-Af-Am-Gm-Am-Um-Gm-Af- Am-Gm-Um-Um | 534 | AmsEVP-Afs-Cm-Um-Um-Cf-Am-Um- Cm-Um-Um-Gm-Gm-Uf-Cm-Uf-Cm-Um- Um-Cms-Ams-Cm | 696 |
| si079-DV25SP | Gms-Ams-Gm-Am-Cm-Cf-Am-Af- Gf-Af-Um-Gm-Am-Am-Gm-Uf- Um-Um-Cm-Am | 535 | UmsEVP-Gfs-Am-Am-Am-Cf-Um-Um- Cm-Am-Um-Cm-Um-Uf-Gm-Gf-Um-Cm- Um-Cms-Ums-Um | 697 |
| si047-DV25SP | Gms-Ams-Gm-Am-Cm-Af-Am-Gf- Cf-Af-Um-Um-Um-Am-Um-Af- Cm-Um-Um-Um | 536 | AmsEVP-Afs-Am-Gm-Um-Af-Um-Am- Am-Am-Um-Gm-Cm-Uf-Um-Gf-Um-Cm- Um-Cms-Cms-Cm | 698 |
| si075-DV26P | Gms-Gms-Am-Gm-Um-Gm-Af-Am- Gf-Af-Gf-Am-Cm-Cm-Am-Am-Gf- Am-Um-Gm-Am | 537 | UmsEVP-Cfs-Af-Uf-Cm-Uf-Um-Gm-Gm- Um-Cm-Um-Cm-Uf-Um-Cf-Am-Cm-Um- Cm-Cms-Ams-Am | 699 |
| si751-DV26P | Ams-Gms-Um-Gm-Am-Am-Gf-Am- Gf-Af-Cf-Cm-Am-Am-Gm-Am-Uf- Gm-Am-Am-Am | 538 | UmsEVP-Ufs-Uf-Cf-Am-Uf-Cm-Um-Um- Gm-Gm-Um-Cm-Uf-Cm-Uf-Um-Cm-Am- Cm-Ums-Cms-Cm | 700 |
| si753-DV26P | Gms-Ams-Am-Gm-Am-Gm-Af-Cm- Cf-Af-Af-Gm-Am-Um-Gm-Am-Af- Gm-Um-Um-Um | 539 | AmsEVP-Afs-Af-Cf-Um-Uf-Cm-Am-Um- Cm-Um-Um-Gm-Gf-Um-Cf-Um-Cm-Um- Um-Cms-Ams-Cm | 701 |
| si791-DV26P | Gms-Ams-Gm-Am-Cm-Cm-Af-Am- Gf-Af-Uf-Gm-Am-Am-Gm-Um-Uf- Um-Cm-Cm-Am | 540 | UmsEVP-Gfs-Gf-Af-Am-Af-Cm-Um-Um- Cm-Am-Um-Cm-Uf-Um-Gf-Gm-Um-Cm- Um-Cms-Ums-Um | 702 |
| si014-DV26P | Ams-Gms-Cm-Um-Am-Um-Uf-Um- Uf-Cf-Af-Um-Am-Am-Um-Am-Af- Um-Am-Cm-Um | 541 | AmsEVP-Gfs-Uf-Af-Um-Uf-Am-Um-Um- Am-Um-Gm-Am-Af-Am-Af-Um-Am-Gm- Cm-Ums-Ums-Um | 703 |
| si076-DV26P | Gms-Ams-Gm-Um-Gm-Am-Af-Gm- Af-Gf-Af-Cm-Cm-Am-Am-Gm-Af- Um-Gm-Am-Am | 542 | UmsEVP-Ufs-Cf-Af-Um-Cf-Um-Um-Gm- Gm-Um-Cm-Um-Cf-Um-Uf-Cm-Am-Cm- Um-Cms-Cms-Am | 704 |
| si077-DV26P | Gms-Ums-Gm-Am-Am-Gm-Af-Gm- Af-Cf-Cf-Am-Am-Gm-Am-Um-Gf- Am-Am-Gm-Um | 543 | AmsEVP-Cfs-Uf-Uf-Cm-Af-Um-Cm-Um- Um-Gm-Gm-Um-Cf-Um-Cf-Um-Um-Cm- Am-Cms-Ums-Cm | 705 |
| si078-DV26P | Ams-Ams-Gm-Am-Gm-Am-Cf-Cm- Af-Af-Gf-Am-Um-Gm-Am-Am-Gf- Um-Um-Um-Am | 544 | UmsEVP-Afs-Af-Af-Cm-Uf-Um-Cm-Am- Um-Cm-Um-Um-Gf-Gm-Uf-Cm-Um-Cm- Um-Ums-Cms-Am | 706 |
| si080-DV26P | Gms-Ams-Cm-Cm-Am-Am-Gf-Am- Uf-Gf-Af-Am-Gm-Um-Um-Um-Cf- Cm-Cm-Am-Am | 545 | UmsEVP-Ufs-Gf-Gf-Gm-Af-Am-Am-Cm- Um-Um-Cm-Am-Uf-Cm-Uf-Um-Gm-Gm- Um-Cms-Ums-Cm | 707 |
| si752-DV26SP | Gms-Ams-Am-Gm-Am-Gf-Am-Cf- Cf-Af-Am-Gm-Am-Um-Gm-Af- Am-Gm-Um-Um | 546 | AmsEVP-Afs-Cf-Uf-Um-Cf-Am-Um-Cm- Um-Um-Gm-Gm-Uf-Cm-Uf-Cm-Um-Um- Cms-Ams-Cm | 708 |
| si079-DV26SP | Gms-Ams-Gm-Am-Cm-Cf-Am-Af- Gf-Af-Um-Gm-Am-Am-Gm-Uf- Um-Um-Cm-Am | 547 | UmsEVP-Gfs-Af-Af-Am-Cf-Um-Um-Cm- Am-Um-Cm-Um-Uf-Gm-Gf-Um-Cm-Um- Cms-Ums-Um | 709 |
| si047-DV26SP | Gms-Ams-Gm-Am-Cm-Af-Am-Gf- Cf-Af-Um-Um-Um-Am-Um-Af- Cm-Um-Um-Um | 548 | AmsEVP-Afs-Af-Gf-Um-Af-Um-Am-Am- Am-Um-Gm-Cm-Uf-Um-Gf-Um-Cm-Um- Cms-Cms-Cm | 710 |
| si075-DV27P | Gms-Gms-Am-Gm-Um-Gm-Af-Am- Gf-Af-Gf-Am-Cm-Cm-Am-Am-Gf- Am-Um-Gm-Am | 549 | UmsEVP-Cfs-Am-Uf-Cf-Uf-Um-Gm-Gm- Um-Cm-Um-Cm-Uf-Um-Cf-Am-Cm-Um- Cm-Cms-Ams-Am | 711 |
| si751-DV27P | Ams-Gms-Um-Gm-Am-Am-Gf-Am- Gf-Af-Cf-Cm-Am-Am-Gm-Am-Uf- Gm-Am-Am-Am | 550 | UmsEVP-Ufs-Um-Cf-Af-Uf-Cm-Um-Um- Gm-Gm-Um-Cm-Uf-Cm-Uf-Um-Cm-Am- Cm-Ums-Cms-Cm | 712 |
| si753-DV27P | Gms-Ams-Am-Gm-Am-Gm-Af-Cm- Cf-Af-Af-Gm-Am-Um-Gm-Am-Af- Gm-Um-Um-Um | 551 | AmsEVP-Afs-Am-Cf-Uf-Uf-Cm-Am-Um- Cm-Um-Um-Gm-Gf-Um-Cf-Um-Cm-Um- Um-Cms-Ams-Cm | 713 |
| si791-DV27P | Gms-Ams-Gm-Am-Cm-Cm-Af-Am- | 552 | UmsEVP-Gfs-Gm-Af-Af-Af-Cm-Um-Um- | 714 |
| | Gf-Af-Uf-Gm-Am-Am-Gm-Um-Uf- Um-Cm-Cm-Am | | Cm-Am-Um-Cm-Uf-Um-Gf-Gm-Um-Cm- Um-Cms-Ums-Um | |
| si014-DV27P | Ams-Gms-Cm-Um-Am-Um-Uf-Um- Uf-Cf-Af-Um-Am-Am-Um-Am-Af- Um-Am-Cm-Um | 553 | AmsEVP-Gfs-Um-Af-Uf-Uf-Am-Um-Um- Am-Um-Gm-Am-Af-Am-Af-Um-Am-Gm- Cm-Ums-Ums-Um | 715 |
| si076-DV27P | Gms-Ams-Gm-Um-Gm-Am-Af-Gm- Af-Gf-Af-Cm-Cm-Am-Am-Gm-Af- Um-Gm-Am-Am | 554 | UmsEVP-Ufs-Cm-Af-Uf-Cf-Um-Um-Gm- Gm-Um-Cm-Um-Cf-Um-Uf-Cm-Am-Cm- Um-Cms-Cms-Am | 716 |
| si077-DV27P | Gms-Ums-Gm-Am-Am-Gm-Af-Gm- Af-Cf-Cf-Am-Am-Gm-Am-Um-Gf- Am-Am-Gm-Um | 555 | AmsEVP-Cfs-Um-Uf-Cf-Af-Um-Cm-Um- Um-Gm-Gm-Um-Cf-Um-Cf-Um-Um-Cm- Am-Cms-Ums-Cm | 717 |
| si078-DV27P | Ams-Ams-Gm-Am-Gm-Am-Cf-Cm- Af-Af-Gf-Am-Um-Gm-Am-Am-Gf- Um-Um-Um-Am | 556 | UmsEVP-Afs-Am-Af-Cf-Uf-Um-Cm-Am- Um-Cm-Um-Um-Gf-Gm-Uf-Cm-Um-Cm- Um-Ums-Cms-Am | 718 |
| si080-DV27P | Gms-Ams-Cm-Cm-Am-Am-Gf-Am- Uf-Gf-Af-Am-Gm-Um-Um-Um-Cf- Cm-Cm-Am-Am | 557 | UmsEVP-Ufs-Gm-Gf-Gf-Af-Am-Am-Cm- Um-Um-Cm-Am-Uf-Cm-Uf-Um-Gm-Gm- Um-Cms-Ums-Cm | 719 |
| si752-DV27SP | Gms-Ams-Am-Gm-Am-Gf-Am-Cf- Cf-Af-Am-Gm-Am-Um-Gm-Af- Am-Gm-Um-Um | 558 | AmsEVP-Afs-Cm-Uf-Uf-Cf-Am-Um-Cm- Um-Um-Gm-Gm-Uf-Cm-Uf-Cm-Um-Um- Cms-Ams-Cm | 720 |
| si079-DV27SP | Gms-Ams-Gm-Am-Cm-Cf-Am-Af- Gf-Af-Um-Gm-Am-Am-Gm-Uf- Um-Um-Cm-Am | 559 | UmsEVP-Gfs-Am-Af-Af-Cf-Um-Um-Cm- Am-Um-Cm-Um-Uf-Gm-Gf-Um-Cm-Um- Cms-Ums-Um | 721 |
| si047-DV27SP | Gms-Ams-Gm-Am-Cm-Af-Am-Gf- Cf-Af-Um-Um-Um-Am-Um-Af- Cm-Um-Um-Um | 560 | AmsEVP-Afs-Am-Gf-Uf-Af-Um-Am-Am- Am-Um-Gm-Cm-Uf-Um-Gf-Um-Cm-Um- Cms-Cms-Cm | 722 |
| si075-DV29P | Gms-Gms-Am-Gm-Um-Gm-Af-Am- Gf-Am-Gf-Am-Cm-Cm-Am-Am- Gf-Am-Um-Gm-Am | 561 | UmsEVP-Cfs-Am-Uf-Cf-Uf-Um-Gm-Gm- Um-Cm-Um-Cm-Uf-Um-Cf-Am-Cm-Um- Cm-Cms-Ams-Am | 723 |
| si751-DV29P | Ams-Gms-Um-Gm-Am-Am-Gf-Am- Gf-Am-Cf-Cm-Am-Am-Gm-Am- Uf-Gm-Am-Am-Am | 562 | UmsEVP-Ufs-Um-Cf-Af-Uf-Cm-Um-Um- Gm-Gm-Um-Cm-Uf-Cm-Uf-Um-Cm-Am- Cm-Ums-Cms-Cm | 724 |
| si753-DV29P | Gms-Ams-Am-Gm-Am-Gm-Af-Cm- Cf-Am-Af-Gm-Am-Um-Gm-Am- Af-Gm-Um-Um-Um | 563 | AmsEVP-Afs-Am-Cf-Uf-Uf-Cm-Am-Um- Cm-Um-Um-Gm-Gf-Um-Cf-Um-Cm-Um- Um-Cms-Ams-Cm | 725 |
| si791-DV29P | Gms-Ams-Gm-Am-Cm-Cm-Af-Am- Gf-Am-Uf-Gm-Am-Am-Gm-Um- Uf-Um-Cm-Cm-Am | 564 | UmsEVP-Gfs-Gm-Af-Af-Af-Cm-Um-Um- Cm-Am-Um-Cm-Uf-Um-Gf-Gm-Um-Cm- Um-Cms-Ums-Um | 726 |
| si014-DV29P | Ams-Gms-Cm-Um-Am-Um-Uf-Um- Uf-Cm-Af-Um-Am-Am-Um-Am- Af-Um-Am-Cm-Um | 565 | AmsEVP-Gfs-Um-Af-Uf-Uf-Am-Um-Um- Am-Um-Gm-Am-Af-Am-Af-Um-Am-Gm- Cm-Ums-Ums-Um | 727 |
| si076-DV29P | Gms-Ams-Gm-Um-Gm-Am-Af-Gm- Af-Gm-Af-Cm-Cm-Am-Am-Gm- Af-Um-Gm-Am-Am | 566 | UmsEVP-Ufs-Cm-Af-Uf-Cf-Um-Um-Gm- Gm-Um-Cm-Um-Cf-Um-Uf-Cm-Am-Cm- Um-Cms-Cms-Am | 728 |
| si077-DV29P | Gms-Ums-Gm-Am-Am-Gm-Af-Gm- Af-Cm-Cf-Am-Am-Gm-Am-Um- | 567 | AmsEVP-Cfs-Um-Uf-Cf-Af-Um-Cm-Um- Um-Gm-Gm-Um-Cf-Um-Cf-Um-Um-Cm- | 729 |
| | Gf-Am-Am-Gm-Um | | Am-Cms-Ums-Cm | |
| si078-DV29P | Ams-Ams-Gm-Am-Gm-Am-Cf-Cm- Af-Am-Gf-Am-Um-Gm-Am-Am- Gf-Um-Um-Um-Am | 568 | UmsEVP-Afs-Am-Af-Cf-Uf-Um-Cm-Am- Um-Cm-Um-Um-Gf-Gm-Uf-Cm-Um-Cm- Um-Ums-Cms-Am | 730 |
| si080-DV29P | Gms-Ams-Cm-Cm-Am-Am-Gf-Am- Uf-Gm-Af-Am-Gm-Um-Um-Um- Cf-Cm-Cm-Am-Am | 569 | UmsEVP-Ufs-Gm-Gf-Gf-Af-Am-Am-Cm- Um-Um-Cm-Am-Uf-Cm-Uf-Um-Gm-Gm- Um-Cms-Ums-Cm | 731 |
| si752-DV29SP | Gms-Ams-Am-Gm-Am-Gf-Am-Cf- Cm-Af-Am-Gm-Am-Um-Gm-Af- Am-Gm-Um-Um | 570 | AmsEVP-Afs-Cm-Uf-Uf-Cf-Am-Um-Cm- Um-Um-Gm-Gm-Uf-Cm-Uf-Cm-Um-Um- Cms-Ams-Cm | 732 |
| si079-DV29SP | Gms-Ams-Gm-Am-Cm-Cf-Am-Af- Gm-Af-Um-Gm-Am-Am-Gm-Uf- Um-Um-Cm-Am | 571 | UmsEVP-Gfs-Am-Af-Af-Cf-Um-Um-Cm- Am-Um-Cm-Um-Uf-Gm-Gf-Um-Cm-Um- Cms-Ums-Um | 733 |
| si047-DV29SP | Gms-Ams-Gm-Am-Cm-Af-Am-Gf- Cm-Af-Um-Um-Um-Am-Um-Af- Cm-Um-Um-Um | 572 | AmsEVP-Afs-Am-Gf-Uf-Af-Um-Am-Am- Am-Um-Gm-Cm-Uf-Um-Gf-Um-Cm-Um- Cms-Cms-Cm | 734 |
| si075-DV32P | Gms-Gms-Am-Gm-Um-Gm-Af-Am- Gf-Am-Gf-Am-Cm-Cm-Am-Am- Gf-Am-Um-Gm-Am | 573 | UmsEVP-Cfs-Am-Um-Cf-Uf-Um-Gm-Gm- Um-Cm-Um-Cm-Uf-Um-Cf-Am-Cm-Um- Cm-Cms-Ams-Am | 735 |
| si751-DV32P | Ams-Gms-Um-Gm-Am-Am-Gf-Am- Gf-Am-Cf-Cm-Am-Am-Gm-Am- Uf-Gm-Am-Am-Am | 574 | UmsEVP-Ufs-Um-Cm-Af-Uf-Cm-Um-Um- Gm-Gm-Um-Cm-Uf-Cm-Uf-Um-Cm-Am- Cm-Ums-Cms-Cm | 736 |
| si753-DV32P | Gms-Ams-Am-Gm-Am-Gm-Af-Cm- Cf-Am-Af-Gm-Am-Um-Gm-Am- Af-Gm-Um-Um-Um | 575 | AmsEVP-Afs-Am-Cm-Uf-Uf-Cm-Am-Um- Cm-Um-Um-Gm-Gf-Um-Cf-Um-Cm-Um- Um-Cms-Ams-Cm | 737 |
| si791-DV32P | Gms-Ams-Gm-Am-Cm-Cm-Af-Am- Gf-Am-Uf-Gm-Am-Am-Gm-Um- Uf-Um-Cm-Cm-Am | 576 | UmsEVP-Gfs-Gm-Am-Af-Af-Cm-Um-Um- Cm-Am-Um-Cm-Uf-Um-Gf-Gm-Um-Cm- Um-Cms-Ums-Um | 738 |
| si014-DV32P | Ams-Gms-Cm-Um-Am-Um-Uf-Um- Uf-Cm-Af-Um-Am-Am-Um-Am- Af-Um-Am-Cm-Um | 577 | AmsEVP-Gfs-Um-Am-Uf-Uf-Am-Um-Um- Am-Um-Gm-Am-Af-Am-Af-Um-Am-Gm- Cm-Ums-Ums-Um | 739 |
| si076-DV32P | Gms-Ams-Gm-Um-Gm-Am-Af-Gm- Af-Gm-Af-Cm-Cm-Am-Am-Gm- Af-Um-Gm-Am-Am | 578 | UmsEVP-Ufs-Cm-Am-Uf-Cf-Um-Um-Gm- Gm-Um-Cm-Um-Cf-Um-Uf-Cm-Am-Cm- Um-Cms-Cms-Am | 740 |
| si077-DV32P | Gms-Ums-Gm-Am-Am-Gm-Af-Gm- Af-Cm-Cf-Am-Am-Gm-Am-Um- Gf-Am-Am-Gm-Um | 579 | AmsEVP-Cfs-Um-Um-Cf-Af-Um-Cm-Um- Um-Gm-Gm-Um-Cf-Um-Cf-Um-Um-Cm- Am-Cms-Ums-Cm | 741 |
| si078-DV32P | Ams-Ams-Gm-Am-Gm-Am-Cf-Cm- Af-Am-Gf-Am-Um-Gm-Am-Am- Gf-Um-Um-Um-Am | 580 | UmsEVP-Afs-Am-Am-Cf-Uf-Um-Cm-Am- Um-Cm-Um-Um-Gf-Gm-Uf-Cm-Um-Cm- Um-Ums-Cms-Am | 742 |
| si080-DV32P | Gms-Ams-Cm-Cm-Am-Am-Gf-Am- Uf-Gm-Af-Am-Gm-Um-Um-Um- Cf-Cm-Cm-Am-Am | 581 | UmsEVP-Ufs-Gm-Gm-Gf-Af-Am-Am-Cm- Um-Um-Cm-Am-Uf-Cm-Uf-Um-Gm-Gm- Um-Cms-Ums-Cm | 743 |
| si752-DV32SP | Gms-Ams-Am-Gm-Am-Gf-Am-Cf- Cm-Af-Am-Gm-Am-Um-Gm-Af- Am-Gm-Um-Um | 582 | AmsEVP-Afs-Cm-Um-Uf-Cf-Am-Um-Cm- Um-Um-Gm-Gm-Uf-Cm-Uf-Cm-Um-Um- Cms-Ams-Cm | 744 |
| si079-DV32SP | Gms-Ams-Gm-Am-Cm-Cf-Am-Af- Gm-Af-Um-Gm-Am-Am-Gm-Uf- Um-Um-Cm-Am | 583 | UmsEVP-Gfs-Am-Am-Af-Cf-Um-Um-Cm- Am-Um-Cm-Um-Uf-Gm-Gf-Um-Cm-Um- Cms-Ums-Um | 745 |
| si047-DV32SP | Gms-Ams-Gm-Am-Cm-Af-Am-Gf- Cm-Af-Um-Um-Um-Am-Um-Af- Cm-Um-Um-Um | 584 | AmsEVP-Afs-Am-Gm-Uf-Af-Um-Am-Am- Am-Um-Gm-Cm-Uf-Um-Gf-Um-Cm-Um- Cms-Cms-Cm | 746 |
| si075-DV34P | Gms-Gms-Am-Gm-Um-Gm-Af-Am- Gf-Am-Gf-Am-Cm-Cm-Am-Am- Gf-Am-Um-Gm-Am | 585 | UmsEVP-Cfs-Af-Um-Cm-Uf-Um-Gm-Gm- Um-Cm-Um-Cm-Uf-Um-Cf-Am-Cm-Um- Cm-Cms-Ams-Am | 747 |
| si751-DV34P | Ams-Gms-Um-Gm-Am-Am-Gf-Am- Gf-Am-Cf-Cm-Am-Am-Gm-Am- Uf-Gm-Am-Am-Am | 586 | UmsEVP-Ufs-Uf-Cm-Am-Uf-Cm-Um-Um- Gm-Gm-Um-Cm-Uf-Cm-Uf-Um-Cm-Am- Cm-Ums-Cms-Cm | 748 |
| si753-DV34P | Gms-Ams-Am-Gm-Am-Gm-Af-Cm- Cf-Am-Af-Gm-Am-Um-Gm-Am- Af-Gm-Um-Um-Um | 587 | AmsEVP-Afs-Af-Cm-Um-Uf-Cm-Am-Um- Cm-Um-Um-Gm-Gf-Um-Cf-Um-Cm-Um- Um-Cms-Ams-Cm | 749 |
| si791-DV34P | Gms-Ams-Gm-Am-Cm-Cm-Af-Am- Gf-Am-Uf-Gm-Am-Am-Gm-Um- Uf-Um-Cm-Cm-Am | 588 | UmsEVP-Gfs-Gf-Am-Am-Af-Cm-Um-Um- Cm-Am-Um-Cm-Uf-Um-Gf-Gm-Um-Cm- Um-Cms-Ums-Um | 750 |
| si014-DV34P | Ams-Gms-Cm-Um-Am-Um-Uf-Um- Uf-Cm-Af-Um-Am-Am-Um-Am- Af-Um-Am-Cm-Um | 589 | AmsEVP-Gfs-Uf-Am-Um-Uf-Am-Um-Um- Am-Um-Gm-Am-Af-Am-Af-Um-Am-Gm- Cm-Ums-Ums-Um | 751 |
| si076-DV34P | Gms-Ams-Gm-Um-Gm-Am-Af-Gm- Af-Gm-Af-Cm-Cm-Am-Am-Gm- Af-Um-Gm-Am-Am | 590 | UmsEVP-Ufs-Cf-Am-Um-Cf-Um-Um-Gm- Gm-Um-Cm-Um-Cf-Um-Uf-Cm-Am-Cm- Um-Cms-Cms-Am | 752 |
| si077-DV34P | Gms-Ums-Gm-Am-Am-Gm-Af-Gm- Af-Cm-Cf-Am-Am-Gm-Am-Um- Gf-Am-Am-Gm-Um | 591 | AmsEVP-Cfs-Uf-Um-Cm-Af-Um-Cm-Um- Um-Gm-Gm-Um-Cf-Um-Cf-Um-Um-Cm- Am-Cms-Ums-Cm | 753 |
| si078-DV34P | Ams-Ams-Gm-Am-Gm-Am-Cf-Cm- Af-Am-Gf-Am-Um-Gm-Am-Am- Gf-Um-Um-Um-Am | 592 | UmsEVP-Afs-Af-Am-Cm-Uf-Um-Cm-Am- Um-Cm-Um-Um-Gf-Gm-Uf-Cm-Um-Cm- Um-Ums-Cms-Am | 754 |
| si080-DV34P | Gms-Ams-Cm-Cm-Am-Am-Gf-Am- Uf-Gm-Af-Am-Gm-Um-Um-Um- Cf-Cm-Cm-Am-Am | 593 | UmsEVP-Ufs-Gf-Gm-Gm-Af-Am-Am-Cm- Um-Um-Cm-Am-Uf-Cm-Uf-Um-Gm-Gm- Um-Cms-Ums-Cm | 755 |
| si752-DV34SP | Gms-Ams-Am-Gm-Am-Gf-Am-Cf- Cm-Af-Am-Gm-Am-Um-Gm-Af- Am-Gm-Um-Um | 594 | AmsEVP-Afs-Cf-Um-Um-Cf-Am-Um-Cm- Um-Um-Gm-Gm-Uf-Cm-Uf-Cm-Um-Um- Cms-Ams-Cm | 756 |
| si079-DV34SP | Gms-Ams-Gm-Am-Cm-Cf-Am-Af- Gm-Af-Um-Gm-Am-Am-Gm-Uf- Um-Um-Cm-Am | 595 | UmsEVP-Gfs-Af-Am-Am-Cf-Um-Um-Cm- Am-Um-Cm-Um-Uf-Gm-Gf-Um-Cm-Um- Cms-Ums-Um | 757 |
| si047-DV34SP | Gms-Ams-Gm-Am-Cm-Af-Am-Gf- Cm-Af-Um-Um-Um-Am-Um-Af- Cm-Um-Um-Um | 596 | AmsEVP-Afs-Af-Gm-Um-Af-Um-Am-Am- Am-Um-Gm-Cm-Uf-Um-Gf-Um-Cm-Um- Cms-Cms-Cm | 758 |
| si075-DV41P | Gms-Gms-Am-Gm-Um-Gm-Af-Am- Gf-Am-Gf-Am-Cm-Cm-Am-Am- Gf-Am-Um-Gm-Am | 597 | UmsEVP-Cfs-Am-Um-Cm-Uf-Um-Gf-Gm- Um-Cm-Um-Cm-Uf-Um-Cf-Am-Cm-Um- Cm-Cms-Ams-Am | 759 |
| si751-DV41P | Ams-Gms-Um-Gm-Am-Am-Gf-Am- | 598 | UmsEVP-Ufs-Um-Cm-Am-Uf-Cm-Uf-Um- | 760 |
| | Gf-Am-Cf-Cm-Am-Am-Gm-Am- Uf-Gm-Am-Am-Am | | Gm-Gm-Um-Cm-Uf-Cm-Uf-Um-Cm-Am- Cm-Ums-Cms-Cm | |
| si791-DV41P | Gms-Ams-Gm-Am-Cm-Cm-Af-Am- Gf-Am-Uf-Gm-Am-Am-Gm-Um- Uf-Um-Cm-Cm-Am | 599 | UmsEVP-Gfs-Gm-Am-Am-Af-Cm-Uf-Um- Cm-Am-Um-Cm-Uf-Um-Gf-Gm-Um-Cm- Um-Cms-Ums-Um | 761 |
| si080-DV41P | Gms-Ams-Cm-Cm-Am-Am-Gf-Am- Uf-Gm-Af-Am-Gm-Um-Um-Um- Cf-Cm-Cm-Am-Am | 600 | UmsEVP-Ufs-Gm-Gm-Gm-Af-Am-Af-Cm- Um-Um-Cm-Am-Uf-Cm-Uf-Um-Gm-Gm- Um-Cms-Ums-Cm | 762 |
| si079-DV41SP | Gms-Ams-Gm-Am-Cm-Cf-Am-Af- Gm-Af-Um-Gm-Am-Am-Gm-Uf- Um-Um-Cm-Am | 601 | UmsEVP-Gfs-Am-Am-Am-Cf-Um-Uf-Cm- Am-Um-Cm-Um-Uf-Gm-Gf-Um-Cm-Um- Cms-Ums-Um | 763 |
| si075-DV42P | Gms-Gms-Am-Gm-Um-Gm-Af-Am- Gf-Am-Gf-Am-Cm-Cm-Am-Am- Gf-Am-Um-Gm-Am | 602 | UmsEVP-Cfs-Am-Uf-Cf-Uf-Um-Gf-Gm- Um-Cm-Um-Cm-Uf-Um-Cf-Am-Cm-Um- Cm-Cms-Ams-Am | 764 |
| si751-DV42P | Ams-Gms-Um-Gm-Am-Am-Gf-Am- Gf-Am-Cf-Cm-Am-Am-Gm-Am- Uf-Gm-Am-Am-Am | 603 | UmsEVP-Ufs-Um-Cf-Af-Uf-Cm-Uf-Um- Gm-Gm-Um-Cm-Uf-Cm-Uf-Um-Cm-Am- Cm-Ums-Cms-Cm | 765 |
| si791-DV42P | Gms-Ams-Gm-Am-Cm-Cm-Af-Am- Gf-Am-Uf-Gm-Am-Am-Gm-Um- Uf-Um-Cm-Cm-Am | 604 | UmsEVP-Gfs-Gm-Af-Af-Af-Cm-Uf-Um- Cm-Am-Um-Cm-Uf-Um-Gf-Gm-Um-Cm- Um-Cms-Ums-Um | 766 |
| si080-DV42P | Gms-Ams-Cm-Cm-Am-Am-Gf-Am- Uf-Gm-Af-Am-Gm-Um-Um-Um- Cf-Cm-Cm-Am-Am | 605 | UmsEVP-Ufs-Gm-Gf-Gf-Af-Am-Af-Cm- Um-Um-Cm-Am-Uf-Cm-Uf-Um-Gm-Gm- Um-Cms-Ums-Cm | 767 |
| si079-DV42SP | Gms-Ams-Gm-Am-Cm-Cf-Am-Af- Gm-Af-Um-Gm-Am-Am-Gm-Uf- Um-Um-Cm-Am | 606 | UmsEVP-Gfs-Am-Af-Af-Cf-Um-Uf-Cm- Am-Um-Cm-Um-Uf-Gm-Gf-Um-Cm-Um- Cms-Ums-Um | 768 |
| si075-DV43P | Gms-Gms-Am-Gm-Um-Gm-Af-Am- Gf-Am-Gf-Am-Cm-Cm-Am-Am- Gf-Am-Um-Gm-Am | 607 | UmsEVP-Cfs-Af-Um-Cm-Uf-Um-Gf-Gm- Um-Cm-Um-Cm-Uf-Um-Cf-Am-Cm-Um- Cm-Cms-Ams-Am | 769 |
| si751-DV43P | Ams-Gms-Um-Gm-Am-Am-Gf-Am- Gf-Am-Cf-Cm-Am-Am-Gm-Am- Uf-Gm-Am-Am-Am | 608 | UmsEVP-Ufs-Uf-Cm-Am-Uf-Cm-Uf-Um- Gm-Gm-Um-Cm-Uf-Cm-Uf-Um-Cm-Am- Cm-Ums-Cms-Cm | 770 |
| si791-DV43P | Gms-Ams-Gm-Am-Cm-Cm-Af-Am- Gf-Am-Uf-Gm-Am-Am-Gm-Um- Uf-Um-Cm-Cm-Am | 609 | UmsEVP-Gfs-Gf-Am-Am-Af-Cm-Uf-Um- Cm-Am-Um-Cm-Uf-Um-Gf-Gm-Um-Cm- Um-Cms-Ums-Um | 771 |
| si080-DV43P | Gms-Ams-Cm-Cm-Am-Am-Gf-Am- Uf-Gm-Af-Am-Gm-Um-Um-Um- Cf-Cm-Cm-Am-Am | 610 | UmsEVP-Ufs-Gf-Gm-Gm-Af-Am-Af-Cm- Um-Um-Cm-Am-Uf-Cm-Uf-Um-Gm-Gm- Um-Cms-Ums-Cm | 772 |
| si079-DV43SP | Gms-Ams-Gm-Am-Cm-Cf-Am-Af- Gm-Af-Um-Gm-Am-Am-Gm-Uf- Um-Um-Cm-Am | 611 | UmsEVP-Gfs-Af-Am-Am-Cf-Um-Uf-Cm- Am-Um-Cm-Um-Uf-Gm-Gf-Um-Cm-Um- Cms-Ums-Um | 773 |
| si075-DV22 | Gms-Gms-Am-Gm-Um-Gm-Af-Am- Gf-Af-Gf-Am-Cm-Cm-Am-Am- Gm-Am-Um-Gm-Am | 612 | Ums-Cfs-Am-Um-Cm-Uf-Um-Gm-Gm- Um-Cm-Um-Cm-Uf-Um-Cf-Am-Cm-Um- Cm-Cms-Ams-Am | 774 |
| si751-DV22 | Ams-Gms-Um-Gm-Am-Am-Gf-Am- Gf-Af-Cf-Cm-Am-Am-Gm-Am- | 613 | Ums-Ufs-Um-Cm-Am-Uf-Cm-Um-Um- Gm-Gm-Um-Cm-Uf-Cm-Uf-Um-Cm-Am- | 775 |
| | Um-Gm-Am-Am-Am | | Cm-Ums-Cms-Cm | |
| si753-DV22 | Gms-Ams-Am-Gm-Am-Gm-Af-Cm- Cf-Af-Af-Gm-Am-Um-Gm-Am- Am-Gm-Um-Um-Um | 614 | Ams-Afs-Am-Cm-Um-Uf-Cm-Am-Um- Cm-Um-Um-Gm-Gf-Um-Cf-Um-Cm-Um- Um-Cms-Ams-Cm | 776 |
| si791-DV22 | Gms-Ams-Gm-Am-Cm-Cm-Af-Am- Gf-Af-Uf-Gm-Am-Am-Gm-Um- Um-Um-Cm-Cm-Am | 615 | Ums-Gfs-Gm-Am-Am-Af-Cm-Um-Um- Cm-Am-Um-Cm-Uf-Um-Gf-Gm-Um-Cm- Um-Cms-Ums-Um | 777 |
| si014-DV22 | Ams-Gms-Cm-Um-Am-Um-Uf-Um- Uf-Cf-Af-Um-Am-Am-Um-Am- Am-Um-Am-Cm-Um | 616 | Ams-Gfs-Um-Am-Um-Uf-Am-Um-Um- Am-Um-Gm-Am-Af-Am-Af-Um-Am-Gm- Cm-Ums-Ums-Um | 778 |
| si076-DV22 | Gms-Ams-Gm-Um-Gm-Am-Af-Gm- Af-Gf-Af-Cm-Cm-Am-Am-Gm- Am-Um-Gm-Am-Am | 617 | Ums-Ufs-Cm-Am-Um-Cf-Um-Um-Gm- Gm-Um-Cm-Um-Cf-Um-Uf-Cm-Am-Cm- Um-Cms-Cms-Am | 779 |
| si077-DV22 | Gms-Ums-Gm-Am-Am-Gm-Af-Gm- Af-Cf-Cf-Am-Am-Gm-Am-Um- Gm-Am-Am-Gm-Um | 618 | Ams-Cfs-Um-Um-Cm-Af-Um-Cm-Um- Um-Gm-Gm-Um-Cf-Um-Cf-Um-Um-Cm- Am-Cms-Ums-Cm | 780 |
| si078-DV22 | Ams-Ams-Gm-Am-Gm-Am-Cf-Cm- Af-Af-Gf-Am-Um-Gm-Am-Am- Gm-Um-Um-Um-Am | 619 | Ums-Afs-Am-Am-Cm-Uf-Um-Cm-Am- Um-Cm-Um-Um-Gf-Gm-Uf-Cm-Um-Cm- Um-Ums-Cms-Am | 781 |
| si080-DV22 | Gms-Ams-Cm-Cm-Am-Am-Gf-Am- Uf-Gf-Af-Am-Gm-Um-Um-Um- Cm-Cm-Cm-Am-Am | 620 | Ums-Ufs-Gm-Gm-Gm-Af-Am-Am-Cm- Um-Um-Cm-Am-Uf-Cm-Uf-Um-Gm-Gm- Um-Cms-Ums-Cm | 782 |
| si752-DV22S | Gms-Ams-Am-Gm-Am-Gf-Am-Cf- Cf-Af-Am-Gm-Am-Um-Gm-Am- Am-Gm-Um-Um | 621 | Ams-Afs-Cm-Um-Um-Cf-Am-Um-Cm- Um-Um-Gm-Gm-Uf-Cm-Uf-Cm-Um-Um- Cms-Ams-Cm | 783 |
| si079-DV22S | Gms-Ams-Gm-Am-Cm-Cf-Am-Af- Gf-Af-Um-Gm-Am-Am-Gm-Um- Um-Um-Cm-Am | 622 | Ums-Gfs-Am-Am-Am-Cf-Um-Um-Cm- Am-Um-Cm-Um-Uf-Gm-Gf-Um-Cm-Um- Cms-Ums-Um | 784 |
| si047-DV22S | Gms-Ams-Gm-Am-Cm-Af-Am-Gf- Cf-Af-Um-Um-Um-Am-Um-Am- Cm-Um-Um-Um | 623 | Ams-Afs-Am-Gm-Um-Af-Um-Am-Am- Am-Um-Gm-Cm-Uf-Um-Gf-Um-Cm-Um- Cms-Cms-Cm | 785 |
| si754-DV29SP | Gms-Ams-Gm-Um-Gm-Af-Am-Gf- Am-Gf-Am-Cm-Cm-Am-Am-Gf- Am-Um-Gm-Am | 624 | UmsEVP-Cfs-Am-Uf-Cf-Uf-Um-Gm-Gm- Um-Cm-Um-Cm-Uf-Um-Cf-Am-Cm-Um- Cms-Cms-Am | 786 |
| si754-DV26SP | Gms-Ams-Gm-Um-Gm-Af-Am-Gf- Af-Gf-Am-Cm-Cm-Am-Am-Gf- Am-Um-Gm-Am | 625 | UmsEVP-Cfs-Af-Uf-Cm-Uf-Um-Gm-Gm- Um-Cm-Um-Cm-Uf-Um-Cf-Am-Cm-Um- Cms-Cms-Am | 787 |
| si754-DV27SP | Gms-Ams-Gm-Um-Gm-Af-Am-Gf- Af-Gf-Am-Cm-Cm-Am-Am-Gf- Am-Um-Gm-Am | 626 | UmsEVP-Cfs-Am-Uf-Cf-Uf-Um-Gm-Gm- Um-Cm-Um-Cm-Uf-Um-Cf-Am-Cm-Um- Cms-Cms-Am | 788 |
| si754-DV25SP | Gms-Ams-Gm-Um-Gm-Af-Am-Gf- Af-Gf-Am-Cm-Cm-Am-Am-Gf- Am-Um-Gm-Am | 627 | UmsEVP-Cfs-Am-Um-Cm-Uf-Um-Gm- Gm-Um-Cm-Um-Cm-Uf-Um-Cf-Am-Cm- Um-Cms-Cms-Am | 789 |
| si754-DV32SP | Gms-Ams-Gm-Um-Gm-Af-Am-Gf- Am-Gf-Am-Cm-Cm-Am-Am-Gf- Am-Um-Gm-Am | 628 | UmsEVP-Cfs-Am-Um-Cf-Uf-Um-Gm-Gm- Um-Cm-Um-Cm-Uf-Um-Cf-Am-Cm-Um- Cms-Cms-Am | 790 |
| si754-DV34SP | Gms-Ams-Gm-Um-Gm-Af-Am-Gf- Am-Gf-Am-Cm-Cm-Am-Am-Gf- Am-Um-Gm-Am | 629 | UmsEVP-Cfs-Af-Um-Cm-Uf-Um-Gm-Gm- Um-Cm-Um-Cm-Uf-Um-Cf-Am-Cm-Um- Cms-Cms-Am | 791 |
| si754-DV22 | Gms-Ams-Gm-Um-Gm-Af-Am-Gf- Af-Gf-Am-Cm-Cm-Am-Am-Gm- Am-Um-Gm-Am | 630 | Ums-Cfs-Am-Um-Cm-Uf-Um-Gm-Gm- Um-Cm-Um-Cm-Uf-Um-Cf-Am-Cm-Um- Cms-Cms-Am | 792 |
| si755-DV29SP | Gms-Ums-Gm-Am-Am-Gf-Am-Gf- Am-Cf-Cm-Am-Am-Gm-Am-Uf- Gm-Am-Am-Am | 631 | UmsEVP-Ufs-Um-Cf-Af-Uf-Cm-Um-Um- Gm-Gm-Um-Cm-Uf-Cm-Uf-Um-Cm-Am- Cms-Ums-Cm | 793 |
| si755-DV26SP | Gms-Ums-Gm-Am-Am-Gf-Am-Gf- Af-Cf-Cm-Am-Am-Gm-Am-Uf- Gm-Am-Am-Am | 632 | UmsEVP-Ufs-Uf-Cf-Am-Uf-Cm-Um-Um- Gm-Gm-Um-Cm-Uf-Cm-Uf-Um-Cm-Am- Cms-Ums-Cm | 794 |
| si755-DV27SP | Gms-Ums-Gm-Am-Am-Gf-Am-Gf- Af-Cf-Cm-Am-Am-Gm-Am-Uf- Gm-Am-Am-Am | 633 | UmsEVP-Ufs-Um-Cf-Af-Uf-Cm-Um-Um- Gm-Gm-Um-Cm-Uf-Cm-Uf-Um-Cm-Am- Cms-Ums-Cm | 795 |
| si755-DV25SP | Gms-Ums-Gm-Am-Am-Gf-Am-Gf- Af-Cf-Cm-Am-Am-Gm-Am-Uf- Gm-Am-Am-Am | 634 | UmsEVP-Ufs-Um-Cm-Am-Uf-Cm-Um- Um-Gm-Gm-Um-Cm-Uf-Cm-Uf-Um-Cm- Am-Cms-Ums-Cm | 796 |
| si755-DV32SP | Gms-Ums-Gm-Am-Am-Gf-Am-Gf- Am-Cf-Cm-Am-Am-Gm-Am-Uf- Gm-Am-Am-Am | 635 | UmsEVP-Ufs-Um-Cm-Af-Uf-Cm-Um-Um- Gm-Gm-Um-Cm-Uf-Cm-Uf-Um-Cm-Am- Cms-Ums-Cm | 797 |
| si755-DV34SP | Gms-Ums-Gm-Am-Am-Gf-Am-Gf- Am-Cf-Cm-Am-Am-Gm-Am-Uf- Gm-Am-Am-Am | 636 | UmsEVP-Ufs-Uf-Cm-Am-Uf-Cm-Um-Um- Gm-Gm-Um-Cm-Uf-Cm-Uf-Um-Cm-Am- Cms-Ums-Cm | 798 |
| si755-DV22 | Gms-Ums-Gm-Am-Am-Gf-Am-Gf- Af-Cf-Cm-Am-Am-Gm-Am-Um- Gm-Am-Am-Am | 637 | Ums-Ufs-Um-Cm-Am-Uf-Cm-Um-Um- Gm-Gm-Um-Cm-Uf-Cm-Uf-Um-Cm-Am- Cms-Ums-Cm | 799 |
| si756-DV29SP | Ams-Ams-Gm-Am-Gm-Af-Cm-Cf- Am-Af-Gm-Am-Um-Gm-Am-Af- Gm-Um-Um-Um | 638 | AmsEVP-Afs-Am-Cf-Uf-Uf-Cm-Am-Um- Cm-Um-Um-Gm-Gf-Um-Cf-Um-Cm-Um- Ums-Cms-Am | 800 |
| si756-DV26SP | Ams-Ams-Gm-Am-Gm-Af-Cm-Cf- Af-Af-Gm-Am-Um-Gm-Am-Af- Gm-Um-Um-Um | 639 | AmsEVP-Afs-Af-Cf-Um-Uf-Cm-Am-Um- Cm-Um-Um-Gm-Gf-Um-Cf-Um-Cm-Um- Ums-Cms-Am | 801 |
| si756-DV27SP | Ams-Ams-Gm-Am-Gm-Af-Cm-Cf- Af-Af-Gm-Am-Um-Gm-Am-Af- Gm-Um-Um-Um | 640 | AmsEVP-Afs-Am-Cf-Uf-Uf-Cm-Am-Um- Cm-Um-Um-Gm-Gf-Um-Cf-Um-Cm-Um- Ums-Cms-Am | 802 |
| si756-DV25SP | Ams-Ams-Gm-Am-Gm-Af-Cm-Cf- Af-Af-Gm-Am-Um-Gm-Am-Af- Gm-Um-Um-Um | 641 | AmsEVP-Afs-Am-Cm-Um-Uf-Cm-Am- Um-Cm-Um-Um-Gm-Gf-Um-Cf-Um-Cm- Um-Ums-Cms-Am | 803 |
| si756-DV32SP | Ams-Ams-Gm-Am-Gm-Af-Cm-Cf- Am-Af-Gm-Am-Um-Gm-Am-Af- Gm-Um-Um-Um | 642 | AmsEVP-Afs-Am-Cm-Uf-Uf-Cm-Am-Um- Cm-Um-Um-Gm-Gf-Um-Cf-Um-Cm-Um- Ums-Cms-Am | 804 |
| si756-DV34SP | Ams-Ams-Gm-Am-Gm-Af-Cm-Cf- Am-Af-Gm-Am-Um-Gm-Am-Af- Gm-Um-Um-Um | 643 | AmsEVP-Afs-Af-Cm-Um-Uf-Cm-Am-Um- Cm-Um-Um-Gm-Gf-Um-Cf-Um-Cm-Um- Ums-Cms-Am | 805 |
| si756-DV22 | Ams-Ams-Gm-Am-Gm-Af-Cm-Cf- | 644 | Ams-Afs-Am-Cm-Um-Uf-Cm-Am-Um- | 806 |
| | Af-Af-Gm-Am-Um-Gm-Am-Am- Gm-Um-Um-Um | | Cm-Um-Um-Gm-Gf-Um-Cf-Um-Cm-Um- Ums-Cms-Am | |
| si792-DV29SP | Ams-Gms-Am-Cm-Cm-Af-Am-Gf- Am-Uf-Gm-Am-Am-Gm-Um-Uf- Um-Cm-Cm-Am | 645 | UmsEVP-Gfs-Gm-Af-Af-Af-Cm-Um-Um- Cm-Am-Um-Cm-Uf-Um-Gf-Gm-Um-Cm- Ums-Cms-Um | 807 |
| si792-DV26SP | Ams-Gms-Am-Cm-Cm-Af-Am-Gf- Af-Uf-Gm-Am-Am-Gm-Um-Uf- Um-Cm-Cm-Am | 646 | UmsEVP-Gfs-Gf-Af-Am-Af-Cm-Um-Um- Cm-Am-Um-Cm-Uf-Um-Gf-Gm-Um-Cm- Ums-Cms-Um | 808 |
| si792-DV27SP | Ams-Gms-Am-Cm-Cm-Af-Am-Gf- Af-Uf-Gm-Am-Am-Gm-Um-Uf- Um-Cm-Cm-Am | 647 | UmsEVP-Gfs-Gm-Af-Af-Af-Cm-Um-Um- Cm-Am-Um-Cm-Uf-Um-Gf-Gm-Um-Cm- Ums-Cms-Um | 809 |
| si792-DV25SP | Ams-Gms-Am-Cm-Cm-Af-Am-Gf- Af-Uf-Gm-Am-Am-Gm-Um-Uf- Um-Cm-Cm-Am | 648 | UmsEVP-Gfs-Gm-Am-Am-Af-Cm-Um- Um-Cm-Am-Um-Cm-Uf-Um-Gf-Gm-Um- Cm-Ums-Cms-Um | 810 |
| si792-DV32SP | Ams-Gms-Am-Cm-Cm-Af-Am-Gf- Am-Uf-Gm-Am-Am-Gm-Um-Uf- Um-Cm-Cm-Am | 649 | UmsEVP-Gfs-Gm-Am-Af-Af-Cm-Um-Um- Cm-Am-Um-Cm-Uf-Um-Gf-Gm-Um-Cm- Ums-Cms-Um | 811 |
| si792-DV34SP | Ams-Gms-Am-Cm-Cm-Af-Am-Gf- Am-Uf-Gm-Am-Am-Gm-Um-Uf- Um-Cm-Cm-Am | 650 | UmsEVP-Gfs-Gf-Am-Am-Af-Cm-Um-Um- Cm-Am-Um-Cm-Uf-Um-Gf-Gm-Um-Cm- Ums-Cms-Um | 812 |
| si792-DV22 | Ams-Gms-Am-Cm-Cm-Af-Am-Gf- Af-Uf-Gm-Am-Am-Gm-Um-Um- Um-Cm-Cm-Am | 651 | Ums-Gfs-Gm-Am-Am-Af-Cm-Um-Um- Cm-Am-Um-Cm-Uf-Um-Gf-Gm-Um-Cm- Ums-Cms-Um | 813 |
| si141-DV29SP | Gms-Cms-Um-Am-Um-Uf-Um-Uf- Cm-Af-Um-Am-Am-Um-Am-Af- Um-Am-Cm-Um | 652 | AmsEVP-Gfs-Um-Af-Uf-Uf-Am-Um-Um- Am-Um-Gm-Am-Af-Am-Af-Um-Am-Gm- Cms-Ums-Um | 814 |
| si141-DV26SP | Gms-Cms-Um-Am-Um-Uf-Um-Uf- Cf-Af-Um-Am-Am-Um-Am-Af- Um-Am-Cm-Um | 653 | AmsEVP-Gfs-Uf-Af-Um-Uf-Am-Um-Um- Am-Um-Gm-Am-Af-Am-Af-Um-Am-Gm- Cms-Ums-Um | 815 |
| si141-DV27SP | Gms-Cms-Um-Am-Um-Uf-Um-Uf- Cf-Af-Um-Am-Am-Um-Am-Af- Um-Am-Cm-Um | 654 | AmsEVP-Gfs-Um-Af-Uf-Uf-Am-Um-Um- Am-Um-Gm-Am-Af-Am-Af-Um-Am-Gm- Cms-Ums-Um | 816 |
| si141-DV25SP | Gms-Cms-Um-Am-Um-Uf-Um-Uf- Cf-Af-Um-Am-Am-Um-Am-Af- Um-Am-Cm-Um | 655 | AmsEVP-Gfs-Um-Am-Um-Uf-Am-Um- Um-Am-Um-Gm-Am-Af-Am-Af-Um-Am- Gm-Cms-Ums-Um | 817 |
| si141-DV32SP | Gms-Cms-Um-Am-Um-Uf-Um-Uf- Cm-Af-Um-Am-Am-Um-Am-Af- Um-Am-Cm-Um | 656 | AmsEVP-Gfs-Um-Am-Uf-Uf-Am-Um-Um- Am-Um-Gm-Am-Af-Am-Af-Um-Am-Gm- Cms-Ums-Um | 818 |
| si141-DV34SP | Gms-Cms-Um-Am-Um-Uf-Um-Uf- Cm-Af-Um-Am-Am-Um-Am-Af- Um-Am-Cm-Um | 657 | AmsEVP-Gfs-Uf-Am-Um-Uf-Am-Um-Um- Am-Um-Gm-Am-Af-Am-Af-Um-Am-Gm- Cms-Ums-Um | 819 |
| si141-DV22 | Gms-Cms-Um-Am-Um-Uf-Um-Uf- Cf-Af-Um-Am-Am-Um-Am-Am- Um-Am-Cm-Um | 658 | Ams-Gfs-Um-Am-Um-Uf-Am-Um-Um- Am-Um-Gm-Am-Af-Am-Af-Um-Am-Gm- Cms-Ums-Um | 820 |
| si761-DV29SP | Ams-Gms-Um-Gm-Am-Af-Gm-Af- Gm-Af-Cm-Cm-Am-Am-Gm-Af- | 659 | UmsEVP-Ufs-Cm-Af-Uf-Cf-Um-Um-Gm- Gm-Um-Cm-Um-Cf-Um-Uf-Cm-Am-Cm- | 821 |
| | Um-Gm-Am-Am | | Ums-Cms-Cm | |
| si761-DV26SP | Ams-Gms-Um-Gm-Am-Af-Gm-Af- Gf-Af-Cm-Cm-Am-Am-Gm-Af- Um-Gm-Am-Am | 660 | UmsEVP-Ufs-Cf-Af-Um-Cf-Um-Um-Gm- Gm-Um-Cm-Um-Cf-Um-Uf-Cm-Am-Cm- Ums-Cms-Cm | 822 |
| si761-DV27SP | Ams-Gms-Um-Gm-Am-Af-Gm-Af- Gf-Af-Cm-Cm-Am-Am-Gm-Af- Um-Gm-Am-Am | 661 | UmsEVP-Ufs-Cm-Af-Uf-Cf-Um-Um-Gm- Gm-Um-Cm-Um-Cf-Um-Uf-Cm-Am-Cm- Ums-Cms-Cm | 823 |
| si761-DV25SP | Ams-Gms-Um-Gm-Am-Af-Gm-Af- Gf-Af-Cm-Cm-Am-Am-Gm-Af- Um-Gm-Am-Am | 662 | UmsEVP-Ufs-Cm-Am-Um-Cf-Um-Um- Gm-Gm-Um-Cm-Um-Cf-Um-Uf-Cm-Am- Cm-Ums-Cms-Cm | 824 |
| si761-DV32SP | Ams-Gms-Um-Gm-Am-Af-Gm-Af- Gm-Af-Cm-Cm-Am-Am-Gm-Af- Um-Gm-Am-Am | 663 | UmsEVP-Ufs-Cm-Am-Uf-Cf-Um-Um-Gm- Gm-Um-Cm-Um-Cf-Um-Uf-Cm-Am-Cm- Ums-Cms-Cm | 825 |
| si761-DV34SP | Ams-Gms-Um-Gm-Am-Af-Gm-Af- Gm-Af-Cm-Cm-Am-Am-Gm-Af- Um-Gm-Am-Am | 664 | UmsEVP-Ufs-Cf-Am-Um-Cf-Um-Um-Gm- Gm-Um-Cm-Um-Cf-Um-Uf-Cm-Am-Cm- Ums-Cms-Cm | 826 |
| si761-DV22 | Ams-Gms-Um-Gm-Am-Af-Gm-Af- Gf-Af-Cm-Cm-Am-Am-Gm-Am- Um-Gm-Am-Am | 665 | Ums-Ufs-Cm-Am-Um-Cf-Um-Um-Gm- Gm-Um-Cm-Um-Cf-Um-Uf-Cm-Am-Cm- Ums-Cms-Cm | 827 |
| si771-DV29SP | Ums-Gms-Am-Am-Gm-Af-Gm-Af- Cm-Cf-Am-Am-Gm-Am-Um-Gf- Am-Am-Gm-Um | 666 | AmsEVP-Cfs-Um-Uf-Cf-Af-Um-Cm-Um- Um-Gm-Gm-Um-Cf-Um-Cf-Um-Um-Cm- Ams-Cms-Um | 828 |
| si771-DV26SP | Ums-Gms-Am-Am-Gm-Af-Gm-Af- Cf-Cf-Am-Am-Gm-Am-Um-Gf- Am-Am-Gm-Um | 667 | AmsEVP-Cfs-Uf-Uf-Cm-Af-Um-Cm-Um- Um-Gm-Gm-Um-Cf-Um-Cf-Um-Um-Cm- Ams-Cms-Um | 829 |
| si771-DV27SP | Ums-Gms-Am-Am-Gm-Af-Gm-Af- Cf-Cf-Am-Am-Gm-Am-Um-Gf- Am-Am-Gm-Um | 668 | AmsEVP-Cfs-Um-Uf-Cf-Af-Um-Cm-Um- Um-Gm-Gm-Um-Cf-Um-Cf-Um-Um-Cm- Ams-Cms-Um | 830 |
| si771-DV25SP | Ums-Gms-Am-Am-Gm-Af-Gm-Af- Cf-Cf-Am-Am-Gm-Am-Um-Gf- Am-Am-Gm-Um | 669 | AmsEVP-Cfs-Um-Um-Cm-Af-Um-Cm- Um-Um-Gm-Gm-Um-Cf-Um-Cf-Um-Um- Cm-Ams-Cms-Um | 831 |
| si771-DV32SP | Ums-Gms-Am-Am-Gm-Af-Gm-Af- Cm-Cf-Am-Am-Gm-Am-Um-Gf- Am-Am-Gm-Um | 670 | AmsEVP-Cfs-Um-Um-Cf-Af-Um-Cm-Um- Um-Gm-Gm-Um-Cf-Um-Cf-Um-Um-Cm- Ams-Cms-Um | 832 |
| si771-DV34SP | Ums-Gms-Am-Am-Gm-Af-Gm-Af- Cm-Cf-Am-Am-Gm-Am-Um-Gf- Am-Am-Gm-Um | 671 | AmsEVP-Cfs-Uf-Um-Cm-Af-Um-Cm-Um- Um-Gm-Gm-Um-Cf-Um-Cf-Um-Um-Cm- Ams-Cms-Um | 833 |
| si771-DV22 | Ums-Gms-Am-Am-Gm-Af-Gm-Af- Cf-Cf-Am-Am-Gm-Am-Um-Gm- Am-Am-Gm-Um | 672 | Ams-Cfs-Um-Um-Cm-Af-Um-Cm-Um- Um-Gm-Gm-Um-Cf-Um-Cf-Um-Um-Cm- Ams-Cms-Um | 834 |
| si781-DV29SP | Ams-Gms-Am-Gm-Am-Cf-Cm-Af- Am-Gf-Am-Um-Gm-Am-Am-Gf- Um-Um-Um-Am | 673 | UmsEVP-Afs-Am-Af-Cf-Uf-Um-Cm-Am- Um-Cm-Um-Um-Gf-Gm-Uf-Cm-Um-Cm- Ums-Ums-Cm | 835 |
| si781-DV26SP | Ams-Gms-Am-Gm-Am-Cf-Cm-Af- Af-Gf-Am-Um-Gm-Am-Am-Gf- Um-Um-Um-Am | 674 | UmsEVP-Afs-Af-Af-Cm-Uf-Um-Cm-Am- Um-Cm-Um-Um-Gf-Gm-Uf-Cm-Um-Cm- Ums-Ums-Cm | 836 |
| si781-DV27SP | Ams-Gms-Am-Gm-Am-Cf-Cm-Af- Af-Gf-Am-Um-Gm-Am-Am-Gf- Um-Um-Um-Am | 675 | UmsEVP-Afs-Am-Af-Cf-Uf-Um-Cm-Am- Um-Cm-Um-Um-Gf-Gm-Uf-Cm-Um-Cm- Ums-Ums-Cm | 837 |
| si781-DV25SP | Ams-Gms-Am-Gm-Am-Cf-Cm-Af- Af-Gf-Am-Um-Gm-Am-Am-Gf- Um-Um-Um-Am | 676 | UmsEVP-Afs-Am-Am-Cm-Uf-Um-Cm- Am-Um-Cm-Um-Um-Gf-Gm-Uf-Cm-Um- Cm-Ums-Ums-Cm | 838 |
| si781-DV32SP | Ams-Gms-Am-Gm-Am-Cf-Cm-Af- Am-Gf-Am-Um-Gm-Am-Am-Gf- Um-Um-Um-Am | 677 | UmsEVP-Afs-Am-Am-Cf-Uf-Um-Cm-Am- Um-Cm-Um-Um-Gf-Gm-Uf-Cm-Um-Cm- Ums-Ums-Cm | 839 |
| si781-DV34SP | Ams-Gms-Am-Gm-Am-Cf-Cm-Af- Am-Gf-Am-Um-Gm-Am-Am-Gf- Um-Um-Um-Am | 678 | UmsEVP-Afs-Af-Am-Cm-Uf-Um-Cm-Am- Um-Cm-Um-Um-Gf-Gm-Uf-Cm-Um-Cm- Ums-Ums-Cm | 840 |
| si781-DV22 | Ams-Gms-Am-Gm-Am-Cf-Cm-Af- Af-Gf-Am-Um-Gm-Am-Am-Gm- Um-Um-Um-Am | 679 | Ums-Afs-Am-Am-Cm-Uf-Um-Cm-Am- Um-Cm-Um-Um-Gf-Gm-Uf-Cm-Um-Cm- Ums-Ums-Cm | 841 |
| si801-DV29SP | Ams-Cms-Cm-Am-Am-Gf-Am-Uf- Gm-Af-Am-Gm-Um-Um-Um-Cf- Cm-Cm-Am-Am | 680 | UmsEVP-Ufs-Gm-Gf-Gf-Af-Am-Am-Cm- Um-Um-Cm-Am-Uf-Cm-Uf-Um-Gm-Gm- Ums-Cms-Um | 842 |
| si801-DV26SP | Ams-Cms-Cm-Am-Am-Gf-Am-Uf- Gf-Af-Am-Gm-Um-Um-Um-Cf- Cm-Cm-Am-Am | 681 | UmsEVP-Ufs-Gf-Gf-Gm-Af-Am-Am-Cm- Um-Um-Cm-Am-Uf-Cm-Uf-Um-Gm-Gm- Ums-Cms-Um | 843 |
| si801-DV27SP | Ams-Cms-Cm-Am-Am-Gf-Am-Uf- Gf-Af-Am-Gm-Um-Um-Um-Cf- Cm-Cm-Am-Am | 682 | UmsEVP-Ufs-Gm-Gf-Gf-Af-Am-Am-Cm- Um-Um-Cm-Am-Uf-Cm-Uf-Um-Gm-Gm- Ums-Cms-Um | 844 |
| si801-DV25SP | Ams-Cms-Cm-Am-Am-Gf-Am-Uf- Gf-Af-Am-Gm-Um-Um-Um-Cf- Cm-Cm-Am-Am | 683 | UmsEVP-Ufs-Gm-Gm-Gm-Af-Am-Am- Cm-Um-Um-Cm-Am-Uf-Cm-Uf-Um-Gm- Gm-Ums-Cms-Um | 845 |
| si801-DV32SP | Ams-Cms-Cm-Am-Am-Gf-Am-Uf- Gm-Af-Am-Gm-Um-Um-Um-Cf- Cm-Cm-Am-Am | 684 | UmsEVP-Ufs-Gm-Gm-Gf-Af-Am-Am-Cm- Um-Um-Cm-Am-Uf-Cm-Uf-Um-Gm-Gm- Ums-Cms-Um | 846 |
| si801-DV34SP | Ams-Cms-Cm-Am-Am-Gf-Am-Uf- Gm-Af-Am-Gm-Um-Um-Um-Cf- Cm-Cm-Am-Am | 685 | UmsEVP-Ufs-Gf-Gm-Gm-Af-Am-Am-Cm- Um-Um-Cm-Am-Uf-Cm-Uf-Um-Gm-Gm- Ums-Cms-Um | 847 |
| si801-DV22 | Ams-Cms-Cm-Am-Am-Gf-Am-Uf- Gf-Af-Am-Gm-Um-Um-Um-Cm- Cm-Cm-Am-Am | 686 | Ums-Ufs-Gm-Gm-Gm-Af-Am-Am-Cm- Um-Um-Cm-Am-Uf-Cm-Uf-Um-Gm-Gm- Ums-Cms-Um | 848 |

### 2. Experimental methods

The inhibitory effects of the test samples on INHBE mRNA expression in Huh7 cells were evaluated by qRT-PCR.

### 2.1 Cell culture

Subcultured Huh7 cells in the logarithmic growth phase were cultured in DMEM culture medium containing 10% fetal bovine serum (supplemented with penicillin and streptomycin at 100 µL/mL), and incubated in a cell incubator at 37°C with 5% CO₂. The medium was exchanged once daily. The cells were digested and passaged using 0.25% trypsin, centrifuged at 1000 r/min for 5 min, the supernatant was discarded, and fresh medium was added for continued passaging and culture.

### 2.2 Cell transfection

Preparation of transfection mixture: Lipofectamine RNAiMAX was mixed with Opti-MEM at a volume ratio of 2:98 and vortexed thoroughly.

siRNA dilution: The siRNA samples were diluted to appropriate concentrations using Opti-MEM to prepare siRNA solutions to be tested.

Preparation of transfection reagent: 60 µL of siRNA solution (diluted in Opti-MEM) was mixed with 60 µL of transfection mixture at a volume ratio of 1:1, vortexed thoroughly, followed by incubation at room temperature for 15 minutes to obtain lipid nanoparticles (LNPs). A 12.5 µL aliquot was taken for encapsulation efficiency analysis.

Blank control (transfection reagent): 60 µL of transfection mixture was added to 60 µL of Opti-MEM, vortexed thoroughly, followed by incubation at room temperature for 15 minutes.

The prepared transfection reagents with different siRNA concentrations were added at 100 µL per well to a 24-well cell culture plate pre-filled with 500 µL of cell suspension per well (containing 1.0 × 10⁵ cells per mL), resulting in a final siRNA concentration of 0.02 nM or 0.07 nM per well. After cross-mixing, the plate was incubated in a cell incubator at 37°C with 5% CO₂ for 40 hours.

### 2.3 INHBE mRNA analysis

The procedure is the same as described in "(2.3) INHBE mRNA analysis" of Example 2.

### 2.4 Calculation of inhibition rate

The calculation was performed as described in Example 2.

### 2.5 IC₅₀ assay

The siRNA derivatives used for IC₅₀ determination were subjected to serial dilution starting from 1.0 nM with 4-fold dilutions, totaling 8 concentration points (1.0 nM, 0.25 nM, 0.0625 nM, 15.6 pM, 3.9 pM, 0.977 pM, 0.244 pM, and 0.061 pM). The inhibition rates of these derivatives at each concentration were measured, and the resulting data were plotted to calculate IC₅₀ values.

### 3. Experimental results

(1) Based on three independent experiments, the inhibition rates of these modified sequences against the INHBE gene in Huh7 cells are shown in Table 13.

The activity results demonstrated that the sequences modified using the templates DV25P to DV27P, DV29P, DV32P, DV34P, DV41P to DV43P, DV25SP to DV27SP, DV29SP, DV32SP, DV34SP, and DV41SP to DV43SP exhibited significant inhibitory effects on INHBE gene expression, with inhibition rates exceeding 40% at a concentration of 20 pM.

**Table 13**

| **siRNA ID** | **Inhibition rate (%)** | | **siRNA ID** | **Inhibition rate (%)** | | **siRNA ID** | **Inhibition rate (%)** | |
|---|---|---|---|---|---|---|---|---|
| | **0.02 nM** | **0.07 nM** | | **0.02 nM** | **0.07 nM** | | **0.02 nM** | **0.07 nM** |
| si791-DV26P | 50.5 | 67.5 | si752-DV25SP | 39.9 | 44.9 | si077-DV34P | 29.2 | 52.1 |
| si047-DV34SP | 50.4 | 65.4 | si791-DV25P | 39.8 | 60.9 | si075-DV25P | 28.8 | 35.4 |
| si079-DV25SP | 50.3 | 68.1 | si076-DV25P | 39.8 | 48.7 | si075-DV41P | 27.4 | 34.9 |
| si047-DV26SP | 50.2 | 60.8 | si079-DV41SP | 39.7 | 44.3 | si075-DV32P | 26.5 | 31.6 |
| si791-DV27P | 50.1 | 58.6 | si791-DV34P | 39.1 | 53.8 | si753-DV29P | 26.4 | 37.8 |
| si014-DV25P | 49.9 | 52.1 | si751-DV34P | 38.8 | 68.2 | si076-DV26P | 25.9 | 33.1 |
| si791-DV29P | 48.9 | 58.9 | si076-DV27P | 37.9 | 42.1 | si080-DV34P | 24.1 | 36.9 |
| si751-DV25P | 48.3 | 70.5 | si751-DV26P | 37.7 | 71.7 | si752-DV32SP | 22.8 | 55.4 |
| si047-DV29SP | 48.2 | 59.6 | si075-DV34P | 36.8 | 48.2 | si080-DV25P | 21.7 | 39.8 |
| si047-DV25SP | 47.5 | 54.3 | si080-DV42P | 36.7 | 46.0 | si077-DV32P | 21.3 | 42.1 |
| si047-DV27SP | 47.4 | 70.1 | si791-DV43P | 36.2 | 51.1 | si751-DV43P | 21.3 | 24.4 |
| si014-DV32P | 47.0 | 50.8 | si077-DV25P | 35.2 | 43.3 | si752-DV27SP | 21.2 | 35.6 |
| si079-DV34SP | 46.0 | 71.6 | si751-DV32P | 35.2 | 66.6 | si075-DV27P | 20.7 | 39.8 |
| si751-DV27P | 44.0 | 72.5 | si753-DV26P | 34.8 | 64.4 | si078-DV29P | 20.6 | 24.9 |
| si079-DV27SP | 44.0 | 72.6 | si014-DV26P | 34.6 | 53.2 | si753-DV34P | 20.6 | 27.7 |
| si014-DV34P | 43.3 | 46.7 | si752-DV34SP | 34.6 | 65.0 | si077-DV29P | 20.5 | 39.0 |
| si079-DV26SP | 43.1 | 69.4 | si080-DV41P | 34.2 | 38.8 | si076-DV32P | 20.5 | 62.8 |
| si079-DV29SP | 43.1 | 68.2 | si791-DV41P | 33.2 | 37.6 | si078-DV34P | 20.2 | 43.9 |
| si079-DV42SP | 42.3 | 44.0 | si791-DV42P | 33.1 | 47.1 | si753-DV32P | 19.7 | 23.4 |
| si751-DV29P | 41.9 | 53.4 | si752-DV26SP | 32.8 | 47.8 | si076-DV29P | 18.7 | 23.5 |
| si753-DV25P | 41.6 | 49.8 | si753-DV27P | 32.7 | 39.6 | si080-DV26P | 18.2 | 29.2 |
| si079-DV43SP | 41.5 | 56.2 | si751-DV41P | 32.7 | 33.0 | si752-DV29SP | 17.4 | 24.7 |
| si078-DV25P | 41.4 | 61.4 | si014-DV29P | 32.5 | 58.1 | si080-DV27P | 16.8 | 32.7 |
| si079-DV32SP | 41.3 | 58.9 | si080-DV43P | 32.5 | 39.8 | si076-DV34P | 15.8 | 39.2 |
| si047-DV32SP | 40.9 | 47.9 | si014-DV27P | 31.9 | 50.9 | si078-DV32P | 15.3 | 21.3 |
| si791-DV32P | 40.8 | 65.9 | si077-DV26P | 31.8 | 55.9 | si075-DV43P | 15.2 | 28.7 |
| si077-DV27P | 40.7 | 40.9 | si080-DV29P | 30.1 | 32.3 | si080-DV32P | 12.9 | 22.1 |
| si078-DV26P | 40.4 | 56.2 | si751-DV42P | 29.8 | 39.0 | si075-DV29P | 11.9 | 32.8 |
| si078-DV27P | 40.3 | 46.5 | si075-DV42P | 29.5 | 42.1 | si075-DV26P | 10.6 | 52.7 |

(2) The same siRNA sequence modified using different templates varies in activity.

**Table 14: Inhibition rates of INHBE mRNA expression by si079 modified using different templates (0.02 nM)**

| siRNA ID | Inhibition rate (%) |
|---|---|
| si079-DV25SP | 50.3 |
| si079-DV26SP | 43.1 |
| si079-DV27SP | 44.0 |
| si079-DV29SP | 43.1 |
| si079-DV32SP | 41.3 |
| si079-DV34SP | 46.0 |
| si079-DV41SP | 39.7 |
| si079-DV42SP | 42.3 |
| si079-DV43SP | 41.5 |

As shown in Table 14, the same siRNA sequence modified using different templates exhibited differences in activity. For example, si079 modified with DV25SP exhibited an inhibition rate 10.6% higher than that of si079 modified with DV41SP.

### (3) IC₅₀ assay

A total of 16 template-modified sequences, including si751-DV25P, si791-DV26P, si047-DV34SP, si791-DV27P, si079-DV25SP, si079-DV27SP, si079-DV29SP, si047-DV26SP, si079-DV34SP, si751-DV27P, si047-DV29SP, si791-DV29P, si047-DV25SP, si014-DV25P, si014-DV34P, and si014-DV32P, were selected for IC₅₀ determination. The experimental results are shown in Table 15.

**Table 15: IC₅₀ assay results for modified sequences**

| siRNA ID | IC₅₀ (nM) | siRNA ID | IC₅₀ (nM) |
|---|---|---|---|
| si751-DV25P | 0.0004 | si079-DV34SP | 0.0058 |
| si791-DV26P | 0.0027 | si751-DV27P | 0.0071 |
| si047-DV34SP | 0.0034 | si047-DV29SP | 0.0113 |
| si791-DV27P | 0.0036 | si791-DV29P | 0.0217 |
| si079-DV25SP | 0.0037 | si047-DV25SP | 0.024 |
| si079-DV27SP | 0.004 | si014-DV25P | N/A |
| si079-DV29SP | 0.0051 | si014-DV34P | N/A |
| si047-DV26SP | 0.0054 | si014-DV32P | N/A |

In Table 15, "N/A" indicates that the inhibition plateau was too low to allow reliable IC₅₀ fitting.

As shown in Table 15, the IC₅₀ values for these 16 sequences ranged from 0.0004 nM to 0.0240 nM, indicating that INHBE gene expression can be effectively inhibited at low concentrations.

### Example 4: Inhibition of target genes and potential off-target genes by siRNA derivatives with anti-off-target modifications

In the prior art, there is a modification pattern in which the nucleotide at position 6 or 7 of the antisense strand of siRNA is replaced with glycol nucleic acid (GNA) to disrupt the seed region of the antisense strand, thereby significantly reducing off-target effects and lowering hepatotoxicity. In addition, studies have shown that replacing all base pairs at positions 1 to 8 from the 5' end of the antisense strand of siRNA with corresponding DNA nucleotides in both the sense and antisense strands can significantly reduce off-target effects without compromising the activity of siRNA.

Therefore, in this example, in order to reduce off-target effects, additional anti-off-target modifications were introduced based on the siRNA derivatives of the present disclosure. Specifically, the nucleotide at position 7, or at positions 7 and 11, of the antisense strand was replaced with DNA, and the nucleotide at the position of the sense strand that is complementary to position 7 of the antisense strand was also replaced with DNA, thereby reducing off-target effects. The codes used and corresponding technical meanings are as follows:
d7B11 anti-off-target modification: the nucleotides at positions 7 and 11 of the antisense strand were replaced with DNA, and the nucleotide at the position of the sense strand that is complementary to position 7 of the antisense strand was also replaced with DNA.
d7B anti-off-target modification: the nucleotide at position 7 of the antisense strand was replaced with DNA, and the nucleotide at the position of the sense strand that is complementary to position 7 of the antisense strand was also replaced with DNA.

**Table 16: Examples of siRNA conjugates disclosed herein with or without anti-off-target modifications**

| siRNA ID | Sense sequence (5'-3') | SEQ ID NO: | Antisense sequence (5'-3') | SEQ ID NO: |
|---|---|---|---|---|
| si751-DV25PG101 | Ams-Gms-Um-Gm-Am-Am-Gf- Am-Gf-Af-Cf-Cm-Am-Am-Gm- Am-Uf-Gm-Am-Am-Am-G101 | 849 | UmsEVP-Ufs-Um-Cm-Am-Uf-Cm- Um-Um-Gm-Gm-Um-Cm-Uf-Cm-Uf- Um-Cm-Am-Cm-Ums-Cms-Cm | 877 |
| si751-DV25Pd7BG101 | Ams-Gms-Um-Gm-Am-Am-Gf- Am-Gf-Af-Cf-Cm-Am-Am-Gd- Am-Uf-Gm-Am-Am-Am-G101 | 850 | UmsEVP-Ufs-Um-Cm-Am-Uf-Cd- Um-Um-Gm-Gm-Um-Cm-Uf-Cm-Uf- Um-Cm-Am-Cm-Ums-Cms-Cm | 878 |
| si755-DV25SPG101 | Gms-Ums-Gm-Am-Am-Gf-Am- Gf-Af-Cf-Cm-Am-Am-Gm-Am- Uf-Gm-Am-Am-Am-G101 | 851 | UmsEVP-Ufs-Um-Cm-Am-Uf-Cm- Um-Um-Gm-Gm-Um-Cm-Uf-Cm-Uf- Um-Cm-Am-Cms-Ums-Cm | 879 |
| si755-DV25SPd7BG101 | Gms-Ums-Gm-Am-Am-Gf-Am- Gf-Af-Cf-Cm-Am-Am-Gd-Am- Uf-Gm-Am-Am-Am-G101 | 852 | UmsEVP-Ufs-Um-Cm-Am-Uf-Cd- Um-Um-Gm-Gm-Um-Cm-Uf-Cm-Uf- Um-Cm-Am-Cms-Ums-Cm | 880 |
| si755-DV25SPd7B11G101 | Gms-Ums-Gm-Am-Am-Gf-Am- Gf-Af-Cf-Cm-Am-Am-Gd-Am- Uf-Gm-Am-Am-Am-G101 | 853 | UmsEVP-Ufs-Um-Cm-Am-Uf-Cd- Um-Um-Gm-Gd-Um-Cm-Uf-Cm-Uf- Um-Cm-Am-Cms-Ums-Cm | 881 |
| si079-DV27SPG101 | Gms-Ams-Gm-Am-Cm-Cf-Am- Af-Gf-Af-Um-Gm-Am-Am-Gm- Uf-Um-Um-Cm-Am-G101 | 854 | UmsEVP-Gfs-Am-Af-Af-Cf-Um-Um- Cm-Am-Um-Cm-Um-Uf-Gm-Gf-Um- Cm-Um-Cms-Ums-Um | 882 |
| si079-DV34SPG101 | Gms-Ams-Gm-Am-Cm-Cf-Am- Af-Gm-Af-Um-Gm-Am-Am-Gm- Uf-Um-Um-Cm-Am-G101 | 855 | UmsEVP-Gfs-Af-Am-Am-Cf-Um- Um-Cm-Am-Um-Cm-Um-Uf-Gm-Gf- Um-Cm-Um-Cms-Ums-Um | 883 |
| si079-DV34SPd7BG101 | Gms-Ams-Gm-Am-Cm-Cf-Am- Af-Gm-Af-Um-Gm-Am-Ad-Gm- Uf-Um-Um-Cm-Am-G101 | 856 | UmsEVP-Gfs-Af-Am-Am-Cf-Td-Um- Cm-Am-Um-Cm-Um-Uf-Gm-Gf-Um- Cm-Um-Cms-Ums-Um | 884 |
| si079-DV34SPd7B11G101 | Gms-Ams-Gm-Am-Cm-Cf-Am- Af-Gm-Af-Um-Gm-Am-Ad-Gm- Uf-Um-Um-Cm-Am-G101 | 857 | UmsEVP-Gfs-Af-Am-Am-Cf-Td-Um- Cm-Am-Td-Cm-Um-Uf-Gm-Gf-Um- Cm-Um-Cms-Ums-Um | 885 |
| si047-DV34SPG101 | Gms-Ams-Gm-Am-Cm-Af-Am- Gf-Cm-Af-Um-Um-Um-Am-Um- Af-Cm-Um-Um-Um-G101 | 858 | AmsEVP-Afs-Af-Gm-Um-Af-Um- Am-Am-Am-Um-Gm-Cm-Uf-Um-Gf- Um-Cm-Um-Cms-Cms-Cm | 886 |
| si047-DV34SPd7BG101 | Gms-Ams-Gm-Am-Cm-Af-Am- Gf-Cm-Af-Um-Um-Um-Ad-Um- Af-Cm-Um-Um-Um-G101 | 859 | AmsEVP-Afs-Af-Gm-Um-Af-Td-Am- Am-Am-Um-Gm-Cm-Uf-Um-Gf-Um- Cm-Um-Cms-Cms-Cm | 887 |
| si047-DV34SPd7B11G101 | Gms-Ams-Gm-Am-Cm-Af-Am- Gf-Cm-Af-Um-Um-Um-Ad-Um- Af-Cm-Um-Um-Um-G101 | 860 | AmsEVP-Afs-Af-Gm-Um-Af-Td-Am- Am-Am-Td-Gm-Cm-Uf-Um-Gf-Um- Cm-Um-Cms-Cms-Cm | 888 |
| si791-DV26PG101 | Gms-Ams-Gm-Am-Cm-Cm-Af- Am-Gf-Af-Uf-Gm-Am-Am-Gm- Um-Uf-Um-Cm-Cm-Am-G101 | 861 | UmsEVP-Gfs-Gf-Af-Am-Af-Cm-Um- Um-Cm-Am-Um-Cm-Uf-Um-Gf-Gm- Um-Cm-Um-Cms-Ums-Um | 889 |
| si791-DV26SPd7BG101 | Ams-Gms-Am-Cm-Cm-Af-Am- Gf-Af-Uf-Gm-Am-Am-Gd-Um- Uf-Um-Cm-Cm-Am-G101 | 862 | UmsEVP-Gfs-Gf-Af-Am-Af-Cd-Um- Um-Cm-Am-Um-Cm-Uf-Um-Gf-Gm- Um-Cm-Ums-Cms-Um | 890 |
| si792-DV26SPG101 | Ams-Gms-Am-Cm-Cm-Af-Am- Gf-Af-Uf-Gm-Am-Am-Gm-Um- Uf-Um-Cm-Cm-Am-G101 | 863 | UmsEVP-Gfs-Gf-Af-Am-Af-Cm-Um- Um-Cm-Am-Um-Cm-Uf-Um-Gf-Gm- Um-Cm-Ums-Cms-Um | 891 |
| si792-DV26SPd7BG101 | Ams-Gms-Am-Cm-Cm-Af-Am- Gf-Af-Uf-Gm-Am-Am-Gd-Um- Uf-Um-Cm-Cm-Am-G101 | 864 | UmsEVP-Gfs-Gf-Af-Am-Af-Cd-Um- Um-Cm-Am-Um-Cm-Uf-Um-Gf-Gm- Um-Cm-Ums-Cms-Um | 892 |
| si079-DV29SPG101 | Gms-Ams-Gm-Am-Cm-Cf-Am- Af-Gm-Af-Um-Gm-Am-Am-Gm- Uf-Um-Um-Cm-Am-G101 | 865 | UmsEVP-Gfs-Am-Af-Af-Cf-Um-Um- Cm-Am-Um-Cm-Um-Uf-Gm-Gf-Um- Cm-Um-Cms-Ums-Um | 893 |
| si047-DV26SPG101 | Gms-Ams-Gm-Am-Cm-Af-Am- Gf-Cf-Af-Um-Um-Um-Am-Um- Af-Cm-Um-Um-Um-G101 | 866 | AmsEVP-Afs-Af-Gf-Um-Af-Um-Am- Am-Am-Um-Gm-Cm-Uf-Um-Gf-Um- Cm-Um-Cms-Cms-Cm | 894 |
| si047-DV26SPd7BG101 | Gms-Ams-Gm-Am-Cm-Af-Am- Gf-Cf-Af-Um-Um-Um-Ad-Um- Af-Cm-Um-Um-Um-G101 | 867 | AmsEVP-Afs-Af-Gf-Um-Af-Td-Am- Am-Am-Um-Gm-Cm-Uf-Um-Gf-Um- Cm-Um-Cms-Cms-Cm | 895 |
| si751-DV27PG101 | Ams-Gms-Um-Gm-Am-Am-Gf- Am-Gf-Af-Cf-Cm-Am-Am-Gm- Am-Uf-Gm-Am-Am-Am-G101 | 868 | UmsEVP-Ufs-Um-Cf-Af-Uf-Cm-Um- Um-Gm-Gm-Um-Cm-Uf-Cm-Uf-Um- Cm-Am-Cm-Ums-Cms-Cm | 896 |
| si751-DV27Pd7BG101 | Ams-Gms-Um-Gm-Am-Am-Gf- Am-Gf-Af-Cf-Cm-Am-Am-Gd- Am-Uf-Gm-Am-Am-Am-G101 | 869 | UmsEVP-Ufs-Um-Cf-Af-Uf-Cd-Um- Um-Gm-Gm-Um-Cm-Uf-Cm-Uf-Um- Cm-Am-Cm-Ums-Cms-Cm | 897 |
| si755-DV27SPG101 | Gms-Ums-Gm-Am-Am-Gf-Am- Gf-Af-Cf-Cm-Am-Am-Gm-Am- Uf-Gm-Am-Am-Am-G101 | 870 | UmsEVP-Ufs-Um-Cf-Af-Uf-Cm-Um- Um-Gm-Gm-Um-Cm-Uf-Cm-Uf-Um- Cm-Am-Cms-Ums-Cm | 898 |
| si755-DV27SPd7BG101 | Gms-Ums-Gm-Am-Am-Gf-Am- Gf-Af-Cf-Cm-Am-Am-Gd-Am- Uf-Gm-Am-Am-Am-G101 | 871 | UmsEVP-Ufs-Um-Cf-Af-Uf-Cd-Um- Um-Gm-Gm-Um-Cm-Uf-Cm-Uf-Um- Cm-Am-Cms-Ums-Cm | 899 |
| si791-DV29PG101 | Gms-Ams-Gm-Am-Cm-Cm-Af- Am-Gf-Am-Uf-Gm-Am-Am-Gm- Um-Uf-Um-Cm-Cm-Am-G101 | 872 | UmsEVP-Gfs-Gm-Af-Af-Af-Cm-Um- Um-Cm-Am-Um-Cm-Uf-Um-Gf-Gm- Um-Cm-Um-Cms-Ums-Um | 900 |
| si792-DV29SPG101 | Ams-Gms-Am-Cm-Cm-Af-Am- Gf-Am-Uf-Gm-Am-Am-Gm-Um- Uf-Um-Cm-Cm-Am-G101 | 873 | UmsEVP-Gfs-Gm-Af-Af-Af-Cm-Um- Um-Cm-Am-Um-Cm-Uf-Um-Gf-Gm- Um-Cm-Ums-Cms-Um | 901 |
| si079-DV25SPG101 | Gms-Ams-Gm-Am-Cm-Cf-Am- Af-Gf-Af-Um-Gm-Am-Am-Gm- Uf-Um-Um-Cm-Am-G101 | 874 | UmsEVP-Gfs-Am-Am-Am-Cf-Um- Um-Cm-Am-Um-Cm-Um-Uf-Gm-Gf- Um-Cm-Um-Cms-Ums-Um | 902 |
| si079-DV25SPd7BG101 | Gms-Ams-Gm-Am-Cm-Cf-Am- Af-Gf-Af-Um-Gm-Am-Ad-Gm- Uf-Um-Um-Cm-Am-G101 | 875 | UmsEVP-Gfs-Am-Am-Am-Cf-Td- Um-Cm-Am-Um-Cm-Um-Uf-Gm-Gf- Um-Cm-Um-Cms-Ums-Um | 903 |
| si079-DV25SPd7B11G101 | Gms-Ams-Gm-Am-Cm-Cf-Am- Af-Gf-Af-Um-Gm-Am-Ad-Gm- Uf-Um-Um-Cm-Am-G101 | 876 | UmsEVP-Gfs-Am-Am-Am-Cf-Td- Um-Cm-Am-Td-Cm-Um-Uf-Gm-Gf- Um-Cm-Um-Cms-Ums-Um | 904 |

The synthesis methods for the modified sense and antisense strands in this example are as described in Example 2. Furthermore, the conjugate moieties such as G101 and G103 described herein are attached to the 3' end of the sense strand of siRNA, and the conjugation methods may refer to those described in the prior art.

The experimental methods for evaluating the inhibitory effect of siRNA conjugates on INHBE gene expression in this example are as described in Examples 2 and 3, except that the test samples are the siRNA conjugates disclosed herein.

The experimental results demonstrated that at concentrations ranging from 40 pM to 25 nM, the siRNA derivatives with anti-off-target modifications exhibited significant inhibitory activity against the target gene INHBE, and the inhibition rates of these derivatives against potential off-target genes were significantly lower than those against the target gene. These findings indicate that the incorporation of anti-off-target modifications does not compromise the inhibitory effect of the template-modified siRNA derivatives disclosed herein on the target gene, but can effectively reduce off-target effects in certain sequences.

### (1) All modified sequences exhibited significant inhibitory effects on INHBE gene expression.

The inhibition rates of INHBE gene expression by all test samples are shown in Table 17.

All template-modified sequences exhibited significant inhibitory effects on INHBE at a concentration of 25 nM. For example, the template-modified sequence si079-DV25SPG101 achieved an inhibition rate of 81.0%.

**Table 17: Inhibition rates of INHBE gene expression by template-modified sequences with anti-off-target modifications**

| siRNA ID | Inhibition rate (%) | siRNA ID | Inhibition rate (%) |
|---|---|---|---|
| si751-DV25PG101 | 72.1 | si792-DV26SPG101 | 61 |
| si751-DV25Pd7BG101 | 71 | si792-DV26SPd7BG101 | 46.7 |
| si755-DV25SPG101 | 59 | si079-DV29SPG101 | 72 |
| si755-DV25SPd7BG101 | 66.3 | si047-DV26SPG101 | 71.7 |
| si755-DV25SPd7B11G101 | 68.8 | si047-DV26SPd7BG101 | 68.9 |
| si079-DV27SPG101 | 72.7 | si751-DV27PG101 | 83.7 |
| si079-DV34SPG101 | 70.4 | si751-DV27Pd7BG101 | 80.3 |
| si079-DV34SPd7BG101 | 69.5 | si755-DV27SPG101 | 75.1 |
| si079-DV34SPd7B11G101 | 70.6 | si755-DV27SPd7BG101 | 71.5 |
| si047-DV34SPG101 | 74.6 | si791-DV29PG101 | 51.4 |
| si047-DV34SPd7BG101 | 74.5 | si792-DV29SPG101 | 67.9 |
| si047-DV34SPd7B11G101 | 72 | si079-DV25SPG101 | 81 |
| si791-DV26PG101 | 45.6 | si079-DV25SPd7BG101 | 77.4 |
| si791-DV26Pd7BG101 | 59 | si079-DV25SPd7B11G101 | 76.9 |

After adopting template modification followed by anti-off-target design, the resulting sequence exhibited significant inhibitory activity against INHBE gene expression. For example, si079 modified with DV25SP and further subjected to anti-off-target design (si079-DV25SPd7B11G101) achieved an inhibition rate of 76.9%.

### (2) After adopting any one or more of the modification patterns disclosed herein followed by anti-off-target design, a significant anti-off-target effect was observed for certain off-target genes, thereby reducing the inhibitory effect on off-target genes.

As shown in Table 18, the experimental results demonstrated that:
The template-modified sequences disclosed herein that incorporate anti-off-target design can reduce the inhibitory effect on off-target genes, thereby achieving an anti-off-target effect. The sequences modified using the templates disclosed herein, incorporating anti-off-target design, exhibited an anti-off-target effect, with the inhibition rate against off-target genes being reduced by up to 26.7%.

**Table 18: Inhibition rates of off-target genes by template-modified sequences with anti-off-target modifications**

| siRNA ID | Off-target genes | Inhibition against off-target genes (%) | Reduction in inhibition compared to without anti-off-target design (%) |
|---|---|---|---|
| si079-DV25SPG101 | MTFR1 | 54.4 | 0.0 |
| si079-DV25SPd7BG101 | | 48.8 | 5.6 |
| si079-DV25SPd7B11G101 | | 27.6 | 26.7 |
| si751-DV27PG101 | SYT12 | 80.1 | 0.0 |
| si751-DV27Pd7BG101 | | 64.8 | 15.2 |

### 1) Template modification alone

Some of the template-modified sequences exhibited measurable off-target effects. For example, the modified sequence si079-DV25SPG101 achieved an inhibition rate of 54.4% against the potential off-target gene MTFR1; the modified sequence si751-DV27PG101 achieved an inhibition rate of 80.1% against the potential off-target gene SYT12.

### 2) Template modification combined with anti-off-target design (denoted as "d7B" or "d7B11" in sequence identifiers)

Some of the sequences incorporating both template modification and anti-off-target modification exhibited significantly reduced inhibition against off-target genes, demonstrating a significant anti-off-target effect. For example, si079-DV25SPd7B11G101 exhibited a 26.7% reduction in inhibition against MTFR1; si751-DV27Pd7BG101 exhibited a 15.2% reduction in inhibition against SYT12.

### Summary:

1. After adopting template modification followed by anti-off-target design, the resulting sequence exhibited significant inhibitory activity against INHBE gene expression. For example, si079 modified with DV25SP and further subjected to anti-off-target design (si079-DV25SPd7B11G101) achieved an inhibition rate of 76.9%.
2. After adopting any one or more of the modification patterns disclosed herein followed by anti-off-target design, a significant anti-off-target effect was observed for certain off-target genes, thereby reducing the inhibitory effect on off-target genes.
   1) Some of the template-modified sequences exhibited measurable off-target effects. For example, the modified sequence si079-DV25SPG101 achieved an inhibition rate of 54.4% against the potential off-target gene MTFR1; the modified sequence si751-DV27PG101 achieved an inhibition rate of 80.1% against the potential off-target gene SYT12.
   2) Some of the sequences incorporating both template modification and anti-off-target modification exhibited significantly reduced inhibition against off-target genes, demonstrating a significant anti-off-target effect. For example, si079-DV25SPd7B11G101 exhibited a 26.7% reduction in inhibition against MTFR1; si751-DV27Pd7BG101 exhibited a 15.2% reduction in inhibition against SYT12.

### Example 5: Inhibition of INHBE mRNA expression in mouse liver by sequences modified using the templates disclosed herein

In this example, the siRNA derivatives described in Example 4 were evaluated using transgenic mice expressing the human INHBE gene. The inhibitory effects of these derivatives on hepatic INHBE expression at different time points were evaluated by qPCR.

### 1. Experimental materials

### Test compounds: siRNA derivatives described in Example 4, as shown in Table 16.

### Solvent: saline.

**Experimental animals:** SPF-grade hINHBE transgenic male mice aged 6 to 8 weeks and weighing 18 g to 28 g, grouped with 10 mice per experimental group.

### 2. Experimental methods

### Dosage design and grouping

Grouping and administration: After acclimatization, the mice were randomly divided into a negative control group (vehicle control) and a test compound group based on body weight, with 10 mice per group. The test compounds were administered at a dose of 1 mg/kg via a single subcutaneous injection (s.c./once), with a dosing volume of 5 mL/kg and a dosing concentration of 0.2 mg/mL. The day of administration was designated as Day 0.

Group information is shown in Table 19 below.

**Table 19: Grouping information**

| Group | Test compound | Dose (mg/kg) | Route/frequency of administration | Duration (days) | Number of animals |
|---|---|---|---|---|---|
| Negative control group | Solvent | / | / | 28 | 10 |
| Test compound group | siRNA | 1 | s.c./once | 28 | 10 |

### Measurement indicators

### (1) General observations

Animals were observed once daily from one week prior to administration until the end of the study.

Cage-side observation parameters included mortality or moribund status, mental state, behavior activities, fecal characteristics, food and water supply, *etc.*

Test animals: all animals in both the negative control group and the test compound group.

### (2) Measurement of body weight and food intake

Changes in body weight and food intake were recorded over time. Body weight (non-fasting body weight) and food intake were measured on the day of administration (Day 0) and once weekly thereafter.

### (3) Measurement of hepatic INHBE mRNA expression

Measurement time points: Day 14 and Day 28.

Method for measuring INHBE mRNA levels: qPCR.

Test animals: 5 animals each from the negative control group and the test compound group at each time point.

### (4) Data processing and statistical analysis

Data are expressed as mean ± standard deviation (Mean ± SD) and analyzed using GraphPad Prism 10 software. Statistical analysis was performed using two-way ANOVA followed by post hoc analysis, including the LSD test for homogeneity of variance and Dunnett's T3 test for unequal variance. *P* < 0.05 was considered statistically significant.

### 3. Experimental results

The detailed experimental results are shown in Table 20.

The results demonstrated that the test compounds significantly inhibited hepatic INHBE mRNA expression. For example, si079-DV25SPd7B11G101 achieved an inhibition rate of 89.9% on Day 14.
**(1) The sequences disclosed herein exhibited significant inhibitory effects on hepatic INHBE mRNA expression. For example, si079-DV25SPd7B11G101 achieved an inhibition rate of 89.9% on Day 14.**

**Table 20: Inhibition rates of hepatic INHBE mRNA by different sequences**

| Group | D14 inhibition rate (%) | D28 inhibition rate (%) |
|---|---|---|
| Vehicle | -3.1 | -3.2 |
| si079-DV34SPd7B11G101 | 74.0 | -20.5 |
| si047-DV34SPG101 | 71.7 | -65.4 |
| si047-DV34SPd7BG101 | 74.5 | -60.8 |
| si047-DV34SPd7B11G101 | 73.2 | -59.9 |
| si791-DV26SPG101 | 64.0 | -8.7 |
| si791-DV26Pd7BG101 | 77.7 | -4.0 |
| si792-DV26SPG101 | 47.8 | -14.8 |
| si792-DV26SPd7BG101 | 80.7 | 12.9 |
| si079-DV29SPG101 | 66.9 | 45.6 |
| si047-DV26SPG101 | 74.6 | 33.7 |
| si751-DV25PG101 | 72.3 | 18.5 |
| si047-DV26SPd7BG101 | 31.0 | -2.1 |
| si751-DV27PG101 | 36.0 | 54.8 |
| si751-DV27Pd7BG101 | 18.1 | 31.8 |
| si752-DV27SPG101 | 12.5 | 60.4 |
| si752-DV27SPd7BG101 | 28.4 | 45.9 |
| si791-DV29PG101 | 67.4 | 42.5 |
| si792-DV29SPG101 | 43.4 | 50.5 |
| si079-DV25SPG101 | 73.0 | 33.5 |
| si079-DV25SPd7BG101 | 81.7 | 17.9 |
| si079-DV25SPd7B11G101 | 89.9 | 22.2 |
| si751-DV25Pd7BG101 | 67.1 | 24.8 |
| si755-DV25SPG101 | 38.9 | 8.9 |
| si755-DV25SPd7BG101 | 74.2 | 10.2 |
| si755-DV25SPd7B11G101 | 78.2 | 36.7 |
| si079-DV27SPG101 | 67.5 | -27.2 |
| si079-DV34SPG101 | 69.9 | -81.4 |
| si079-DV34SPd7BG101 | 47.8 | -63.3 |

As shown in Table 20, the conjugates formed by conjugating the sequences with GalNAc compounds exhibited significant inhibitory effects on hepatic INHBE mRNA expression. For example, si079-DV25SPd7B11G101 achieved inhibition rates of 89.9% and 22.2% on Day 14 and Day 21, respectively.

These findings indicate that the sequences designed in the present disclosure, when modified using the templates described herein, or further incorporating anti-off-target design, and conjugated with GalNAc compounds, can be efficiently delivered to the liver of animals, significantly inhibiting INHBE gene expression.

### Example 6: Inhibition of body weight gain in mice by sequences modified using the templates disclosed herein

In this example, some of the siRNA derivatives described in Example 4 were selected to establish a diet-induced obesity (DIO) model using transgenic mice expressing the human INHBE gene. Body weight and body fat percentage were measured at different time points. The inhibitory effects of these derivatives on hepatic INHBE expression at different time points were evaluated by qPCR.

### 1. Experimental materials

### Test compounds:

siRNA derivatives described in Example 4, including si079-DV25SPd7B11G101, si752-DV25SPd7B11G101, si791-DV26Pd7BG101, si047-DV26SPG101, si079-DV25SPG101, si791-DV29PG101, si079-DV29SPG101, and si079-DV25SPd7BG101. The sequences of these derivatives are shown in Table 16.

Positive control AC004285 is derived from Table 5C of US20250075214A1, whose sense sequence is 5'-(NAG37)s-(invAb)s-Cm-Um-Gm-Gm-Cm-Um-Um-Am-Uf-Am-Cf-Uf-Um-Um-Cm-Um-Um-Am-Am-Um-Ams-(invAb)-3' (SEQ ID NO: 908) and whose antisense sequence is 5'-Ums-Afs-Um-Um-Af-Am-Gm-Am-Am-Am-Gm-Uf-Am-Uf-Am-Af-Gm-Cm-Cm-Amss-Gm-3' (SEQ ID NO: 909), wherein NAG37 and invAb are as defined in the referenced patent, and the letters s, f, and m have the same meanings as defined elsewhere in the present disclosure.

### Solvent: saline.

**Experimental animals:** SPF-grade hINHBE transgenic male mice aged 6 to 8 weeks and weighing 18 g to 28 g, grouped as follows: 15 mice per control group, 8 mice per 1 mpk dose group, and 9 mice per 3 mpk dose group.

### 2. Experimental methods

### Dosage design and grouping

Grouping and administration: After acclimatization, the mice were randomly divided into a negative control group and a test compound group based on body weight and body fat percentage. The test compounds were administered at a dose of 1 mg/kg or 3 mg/kg via a single subcutaneous injection, with a dosing volume of 5 mL/kg and a dosing concentration of 0.2 mg/mL or 0.6 mg/mL. The day of administration was designated as Day 0.

Group information is shown in Table 21 below.

**Table 21: Grouping information**

| Group | Test compound | Dose (mg/kg) | Route/frequency of administration | Duration (days) | Number of animals |
|---|---|---|---|---|---|
| Negative control group | Saline | / | / | 70 | 15 |
| Test compound group | siRNA | 1 | s.c., D0, D41 | 70 | 8 |
| | | 3 | s.c., D0, D41 | 70 | 9 |

### Measurement indicators

### (1) General observations

Animals were observed once daily from one week prior to administration until the end of the study.

Cage-side observation parameters included mortality or moribund status, mental state, behavior activities, fecal characteristics, food and water supply, *etc.*

Test animals: all animals in both the negative control group and the test compound group.

### (2) Measurement of body weight, body fat, and food intake

Changes in body weight and food intake were recorded over time. Body weight (non-fasting body weight) and food intake were measured on the day of administration (Day 0) and twice weekly thereafter. Body fat percentage was measured once weekly.

### (3) Measurement of hepatic INHBE mRNA expression

Measurement time points: D28 (4 weeks after the first dose) and D55 (2 weeks after the second dose)

Method for measuring INHBE mRNA levels: qPCR.

Test animals: 3 to 5 animals each from the negative control group and the test compound group at each time point.

### (4) Data processing and statistical analysis

Data are expressed as mean ± standard deviation (Mean ± SD) and analyzed using GraphPad Prism 10 software. Statistical analysis was performed using two-way ANOVA followed by post hoc analysis, including the LSD test for homogeneity of variance and Dunnett's T3 test for unequal variance. *P* < 0.05 was considered statistically significant.

### 3. Experimental results

The results of hepatic INHBE mRNA expression are shown in Table 22.

The results demonstrated that these sequences significantly inhibited hepatic INHBE mRNA expression. For example, at a dose of 1 mg/kg, si079-DV25SPd7B11G101 achieved inhibition rates of 46.6% and 84.9% on Day 28 and Day 55, respectively.
**(1) The sequences disclosed herein exhibited significant inhibitory effects on hepatic INHBE mRNA expression. For example, at a dose of 1 mg/kg, si079-DV25SPd7B11G101 achieved inhibition rates of 46.6% and 84.9% on Day 28 and Day 55, respectively.**

**Table 22: Inhibition rates of hepatic INHBE mRNA by different sequences**

| siRNA ID | Dose | D28 inhibition rate | D55 inhibition rate |
|---|---|---|---|
| si079-DV25SPd7B11G101 | 1 mg/kg | 46.6 | 84.9 |
| | 3 mg/kg | N/A | 78.7 |
| si755-DV25SPd7B11G101 | 1 mg/kg | 16.2 | 82.7 |
| | 3 mg/kg | N/A | 81.1 |
| si791-DV26Pd7BG101 | 1 mg/kg | 25.5 | 77.9 |
| | 3 mg/kg | N/A | 81.0 |
| si047-DV26SPG101 | 1 mg/kg | -98.7 | 50.3 |
| | 3 mg/kg | N/A | 73.2 |
| si079-DV25SPG101 | 1 mg/kg | 22.0 | 73.9 |
| | 3 mg/kg | N/A | 88.2 |
| si791-DV29PG101 | 1 mg/kg | -36.4 | 83.1 |
| | 3 mg/kg | N/A | 90.8 |
| si079-DV29SPG101 | 1 mg/kg | -15.3 | 82.3 |
| | 3 mg/kg | N/A | 86.9 |
| si079-DV25SPd7BG101 | 1 mg/kg | -3.6 | 87.7 |
| | 3 mg/kg | N/A | 89.5 |
| AC004285 | 1 mg/kg | 34.0 | 74.6 |
| | 3 mg/kg | N/A | 77.3 |

**(2) The sequences disclosed herein exhibited significant inhibitory effects on body weight gain and body fat accumulation in DIO mice (see Table 23). For example, at a dose of 1 mg/kg or 3 mpk, si791-DV26Pd7BG101 exhibited a 12.7% reduction in body weight and a 22.7% reduction in body fat on Day 21 compared to the saline control group.**

**Table 23: Percentage reduction in body weight and body fat by different sequences**

| siRNA ID | D21 reduction in body weight (%) | | D21 reduction in body fat (%) | |
|---|---|---|---|---|
| | 1 mg/kg | 3 mg/kg | 1 mg/kg | 3 mg/kg |
| si079-DV25SPd7B11G101 | 8.5 | 10.8 | 19.9 | 26.7 |
| si755-DV25SPd7B11G101 | 14.6 | 15.2 | 31.4 | 40.0 |
| si791-DV26Pd7BG101 | 12.7 | 15.7 | 22.7 | 34.1 |
| si047-DV26SPG101 | 11.5 | 16.0 | 29.9 | 29.2 |
| si079-DV25SPG101 | 10.9 | 12.3 | 23.5 | 31.2 |
| si791-DV29PG101 | 9.5 | 11.4 | 22.0 | 35.0 |
| si079-DV29SPG101 | 13.7 | 13.7 | 31.5 | 30.2 |
| si079-DV25SPd7BG101 | 15.0 | 15.8 | 41.5 | 34.8 |
| AC004285 | 9.2 | 11.8 | 20.5 | 28.9 |

As shown in Table 23, the conjugates formed by conjugating the sequences with GalNAc compounds exhibited significant inhibitory effects on body weight gain and body fat accumulation. For example, at a dose of 1 mg/kg or 3 mpk, si791-DV26Pd7BG101 exhibited a 12.7% reduction in body weight and a 22.7% reduction in body fat on Day 21 compared to the saline control group.

These findings indicate that the sequences designed in the present disclosure, when modified using the templates described herein, or further incorporating anti-off-target design, and conjugated with GalNAc compounds, can be efficiently delivered to the liver of animals, significantly inhibiting body weight gain and body fat accumulation.

In the present disclosure, a series of siRNAs were designed based on the INHBE mRNA sequence and modified using a set of specific modification templates, with some of these sequences further incorporating anti-off-target design. The results show that the multiply modified sequences can achieve inhibition rates of up to greater than 70% Furthermore, conjugation of the modified sequences with GalNAc compounds enables efficient delivery to the liver of animals, significantly inhibiting INHBE gene expression, body weight gain, and body fat accumulation.

## Claims

1. An siRNA duplex, wherein the siRNA duplex comprises a sense strand and an antisense strand that form a reverse complementary duplex region, wherein the antisense strand comprises at least 15, 16, 17, 18, or 19 consecutive nucleotides complementary to the target sequence of SEQ ID NO: 907, or wherein the antisense strand comprises a fragment of at least 15, 16, 17, 18, or 19 consecutive nucleotides from any one of the sequences of SEQ ID NOs: 23, 16, 14, 13, 15, 18-22, 17, 24, 134 to 236, 238 to 240, and 252 to 260.

2. The siRNA duplex according to claim 1, wherein the sense strand comprises a fragment of at least 17, 18, or 19 consecutive nucleotides from any one of the sequences of SEQ ID NOs: 11, 4, 2, 1, 3, 6-10, 5, 12, 25 to 127, 129 to 131, and 243 to 251.

3. The siRNA duplex according to claim 1, wherein the siRNA duplex satisfies one or more of the following conditions:
(1) the reverse complementary duplex region is 17 to 21 bp in length;
(2) the sense strand and the antisense strand each independently comprise 19 to 23 nucleotides;
(3) at least one strand comprises a 3' overhang of at least one or at least two nucleotides; and
(4) the siRNA duplex is an siRNA for inhibiting INHBE gene expression.

4. The siRNA duplex according to claim 1, wherein the siRNA duplex comprises any one oligonucleotide duplex selected from the following pairings of a sense strand and an antisense strand, or a combination of at least two thereof:
the nucleotide sequence of the sense strand is set forth in SEQ ID NO: 11; and the nucleotide sequence of the antisense strand is set forth in SEQ ID NO: 23;
the nucleotide sequence of the sense strand is set forth in SEQ ID NO: 4; and the nucleotide sequence of the antisense strand is set forth in SEQ ID NO: 16;
the nucleotide sequence of the sense strand is set forth in SEQ ID NO: 2; and the nucleotide sequence of the antisense strand is set forth in SEQ ID NO: 14;
the nucleotide sequence of the sense strand is set forth in SEQ ID NO: 1; and the nucleotide sequence of the antisense strand is set forth in SEQ ID NO: 13;
the nucleotide sequence of the sense strand is set forth in SEQ ID NO: 3; and the nucleotide sequence of the antisense strand is set forth in SEQ ID NO: 15;
the nucleotide sequence of the sense strand is set forth in SEQ ID NO: 6; and the nucleotide sequence of the antisense strand is set forth in SEQ ID NO: 18;
the nucleotide sequence of the sense strand is set forth in SEQ ID NO: 7; and the nucleotide sequence of the antisense strand is set forth in SEQ ID NO: 19;
the nucleotide sequence of the sense strand is set forth in SEQ ID NO: 8; and the nucleotide sequence of the antisense strand is set forth in SEQ ID NO: 20;
the nucleotide sequence of the sense strand is set forth in SEQ ID NO: 9; and the nucleotide sequence of the antisense strand is set forth in SEQ ID NO: 21;
the nucleotide sequence of the sense strand is set forth in SEQ ID NO: 10; and the nucleotide sequence of the antisense strand is set forth in SEQ ID NO: 22;
the nucleotide sequence of the sense strand is set forth in SEQ ID NO: 5; and the nucleotide sequence of the antisense strand is set forth in SEQ ID NO: 17;
the nucleotide sequence of the sense strand is set forth in SEQ ID NO: 12; and the nucleotide sequence of the antisense strand is set forth in SEQ ID NO: 24;
the nucleotide sequence of the sense strand is set forth in SEQ ID NO: 25; and the nucleotide sequence of the antisense strand is set forth in SEQ ID NO: 134;
the nucleotide sequence of the sense strand is set forth in SEQ ID NO: 26; and the nucleotide sequence of the antisense strand is set forth in SEQ ID NO: 135;
the nucleotide sequence of the sense strand is set forth in SEQ ID NO: 27; and the nucleotide sequence of the antisense strand is set forth in SEQ ID NO: 136;
the nucleotide sequence of the sense strand is set forth in SEQ ID NO: 28; and the nucleotide sequence of the antisense strand is set forth in SEQ ID NO: 137;
the nucleotide sequence of the sense strand is set forth in SEQ ID NO: 29; and the nucleotide sequence of the antisense strand is set forth in SEQ ID NO: 138;
the nucleotide sequence of the sense strand is set forth in SEQ ID NO: 30; and the nucleotide sequence of the antisense strand is set forth in SEQ ID NO: 139;
the nucleotide sequence of the sense strand is set forth in SEQ ID NO: 31; and the nucleotide sequence of the antisense strand is set forth in SEQ ID NO: 140;
the nucleotide sequence of the sense strand is set forth in SEQ ID NO: 32; and the nucleotide sequence of the antisense strand is set forth in SEQ ID NO: 141;
the nucleotide sequence of the sense strand is set forth in SEQ ID NO: 33; and the nucleotide sequence of the antisense strand is set forth in SEQ ID NO: 142;
the nucleotide sequence of the sense strand is set forth in SEQ ID NO: 34; and the nucleotide sequence of the antisense strand is set forth in SEQ ID NO: 143;
the nucleotide sequence of the sense strand is set forth in SEQ ID NO: 35; and the nucleotide sequence of the antisense strand is set forth in SEQ ID NO: 144;
the nucleotide sequence of the sense strand is set forth in SEQ ID NO: 36; and the nucleotide sequence of the antisense strand is set forth in SEQ ID NO: 145;
the nucleotide sequence of the sense strand is set forth in SEQ ID NO: 37; and the nucleotide sequence of the antisense strand is set forth in SEQ ID NO: 146;
the nucleotide sequence of the sense strand is set forth in SEQ ID NO: 38; and the nucleotide sequence of the antisense strand is set forth in SEQ ID NO: 147;
the nucleotide sequence of the sense strand is set forth in SEQ ID NO: 39; and the nucleotide sequence of the antisense strand is set forth in SEQ ID NO: 148;
the nucleotide sequence of the sense strand is set forth in SEQ ID NO: 40; and the nucleotide sequence of the antisense strand is set forth in SEQ ID NO: 149;
the nucleotide sequence of the sense strand is set forth in SEQ ID NO: 41; and the nucleotide sequence of the antisense strand is set forth in SEQ ID NO: 150;
the nucleotide sequence of the sense strand is set forth in SEQ ID NO: 42; and the nucleotide sequence of the antisense strand is set forth in SEQ ID NO: 151;
the nucleotide sequence of the sense strand is set forth in SEQ ID NO: 43; and the nucleotide sequence of the antisense strand is set forth in SEQ ID NO: 152;
the nucleotide sequence of the sense strand is set forth in SEQ ID NO: 44; and the nucleotide sequence of the antisense strand is set forth in SEQ ID NO: 153;
the nucleotide sequence of the sense strand is set forth in SEQ ID NO: 45; and the nucleotide sequence of the antisense strand is set forth in SEQ ID NO: 154;
the nucleotide sequence of the sense strand is set forth in SEQ ID NO: 46; and the nucleotide sequence of the antisense strand is set forth in SEQ ID NO: 155;
the nucleotide sequence of the sense strand is set forth in SEQ ID NO: 47; and the nucleotide sequence of the antisense strand is set forth in SEQ ID NO: 156;
the nucleotide sequence of the sense strand is set forth in SEQ ID NO: 48; and the nucleotide sequence of the antisense strand is set forth in SEQ ID NO: 157;
the nucleotide sequence of the sense strand is set forth in SEQ ID NO: 49; and the nucleotide sequence of the antisense strand is set forth in SEQ ID NO: 158;
the nucleotide sequence of the sense strand is set forth in SEQ ID NO: 50; and the nucleotide sequence of the antisense strand is set forth in SEQ ID NO: 159;
the nucleotide sequence of the sense strand is set forth in SEQ ID NO: 51; and the nucleotide sequence of the antisense strand is set forth in SEQ ID NO: 160;
the nucleotide sequence of the sense strand is set forth in SEQ ID NO: 52; and the nucleotide sequence of the antisense strand is set forth in SEQ ID NO: 161;
the nucleotide sequence of the sense strand is set forth in SEQ ID NO: 53; and the nucleotide sequence of the antisense strand is set forth in SEQ ID NO: 162;
the nucleotide sequence of the sense strand is set forth in SEQ ID NO: 54; and the nucleotide sequence of the antisense strand is set forth in SEQ ID NO: 163;
the nucleotide sequence of the sense strand is set forth in SEQ ID NO: 55; and the nucleotide sequence of the antisense strand is set forth in SEQ ID NO: 164;
the nucleotide sequence of the sense strand is set forth in SEQ ID NO: 56; and the nucleotide sequence of the antisense strand is set forth in SEQ ID NO: 165;
the nucleotide sequence of the sense strand is set forth in SEQ ID NO: 57; and the nucleotide sequence of the antisense strand is set forth in SEQ ID NO: 166;
the nucleotide sequence of the sense strand is set forth in SEQ ID NO: 58; and the nucleotide sequence of the antisense strand is set forth in SEQ ID NO: 167;
the nucleotide sequence of the sense strand is set forth in SEQ ID NO: 59; and the nucleotide sequence of the antisense strand is set forth in SEQ ID NO: 168;
the nucleotide sequence of the sense strand is set forth in SEQ ID NO: 60; and the nucleotide sequence of the antisense strand is set forth in SEQ ID NO: 169;
the nucleotide sequence of the sense strand is set forth in SEQ ID NO: 61; and the nucleotide sequence of the antisense strand is set forth in SEQ ID NO: 170;
the nucleotide sequence of the sense strand is set forth in SEQ ID NO: 62; and the nucleotide sequence of the antisense strand is set forth in SEQ ID NO: 171;
the nucleotide sequence of the sense strand is set forth in SEQ ID NO: 63; and the nucleotide sequence of the antisense strand is set forth in SEQ ID NO: 172;
the nucleotide sequence of the sense strand is set forth in SEQ ID NO: 64; and the nucleotide sequence of the antisense strand is set forth in SEQ ID NO: 173;
the nucleotide sequence of the sense strand is set forth in SEQ ID NO: 65; and the nucleotide sequence of the antisense strand is set forth in SEQ ID NO: 174;
the nucleotide sequence of the sense strand is set forth in SEQ ID NO: 66; and the nucleotide sequence of the antisense strand is set forth in SEQ ID NO: 175;
the nucleotide sequence of the sense strand is set forth in SEQ ID NO: 67; and the nucleotide sequence of the antisense strand is set forth in SEQ ID NO: 176;
the nucleotide sequence of the sense strand is set forth in SEQ ID NO: 68; and the nucleotide sequence of the antisense strand is set forth in SEQ ID NO: 177;
the nucleotide sequence of the sense strand is set forth in SEQ ID NO: 69; and the nucleotide sequence of the antisense strand is set forth in SEQ ID NO: 178;
the nucleotide sequence of the sense strand is set forth in SEQ ID NO: 70; and the nucleotide sequence of the antisense strand is set forth in SEQ ID NO: 179;
the nucleotide sequence of the sense strand is set forth in SEQ ID NO: 71; and the nucleotide sequence of the antisense strand is set forth in SEQ ID NO: 180;
the nucleotide sequence of the sense strand is set forth in SEQ ID NO: 72; and the nucleotide sequence of the antisense strand is set forth in SEQ ID NO: 181;
the nucleotide sequence of the sense strand is set forth in SEQ ID NO: 73; and the nucleotide sequence of the antisense strand is set forth in SEQ ID NO: 182;
the nucleotide sequence of the sense strand is set forth in SEQ ID NO: 74; and the nucleotide sequence of the antisense strand is set forth in SEQ ID NO: 183;
the nucleotide sequence of the sense strand is set forth in SEQ ID NO: 75; and the nucleotide sequence of the antisense strand is set forth in SEQ ID NO: 184;
the nucleotide sequence of the sense strand is set forth in SEQ ID NO: 76; and the nucleotide sequence of the antisense strand is set forth in SEQ ID NO: 185;
the nucleotide sequence of the sense strand is set forth in SEQ ID NO: 77; and the nucleotide sequence of the antisense strand is set forth in SEQ ID NO: 186;
the nucleotide sequence of the sense strand is set forth in SEQ ID NO: 78; and the nucleotide sequence of the antisense strand is set forth in SEQ ID NO: 187;
the nucleotide sequence of the sense strand is set forth in SEQ ID NO: 79; and the nucleotide sequence of the antisense strand is set forth in SEQ ID NO: 188;
the nucleotide sequence of the sense strand is set forth in SEQ ID NO: 80; and the nucleotide sequence of the antisense strand is set forth in SEQ ID NO: 189;
the nucleotide sequence of the sense strand is set forth in SEQ ID NO: 81; and the nucleotide sequence of the antisense strand is set forth in SEQ ID NO: 190;
the nucleotide sequence of the sense strand is set forth in SEQ ID NO: 82; and the nucleotide sequence of the antisense strand is set forth in SEQ ID NO: 191;
the nucleotide sequence of the sense strand is set forth in SEQ ID NO: 83; and the nucleotide sequence of the antisense strand is set forth in SEQ ID NO: 192;
the nucleotide sequence of the sense strand is set forth in SEQ ID NO: 84; and the nucleotide sequence of the antisense strand is set forth in SEQ ID NO: 193;
the nucleotide sequence of the sense strand is set forth in SEQ ID NO: 85; and the nucleotide sequence of the antisense strand is set forth in SEQ ID NO: 194;
the nucleotide sequence of the sense strand is set forth in SEQ ID NO: 86; and the nucleotide sequence of the antisense strand is set forth in SEQ ID NO: 195;
the nucleotide sequence of the sense strand is set forth in SEQ ID NO: 87; and the nucleotide sequence of the antisense strand is set forth in SEQ ID NO: 196;
the nucleotide sequence of the sense strand is set forth in SEQ ID NO: 88; and the nucleotide sequence of the antisense strand is set forth in SEQ ID NO: 197;
the nucleotide sequence of the sense strand is set forth in SEQ ID NO: 89; and the nucleotide sequence of the antisense strand is set forth in SEQ ID NO: 198;
the nucleotide sequence of the sense strand is set forth in SEQ ID NO: 90; and the nucleotide sequence of the antisense strand is set forth in SEQ ID NO: 199;
the nucleotide sequence of the sense strand is set forth in SEQ ID NO: 91; and the nucleotide sequence of the antisense strand is set forth in SEQ ID NO: 200;
the nucleotide sequence of the sense strand is set forth in SEQ ID NO: 92; and the nucleotide sequence of the antisense strand is set forth in SEQ ID NO: 201;
the nucleotide sequence of the sense strand is set forth in SEQ ID NO: 93; and the nucleotide sequence of the antisense strand is set forth in SEQ ID NO: 202;
the nucleotide sequence of the sense strand is set forth in SEQ ID NO: 94; and the nucleotide sequence of the antisense strand is set forth in SEQ ID NO: 203;
the nucleotide sequence of the sense strand is set forth in SEQ ID NO: 95; and the nucleotide sequence of the antisense strand is set forth in SEQ ID NO: 204;
the nucleotide sequence of the sense strand is set forth in SEQ ID NO: 96; and the nucleotide sequence of the antisense strand is set forth in SEQ ID NO: 205;
the nucleotide sequence of the sense strand is set forth in SEQ ID NO: 97; and the nucleotide sequence of the antisense strand is set forth in SEQ ID NO: 206;
the nucleotide sequence of the sense strand is set forth in SEQ ID NO: 98; and the nucleotide sequence of the antisense strand is set forth in SEQ ID NO: 207;
the nucleotide sequence of the sense strand is set forth in SEQ ID NO: 99; and the nucleotide sequence of the antisense strand is set forth in SEQ ID NO: 208;
the nucleotide sequence of the sense strand is set forth in SEQ ID NO: 100; and the nucleotide sequence of the antisense strand is set forth in SEQ ID NO: 209;
the nucleotide sequence of the sense strand is set forth in SEQ ID NO: 101; and the nucleotide sequence of the antisense strand is set forth in SEQ ID NO: 210;
the nucleotide sequence of the sense strand is set forth in SEQ ID NO: 102; and the nucleotide sequence of the antisense strand is set forth in SEQ ID NO: 211;
the nucleotide sequence of the sense strand is set forth in SEQ ID NO: 103; and the nucleotide sequence of the antisense strand is set forth in SEQ ID NO: 212;
the nucleotide sequence of the sense strand is set forth in SEQ ID NO: 104; and the nucleotide sequence of the antisense strand is set forth in SEQ ID NO: 213;
the nucleotide sequence of the sense strand is set forth in SEQ ID NO: 105; and the nucleotide sequence of the antisense strand is set forth in SEQ ID NO: 214;
the nucleotide sequence of the sense strand is set forth in SEQ ID NO: 106; and the nucleotide sequence of the antisense strand is set forth in SEQ ID NO: 215;
the nucleotide sequence of the sense strand is set forth in SEQ ID NO: 107; and the nucleotide sequence of the antisense strand is set forth in SEQ ID NO: 216;
the nucleotide sequence of the sense strand is set forth in SEQ ID NO: 108; and the nucleotide sequence of the antisense strand is set forth in SEQ ID NO: 217;
the nucleotide sequence of the sense strand is set forth in SEQ ID NO: 109; and the nucleotide sequence of the antisense strand is set forth in SEQ ID NO: 218;
the nucleotide sequence of the sense strand is set forth in SEQ ID NO: 110; and the nucleotide sequence of the antisense strand is set forth in SEQ ID NO: 219;
the nucleotide sequence of the sense strand is set forth in SEQ ID NO: 111; and the nucleotide sequence of the antisense strand is set forth in SEQ ID NO: 220;
the nucleotide sequence of the sense strand is set forth in SEQ ID NO: 112; and the nucleotide sequence of the antisense strand is set forth in SEQ ID NO: 221;
the nucleotide sequence of the sense strand is set forth in SEQ ID NO: 113; and the nucleotide sequence of the antisense strand is set forth in SEQ ID NO: 222;
the nucleotide sequence of the sense strand is set forth in SEQ ID NO: 114; and the nucleotide sequence of the antisense strand is set forth in SEQ ID NO: 223;
the nucleotide sequence of the sense strand is set forth in SEQ ID NO: 115; and the nucleotide sequence of the antisense strand is set forth in SEQ ID NO: 224;
the nucleotide sequence of the sense strand is set forth in SEQ ID NO: 116; and the nucleotide sequence of the antisense strand is set forth in SEQ ID NO: 225;
the nucleotide sequence of the sense strand is set forth in SEQ ID NO: 117; and the nucleotide sequence of the antisense strand is set forth in SEQ ID NO: 226;
the nucleotide sequence of the sense strand is set forth in SEQ ID NO: 118; and the nucleotide sequence of the antisense strand is set forth in SEQ ID NO: 227;
the nucleotide sequence of the sense strand is set forth in SEQ ID NO: 119; and the nucleotide sequence of the antisense strand is set forth in SEQ ID NO: 228;
the nucleotide sequence of the sense strand is set forth in SEQ ID NO: 120; and the nucleotide sequence of the antisense strand is set forth in SEQ ID NO: 229;
the nucleotide sequence of the sense strand is set forth in SEQ ID NO: 121; and the nucleotide sequence of the antisense strand is set forth in SEQ ID NO: 230;
the nucleotide sequence of the sense strand is set forth in SEQ ID NO: 122; and the nucleotide sequence of the antisense strand is set forth in SEQ ID NO: 231;
the nucleotide sequence of the sense strand is set forth in SEQ ID NO: 123; and the nucleotide sequence of the antisense strand is set forth in SEQ ID NO: 232;
the nucleotide sequence of the sense strand is set forth in SEQ ID NO: 124; and the nucleotide sequence of the antisense strand is set forth in SEQ ID NO: 233;
the nucleotide sequence of the sense strand is set forth in SEQ ID NO: 125; and the nucleotide sequence of the antisense strand is set forth in SEQ ID NO: 234;
the nucleotide sequence of the sense strand is set forth in SEQ ID NO: 126; and the nucleotide sequence of the antisense strand is set forth in SEQ ID NO: 235;
the nucleotide sequence of the sense strand is set forth in SEQ ID NO: 127; and the nucleotide sequence of the antisense strand is set forth in SEQ ID NO: 236;
the nucleotide sequence of the sense strand is set forth in SEQ ID NO: 129; and the nucleotide sequence of the antisense strand is set forth in SEQ ID NO: 238;
the nucleotide sequence of the sense strand is set forth in SEQ ID NO: 130; and the nucleotide sequence of the antisense strand is set forth in SEQ ID NO: 239;
the nucleotide sequence of the sense strand is set forth in SEQ ID NO: 131; and the nucleotide sequence of the antisense strand is set forth in SEQ ID NO: 240;
the nucleotide sequence of the sense strand is set forth in SEQ ID NO: 243; and the nucleotide sequence of the antisense strand is set forth in SEQ ID NO: 252;
the nucleotide sequence of the sense strand is set forth in SEQ ID NO: 244; and the nucleotide sequence of the antisense strand is set forth in SEQ ID NO: 253;
the nucleotide sequence of the sense strand is set forth in SEQ ID NO: 245; and the nucleotide sequence of the antisense strand is set forth in SEQ ID NO: 254;
the nucleotide sequence of the sense strand is set forth in SEQ ID NO: 246; and the nucleotide sequence of the antisense strand is set forth in SEQ ID NO: 255;
the nucleotide sequence of the sense strand is set forth in SEQ ID NO: 247; and the nucleotide sequence of the antisense strand is set forth in SEQ ID NO: 256;
the nucleotide sequence of the sense strand is set forth in SEQ ID NO: 248; and the nucleotide sequence of the antisense strand is set forth in SEQ ID NO: 257;
the nucleotide sequence of the sense strand is set forth in SEQ ID NO: 249; and the nucleotide sequence of the antisense strand is set forth in SEQ ID NO: 258;
the nucleotide sequence of the sense strand is set forth in SEQ ID NO: 250; and the nucleotide sequence of the antisense strand is set forth in SEQ ID NO: 259; and
the nucleotide sequence of the sense strand is set forth in SEQ ID NO: 251; and the nucleotide sequence of the antisense strand is set forth in SEQ ID NO: 260.

5. The siRNA duplex according to claim 1, wherein the sense strand or the antisense strand comprises at least one modified nucleotide, wherein the at least one modified nucleotide is any one selected from the group consisting of a deoxy-nucleotide, a 3'-deoxy-thymidine (dT) nucleotide, a 2'-O-methyl-modified nucleotide, a 2'-fluoro-modified nucleotide, a 2'-deoxy-modified nucleotide, a locked nucleotide, an unlocked nucleotide, a conformationally restricted nucleotide, a constrained ethyl nucleotide, an abasic nucleotide, a 2'-amino-modified nucleotide, a 2'-O-allyl-modified nucleotide, a 2'-C-alkyl-modified nucleotide, a 2'-hydroxy-modified nucleotide, a 2'-methoxyethyl-modified nucleotide, a 2'-O-alkyl-modified nucleotide, a morpholino nucleotide, a phosphoramidate, a nucleotide comprising a non-natural base, a tetrahydropyran-modified nucleotide, a 1,5-anhydrohexitol-modified nucleotide, a cyclohexenyl-modified nucleotide, a nucleotide comprising a phosphorothioate group, a nucleotide comprising a methylphosphonate group, a nucleotide comprising a 5'-phosphate group, and a nucleotide comprising a 5'-phosphate mimic, or a combination of at least two thereof;
preferably, modification of the nucleotides on the sense strand and the antisense strand is a modification at the 2' position of the ribose of the nucleotide;
more preferably, the modification at the 2' position of the ribose of the nucleotide is any one selected from 2'-O-methyl, 2'-methoxyethyl, 2'-fluoro, 2'-benzyloxy, 2'-methylcarbonylamino, and 2'-pyridinylmethoxy, or a combination thereof;
further preferably, the modification at the 2' position of the ribose of the nucleotide is an alternating combination of 2'-O-methyl and 2'-fluoro;
still more preferably, the modification pattern at the 2' position of the ribose of the nucleotide is as follows: on the sense strand, all odd-numbered positions are modified with 2'-fluoro, and all even-numbered positions are modified with 2'-O-methyl; and on the antisense strand, all odd-numbered positions are modified with 2'-O-methyl, and all even-numbered positions are modified with 2'-fluoro.

6. The siRNA duplex according to claim 1, wherein the siRNA duplex satisfies one of the conditions (a) to (d):
(a) nucleotide monomers are linked to one another via a 3',5'-phosphodiester bond or a 3',5'-phosphorothioate bond;
(b)the 3',5'-phosphodiester bond between nucleotides at the terminus of the siRNA duplex comprises a phosphorothioate modification to form a stereochemically pure 3',5'-phosphorothioate bond;
preferably, one, two, or three 3',5'-phosphorothioate bonds are present among nucleotides at positions 1 to 4 from the 5' end of the sense strand and/or the antisense strand, and one, two, or three 3',5'-phosphorothioate bonds are present among nucleotides at positions 1 to 4 from the 3' end of the antisense strand;
(c) 5' carbon atom of the nucleotide glycoside at the 5' end of the modified antisense strand is phosphorylated, wherein the phosphorylation of the 5' carbon atom of the nucleotide glycoside is selected from the following 5'-phosphorylation groups: 5'-vinylphosphonate group (5'-E-VP), 5'-methylphosphonate group (5'-MP), 5'-C-methylphosphonate group, 5'-phosphorothioate group (5'-PS), and 5'-phosphate group (5'-P), the structures of which are as follows: 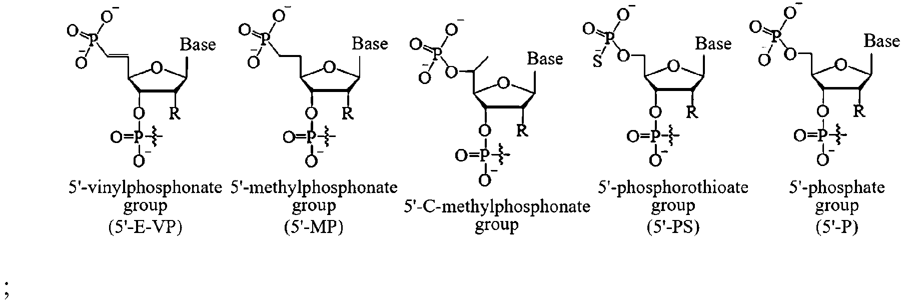
wherein R is hydrogen, hydroxy, amino, C₁₋₄ alkyl, aryl, C₁₋₄ alkoxy, C₁₋₄ alkylcarbonylamino, or halogen;
the base is selected from adenine, guanine, cytosine, thymine, and uracil;
(d) the siRNA duplex comprises the following modifications:
the antisense strand has any one of the following modification patterns:
| No. | **Antisense strand (AS) 5'-3'** | | | | | | | | | | | |
|---|---|---|---|---|---|---|---|---|---|---|---|---|
| **A** | **1** | **2** | **3** | **4** | **5** | **6** | **7** | **8** | **9** | **10** | **11** | **12** |
| | 2'-OMe +PS +EVP | **2'-F +PS** | 2'-OMe | 2'-OMe | 2'-OMe | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **B** | **1** | **2** | **3** | **4** | **5** | **6** | **7** | **8** | **9** | **10** | **11** | **12** |
| | 2'-OMe +PS +EVP | **2'-F +PS** | **2'-F** | **2'-F** | 2'-OMe | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe- | 2'-OMe | 2'-OMe | 2'-OMe | 2'-OMe | |
| | | | | | | | | | | | | |
|---|---|---|---|---|---|---|---|---|---|---|---|---|
| | | | | | | | | | +PS | +PS | | |
| **C** | **1** | **2** | **3** | **4** | **5** | **6** | **7** | **8** | **9** | **10** | **11** | **12** |
| | 2'-OMe +PS +EVP | **2'-F +PS** | 2'-OMe | **2'-F** | **2'-F** | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **+22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **D** | **1** | **2** | **3** | **4** | **5** | **6** | **7** | **8** | **9** | **10** | **11** | **12** |
| | 2'-OMe +PS +EVP | **2'-F +PS** | 2'-OMe | 2'-OMe | **2'-F** | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **E** | **1** | **2** | **3** | **4** | **5** | **6** | **7** | **8** | **9** | **10** | **11** | **12** |
| | 2'-OMe +PS +EVP | **2'-F +PS** | **2'-F** | 2'-OMe | 2'-OMe | **2'-F** | 2'-OMe | 2'-OMe | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **F** | **1** | **2** | **3** | **4** | **5** | **6** | **7** | **8** | **9** | **10** | **11** | **12** |
| | 2'-OMe +PS +EVP | **2'-F +PS** | 2'-OMe | 2'-OMe | 2'-OMe | **2'-F** | 2'-OMe | **2'-F** | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **G** | **1** | **2** | **3** | **4** | **5** | **6** | **7** | **8** | **9** | **10** | **11** | **12** |
| | 2'-OMe +PS +EVP | **2'-F +PS** | 2'-OMe | **2'-F** | **2'-F** | **2'-F** | 2'-OMe | **2'-F** | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
| **H** | **1** | **2** | **3** | **4** | **5** | **6** | **7** | **8** | **9** | **10** | **11** | **12** |
| | 2'-OMe +PS +EVP | **2'-F +PS** | **2'-F** | 2'-OMe | 2'-OMe | **2'-F** | 2'-OMe | **2'-F** | 2'-OMe | 2'-OMe | 2'-OMe | 2'-OMe |
| | **13** | **14** | **15** | **16** | **17** | **18** | **19** | **20** | **21** | **22** | **23** | |
| | 2'-OMe | **2'-F** | 2'-OMe | **2'-F** | 2'-OMe | 2'-OMe | 2'-OMe | 2'-OMe | 2'-OMe +PS | 2'-OMe +PS | 2'-OMe | |
and/or, the sense strand has any one of the following modification patterns:
| No. | **Sense strand (SS) 5'-3'** | | | | | | | | | | |
|---|---|---|---|---|---|---|---|---|---|---|---|
| **a** | **1** | **2** | **3** | **4** | **5** | **6** | **7** | **8** | **9** | **10** | **11** |
| | 2'-OMe+PS | 2'-OMe+PS | 2'-OMe | 2'-OMe | 2'-OMe | 2'-OMe | **2'-F** | 2'-OMe | **2'-F** | **2'-F** | **2'-F** |
| | **12** | **13** | **14** | **15** | **16** | **17** | **18** | **19** | **20** | **21** | |
| | 2'-OMe | 2'-OMe | 2'-OMe | 2'-OMe | 2'-OMe | 2'-F | 2'-OMe | 2'-OMe | 2'-OMe | 2'-OMe | |
| **b** | **1** | **2** | **3** | **4** | **5** | **6** | **7** | **8** | **9** | **10** | **11** |
| | 2'-OMe+PS | 2'-OMe+PS | 2'-OMe | 2'-OMe | 2'-OMe | 2'-OMe | **2'-F** | 2'-OMe | **2'-F** | 2'-OMe | **2'-F** |
| | **12** | **13** | **14** | **15** | **16** | **17** | **18** | **19** | **20** | **21** | |
| | 2'-OMe | 2'-OMe | 2'-OMe | 2'-OMe | 2'-OMe | **2'-F** | 2'-OMe | 2'-OMe | 2'-OMe | 2'-OMe | |
wherein 2'-OMe represents a 2'-O-methyl modification; 2'-F represents a 2'-fluoro modification; PS indicates that, starting from the 5' end, the nucleotide at the position is linked to the adjacent nucleotide on the right via a 3',5'-phosphorothioate bond; EVP represents a 5'-vinyl-(E)-phosphonate modification; or
the antisense strand has any one of the following modification patterns:
| No. | **Antisense strand (AS) 5'-3'** | | | | | | | | | | |
|---|---|---|---|---|---|---|---|---|---|---|---|
| **A'** | **1** | **2** | **3** | **4** | **5** | **6** | **7** | **8** | **9** | **10** | **11** |
| | 2'-OMe +PS +EVP | **2'-F +PS** | 2'-OMe | 2'-OMe | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **B'** | **1** | **2** | **3** | **4** | **5** | **6** | **7** | **8** | **9** | **10** | **11** |
| | 2'-OMe +PS +EVP | **2'-F +PS** | **2'-F** | **2'-F** | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **C'** | **1** | **2** | **3** | **4** | **5** | **6** | **7** | **8** | **9** | **10** | **11** |
| | 2'-OMe +PS +EVP | **2'-F +PS** | 2'-OMe | **2'-F** | **2'-F** | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **D'** | **1** | **2** | **3** | **4** | **5** | **6** | **7** | **8** | **9** | **10** | **11** |
| | 2'-OMe +PS +EVP | **2'-F +PS** | 2'-OMe | 2'-OMe | **2'-F** | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| | | | | | | | | | | | |
|---|---|---|---|---|---|---|---|---|---|---|---|
| **E'** | **1** | **2** | **3** | **4** | **5** | **6** | **7** | **8** | **9** | **10** | **11** |
| | 2'-OMe +PS +EVP | **2'-F +PS** | **2'-F** | 2'-OMe | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **F'** | **1** | **2** | **3** | **4** | **5** | **6** | **7** | **8** | **9** | **10** | **11** |
| | 2'-OMe +PS +EVP | **2'-F +PS** | 2'-OMe | 2'-OMe | 2'-OMe | **2'-F** | 2'-OMe | **2'-F** | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **G'** | **1** | **2** | **3** | **4** | **5** | **6** | **7** | **8** | **9** | **10** | **11** |
| | 2'-OMe +PS +EVP | 2'-F **+PS** | 2'-OMe | **2'-F** | **2'-F** | **2'-F** | 2'-OMe | **2'-F** | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **H'** | **1** | **2** | **3** | **4** | **5** | **6** | **7** | **8** | **9** | **10** | **11** |
| | 2'-OMe +PS +EVP | **2'-F +PS** | **2'-F** | 2'-OMe | 2'-OMe | **2'-F** | 2'-OMe | **2'-F** | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
| **I'** | **1** | **2** | **3** | **4** | **5** | **6** | **7** | **8** | **9** | **10** | **11** |
| | 2'-OMe +PS | **2'-F +PS** | 2'-OMe | 2'-OMe | 2'-OMe | **2'-F** | 2'-OMe | 2'-OMe | 2'-OMe | 2'-OMe | 2'-OMe |
| | **12** | **13** | **14** | **15** | **16** | **17** | **18** | **19** | **20** | **21** | **22** |
| | 2'-OMe | 2'-OMe | **2'-F** | 2'-OMe | **2'-F** | 2'-OMe | 2'-OMe | 2'-OMe | 2'-OMe +PS | 2'-OMe +PS | 2'-OMe |
and/or, the sense strand has any one of the following modification patterns:
| No. | **Sense strand (SS) 5'-3'** | | | | | | | | | |
|---|---|---|---|---|---|---|---|---|---|---|
| **a'** | **1** | **2** | **3** | **4** | **5** | **6** | **7** | **8** | **9** | **10** |
| | 2'-OMe+PS | 2'-OMe+PS | 2'-OMe | 2'-OMe | 2'-OMe | **2'-F** | 2'-OMe | **2'-F** | **2'-F** | **2'-F** |
| | **11** | **12** | **13** | **14** | **15** | **16** | **17** | **18** | **19** | **20** |
| | 2'-OMe | 2'-OMe | 2'-OMe | 2'-OMe | 2'-OMe | 2'-F | 2'-OMe | 2'-OMe | 2'-OMe | 2'-OMe |
| **b'** | **1** | **2** | **3** | **4** | **5** | **6** | **7** | **8** | **9** | **10** |
| | 2'-OMe+PS | 2'-OMe+PS | 2'-OMe | 2'-OMe | 2'-OMe | **2'-F** | 2'-OMe | **2'-F** | 2'-OMe | **2'-F** |
| | **11** | **12** | **13** | **14** | **15** | **16** | **17** | **18** | **19** | **20** |
| | 2'-OMe | 2'-OMe | 2'-OMe | 2'-OMe | 2'-OMe | **2'-F** | 2'-OMe | 2'-OMe | 2'-OMe | 2'-OMe |
| **c'** | **1** | **2** | **3** | **4** | **5** | **6** | **7** | **8** | **9** | **10** |
| | 2'-OMe+PS | 2'-OMe+PS | 2'-OMe | 2'-OMe | 2'-OMe | **2'-F** | 2'-OMe | **2'-F** | **2'-F** | **2'-F** |
| | **11** | **12** | **13** | **14** | **15** | **16** | **17** | **18** | **19** | **20** |
| | 2'-OMe | 2'-OMe | 2'-OMe | 2'-OMe | 2'-OMe | 2'-OMe | 2'-OMe | 2'-OMe | 2'-OMe | 2'-OMe |
wherein 2'-OMe represents a 2'-O-methyl modification; 2'-F represents a 2'-fluoro modification; PS indicates that, starting from the 5' end, the nucleotide at the position is linked to the adjacent nucleotide on the right via a 3',5'-phosphorothioate bond; EVP represents a 5'-vinyl-(E)-phosphonate modification;
preferably,
the antisense strand is modified according to modification pattern B, and the sense strand is modified according to modification pattern a;
the antisense strand is modified according to modification pattern C, and the sense strand is modified according to modification pattern a;
the antisense strand is modified according to modification pattern C, and the sense strand is modified according to modification pattern b;
the antisense strand is modified according to modification pattern D, and the sense strand is modified according to modification pattern b;
the antisense strand is modified according to modification pattern E, and the sense strand is modified according to modification pattern b;
the antisense strand is modified according to modification pattern F, and the sense strand is modified according to modification pattern b;
the antisense strand is modified according to modification pattern G, and the sense strand is modified according to modification pattern b; or
the antisense strand is modified according to modification pattern H, and the sense strand is modified according to modification pattern b;
or,
the antisense strand is modified according to modification pattern B', and the sense strand is modified according to modification pattern a';
the antisense strand is modified according to modification pattern C', and the sense strand is modified according to modification pattern a';
the antisense strand is modified according to modification pattern C', and the sense strand is modified according to modification pattern b';
the antisense strand is modified according to modification pattern D', and the sense strand is modified according to modification pattern b';
the antisense strand is modified according to modification pattern E', and the sense strand is modified according to modification pattern b';
the antisense strand is modified according to modification pattern F', and the sense strand is modified according to modification pattern b';
the antisense strand is modified according to modification pattern G', and the sense strand is modified according to modification pattern b'; or
the antisense strand is modified according to modification pattern H', and the sense strand is modified according to modification pattern b'.

7. The siRNA duplex according to any one of claims 1 to 6, wherein the siRNA duplex comprises any one oligonucleotide duplex selected from the pairings of a sense strand and an antisense strand shown in Table 5 and Table 12, or a combination of at least two thereof.

8. The siRNA duplex according to claim 1, wherein any one or at least two positions selected from positions 2 to 11 from the 5' end of the antisense strand and/or the sense strand are modified with a modifying moiety, wherein the modifying moiety is selected from UNA, GNA, and DNA, wherein the structures of UNA and GNA are as follows: 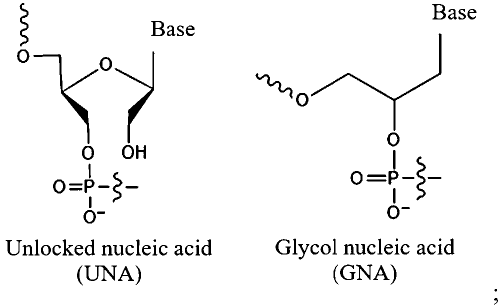
wherein the base is selected from adenine, guanine, cytosine, thymine, and uracil;
preferably, the nucleotide at position 7, or at positions 7 and 11, of the antisense strand is replaced with DNA, while the nucleotide at the position of the sense strand that is complementary to position 7 of the antisense strand is also replaced with DNA.

9. An siRNA conjugate, wherein the siRNA conjugate comprises the siRNA duplex according to any one of claims 1 to 8 and a conjugate moiety attached thereto;
preferably, the siRNA conjugate satisfies one of the conditions (i) to (iii):
(i) the conjugate moiety is attached to the 3' end or 5' end of the sense strand of the oligonucleotide; and/or, the conjugate moiety is a GalNAc derivative attached via a bivalent or trivalent branched linker arm;
(ii) the conjugate moiety is as follows: 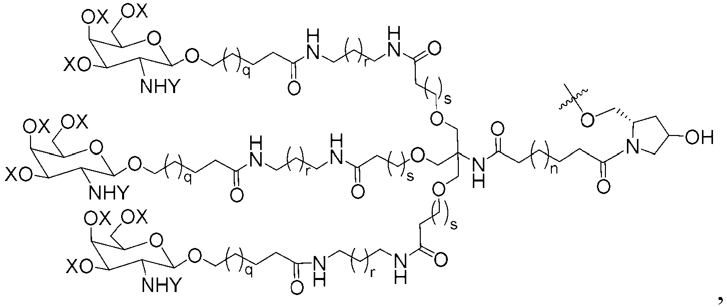
wherein X is a hydroxyl protecting group or H, the hydroxyl protecting group being selected from acetyl, benzoyl, and isobutyryl; Y is an amino protecting group or H, the amino protecting group being selected from formyl, acetyl, propionyl, n-butyryl, and isobutyryl; n is an integer from 0 to 20; q, r, and s are independently integers from 1 to 7;
preferably, the conjugate moiety is as follows: 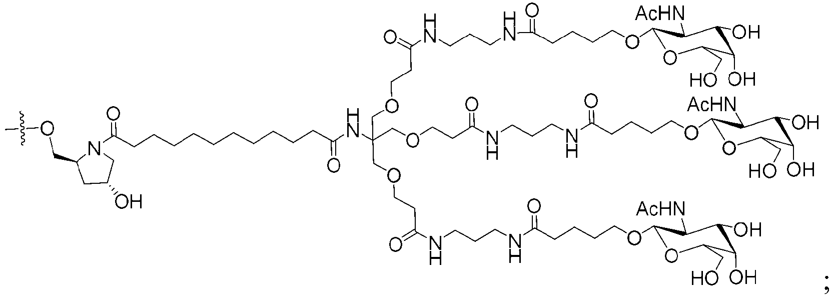
(iii) the conjugate moiety is as follows: 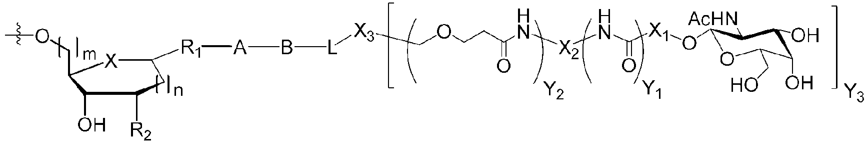
wherein X is oxygen, -N(Y)-, or sulfur;
Y is C₁₋₄ alkyl or C₆₋₁₀ aryl;
R₁ is oxygen or sulfur;
R₂ is hydrogen, -NH₂, C₁₋₄ alkyl, C₆₋₁₀ aryl, C₁₋₄ alkoxy, or halogen;
A is -(CH₂)ₐ-, -(CH₂CH₂O)_{b}-, -((CH₂)_{c}NHCO)_{d}-, or -((CH₂)_{c}CONH)_{d}-, wherein a is an integer from 1 to 15, b is an integer from 1 to 7, c is an integer from 1 to 7, and d is an integer from 1 to 5;
B is -(CH₂)ₑ-, wherein e is an integer from 0 to 7;
L is -CONH- or -NHCO-;
X₁ is -(CH₂)_{f}- or -(CH₂CH₂O)_{f}CH₂-, wherein f is an integer from 1 to 5;
X₂ is -(CH₂)_{g}-, wherein g is an integer from 1 to 6;
Y₁ is 0 or 1;
Y₂ is 0, 1, or 2;
when Y₃ is 1, X₃ is CH₂; when Y₃ is 2, X₃ is CH; when Y₃ is 3, X₃ is carbon;
m is an integer from 0 to 4;
n is an integer from 0 to 4;preferably, the conjugate moiety is G4, G5, G6, or G7, the structures of which are as follows:

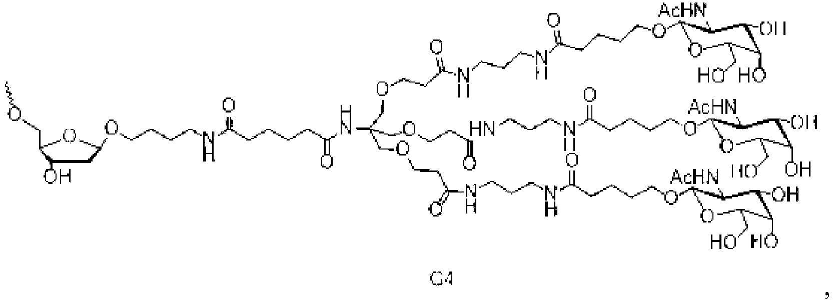

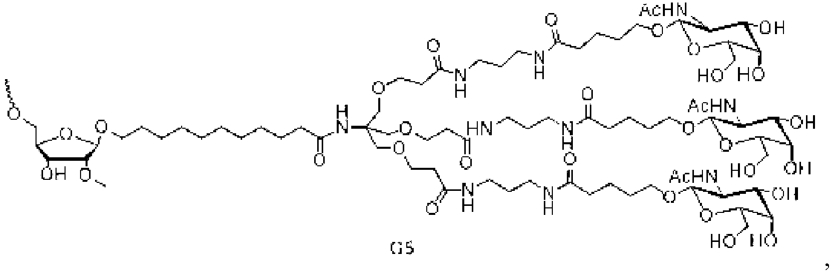

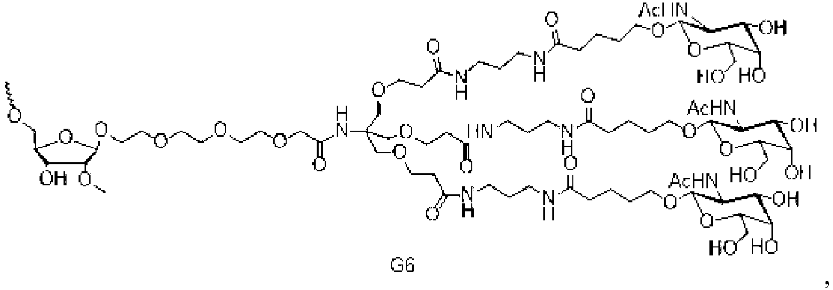

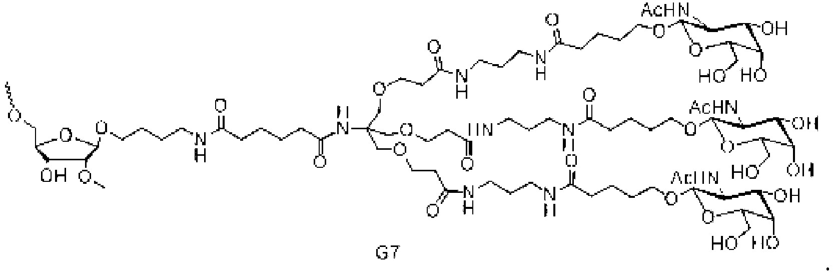

10. The conjugate according to claim 9, wherein the conjugate has any one of the following structures: 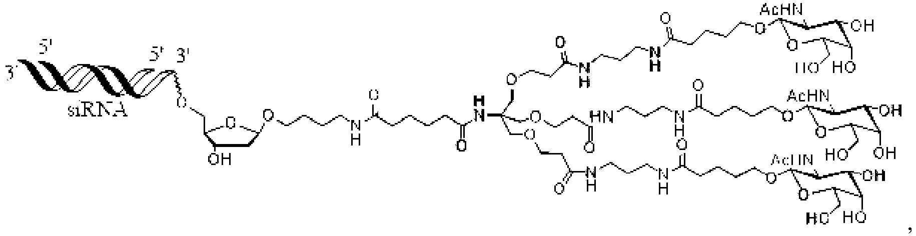 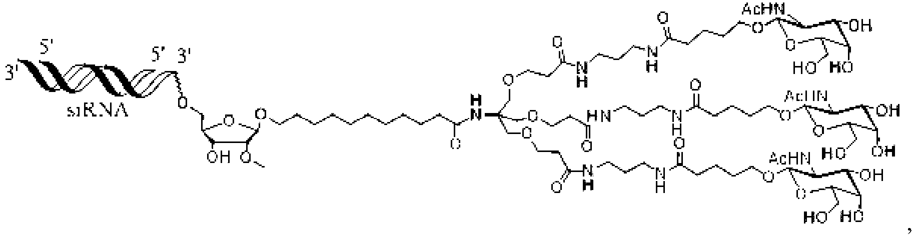 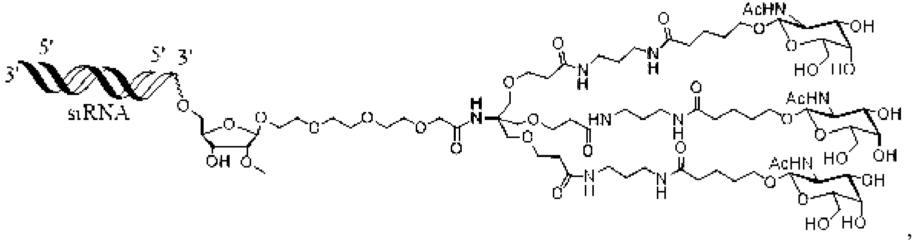 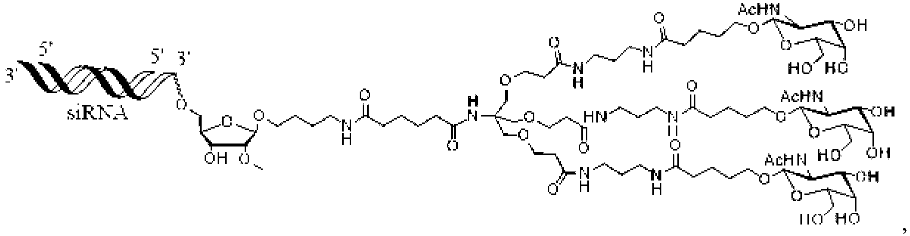 and

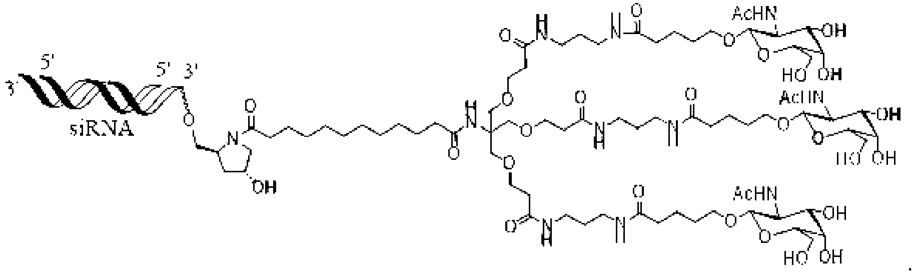

11. The conjugate according to claim 9, wherein the conjugate moiety is as follows: 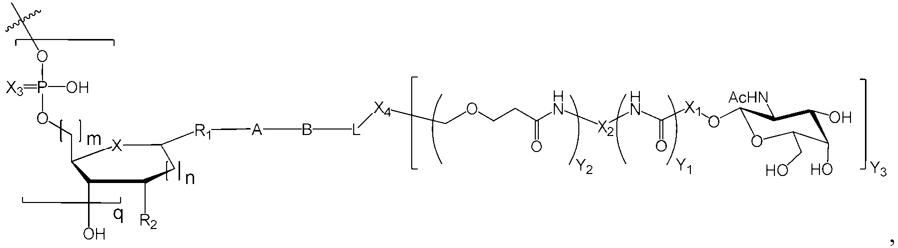
wherein X is oxygen, -N(Y)-, or sulfur;
Y is C₁₋₄ alkyl or C₆₋₁₀ aryl;
R₁ is oxygen or sulfur;
R₂ is hydrogen, -NH₂, C₁₋₄ alkyl, C₆₋₁₀ aryl, C₁₋₄ alkoxy, or halogen;
A is -(CH₂)ₐ-, -(CH₂CH₂O)_{b}-, -((CH₂)_{c}NHCO)_{d}-, or -((CH₂)_{c}CONH)_{d}-, wherein a is an integer from 1 to 15, b is an integer from 1 to 7, c is an integer from 1 to 7, and d is an integer from 1 to 5;
B is -(CH₂)ₑ-, wherein e is an integer from 0 to 7;
L is -CONH- or -NHCO-;
X₁ is -(CH₂)_{f}- or -(CH₂CH₂O)_{f}CH₂-, wherein f is an integer from 1 to 5;
X₂ is -(CH₂)_{g}-, wherein g is an integer from 1 to 6;
X₃ is oxygen or sulfur;
Y₁ is 0 or 1;
Y₂ is 0, 1, or 2;
when Y₃ is 1, X₄ is CH₂; when Y₃ is 2, X₄ is CH; when Y₃ is 3, X₃ is carbon;
m is an integer from 0 to 4;
n is an integer from 0 to 4;
q is an integer from 0 to 4;
preferably, the conjugate moiety is G101, G102, G103, G105, or G106: 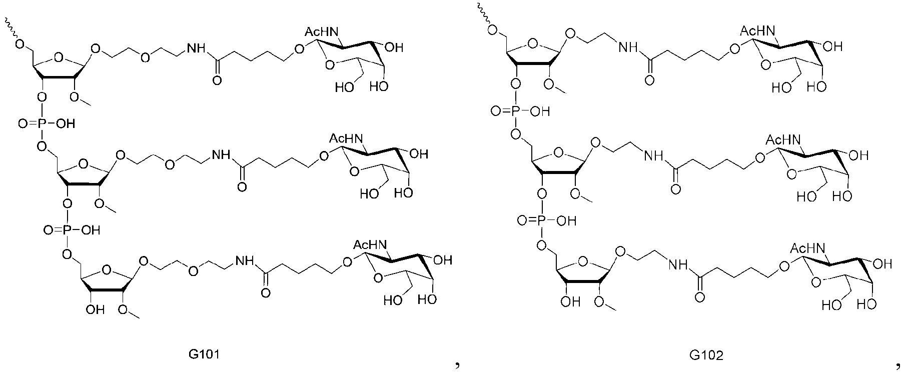 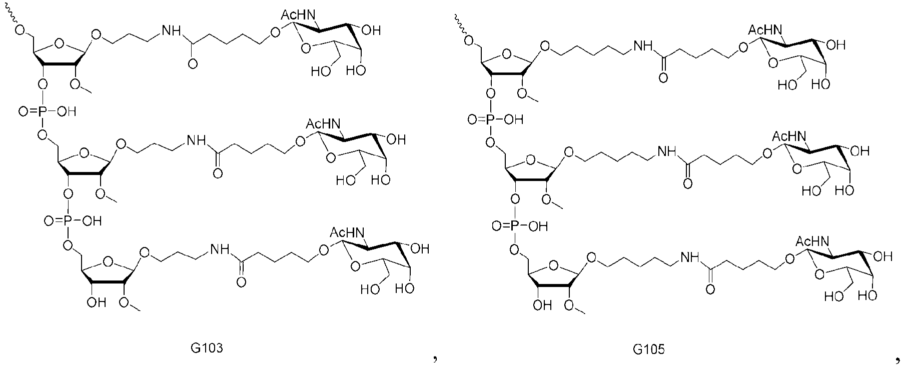 and 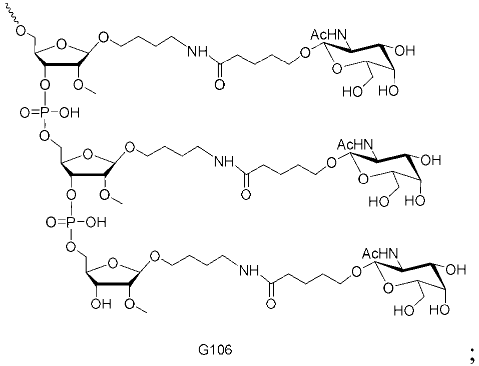
more preferably, the conjugate has any one of the following structures: 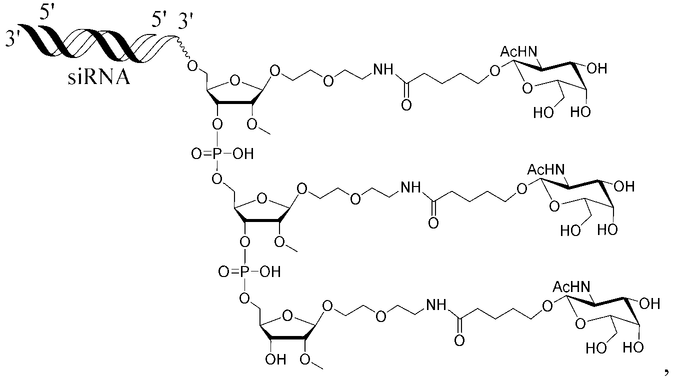 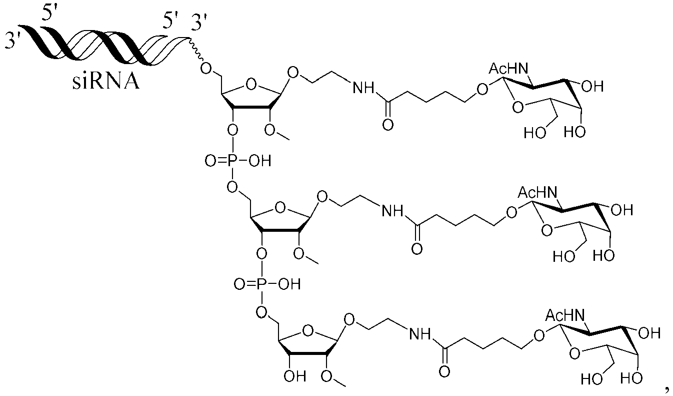 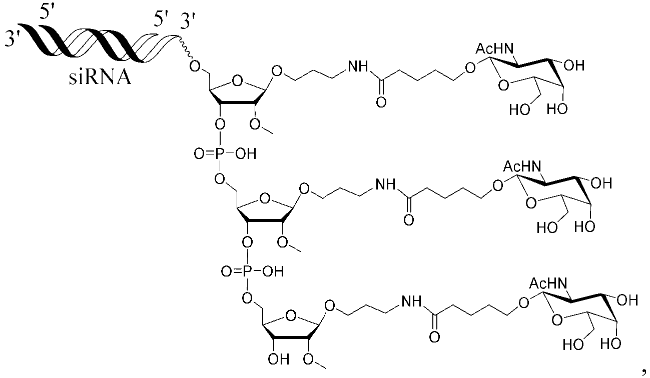 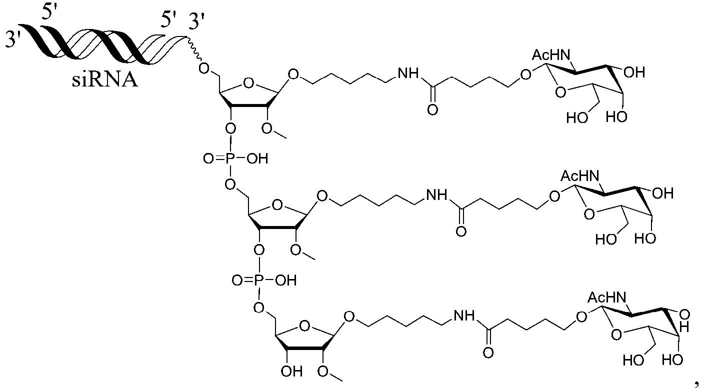 or

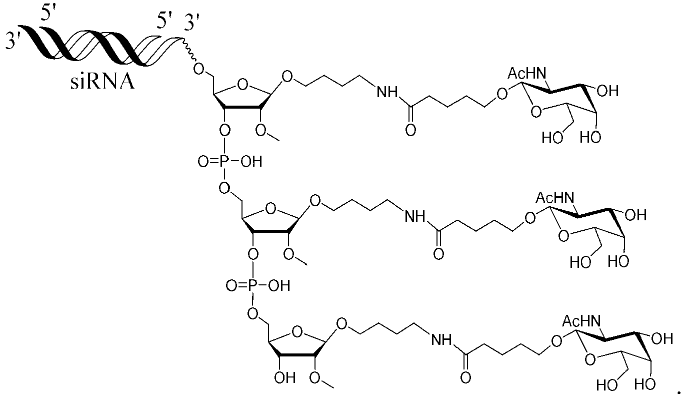

12. The conjugate according to claim 9, wherein the siRNA duplex comprises any one oligonucleotide duplex selected from the pairings of a sense strand and an antisense strand shown in Table 5, Table 12, and Table 16, or a combination of at least two thereof;
wherein either or both of the sense strand and the antisense strand are linked to the conjugate moiety G4, G5, G6, G7, G101, G102, G103, G105, or G106;
preferably, the conjugate moiety is attached to the 3' end of the sense strand.

13. A nucleic acid-protein complex, wherein the nucleic acid-protein complex comprises a duplex region of the siRNA duplex according to any one of claims 1 to 8, and a nuclease; or, the nucleic acid-protein complex comprises an antisense strand of the duplex region of the siRNA duplex according to any one of claims 1 to 8, and a nuclease;
preferably, the nuclease is an AGO protein.

14. A pharmaceutical composition, wherein the pharmaceutical composition comprises the siRNA duplex according to any one of claims 1 to 8, the conjugate according to any one of claims 9 to 12, or the nucleic acid-protein complex according to claim 13, and a pharmaceutically acceptable carrier.

15. The siRNA duplex according to any one of claims 1 to 8, the conjugate according to any one of claims 9 to 12, the nucleic acid-protein complex according to claim 13, or the pharmaceutical composition according to claim 14 for use in preventing and/or treating a disease associated with INHBE gene expression, or for use in for preventing and/or treating diabetes and obesity, as well as hyperlipidemia or dyslipidemia, insulin resistance or elevated or altered insulin levels during fasting or metabolic challenge, hepatic fat deposition or fatty liver disease, non-alcoholic steatohepatitis, elevated blood pressure and/or hypertension, metabolic syndrome, coronary artery disease and other atherosclerotic conditions, and complications associated with these diseases.
